# EUROPEAN PATENT APPLICATION

(11) **EP 4 378 954 A1**
(43) Date of publication of application: **05.06.2024**
(21) Application number: 22848645.2
(22) Date of filing: 28.07.2022
(51) Int. Cl.: C07K 16/28, A61P 35/00

(54) **ANTI-PVRIG/ANTI-TIGIT BISPECIFIC ANTIBODY AND APPLICATION**

(30) Priority: 30.07.2021 CN 202110872620; 30.07.2021 CN 202110874745; 06.08.2021 CN 202110903850; 21.03.2022 CN 202210276638
(71) Applicant: Shandong Simcere Biopharmaceutical Co., Ltd., Shandong 264006 (CN)
(72) Inventor: ZHAO, Xiaofeng, Shanghai 201318 (CN); LIU, Lei, Shanghai 201318 (CN); LIU, Yang, Jiangsu 210042 (CN); FU, Yayuan, Shanghai 201318 (CN); CAO, Zhuoxiao, Shanghai 201318 (CN); TANG, Renhong, Shanghai 201318 (CN); REN, Jinsheng, Jiangsu 210041 (CN)
(74) Representative: KATZAROV S.A.
(86) International application number: PCT/CN2022/108648
(87) International publication number: WO 2023/006040

(57) **Abstract**

The present disclosure relates to a bispecific antibody capable of specifically binding PVRIG and TIGIT, which can modulate the function of immune cells and can be used as a drug to treat diseases related to immune abnormalities, such as tumors.

## Description

### FIELD

The disclosure relates to the field of antibodies, in particular to anti-PVRIG/anti-TIGIT bispecific antibodies.

### BACKGROUND

Immunotherapy is based on manipulating and/or regulating the immune system, including both innate immune responses and acquired immune responses. The general goal of immunotherapy is to treat diseases by controlling the immune response to "foreign agents" (such as pathogens or tumor cells). The immune system is a highly complex system composed of multiple cell types, which have complex and subtle systems to control interactions and reactions. The concept of cancer immunosurveillance is based on the theory that the immune system can identify tumor cells, initiate immune responses and inhibit tumor development and/or progression. However, it is clear that many cancer cells have developed mechanisms to evade the immune system, which can allow uninhibited tumor growth. Cancer/tumor immunotherapy focuses on the development of novel agonists and/or antagonists that can activate the immune system, so as to achieve more effective anti-tumor responses, enhance the killing effect on tumor cells and/or inhibit tumor growth.

PVRIG is expressed on natural killer (NK) cells and T cells, and has several similarities with other known immune checkpoints. The identification and methods for demonstrating that PVRIG is an immune checkpoint receptor are described in WO2016/134333, which is expressly incorporated herein by reference. When PVRIG binds to its ligand (PVRL2), it triggers an inhibitory signal, which acts to weaken the immune response of NK cells and T cells against target cells (i.e., similar to PD-1/PD-L1). Blocking the binding of PVRL2 and PVRIG will cut off the inhibitory signal of PVRIG, and thus regulate the immune response of NK cells and T cells. PVRIG antibodies that blocks the binding of PVRIG and PVRL2 is used as a therapeutic method to enhance the ability of NK cells and T cells to kill cancer cells. Blocking antibodies that bind PVRIG and block its binding to ligand PVRL2 have been produced.

Similarly, TIGIT is another target of interest. It has been demonstrated that TIGIT binds to its cognate ligand PVR and directly inhibits the cytotoxicity of NK cells and T cells through its intracellular ITIM domain. Knockout of TIGIT gene or TIGIT/PVR interaction blocking antibodies have been shown to enhance the killing effect of NK cells in vitro or aggravate autoimmune diseases in vivo. In addition to its direct effects on T cells and NK cells, TIGIT can also induce PVR-mediated signal transduction in dendritic cells or tumor cells, result in increased production of anti-inflammatory cytokines (such as IL10). Significantly, the expression of TIGIT is closely related to the expression of another important co-inhibitory receptor PD-1. TIGIT and PD-1 are co-expressed on many human and murine tumor infiltrating lymphocytes (TIL).

TIGIT and PVRIG belong to DNAM superfamily, and have been demonstrated to be co-expressed in various tumor infiltrating lymphocytes to exert immunosuppressive effects. Meanwhile, tumor infiltrating effector T cells co-expressing TIGIT, PVRIG and PD-1 are considered to be the most important effector T cells in the infiltrating T cell population. Therefore, bispecific antibodies that can simultaneously target PVRIG and TIGIT have potential synergistic effects, which can be an attractive therapeutic method for single antibody therapy. These bispecific antibodies will allow simultaneously targeting two immune checkpoint receptors, and have a potential further synergistic effect with the existing anti-PD-1/L-1 antibody therapy at the same time, which play an important role in providing new therapeutic means in cancer treatment.

### SUMMARY

In view of the potential synergistic effect of bispecific antibodies, the present invention is proposed in order to improve the immunosuppressive effect and to solve the problem of poor response efficiency of immune checkpoint inhibitors.

The present disclosure provides anti-PVRIG/anti-TIGIT antibodies, nucleic acids encoding the antibodies, a method for preparation of the antibodies, pharmaceutical compositions containing the antibodies and related uses of the pharmaceutical compositions for treating tumors.

In a first aspect, the disclosure provides an anti-PVRIG/anti-TIGIT bispecific antibody, which comprises:
(a) a first antigen binding fragment, which comprise a heavy chain variable region (VH) and a light chain variable region (VL) that form an anti-TIGIT antigen binding domain; wherein the TIGIT VH comprises HCDR1, HCDR2 and HCDR3 of the VH shown in SEQ ID NO: 72 or 87; the TIGIT VL comprises LCDR1, LCDR2 and LCDR3 of the VL shown in SEQ ID NO: 68 or 91;
(b) a second antigen binding fragment, which comprise VHH that specifically binds to PVRIG, the VHH comprises CDR1, CDR2 and CDR3 of the sequences shown in SEQ ID NO: 200 or 211.

In some embodiments, (a) the HCDR1 of the first antigen binding fragment comprises the sequence shown in any one of SEQ ID NO: 21 or 33; the HCDR2 comprises the sequence shown in any one of SEQ ID NO: 22 or 34; the HCDR3 comprises the sequence shown in any one of SEQ ID NO: 23 or 35;
(b) the LCDR1 of the first antigen binding fragment comprises the sequence shown in any one of SEQ ID NO: 18 or 96; the LCDR2 comprises the sequence shown in any one of SEQ ID NO: 19 or 31; the LCDR3 comprises the sequence shown in any one of SEQ ID NO: 20 or 32;
(c) the CDR1 of the second antigen binding fragment comprises the sequence shown in any one of SEQ ID NO: 168 or 147; the CDR2 comprises the sequence shown in any one of SEQ ID NO: 207 or 148; the CDR3 comprises the sequence shown in any one of SEQ ID NO: 208 or 149.

In some embodiments, the first antigen binding fragment comprises HCDR1, HCDR2, HCDR3, LCDR1, LCDR2 and LCDR3 of the following sequences:
(1) SEQ ID NO: 21, 22, 23, 18, 19 and 20, respectively; or
(2) SEQ ID NO: 33, 34, 35, 96, 31 and 32, respectively; or
(3) a sequence having at least 90% identity or having 1, 2, 3 or more amino acid insertions, deletions and/or substitutions compared with the sequences shown in (1) or (2), preferably, the substitutions are conservative amino acid substitutions.

In some embodiments, the second antigen binding fragment comprises CDR1, CDR2 and CDR3 of the following sequences:
(1) SEQ ID NO: 168, 207 and 208 respectively; or
(2) SEQ ID NO: 147, 148 and 149 respectively; or
(3) a sequence having at least 90% identity or having 1, 2, 3 or more amino acid insertions, deletions and/or substitutions compared with the sequences shown in (1) or (2), preferably, the substitutions are conservative amino acid substitutions.

In some embodiments, the VH of the first antigen binding fragment comprises a sequence having at least 90% identity to the amino acid sequence shown in SEQ ID NO: 72 or 87; the VL of the first antigen binding fragment comprises a sequence having at least 90% identity to the amino acid sequence shown in SEQ ID NO: 68 or 91.

In some embodiments, the second antigen binding fragment comprises a sequence having at least 90% identity to the amino acid sequence shown in SEQ ID NO: 200 or 211.

In some embodiments, the first antigen binding fragment is a full-length antibody, comprising two heavy chains and two light chains; the C-terminus of the second antigen binding fragment is fused to the N-terminus of at least one heavy chain of the first antigen binding fragment.

In some embodiments, the heavy chain fusion polypeptide comprises PVRIG VHH-(G4S)4 Linker-TIGIT VH-CH1-hinge-CH2-CH3 from the N-terminus to the C-terminus, and the light chain polypeptide comprises TIGIT VL-CL from the N-terminus to the C-terminus.

In some embodiments, the heavy chain fusion polypeptide comprises a sequence having at least 80% identity to the amino acid sequences shown in SEQ ID NO: 227, 229, 231 or 233, and the light chain polypeptide comprises a sequence having at least 80% identity to the amino acid sequence shown in SEQ ID NO: 226, 228, 230 or 232.

In some embodiments, the bispecific antibodies are humanized antibodies.

In some embodiments, the bispecific antibodies specifically bind to PRVIG or TIGIT protein of human and/or monkey; preferably, the KD value between bispecific antibodies and TIGIT protein of human and/or monkey is better than 1.00E-7M, and the KD value between bispecific antibodies and PRVIG protein of human and/or monkey is better than 1.00E-8M; more preferably, the bispecific antibodies can simultaneously combine with TIGIT and PVRIG.

In another aspect, the present disclosure provides an antibody or antigen-binding fragment specifically binding to TIGIT, which comprises:
(1) a heavy chain variable region (VH), wherein the heavy chain variable region comprises three complementary determining regions (HCDRs): HCDR1, HCDR2 and HCDR3, wherein, according to the Kabat numbering scheme, the HCDR1 comprises the amino acid sequence shown in SEQ ID NO: 21, 27, 33 or 39, the HCDR2 comprises the amino acid sequence shown in SEQ ID NO: 22, 28, 34 or 40, and the HCDR3 comprises the amino acid sequence shown in SEQ ID NO: 23, 29, 35 or 41; according to the IMGT numbering scheme, the HCDR1 comprises the amino acid sequence shown in SEQ ID NO: 45, 51, 57 or 63, the HCDR2 comprises the amino acid sequence shown in SEQ ID NO:46, 52, 58 or 64, and the HCDR3 comprises the amino acid sequence shown in SEQ ID NO:47, 53, 59 or 65; and,
(2) a light chain variable region (VL), wherein the light chain variable region comprises three complementary determining regions (LCDRs): LCDR1, LCDR2 and LCDR3, wherein, according to the Kabat numbering scheme, the LCDR1 comprises the amino acid sequence shown in SEQ ID NO: 18, 24, 30, 36, 93, 94, 95 or 96, the LCDR2 comprises the amino acid sequence shown in SEQ ID NO: 19, 25, 31 or 37, and the LCDR3 comprises the amino acid sequence shown in SEQ ID NO: 20, 26, 32 or 38; according to the IMGT numbering scheme, the LCDR1 comprises the amino acid sequence shown in SEQ ID NO: 42, 48, 54 or 60, the LCDR2 comprises the amino acid sequence shown in SEQ ID NO: 43, 49, 55 or 61, and the LCDR3 comprises the amino acid sequence shown in SEQ ID NO: 44, 50, 56 or 62.

In some embodiments, the antibody or antigen-binding fragment comprises LCDR1, LCDR2, LCDR3, HCDR1, HCDR2 and HCDR3 of the following sequences:
(1) SEQ ID NO: 18, 19, 20, 21, 22 and 23, respectively; or
(2) SEQ ID NO: 24, 25, 26, 27, 28 and 29, respectively; or
(3) SEQ ID NO: 30, 31, 32, 33, 34 and 35, respectively; or
(4) SEQ ID NO: 36, 37, 38, 39, 40 and 41, respectively; or
(5) SEQ ID NO: 42, 43, 44, 45, 46 and 47, respectively; or
(6) SEQ ID NO: 48, 49, 50, 51, 52 and 53, respectively; or
(7) SEQ ID NO: 54, 55, 56, 57, 58 and 59, respectively; or
(8) SEQ ID NO: 60, 61, 62, 63, 64 and 65, respectively; or
(9) SEQ ID NO: 93, 31, 32, 33, 34 and 35, respectively; or
(10) SEQ ID NO: 94, 31, 32, 33, 34 and 35, respectively; or
(11) SEQ ID NO: 95, 31, 32, 33, 34 and 35, respectively; or
(12) SEQ ID NO: 96, 31, 32, 33, 34 and 35, respectively; or
(13) a sequence having at least 80% identity to or having 1, 2, 3 or more amino acid insertions, deletions and/or substitutions with the sequences shown in the above (1) to (12), preferably, the substitutions are conservative amino acid substitutions.

In some embodiments, the antibody or antigen-binding fragment comprises:
(1) a heavy chain variable region, which comprises amino acid sequences having at least 80% identity to SEQ ID NO: 10, 11, 12, 13, 69, 70, 71, 72, 81, 82, 83, 84, 85, 87, 101,102 or 103; or / and
(2) a light chain variable region, which comprises amino acid sequences having at least 80% identity to SEQ ID NO: 14, 15, 16, 17, 66, 67, 68, 78, 79, 80, 86, 88, 89, 90, 91, 98, 99 or 100.

In some embodiments, the antibody or antigen-binding fragment comprises:
(1) a heavy chain variable region comprising the amino acid sequence shown in SEQ ID NO:10 and a light chain variable region comprising the amino acid sequence shown in SEQ ID NO:14; or
(2) a heavy chain variable region comprising the amino acid sequence shown in SEQ ID NO:11 and a light chain variable region comprising the amino acid sequence shown in SEQ ID NO:15; or
(3) a heavy chain variable region comprising the amino acid sequence shown in SEQ ID NO: 12 and a light chain variable region comprising the amino acid sequence shown in SEQ ID NO:16; or
(4) a heavy chain variable region comprising the amino acid sequence shown in SEQ ID NO: 13 and a light chain variable region comprising the amino acid sequence shown in SEQ ID NO: 17; or
(5) a heavy chain variable region comprising the amino acid sequence shown in SEQ ID NO:69 and a light chain variable region comprising the amino acid sequence shown in SEQ ID NO:66, 67 or 68; or
(6) a heavy chain variable region comprising the amino acid sequence shown in SEQ ID NO:70 and a light chain variable region comprising the amino acid sequence shown in SEQ ID NO:66, 67 or 68; or
(7) a heavy chain variable region comprising the amino acid sequence shown in SEQ ID NO:71 and a light chain variable region comprising the amino acid sequence comprising SEQ ID NO:66, 67 or 68; or
(8) a heavy chain variable region comprising the amino acid sequence shown in SEQ ID NO:72 and a light chain variable region comprising the amino acid sequence shown in SEQ ID NO:66, 67 or 68; or
(9) a heavy chain variable region comprising the amino acid sequence shown in SEQ ID NO:81, 82, 83, 84 or 85 and a light chain variable region comprising the amino acid sequence shown in SEQ ID NO:78; or
(10) a heavy chain variable region comprising the amino acid sequence shown in SEQ ID NO:81, 82, 83, 84 or 85 and a light chain variable region comprising the amino acid sequence shown in SEQ ID NO:79; or
(11) a heavy chain variable region comprising the amino acid sequence shown in SEQ ID NO:81, 82, 83, 84 or 85 and a light chain variable region comprising the amino acid sequence shown in SEQ ID NO:80; or
(12) a heavy chain variable region comprising the amino acid sequence shown in SEQ ID NO:87 and a light chain variable region comprising the amino acid sequence shown in SEQ ID NO:86, 88, 89, 90 or 91; or
(13) a heavy chain variable region comprising the amino acid sequence shown in SEQ ID NO:101 and a light chain variable region comprising the amino acid sequence shown in SEQ ID NO:98, 99 or 100; or
(14) a heavy chain variable region comprising the amino acid sequence shown in SEQ ID NO:102 and a light chain variable region comprising the amino acid sequence shown in SEQ ID NO:98, 99 or 100
(15) a heavy chain variable region comprising the amino acid sequence shown in SEQ ID NO:103 and a light chain variable region comprising the amino acid sequence shown in SEQ ID NO:98, 99 or 100; or
(16) a sequence having at least 80% identity or at most 20 mutations to the sequences shown in the above (1) to (15); the mutation may be selected from insertion, deletion and/or substitution, and the substitution is preferably a conservative amino acid substitution.

In some embodiments, the antibody or antigen-binding fragment comprises a heavy chain variable region, wherein the heavy chain variable region has at least a mutation selected from the following group compared with the VH shown in SEQ ID NO.10: S30T, G44K, W47Y, I48M, V67I or V71R; preferably, at least S30T and V71R mutations; more preferably, at least S30T, G44K and V71R mutations; more preferably, at least S30T, G44K, I48M, V67I and V71R mutations; more preferably, at least S30T, G44K, W47Y and V71R mutations, which are numbered in natural order; or
the heavy chain variable region has at least a mutation selected from the following group compared with the VH shown in SEQ ID NO.11: T28A, R72A, T74K or A76S; preferably, at least T28A, R72A, T74K and A76S, which are numbered in natural order; or
the heavy chain variable region has at least a mutation selected from the following group compared with the VH shown in SEQ ID NO.12: I29M, S30T, G44K, W47Y, I48M, V67I or V71R mutations; preferably, at least S30T and V71R mutations; more preferably, at least I29M, S30T and V71R mutations; more preferably, at least I29M, S30T, G44K and V71R mutations; more preferably, at least I29M, S30T, G44K, I48M, V67I and V71R mutations; more preferably, at least I29M, S30T, G44K, W47Y and V71R mutations, which are numbered in natural order; or
the heavy chain variable region has at least a mutation selected from the following group compared with the VH shown in SEQ ID NO.13: R44G, R72V, T74K, S75L or A76S; preferably, at least R72V and T74K mutations; more preferably, at least R72V, T74K, S75L and A76S mutations; more preferably, at least R44G, R72V, T74K, S75L and A76S mutations, which are numbered in natural order.

In some embodiments, the antibody or antigen-binding fragment comprises a light chain variable region, wherein the light chain variable region has at least a mutation selected from the following group compared with the VL shown in SEQ ID NO.14: L37Q, P43S or L47M; preferably, at least L47M mutation; more preferably, at least L37Q and L47M mutations; more preferably, at least P43S and L47M mutations, which are numbered in natural order; or
the light chain variable region has at least a mutation selected from the following group compared with the VL shown in SEQ ID NO.15 : N31Q, N31T, N31D, G32A, Q38H or P43S; preferably, at least Q38H and P43S mutations; more preferably, at least N31Q, Q38H and P43S mutations; more preferably, at least N31T, Q38H and P43S mutations; more preferably, at least N31D, Q38H and P43S mutations; more preferably, at least G32A, Q38H and P43S mutations; more preferably, at least G32A, Q38H and P43S mutations, which are numbered in natural order; or
the light chain variable region has at least a mutation selected from the following group compared with the VL shown in SEQ ID NO.16: L37Q, P43S or Q45K; preferably, at least L37Q and Q45K mutations; more preferably, at least P43S mutation, which are numbered in natural order; or
the light chain variable region has at least a mutation selected from the following group compared with the VL shown in SEQ ID NO.17: A43 S, P43 S or I48V; preferably, at least A43 S mutation; more preferably, at least A43S and I48V mutations; more preferably, at least P43S and I48V mutations, which are numbered in natural order.

In some embodiments, the antibody or antigen-binding fragment specifically binds to human and/or monkey TIGIT proteins; preferably, the KD value between the antibody or antigen-binding fragment and human and/or monkey TIGIT proteins is better than 1.00E-8M.

In some embodiments, the antibody or antigen-binding fragment is mouse antibodies, humanized antibodies, fully human antibodies or chimeric antibodies.

In some embodiments, the antibody or antigen-binding fragment is selected from monoclonal antibodies, polyclonal antibodies, natural antibodies, engineered antibodies, monospecific antibodies, multispecific antibodies (such as bispecific antibodies), univalent antibodies, multivalent antibodies, intact antibodies, fragments of intact antibodies, naked antibodies, conjugated antibodies, chimeric antibodies, humanized antibodies, fully human antibodies, Fab, Fab', Fab'-SH, F (ab') 2, Fd, Fv, scFv, bispecific antibodies (diabody) or single-domain antibody.

In another aspect, the present disclosure provides a nanobody or antigen-binding fragment specifically binding to PVRIG, which comprises HCDR1, HCDR2 and HCDR3 of the VH sequences shown in SEQ ID NO: 107-119, 198-204, 211-216, 219-225.

In some embodiments, according to the IMGT numbering scheme, wherein LCDR1, LCDR2 and LCDR3 of the nanobody or antigen-binding fragment are selected from, for example, Table 21; according to the Kabat numbering scheme, HCDR1, HCDR2 and HCDR3 are selected from, for example, Table 22 or Table 29.

In some embodiments, wherein the HCDR1∼3 of the VH shown in SEQ ID NO. 107 have sequences as shown in SEQ ID NO: 120-122 or SEQ ID NO: 159-161 according to the IMGT or Kabat numbering system;
the HCDR1-3 of the VH shown in SEQ ID NO. 108 have sequences as shown in SEQ ID NO: 123-125 or SEQ ID NO: 162-164 according to the IMGT or Kabat numbering system;
the HCDR1-3 of the VH shown in SEQ ID NO. 109 have sequences as shown in SEQ ID NO: 126-128 or SEQ ID NO: 165-167 according to the IMGT or Kabat numbering system;
the HCDR1-3 of the VH shown in SEQ ID NO. 110 have sequences as shown in SEQ ID NO: 129-131 or SEQ ID NO: 168-170 according to the IMGT or Kabat numbering system;
the HCDR1-3 of the VH shown in SEQ ID NO. 111 have sequences as shown in SEQ ID NO: 132-134 or SEQ ID NO: 171-173 according to the IMGT or Kabat numbering system;
the HCDR1-3 of the VH shown in SEQ ID NO. 112 have sequences as shown in SEQ ID NO: 135-137 or SEQ ID NO: 174-176 according to the IMGT or Kabat numbering system;
the HCDR1-3 of the VH shown in SEQ ID NO. 113 have sequences as shown in SEQ ID NO: 138-140 or SEQ ID NO: 177-179 according to the IMGT or Kabat numbering system;
the HCDR1-3 of the VH shown in SEQ ID NO. 114 have sequences as shown in SEQ ID NO: 141-143 or SEQ ID NO: 180-182 according to the IMGT or Kabat numbering system;
the HCDR1-3 of the VH shown in SEQ ID NO. 115 have sequences as shown in SEQ ID NO: 144-146 or SEQ ID NO: 183-185 according to the IMGT or Kabat numbering system;
the HCDR1-3 of the VH shown in SEQ ID NO. 116 have sequences as shown in SEQ ID NO: 147-149 or SEQ ID NO: 186-188 according to the IMGT or Kabat numbering system;
the HCDR1-3 of the VH shown in SEQ ID NO. 117 have sequences as shown in SEQ ID NO: 150-152 or SEQ ID NO: 189-191 according to the IMGT or Kabat numbering system;
the HCDR1-3 of the VH shown in SEQ ID NO. 118 have sequences as shown in SEQ ID NO: 153-155 or SEQ ID NO: 192-194 according to the IMGT or Kabat numbering system;
the HCDR1-3 of the VH shown in SEQ ID NO. 119 have sequences as shown in SEQ ID NO: 156-158 or SEQ ID NO: 195-197 according to the IMGT or Kabat numbering system;
the HCDR1-3 of the VH shown in SEQ ID NO. 198 have sequences as shown in SEQ ID NO: 168-170 according to the Kabat numbering system;
the HCDR1-3 of the VH shown in SEQ ID NO. 199 have sequences as shown in SEQ ID NO: 168, 207 and 170 according to the Kabat numbering system;
the HCDR1-3 of the VH shown in SEQ ID NO. 200 have sequences as shown in SEQ ID NO: 168, 207 and 208 according to the Kabat numbering system;
the HCDR1-3 of the VH shown in SEQ ID NO. 201 have sequences as shown in SEQ ID NO: 168, 207 and 209 according to the Kabat numbering system;
the HCDR1-3 of the VH shown in SEQ ID NO. 202 have sequences as shown in SEQ ID NO: 168, 169 and 208 according to the Kabat numbering system;
the HCDR1-3 of the VH shown in SEQ ID NO. 203 or 204 have sequences as shown in SEQ ID NO: 168, 210 and 208 according to the Kabat numbering system;
the HCDR1-3 of the VH shown in SEQ ID NO. 211-215 have sequences as shown in SEQ ID NO: 147-149 according to the IMGT numbering system;
the HCDR1-3 of the VH shown in SEQ ID NO. 216 have sequences as shown in SEQ ID NO: 147, 148 and 218 according to the IMGT numbering system;
the HCDR1-3 of the VH shown in SEQ ID NO. 219-225 have sequences as shown in SEQ ID NO: 156-158 according to the IMGT numbering system.

In some embodiments, the nanobody or antigen-binding fragment comprises CDRs sequences having at least 80% identity or having 1, 2, 3 or more amino acid insertions, deletions and/or substitutions compared with the HCDR1, HCDR2 and HCDR3, preferably, the substitutions are conservative amino acid substitutions.

In some embodiments, the nanobody or antigen-binding fragment comprises VH as shown in any one of SEQ ID NO. 107-119, 198-204, 211-216, 219-225, or a sequence having at least 80% identity or having at most 20 mutations compared with the VH as shown in any one of SEQ ID NO. 107-119, 198-204, 211-216, 219-225; the mutation may be selected from insertion, deletion and/or substitution, and the substitution is preferably a conservative amino acid substitution.

In some embodiments, the nanobody or antigen-binding fragment comprises at least a mutant sequence selected from the following group compared with the VH shown in SEQ ID NO. 110: A97V, K98E, N54D, N108S, S110A, G55A or S75T; preferably, at least A97V and K98E mutations; more preferably, at least A97V, K98E and N54D mutations; more preferably, at least A97V, K98E, N54D and N108S mutations; more preferably, at least A97V, K98E, N54D and S110A mutations; more preferably, at least A97V, K98E and N108S mutations; more preferably, at least A97V, K98E, G55A and N108S mutations; more preferably, at least S75T, A97V, K98E, G55A and N108S mutations, which are numbered in natural order;
or comprises at least a mutant sequence selected from the following group compared with the VH shown in SEQ ID NO. 116: S35T, V37F, G44E, L45R, W47F, N50T, L79V, V61S, D62H, T122I or M123Q; preferably, at least V37F, G44E, L45R, W47F and N50T mutations; more preferably, at least S35T, V37F, G44E, L45R, W47F and N50T mutations; more preferably, at least S35T, V37F, G44E, L45R, W47F, N50T and L79V mutations; more preferably, at least S35T, V37F, G44E, L45R, W47F, N50T, V61S and D62H mutations; more preferably, at least S35T, V37F, G44E, L45R, W47F, N50T, T122I and M123Q, which are numbered in natural order;
or comprises at least a mutant sequence selected from the following group compared with the VH shown in SEQ ID NO. 119: S35G, V37Y, G44D, L45R, W47L, N50T, Y58K, Y59I, D72G, N73D, Y79S, L78V or Y94F; preferably, at least S35G, V37Y, G44D, L45R, W47L and N50T mutations; more preferably, at least S35G, V37Y, G44D, L45R, W47L, N50T and Y58K mutations; more preferably, at least S35G, V37Y, G44D, L45R, W47L, N50T, Y58K, D72G and N73D mutations; more preferably, at least S35G, V37Y, G44D, L45R, W47L, N50T, Y58K, D72G, N73D and Y79S mutations; more preferably, at least S35G, V37Y, G44D, L45R, W47L, N50T, Y58K, D72G, N73D and L78V mutations; more preferably, at least S35G, V37Y, G44D, L45R, W47L, N50T, Y58K, Y59I, D72G and N73D mutations; more preferably, at least S35G, V37Y, G44D, L45R, W47L, N50T, Y58K, D72G, N73D and Y94F, which are numbered in natural order.

In some embodiments, the nanobody or antigen-binding fragment specifically binds to human and/or monkey PVRIG proteins; preferably, the KD value between the nanobody or antigen-binding fragment and human and/or monkey PVRIG proteins is better than 2.00E-9M.

In some embodiments, the nanobody or antigen-binding fragment is (1) chimeric nanobody or fragment thereof; (2) humanized nanobody or fragment thereof; or (3) fully human nanobody or fragment thereof.

In some embodiments, the nanobody or antigen binding fragment includes or does not include an antibody heavy chain constant region; optionally, the antibody heavy chain constant region is selected from human, alpaca, mouse, rat, rabbit or sheep; optionally, the antibody heavy chain constant region may be selected from IgG, IgM, IgA, IgE or IgD, and the IgG may be selected from IgG1, IgG2, IgG3 or IgG4; optionally, the heavy chain constant region may be selected from the Fc region, the CH3 region or the intact heavy chain constant region, preferably, the heavy chain constant region is a human Fc region; preferably, the nanobody or antigen binding fragment is a heavy chain antibody.

In another aspect, wherein the anti-PVRIG/anti-TIGIT bispecific antibody, the antibody or antigen-binding fragment specifically binding to TIGIT, the nanobody or antigen-binding fragment specifically binding to PVRIG of present disclosure are also coupled with a therapeutic agent or a tracer; preferably, the therapeutic agent is selected from drugs, toxins, radioisotopes, chemotherapeutic drugs or immunomodulators, and the tracer is selected from radiocontrast agents, paramagnetic ions, metals, fluorescent labels, chemiluminescent labels, ultrasound contrast agents and photosensitizers.

In another aspect, the present disclosure provides a multispecific molecule comprising the anti-PVRIG/anti-TIGIT bispecific antibody, the antibody or antigen-binding fragment specifically binding to TIGIT or the nanobodies or antigen-binding fragments specifically binding to PVRIG described above; preferably, the multispecific molecule may be bispecific, trispecific or tetraspecific, and more preferably, the multispecific molecule may be bivalent, tetravalent or hexavalent.

In some embodiments, the multispecific molecule may be tandem scFv, bifunctional antibody (Db), single chain bifunctional antibody (scDb), dual-affinity retargeting (DART) antibody, F(ab')2, dual variable domain (DVD) antibody, knob-into-holes (KIH) antibody, dock-and-lock (DNL) antibody, chemically crosslinked antibody, heteropolymeric nanoantibody or heteroconjugate antibody.

In another aspect, the present disclosure provides a chimeric antigen receptor (CAR), which comprises at least an extracellular antigen-binding domain, a transmembrane domain and an intracellular signaling domain, wherein the extracellular antigen-binding domain comprises the antibody or antigen-binding fragment specifically binding to TIGIT, or comprises any of the nanobodies or antigen-binding fragments specifically binding to PVRIG described above.

In another aspect, the present disclosure provides an immune effector cell which expresses the above chimeric antigen receptor or comprises a nucleic acid fragment encoding thereof; preferably, the immune effector cells are selected from T cells, natural killer cells (NK cells), natural killer T cells (NKT cells), double negative T cells (DNT cells), monocytes, macrophages, dendritic cells or mast cells, the T cells are preferably selected from cytotoxic T cells, regulatory T cells or T helper cells; preferably, the immune effector cells are autoimmune effector cells or allogeneic immune effector cells.

In another aspect, the present disclosure provides an isolated nucleic acid fragmentencoding any of the above bispecific antibodies, any of the above antibodies or antigen-binding fragments specifically binding to TIGIT, any of the above nanobodies or antigen-binding fragments specifically binding to PVRIG, any of the above multispecific molecules, or any of the above chimeric antigen receptor.

In another aspect, the present disclosure provides a vector comprising the above nucleic acid fragment.

In another aspect, the present disclosure provides a host cell comprising the above vector; preferably, the cell is a prokaryotic or eukaryotic cell, such as a bacterium (*Escherichia coli*), fungus (yeast), insect or mammalian cell (CHO or 293T cell line).

In another aspect, the present disclosure provides a method for preparing any of the above bispecific antibodies, any of the above antibodies or antigen-binding fragments specifically binding to TIGIT, any of the above nanobodies or antigen-binding fragments specifically binding to PVRIG, or any of the above multispecific molecule , which comprises culturing the above host cells and isolating the antibodies or molecules expressed by the cells.

In another aspect, the present disclosure provides a method for preparing the immune effector cells, which introduces the nucleic acid fragment encoding the CAR into the immune effector cells, and optionally, further initiates the immune effector cells to express the CAR.

In another aspect, the disclosure provides a pharmaceutical composition, which comprises any one of the above bispecific antibodies, any of the above antibodies or antigen-binding fragments specifically binding to TIGIT, any of the above nanobodies or antigen-binding fragments specifically binding to PVRIG, any of the above multispecific molecule, above the immune effector cells, nucleic acid fragments, vectors, host cells, or products prepared by the method, and pharmaceutically acceptable carriers.

In some embodiments, the pharmaceutical composition further comprises an additional therapeutic agent; preferably, the additional therapeutic agent is an antitumor agent; more preferably, the antitumor agent is a PD-1 axis binding antagonist.

In another aspect, the disclosure also provides the use of any of the above-mentioned bispecific antibodies, any of the above antibodies or antigen-binding fragments specifically binding to TIGIT, any of the above nanobodies or antigen-binding fragments specifically binding to PVRIG, any of the above multispecific molecule, the above immune effector cells, nucleic acid fragments, vectors and host cells, the products prepared by the method, or the pharmaceutical composition in the preparation of drugs for treating cancer or infectious diseases; wherein the cancer is selected from solid tumors and blood tumors, preferably, wherein the tumor is selected from leukemia, multiple myeloma, lymphoma, myelodysplastic syndrome, prostate cancer, liver cancer, colorectal cancer, anal cancer, ovarian cancer, endometrial carcinoma, cervical cancer, abdominal cancer, breast cancer, pancreatic cancer, gastric cancer, head and neck cancer, thyroid cancer, testicular cancer, urinary tract epithelial cancer, lung cancer, melanoma, non-melanoma skin cancer, glioma, kidney cancer, mesothelioma, esophageal cancer, non-small cell lung cancer, small cell lung cancer, bladder cancer, sarcoma, glioblastoma, thymic carcinoma, mycosis fungoides, Merkel cell carcinoma, high MSI cancer and KRAS mutant tumors.

In some embodiments, the drug is used in combination with an additional therapeutic agent or operation, wherein the additional therapeutic agent or operation is selected from radiotherapy, chemotherapy, oncolytic drugs, cytotoxic agents, cytokines, surgery, immunostimulatory antibodies, immunomodulatory drugs, activators of costimulatory molecules, inhibitors of inhibitory molecules, vaccines or cellular immunotherapy.

In some embodiments, the additional therapeutic agent is administered before or after the administration of the drug, or simultaneously with the drug.

In some embodiments, the drug is used in combination with a PD-1 axis binding antagonist.

In some embodiments, the PD-1 axis binding antagonist is selected from the group consisting of a PD-1 binding antagonist, a PD-L1 binding antagonist, and a PD-L2 binding antagonist; preferably, the PD-1 binding antagonist is an anti-PD-1 antibody; more preferably, the PD-1 binding antagonist is selected from the group consisting of MDX 1106 (nivolumab), MK-3475 (pembrolizumab), CT-011 (pidilizumab), MEDI-0680 (AMP-514), PDR001, REGN2810, and BGB-108; preferably, the PD-L1 binding antagonist is an anti-PD-L1 antibody; more preferably, the PD-L1 binding antagonist is selected from the group consisting of MPDL3280A (atezolizumab), YW243.55.S70, MDX-1105, MEDI4736 (durvalumab), Tecentriq and MSB0010718C (avelumab); preferably, the PD-L2 binding antagonist is an anti-PD-L2 antibody; more preferably, the PD-L2 binding antagonist is an immunoadhesin.

In another aspect, the disclosure further provides a method for treating cancer or infectious diseases, which comprises administering an effective amount of any of the bispecific antibodies, any of the above antibodies or antigen-binding fragments specifically binding to TIGIT, any of the above nanobodies or antigen-binding fragments specifically binding to PVRIG, any of the above multispecific molecule, the above immune effector cells, nucleic acid fragments, vectors, host cells, products prepared by the method or pharmaceutical compositions to patients in need; wherein the cancer is selected from solid tumors and blood tumors, preferably, wherein the tumor is selected from leukemia, multiple myeloma, lymphoma, myelodysplastic syndrome, prostate cancer, liver cancer, colorectal cancer, anal cancer, ovarian cancer, endometrial carcinoma, cervical cancer, abdominal cancer, breast cancer, pancreatic cancer, gastric cancer, head and neck cancer, thyroid cancer, testicular cancer, urinary tract epithelial cancer, lung cancer, melanoma, non-melanoma skin cancer, glioma, kidney cancer, mesothelioma, esophageal cancer, non-small cell lung cancer, small cell lung cancer, bladder cancer, sarcoma, glioblastoma, thymic carcinoma, mycosis fungoides, Merkel cell carcinoma, high MSI cancer and KRAS mutant tumors.

In some embodiments, the method further comprises administering an effective amount of a PD-1 axis binding antagonist to patients in need, wherein the PD-1 axis binding antagonist is selected from the group consisting of a PD-1 binding antagonist, a PD-L1 binding antagonist, and a PD-L2 binding antagonist; preferably, the PD-1 binding antagonist is an anti-PD-1 antibody; more preferably, the PD-1 binding antagonist is selected from the group consisting of MDX 1106 (nivolumab), MK-3475 (pembrolizumab), CT-011 (pidilizumab), MEDI-0680 (AMP-514), PDR001, REGN2810, and BGB-108; preferably, the PD-L1 binding antagonist is an anti-PD-L1 antibody; more preferably, the PD-L1 binding antagonist is selected from the group consisting of MPDL3280A (atezolizumab), YW243.55.S70, MDX-1105, MEDI4736 (durvalumab), Tecentriq and MSB0010718C (avelumab); preferably, the PD-L2 binding antagonist is an anti-PD-L2 antibody; more preferably, the PD-L2 binding antagonist is an immunoadhesin.

In another aspect, the disclosure also provides any of the above-mentioned bispecific antibodies, any of the above antibodies or antigen-binding fragments specifically binding to TIGIT, any of the above nanobodies or antigen-binding fragments specifically binding to PVRIG, any of the above multispecific molecule, the above immune effector cells, nucleic acid fragments, vectors and host cells, the products prepared by the method, or the pharmaceutical composition for treating cancer or infectious diseases, wherein the cancer is selected from solid tumors and blood tumors, preferably, wherein the tumor is selected from leukemia, multiple myeloma, lymphoma, myelodysplastic syndrome, prostate cancer, liver cancer, colorectal cancer, anal cancer, ovarian cancer, endometrial carcinoma, cervical cancer, abdominal cancer, breast cancer, pancreatic cancer, gastric cancer, head and neck cancer, thyroid cancer, testicular cancer, urinary tract epithelial cancer, lung cancer, melanoma, non-melanoma skin cancer, glioma, kidney cancer, mesothelioma, esophageal cancer, non-small cell lung cancer, small cell lung cancer, bladder cancer, sarcoma, glioblastoma, thymic carcinoma, mycosis fungoides, Merkel cell carcinoma, high MSI cancer and KRAS mutant tumors.

The anti-PVRIGxTIGIT humanized bispecific antibody of the present disclosure specifically targets tumor cells, effectively mediates the killing effect on tumor cell lines, and possesses a good safety profile while achieving excellent tumor suppression.

### DEFINITION OF TERMS

Unless otherwise defined by the present disclosure, the scientific and technical terms related to the present disclosure shall have the meanings understood by those skilled in the art.

In addition, unless otherwise specified herein, the terms in the singular form herein shall include the plural form, and the terms in the plural form shall include the singular form. More specifically, as used in the description and the appended claims, the singular forms "a" and "this" include pluralities unless otherwise expressly stated.

As used herein, the terms "comprise", "contain" and "include" are used interchangeably to indicate the inclusiveness of the embodiment, which suggests that the embodiment may have other elements besides the listed elements. Meanwhile, it should be understood that the descriptions of "comprise", "contain" and "include" used herein also refer to the embodiment of "consisting of ...". For example, "a composition comprising A and B" should be understood as the following technical solutions: a composition consisting of A and B, and a composition containing other components besides A and B, which all fall within the scope of the aforementioned "a composition".

As used herein, the term "and/or" includes the meanings of "and", "or" and "all or any other combination of elements linked by the terms".

The terms "T cell immune receptor with Ig and ITIM domains", "TIGIT", "TIGIT antigen", "Vstm3" and "WUCAM" can be used interchangeably and include various mammalian isoforms, such as human Tigit, orthologs of human Tigit, analogs containing at least one epitope in Tigit, and analogs having at least one epitope in common with TIGIT. The amino acid sequence of TIGIT (such as human TIGIT) and the nucleotide sequence encoding thereof are known in the art.

The terms "PVRIG" or "PVRIG protein" used herein may optionally include any such protein or variants, conjugates or fragments thereof, including but not limited to any known or wild-type PVRIG as described herein, and any naturally occurring splice variants, amino acid variants or isoforms, and especially the ECD fragment of PVRIG. "Anti-PVRIG antibodies" (including antigen-binding fragments) that bind to PVRIG and prevent activation by PVRL2 (for example, most often by blocking the interaction between PVRIG and PVLR2) are used to enhance the activation of T cells and/or NK cells, and thus used to treat diseases such as cancer and pathogen infections.

The terms "anti-PVRIG/anti-TIGIT antibody" and "bispecific PVRIG/TIGIT antibody" and "anti-PVRIG/anti-TIGIT bispecific antibody" can be used interchangeably herein. The anti-PVRIG/anti-TIGIT bispecific antibody of the present disclosure specifically binds to human TIGIT, preferably the ECD of human TIGIT, and PVRIG, even more preferably the ECD of human PVRIG.

The term "specific binding" herein refers to that an antigen-binding molecule (such as an antibody) usually specifically binds to an antigen and substantially the same antigen with high affinity, but does not bind to an unrelated antigen with high affinity. Affinity is usually reflected by equilibrium dissociation constant (KD), wherein a lower KD value suggests a higher affinity. Taking antibodies as an example, high affinity usually refers to a KD value of about 1 × 10⁻⁷M or lower, about 1×10⁻⁸M or lower, about 1×10⁻⁹M or lower, about 1×10⁻¹⁰M or lower, 1×10⁻¹M or lower or 1×10⁻¹²M or lower. KD value is calculated as follows: KD=Kd/Ka, where Kd represents the dissociation rate, and Ka represents the binding rate. The equilibrium dissociation constant (KD) can be measured by methods known in the art, such as surface plasmon resonance (e.g., Biacore) or equilibrium dialysis.

The term "antigen-binding molecule" is used herein in the broadest sense and refers to a molecule that specifically binds to an antigen. Illustratively, antigen binding molecules include, but are not limited to, antibodies or antibody mimetics. "Antibody mimics" refer to organic compounds or binding domains that can specifically bind to an antigen but are not structurally related to antibodies. Illustratively, antibody mimics include, but are not limited to, affibody, affitin, affilin, and designed ankyrin repeat proteins (DARPin), aptamer or Kunitz-type domain peptide.

The term "antibody" is used herein in the broadest sense and refers to a polypeptide or a combination of peptides that comprises sufficient sequences from the variable region of an immunoglobulin heavy chain and/or sufficient sequence from the variable region of an immunoglobulin light chain to specifically bind to an antigen. "Antibody" herein covers various forms and structures as long as they exhibit the desired antigen-binding activity. "Antibody" herein includes alternative protein scaffolds or artificial scaffolds with grafted complementarity determining regions (CDRs) or CDR derivatives. Such scaffolds include antibody-derived scaffolds (containing introduced mutations to, for example, stabilize the three-dimensional structure of the antibody) and fully synthetic scaffolds containing, for example, biocompatible polymers. Refer to, for example, Korndorfer et al., 2003, Proteins: Structure, Function, and Bioinformatics, 53(1): 121-129 (2003); Roque et al., Biotechnol. Prog. 20:639-654 (2004). Such scaffolds may also include non-antibody-derived scaffolds, such as scaffold proteins known in the art that can be used for CDR grafting, including but not limited to tenascin, fibronectin, peptide aptamers, etc.

The "antibody" herein includes a typical "four-chain antibody", which belongs to immunoglobulin consisting of two heavy chains (HCs) and two light chains (LCs). Heavy chain is comprised of a heavy chain variable region (VH), a heavy chain constant region CH1 domain, a hinge region (HR), a heavy chain constant region CH2 domain, and a heavy chain constant region CH3 domain in the direction from the N-terminus to the C-terminus. In addition, when the full-length antibody is IgE isotype, the heavy chain optionally further includes a heavy chain constant region CH4 domain. The light chain is a polypeptide chain comprised of the light chain variable region (VL) and the light chain constant region (CL) in the direction from N-terminus to the C-terminus. Heavy chain and heavy chain, and heavy chain and light chain are connected by disulfide bonds to form a "Y"-shaped structure. Antibodies have different amino acid composition and sequence in the constant region of the immunoglobulin heavy chain; thus, their antigenicity is also different. Accordingly, the "immunoglobulins" herein can be divided into five classes, or isotypes of immunoglobulins, namely IgM, IgD, IgG, IgA and IgE, and their corresponding heavy chains are µ chain, δ chain, γ chain, α chain and ε chain, respectively. The same type of Ig can be further divided into different subclasses according to the composition of amino acid in its hinge region and the number and position of heavy chain disulfide bonds, for example, IgG can be divided into IgG1, IgG2, IgG3, and IgG4, and IgA can be divided into IgA1 and IgA2. The light chain is divided into a κ chain or a λ chain by the difference of the constant region. Each of the five classes of IgG can have a κ chain or a λ chain.

"Antibodies" herein also include antibodies that do not contain light chains, such as heavy-chain antibodies (HCAbs) produced by camels such as Camelus dromedarius, Camelus bactrianus, Lama glama, Lama guanicoe and Vicugna pacos, and Ig new antigen receptor (IgNAR) found in Chondrichthyes such as sharks.

As used herein, the term "heavy chain antibody" refers to an antibody lacking a light chain of conventional antibodies. The term specifically includes but is not limited to homodimer antibodies comprising VH antigen binding domains and CH2 and CH3 constant domains in the absence of the CH1 domain.

As used herein, the term "nanobody" refers to a heavy chain antibody that is naturally present in camels and lacks light chains. The variable region can be cloned to obtain a single domain antibody consisting of only the variable region of the heavy chain, also known as VHH (Variable domain of heavy chain of heavy chain antibody), which is the smallest functional antigen-binding fragment.

The terms "nanobody" and "single domain antibody" (sdAb) have the same meaning and can be used interchangeably, which refers to the cloned variable region of heavy chain antibody, and constructs a single domain antibody composed of only one heavy chain variable region, which is the smallest antigen binding fragment with complete function. Usually, heavy chain antibodies with natural missing of light chain and heavy chain constant region 1 (CH1) are obtained first, and then the variable region of heavy chain of the antibodies is cloned to construct single domain antibodies composed of only one heavy chain variable region.

For further descriptions of "heavy chain antibodies" and "nanobodies", see: Hamers-Casterman et al., Nature. 1993; 363; 446-8; a review article by Muyldermans (Reviews in Molecular Biotechnology 74: 277-302, 2001); and the following patent applications, which are mentioned as general background technology: WO 94/04678, WO 95/04079 and WO 96/34103; WO 94/25591, WO 99/37681, WO 00/40968, WO 00/43507, WO 00/ 65057, WO 01/40310, WO 01/44301, EP 1134231 and WO 02/48193; WO 97/49805, WO 01/21817, WO 03/035694, WO 03/054016 and WO 03/055527; WO 03/050531; WO 01/90190; WO 03/025020; and WO 04/041867, WO 04/041862, WO 04/041865, WO 04/041863, WO 04/062551, WO 05/044858, WO 06/40153, WO 06/079372, WO 06/122786, WO 06/122787 and WO 06/122825 and other prior art mentioned in these applications.

The "antibody" herein can be derived from any animals, including but not limited to humans and non-human animals. The non-human animals can be selected from primates, mammals, rodents and vertebrates, such as camelids, llamas, cassowary, alpaca, sheep, rabbit, mouse, rat or cartilaginous fishes (such as sharks).

The "antibody" herein includes, but is not limited to, monoclonal antibodies, polyclonal antibodies, monospecific antibodies, multispecific antibodies (such as bispecific antibodies), univalent antibodies, multivalent antibodies, intact antibodies, fragments of intact antibodies, naked antibodies, conjugated antibodies, chimeric antibodies, humanized antibodies or fully human antibodies.

The term "monoclonal antibody" herein refers to an antibody obtained from a substantially homogeneous antibody population, that is, except for possible variants (for example, naturally-occurring mutations or mutations generated during the process of production, which are usually presented in a small amount), each antibody in the population is same and/or binds to the same epitope. In contrast to polyclonal antibody preparations which usually comprise different antibodies against different determinants (epitopes), each monoclonal antibody in a monoclonal antibody preparation targets a single determinant on the antigen. The modifier "monoclonal' herein should not be understood as requiring any specific methods to produce the antibody or antigen-binding molecule. For example, monoclonal antibodies can be produced by a variety of techniques, including (but not limited to) hybridoma technology, recombinant DNA technology, phage display library technology, and methods using transgenic animals containing all or part of human immunoglobulin loci and other methods known in the art.

The term "monospecific" herein refers to having one or more binding sites, wherein each binding site binds to the same epitope of the same antigen.

The term "multispecific" herein refers to having at least two antigen binding sites, each of the at least two antigen binding sites binds to a different epitope of the same antigen or to a different epitope of a different antigen. Therefore, terms such as "bispecific", "trispecific" and "tetraspecific" refer to the number of different epitopes that an antibody/antigen binding molecule can bind to.

The term "valency" herein refers to a specified number of binding sites in the antibody/antigen binding molecule. Therefore, the terms "monovalent", "bivalent", "tetravalent" and "hexavalent" refer to one binding site, two binding sites, four binding sites and six binding sites in an antibody/antigen binding molecule, respectively.

The "full-length antibody", "intact antibody" and "whole antibody" are used interchangeably herein and refer to having a structure that is substantially similar to the structure of a natural antibody.

The "antigen-binding fragment" and "antibody fragment" are used interchangeably herein, which do not have the complete structure of an intact antibody, but only contain parts of an intact antibody or variants thereof. The parts or variants thereof have the ability to bind to antigens. The "antigen-binding fragment" or "antibody fragment" herein includes but is not limited to Fab, Fab', Fab'-SH, F(ab')2, Fd, Fv, scFv, diabody and single-domain antibody.

The intact antibody is digested by papain and produces two identical antigen-binding fragments, called "Fab" fragments, each containing the variable domains of the heavy and light chains, as well as the constant domain of the light chain and the first constant domain of the heavy chain (CH1). As such, the term "Fab fragment" herein refers to an antibody fragment comprising the VL domain and the constant domain (CL) of the light chain, and the VH domain and the first constant domain (CH1) of the heavy chain. Fab' fragments differ from Fab fragments by adding a few residues to the carboxyl-terminus of the CH1 domain of the heavy chain, including one or more cysteines from the hinge region of the antibody. Fab'-SH is a Fab' fragment in which the cysteine residue of the constant domain carries a free thiol group. The F(ab')2 fragment is produced by pepsin treatment, which has two antigen binding sites (two Fab fragments) and a part of the Fc region.

The term "Fd" herein refers to an antibody composed of VH and CH1 domains. The term "Fv" herein refers to an antibody fragment composed of single-armed VL and VH domains. Fv fragments are generally considered to be the smallest antibody fragment that can form a complete antigen binding site. It is generally acknowledged that the antigen binding specificity of an antibody is provided by the six CDRs. However, even a variable region (such as an Fd fragment, which contains only three antigen-specific CDRs) can recognize and bind to antigens, although its affinity may be lower than the complete binding site.

The term "scFv" (single-chain variable fragment) herein refers to a single polypeptide chain comprising VL and VH domains, wherein the VL and VH are connected by a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, Vol. 113, Edited by Roseburg and Moore, Springer-Verlag, New York, pp. 269-315 (1994)). Such scFv molecules may have a general structure: NH2-VL-linker-VH-COOH or NH2-VH-linker-VL-COOH. Suitable linkers in the prior art consist of repeated GGGGS amino acid sequences or variants thereof. For example, a linker with the amino acid sequence (GGGGS) 4 or variants thereof can be used (Holliger et al. (1993), Proc. Natl. Acad. Sci. USA 90: 6444-6448). Other linkers that can be used in the present disclosure are described in Alfthan et al. (1995), Protein Eng. 8: 725-731, Choi et al. (2001), Eur. J. Immunol. 31: 94-106, Hu et al. (1996), Cancer Res 56:3055-3061, Kipriyanov et al. (1999), J. Mol. Biol. 293:41-56 and Roovers et al. (2001), Cancer Immunol. In some examples, there may also be disulfide bonds between the VH and VL of the scFv to form a disulfide-stabilized Fv (dsFv).

As for the term "diabody" herein, the VH and VL domains of which are expressed on a single polypeptide chain, but the linker is too short to allow pairing between the two domains on the same chain, thus forcing the domains to pair with the complementary domains of another chain and producing two antigen binding sites (see, for example, Holliger P. et al., Proc. Natl. Acad. Sci. USA 90: 6444-6448 (1993), and Poljak R. J. et al., Structure 2: 1121-1123 (1994)).

The term "naked antibody" herein refers to an antibody that is not conjugated to a therapeutic agent or a tracer; the term "conjugated antibody" refers to an antibody that is conjugated to a therapeutic agent or a tracer, preferably, the therapeutic agent is selected from drugs, toxins, radioisotopes, chemotherapeutic drugs or immunomodulators, and the tracer is selected from radiocontrast agents, paramagnetic ions, metals, fluorescent labels, chemiluminescent labels, ultrasound contrast agents and photosensitizers.

The term "chimeric antibody" herein refers to an antibody, wherein part of its light chain or/and heavy chain is derived from an antibody (which may be derived from a specific species or belongs to a specific antibody class or subclass), and the other part of the light chain or/and heavy chain is derived from another antibody (which may be derived from the same or different species or belongs to the same or different antibody class or subclass), but it still retains the binding activity to the antigen of interest in any case (U.S.P 4,816,567 to Cabilly et al.; Morrison et al., Proc. Natl. Acad. Sci. USA, 81:6851 6855 (1984)). For example, the term "chimeric antibody" may include antibodies (e.g., human-mouse chimeric antibodies) in which the variable regions of the heavy and light chain are derived from a first antibody (e.g., murine antibody), and the constant regions of the heavy chain and light chain are derived from a second antibody (e.g., a human antibody).

The term "humanized antibody" herein refers to a genetically engineered non-human antibody in which amino acid sequence has been modified to increase homology with the sequence of human antibodies. Usually, all or part of the CDR regions of a humanized antibody are derived from a non-human antibody (donor antibody), and all or part of the non-CDRs regions (for example, variable region FR and/or constant region) are derived from human immunoglobulin (receptor antibody). Humanized antibodies generally retain or partially retain the expected properties of the donor antibody, including but not limited to, antigen specificity, affinity, reactivity, ability to improve immune cell activity, and ability to enhance immune response.

The term "fully human antibody" herein refers to an antibody having variable regions in which both FR and CDR are derived from human germline immunoglobulin sequences. In addition, if the antibody contains a constant region, the constant region is also derived from human germline immunoglobulin sequences. Fully human antibodies herein may comprise amino acid residues that are not encoded by human germline immunoglobulin sequences (e.g., mutations introduced by random or site-specific mutagenesis in vitro or by somatic mutation in vivo). However, the "fully human antibodies" herein do not include antibodies in which CDR sequences derived from the germline of another mammalian species (e.g., mouse) have been grafted onto human framework sequences.

The term "variable region" herein refers to regions involved in antigen-antibody binding in the heavy or light chain of an antibody. The "variable region of the heavy chain" can be used interchangeably with "VH" and "HCVR", and the "variable region of the light chain" can be used interchangeably with "VL" and "LCVR". The variable domains of the heavy and light chains of natural antibodies (VH and VL, respectively) generally have similar structures, and each domain contains four conserved framework regions (FR) and three hypervariable regions (HVR) (see, for example, Kindt et al., Kuby Immunology, 6th ed., W.H. Freeman and Co., p. 91 (2007)). A single VH or VL domain may be sufficient to provide antigen binding specificity. The terms "complementarity determining region" and "CDR" can be used interchangeably herein, which usually refer to the hypervariable region (HVR) of the heavy chain variable region (VH) or light chain variable region (VL). The specific region is also called a complementarity determining region as it can form a precise complementation with an epitope in space structure, wherein the variable region (CDR) of the heavy chain can be abbreviated as HCDR, and the variable region of the light chain can be abbreviated as LCDR. The terms "framework region" or "FR region" can be used interchangeable, which refer to those amino acid residues other than CDR in the variable region of the heavy chain or the light chain. Generally, a typical antibody variable region consists of 4 FR regions and 3 CDR regions in the following order: FR 1-CDR 1-FR2-CDR2-FR3 -CDR3 -FR4.

For a further description of CDR, refer to Kabat et al., J. Biol. Chem., 252: 6609-6616 (1977); Kabat et al., U.S. Department of Health and Human Services, "Sequences of proteins of immunological interest" (1991); Chothia et al., J. Mol. Biol. 196: 901-917 (1987); Al-Lazikani B. et al., J. Mol. Biol., 273: 927-948 (1997); MacCallum et al., J. Mol Biol. 262: 732-745 (1996); Abhinandan and Martin, Mol. Immunol., 45: 3832-3839 (2008); Lefranc M.P. et al., Dev. Comp. Immunol., 27: 55-77 (2003 ); and Honegger and Plückthun, J. Mol. Biol., 309:657-670 (2001). The "CDR" herein can be labeled and defined in methods known in the art, including but not limited to the Kabat numbering scheme, Chothia numbering scheme or IMGT numbering scheme. The website tools used herein includes but not limited to the AbRSA website (http://cao.lab share.cn/AbRSA/cdrs.php), abYsis website (http://www.abysis.org/abysis/sequence_input/key_annotation/key_annotation.cgi) and IMGT website (http://www.imgt.org/3Dstructure-DB/cgi/DomainGapAlign. cgi#results). The CDRs herein include overlaps and subsets of amino acid residues defined in different methods.

The term "Kabat numbering scheme" herein generally refers to the immunoglobulin alignment and numbering scheme proposed by Elvin A. Kabat (see, for example, Kabat et al., Sequences of Proteins of Immunological Interest, 5th Ed. Public Health Service, National Institutes of Health, Bethesda, Md., 1991).

The term "IMGT numbering scheme" herein generally refers to the numbering scheme based on the international ImMunoGeneTics information system (IMGT)) initiated by Lefranc et al., see Lefranc et al., Dev.Comparat.Immunol. 27:55-77, 2003.

The term "heavy chain constant region" herein refers to the carboxyl-terminal part of the antibody heavy chain, which does not directly participate in the antigen-antibody binding, but shows effector functions, such as the interaction with the Fc receptor. The heavy chain constant region has a more conservative amino acid sequence compared with the variable domain of antibody. The "heavy chain constant region" includes at least: CH1 domain, hinge region, CH2 domain, CH3 domain, or variants or fragments thereof. The "heavy chain constant region" comprises "full-length heavy chain constant region" and "heavy chain constant region fragment". The former has a structure basically similar to the natural antibody constant regions, while the latter only includes "part of full-length heavy chain constant regions". Exemplarily, a typical "full-length antibody heavy chain constant region" consists of CH1 domain-hinge region-CH2 domain-CH3 domain. When the antibody is IgE, it also comprises CH4 domain. When the antibody is a heavy-chain antibody, it does not comprise the CH1 domain. Exemplarily, a typical "heavy chain constant region fragment" can be selected from CH1, Fc or CH3 domains.

The term "light chain constant region" herein refers to the carboxyl-terminal part of the antibody light chain, which does not directly participate in the antigen-antibody binding, and the light chain constant region can be selected from a constant κ domain or a constant λ domain.

The term "Fc" herein refers to the carboxyl-terminal part of a whole antibody hydrolyzed by papain, which typically comprises the CH3 and CH2 domains of the antibody. The Fc region includes, for example, a native sequence Fc region, a recombinant Fc region, and a variant Fc region. Although the boundaries of the Fc sequence of an immunoglobulin heavy chain might slightly change, the Fc region of a human IgG heavy chain is usually defined to stretch from an amino acid residue at position Cys226, or from position Pro230, to the carboxyl-terminus of the Fc sequence. The C-terminal lysine of the Fc region (residue 447 according to the Kabat numbering scheme) can be removed, for example, during the production or purification of the antibody, or by recombinant engineering of the nucleic acid encoding the antibody heavy chain. Therefore, the Fc region may or may not comprise Lys447.

The term "conservative amino acids" herein usually refers to amino acids that belong to the same class or have similar characteristics (such as charge, side chain size, hydrophobicity, hydrophilicity, main-chain conformation, and rigidity). Exemplarily, the amino acids in each of the following groups are conservative amino acid residues of each other, and the substitutions of amino acid residues within the groups belong to conservative amino acid substitutions:

Exemplarily, the following six groups are examples of amino acids that are considered to be conservative substitutions for each other:
1) Alanine (A), Serine (S), Threonine (T);
2) Aspartic acid (D), Glutamic acid (E);
3) Asparagine (N), Glutamine (Q);
4) Arginine (R), Lysine (K), Histidine (H);
5) Isoleucine (I), Leucine (L), Methionine Acid (M), Valine (V); and
6) Phenylalanine (F), Tyrosine (Y), Tryptophan (W).

The term "identity" herein can be calculated as follows: to determine the percentage of "identity" of two amino acid sequences or two nucleic acid sequences, the sequences are aligned for the best alignment (for example, gaps may be introduced into one or both of the first and second amino acid sequences or nucleic acid sequences for the best alignment, or non-homologous sequences may be discarded for comparison purpose). The amino acid residues or nucleotides at corresponding positions are then compared. When a position in the first sequence is occupied by the same amino acid residue or nucleotide at the corresponding position in the second sequence, then the molecules are identical at this position.

Considering the number of gaps and the length of each gap that need to be introduced for the best alignment between the two sequences, the percentage of identity between the two sequences varies with the same position shared by the sequences.

The sequence comparison and calculation of the percentage of identity between two sequences can be achieved by using mathematical algorithms. For example, using the Needlema and Wunsch ((1970) J.Mol.Biol.48:444-453) algorithm (available at www.gcg.com) in the GAP program, which has been integrated into GCG software package, and using the Blossum62 matrix or PAM250 matrix and gap weights of 16, 14, 12, 10, 8, 6, or 4 and length weights of 1, 2, 3, 4, 5, or 6, to determine the percentage of identity between two amino acid sequences. For another example, using the GAP program in the GCG software package (available at www.gcg.com), and using the NWSgapdna.CMP matrix and gap weights of 40, 50, 60, 70 or 80 and length weights of 1, 2, 3, 4, 5 or 6, to determine the percentage of identity between two nucleotide sequences. A particularly preferred parameter set (and a parameter set that should be used unless otherwise specified) is the Blossum62 scoring matrix with a gap penalty of 12, a gap extension penalty of 4, and a frameshift gap penalty of 5.

The PAM120 weighted remainder table, gap length penalty 12 and gap penalty 4 can also be used to determine the percentage of identity between two amino acid sequences or nucleotide sequences by using E. Meyers and W. Miller algorithms ((1989) CABIOS, 4:11-17) that have been incorporated into the ALIGN program (version 2.0).

Additionally or alternatively, the nucleic acid sequences and protein sequences of the present disclosure can be further used as "query sequences" to perform searches against public databases, for example, to identify the sequences of other family member or related sequences. For example, such searches can be performed by using NBLAST and XBLAST programs (version 2.0) (Altschul et al. (1990) J. Mol. Biol. 215:403-10). BLAST nucleotide search can be performed by NBLAST program with score=100 and word length=12 to obtain a nucleotide sequence homologous to the nucleic acid molecule of the present disclosure. BLAST protein search can be performed by XBLAST program with score=50, word length=3 to obtain amino acid sequences homologous to the protein molecule of the present disclosure. To obtain alignment results with gaps for comparison purposes, gapped BLAST can be used as described in Altschul et al., (1997) Nucleic Acids Res. 25:3389-3402. When using BLAST and gapped BLAST programs, the default parameters of the corresponding programs (for example, XBLAST and NBLAST) can be used. See www.ncbi.nlm.nih.gov.

The term "chimeric antigen receptor (CAR)" herein refers to an artificial cell surface receptor engineered to be expressed on an immune effector cell and specifically binds to an antigen, comprising at least (1) an extracellular antigen binding domain, such as a variable heavy or light chain of an antibody, (2) a transmembrane domain that anchors CAR into an immune effector cell, and (3) an intracellular signaling domain. CAR can use extracellular antigen binding domains to redirect T cells and other immune effector cells to selected targets, such as cancer cells, in a non-MHC limiting manner.

The term "immunostimulatory antibody" herein includes: 1) antagonistic antibodies targeting inhibitory immune checkpoints, including anti-CTLA4 mAb (such as ipilimumab, tremelimumab), anti-PD-1 (such as nivolumab BMS -936558/MDX-1106/ONO-4538, CT-011, lambrozilumab MK-3475, MEDI-0680 (AMP-514), PDR001, REGN2810, BGB-108), anti-PD-L1 antagonist (such as BMS-936559/MDX-1105, MEDI4736, RG-7446/MPDL3280A, MSB0010718C, YW243.55.S70), anti-LAG-3 (such as IMP-321), anti-TIM-3, anti-BTLA, anti-B7-H4, anti-B7-H3 and anti-VISTA; 2) agonistic antibodies for enhancing immunostimulatory proteins, including anti-CD40 mAb (such as CP-870,893, lucatumumab, dacetuzumab), anti-CD137 mAb (such as BMS-663513 urelumab, PF-05082566) , anti-OX40 mAb (such as anti-OX40), anti-GITR mAb (such as TRX518), anti-CD27 mAb (such as CDX-1127) and anti-ICOS mAb. The "immunostimulatory antibody" can promote anti-tumor immunity by directly regulating immune function, that is, blocking other inhibitory targets or enhancing immune stimulatory proteins.

The term "immunomodulatory drug" herein may be, for example, thymosin α1. Principle: thymosin α1 (Tα1) is a naturally occurring thymosin peptide that acts as an endogenous regulator of the innate and adaptive immune system. It is used worldwide to treat diseases related to immune dysfunction, including viral infections, such as hepatitis B and C, certain cancers, and to enhance the effect of vaccine. In particular, the latest progress in immunomodulation research has pointed out the beneficial effects of Ta1 treatment in patients with sepsis (Wu et al. "Critical Care" 2013, 17: R8).

The term "nucleic acid" herein includes any compound and/or substance comprising a polymer of nucleotides. Each nucleotide consists of bases, particularly purine or pyrimidine bases (i.e., cytosine (C), guanine (G), adenine (A), thymine (T) or uracil (U)), sugar (i.e., deoxyribose or ribose) and phosphate groups. Generally, a nucleic acid molecule is described by a base sequence, whereby the bases represent the primary structure (linear structure) of the nucleic acid molecule. The base sequence is usually represented as 5' to 3'. As used herein, the term "nucleic acid molecule" contains deoxyribonucleic acid (DNA), including, for example, complementary DNA (cDNA) and genomic DNA, ribonucleic acid (RNA), especially synthetic forms of messenger RNA (mRNA), DNA or RNA, and any mixed polymers containing two or more of these molecules. Nucleic acid molecules can be linear or cyclic. In addition, the term "nucleic acid molecule" includes the sense strand and the antisense strand, as well as single-stranded and double-stranded forms. Furthermore, the nucleic acid molecules described herein may comprise naturally-occurring or non-naturally-occurring nucleotides. Examples of non-naturally occurring nucleotides include modified nucleotide bases with derived sugar or phosphate backbone bonding or chemically modified residues. Nucleic acid molecules also include DNA and RNA molecules, which are suitable as vectors for directly expressing the antibody of the present disclosure in vitro and/or in vivo, for example, in hosts or patients. Such DNA (e.g. cDNA) or RNA (e.g. mRNA) vectors can be unmodified or modified. For example, the mRNA can be chemically modified to enhance the stability of the RNA vector and/or the expression of the encoded molecule, so that the mRNA can be injected into the subject to produce antibodies in vivo (see, for example, Stadler et al., Nature Medicine 2017, published online 2017 June 12, doi: 10.1038/nm.4356 or EP2101823B1). The "isolated" nucleic acid herein refers to a nucleic acid molecule that has been separated from components of its natural environment. An isolated nucleic acid includes a nucleic acid molecule contained in the following cells, which usually contains the nucleic acid molecule, but the nucleic acid molecule present outside the chromosome or at a chromosomal location different from its natural chromosomal location.

The term "vector" herein refers to a nucleic acid molecule, which is capable of amplifying another nucleic acid linked thereto. The term includes a vector as a self-replicating nucleic acid structure as well as a vector integrated into the genome of a host cell. Some vectors are capable of directing the expression of nucleic acids to which they are operably linked. Such vectors are referred to herein as "expression vectors".

The term "host cell" herein refers to a cell into which exogenous nucleic acids are introduced, including the offspring of such cells. Host cells include "transformants" and "transformed cells", which include the primary transformed cell and the offspring thereof, regardless of the number of passages. The offspring may not be identical with their parent cell in nucleic acid content, and may contain mutations. Included herein are mutant offspring that have the same function or biological activity as those screened or selected in the initially transformed cell.

The term "pharmaceutical composition" herein refers to a preparation that presents in a form that allows the biological activity of the active ingredients contained therein to be effective, and does not contain other ingredients that have unacceptable toxicity to the subject administered with the pharmaceutical composition.

The term "treatment" herein refers to surgical or therapeutic treatment, which aims to to prevent, mitigate (reduce) undesirable physiological changes or the progression of lesions, such as the progression of cancer. Beneficial or desired clinical results include, but are not limited to, relief of symptoms, alleviation of disease severity, stable disease status (i.e., no deterioration), prevention or slowdown of disease progression, improvement or mitigation of disease status, and remission (whether partial or complete), whether detectable or undetectable. The subjects in need of treatment include those who suffer from diseases or disorders, those who are prone to diseases or disorders or those who intend to prevent diseases or disorders. When the terms "mitigate, slow, relieve, reduce and alleviate" are mentioned, they also mean elimination, disappearance and non-occurrence.

The term "subject" herein refers to an organism receiving treatment for a specific diseases or disorders as described herein. Examples of subjects and patients include mammals receiving treatment for diseases or disorders, such as humans, primates (e.g., monkeys), or non-primate mammals.

The term "effective amount" herein refers to an amount of a therapeutic agent that is effective in preventing or alleviating the condition of a disease or the progression of the disease, when administered to a cell, tissue or subject alone or in combination with another therapeutic agent. The "effective amount" also refers to an amount of a compound that is sufficient to relieve symptoms (such as to treat, cure, prevent or relieve related medical conditions), or to treat, cure, prevent or relieve those conditions at an increased rate. When the active ingredient is administered to an individual alone, the therapeutically effective dose refers solely to the active ingredient. When a certain combination is administrated, the therapeutically effective dose refers to the combined amount of active ingredients that have a therapeutic effect, whether it is administered in a combination, continuously or simultaneously.

The term "cancer" herein refers to a physiological condition in mammals that is typically characterized by unregulated cell growth. Both benign and malignant cancers are included in this definition. The term "tumor" herein refers to all the neoplastic cell growth and proliferation, whether malignant or benign, and all pre-cancerous and cancerous cells and tissues. The terms "cancer" and "tumor" are not mutually exclusive when mentioned herein.

The term "EC50" herein refers to the half maximal effective concentration, which includes the concentration of antibody that induces a response halfway between the baseline and maximum after a specified exposure time. EC50 essentially represents the antibody concentration at which 50% of its maximum effect is observed, and can be measured by methods known in the art.

The term "G4S linker peptide" herein refers to the GS combination of glycine (G) and serine (S), which is used to link multiple proteins together to form a fusion protein. The commonly used GS combination is (GGGGS)n, and the length of the linker sequence can be changed by changing n. Meanwhile, glycine and serine can also be combined to produce different linker sequences, such as the GS combination of (G4S)4 Linker used in the present disclosure is GGGGS.

### DESCRIPTION OF THE FIGURES

FIG. 1 shows the binding activity of anti-TIGIT human-mouse chimeric antibodies to human TIGIT ECD-mFc fusion proteins.
FIG. 2 shows the binding activity of anti-TIGIT human-mouse chimeric antibodies to cynomolgus monkey TIGIT ECD-mFc fusion proteins.
FIG. 3 shows the binding activity of anti-TIGIT human-mouse chimeric antibodies to CHO-K1 human TIGIT high expression cell strains.
FIG. 4 shows the binding activity of anti-TIGIT human-mouse chimeric antibodies to CHO-K1 human TIGIT medium expression cell strains.
FIG. 5 shows the binding activity of anti-TIGIT human-mouse chimeric antibodies to CHO-K1 human TIGIT low expression cell strains.
FIG. 6 shows the binding activity of anti-TIGIT human-mouse chimeric antibodies to cynomolgus monkey TIGIT cells.
FIG. 7 shows the effect of anti-TIGIT human-mouse chimeric antibodies blocking the interaction between Bio-CD155-His and CHO-K1 human TIGIT.
FIG. 8 shows the effect of anti-TIGIT human-mouse chimeric antibodies blocking the interaction between TIGIT ECD-mFc and CHO-K1 CD155.

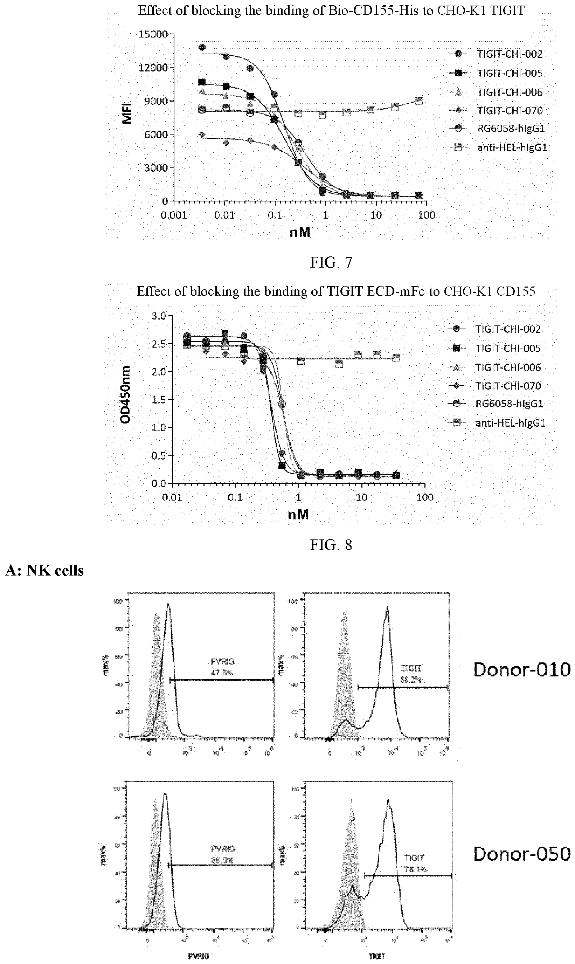

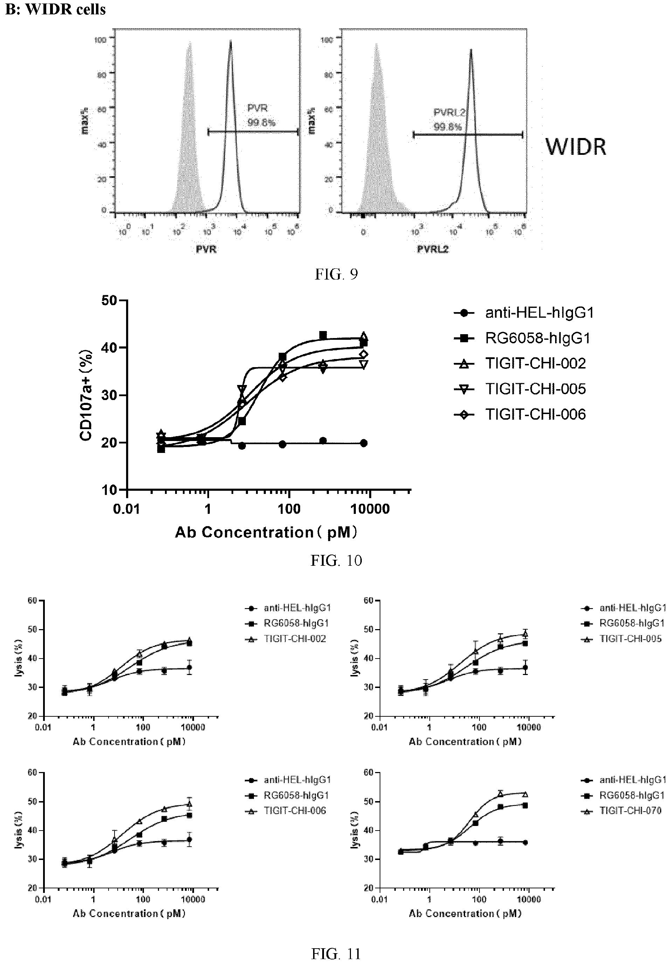

FIG. 9 shows the expression levels of PVRIG and TIGIT on the surface of NK cells from different donor sources (donor-010 and donor-050), and the expression levels of PVR and PVRL2 on the surface of tumor cell line WIDR cells detected by FACS; Panel A, in the graph, the black hollow peak refers to the expression level of PVRIG/TIGIT on the surface of NK cells, and the gray solid peak refers to the isotype controls corresponding to two detected antibodies. Panel B, in the graph, the black hollow peak refers to the expression level of PVR/PVRL2 on the surface of WIDR cells, and the gray solid peak refers to isotype controls corresponding to two detected antibodies.
FIG. 10 shows the effect of co-incubation of anti-TIGIT human-mouse chimeric antibodies with WIDR cells and NK cells on NK cell degranulation (CD107a). The abscissa is the concentration of tested antibodies, and the ordinate is the percentage of CD107a positive cells, wherein RG6058-hIgG1 is a positive control antibody, and anti-HEL-hIgG1 is a negative isotype control antibody.
FIG. 11 shows the effect of anti-TIGIT human-mouse chimeric antibodies on cytotoxicity for WIDR target cells by NK cells. The abscissa shows the concentration of tested antibodies, the ordinate shows the death rate of target cells, RG6058-hIgG1 is a positive control antibody, and anti-HEL-hIgG1 is a negative isotype control antibody.

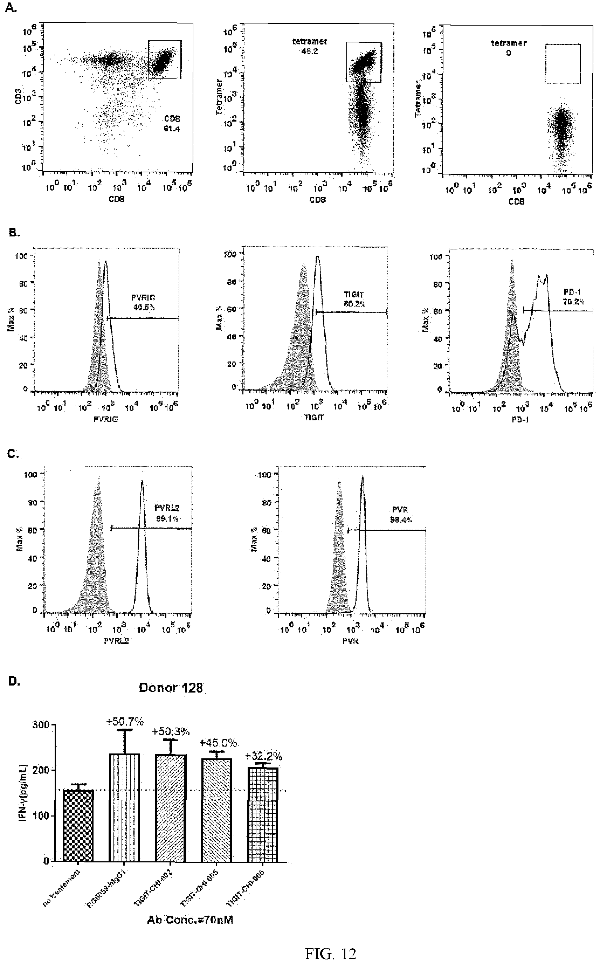

FIG. 12 shows the effect of anti-TIGIT human-mouse chimeric antibodies on the functional activity of antigen-specific CD8 T cells detected by CMV antigen-recall assay. Panel A, the percentage of CD8 T cells, CMV pp65-specific CD8 T cells and FMO control in CMV IgG positive donor 128 PBMC after being induced by CMV pp65 (495-503) for 11 days; Panel B, the expression of PVRIG, TIGIT and PD-1 on CMV pp65-specific CD8 T cells; Panel C, the expression of PVRL2 and PVR on Colo205; Panel D, the secretion level of IFN-γ in cell supernatant after 18 hours co-incubation, the positive control is RG6058-hIgG1, and the negative control is no treatment (without any drug treatment), the histogram shows the percentage of IFN-γ secretion increased in the experimental group compared with no treatment group.
FIG. 13 shows the binding activity of anti-TIGIT humanized antibodies to human TIGIT ECD-mFc fusion proteins.
FIG. 14 shows the binding activity of anti-TIGIT humanized antibodies to cynomolgus monkey TIGIT ECD-mFc fusion proteins.
FIG. 15 shows the binding activity of anti-TIGIT humanized antibodies to CHO-K1 human TIGIT high expression cell strains.
FIG. 16 shows the binding activity of anti-TIGIT humanized antibodies to CHO-K1 human TIGIT medium expression cell strains.
FIG. 17 shows the binding activity of anti-TIGIT humanized antibodies to CHO-K1 human TIGIT low expression cell strains.
FIG. 18 shows the binding activity of anti-TIGIT humanized antibodies to cynomolgus monkey TIGIT cells.
FIG. 19 shows the effect of anti-TIGIT humanized antibodies blocking the interaction between Bio-CD155-His and CHO-K1 human TIGIT.
FIG. 20 shows the effect of anti-TIGIT humanized antibodies blocking the interaction between TIGIT ECD-mFc and CHO-K1 CD155.
FIG. 21 shows the effect of anti-TIGIT humanized antibodies blocking the interaction between TIGIT ECD-mFc and CHO-K1 CD112.
FIG. 22 shows the binding activity of anti-TIGIT humanized antibodies to human PBMCs.
FIG. 23 shows the effect of anti-TIGIT humanized antibodies on cytotoxicity for WIDR target cells by NK cells. The abscissa shows the concentration of tested antibodies, the ordinate shows the death rate of target cells, TIGIT-CHI-002, TIGIT-CHI-005, TIGIT-CHI-006 and TIGIT-CHI-070 are chimeric antibodies before humanization, RG6058-hIgG1 is a positive control antibody, and anti-HA HcAb-hIgG 1 is a negative isotype control antibody.

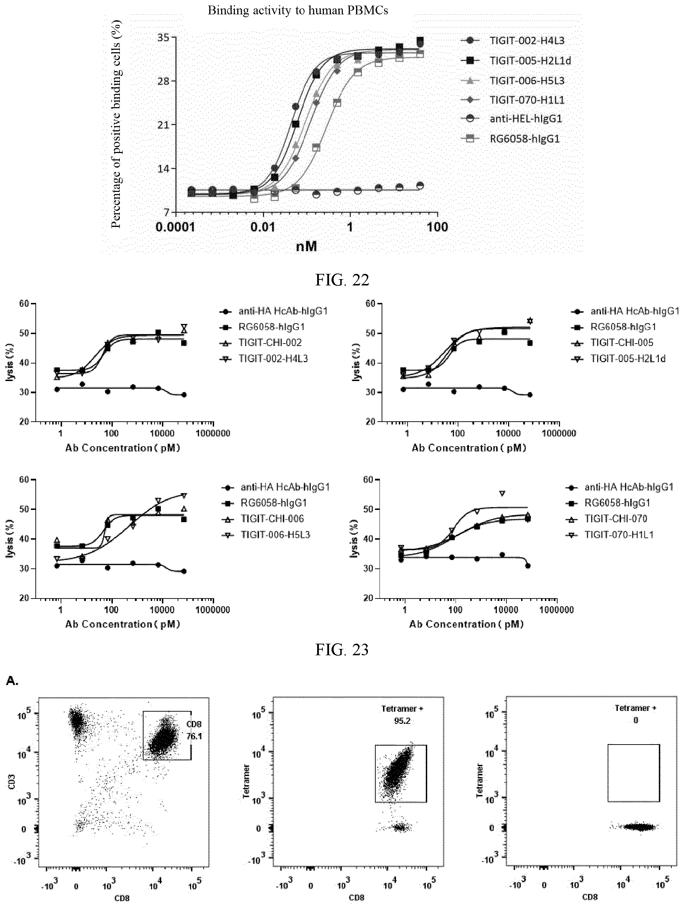

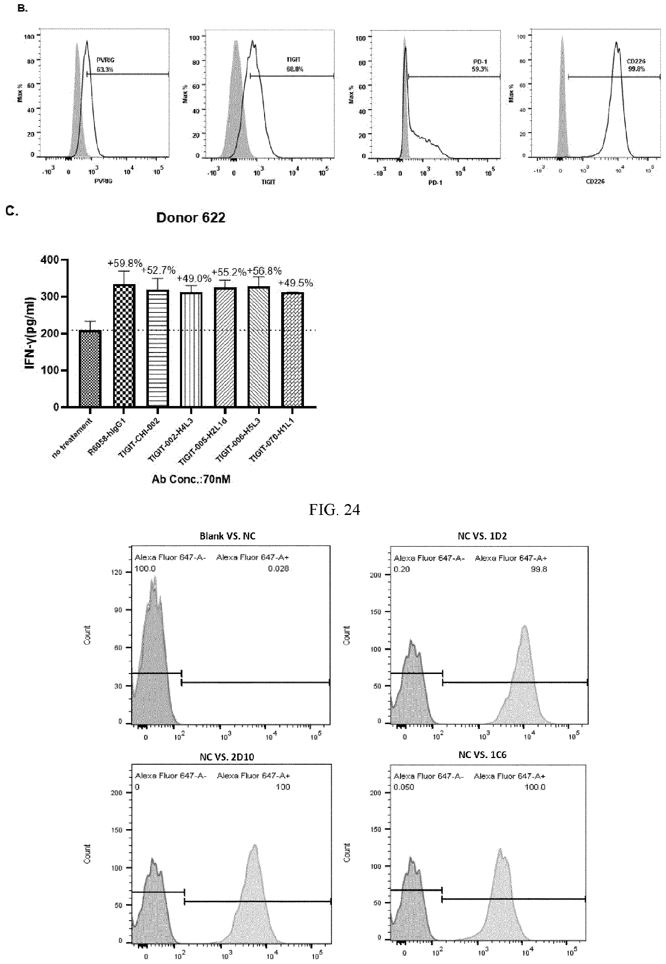

FIG. 24 shows the effect of anti-TIGIT humanized antibodies on the functional activity of antigen-specific CD8 T cells detected by CMV antigen-recall assay. Panel A, the percentage of CD8 T cells, CMV pp65-specific CD8 T cells and FMO control in CMV IgG positive donor 622 PBMC after being induced by CMV pp65 (495-503) for 11 days; Panel B, the expression of PVRIG, TIGIT, PD-1 and CD226 on CMV pp65-specific CD8 T cells; Panel C, the secretion level of IFN-γ in cell supernatant after 18 hours co-incubation, the positive control is RG6058-hIgG1 and TIGIT-CHI-002, and the negative control is no treatment (without any drug treatment), the histogram shows the percentage of IFN-γ secretion increased in the experimental group compared with that in no treatment group.
FIG. 25 shows the result of the human TIGIT overexpressing cell strain (CHO-K1 human TIGIT) detected by FACS.
FIG. 26A shows the binding ability of test PVRIG antibodies to human PVRIG recombinant proteins. The figure shows the binding ability of the tested antibodies PVRIG-A11, A15, A30, A35, A43, A50, A60, A75, A104, A105, A113, A117 and A118 to human PVRIG protein, wherein COM701-hIgG1, COM701-hIgG4 and SRF813-hIgG1 are the positive controls of this experiment; anti-HA HcAb-hIgG1 and anti-CD38 HcAb-hIgG1 are the negative controls of this experiment.
FIG. 26B shows the binding ability of tested PVRIG antibodies to cynomolgus monkey PVRIG recombinant proteins. The figure shows the binding ability of the tested antibodies PVRIG-A11, A15, A30, A35, A43, A50, A60, A75, A104, A105, A113, A117 and A118 to cynomolgus monkey PVRIG protein, wherein COM701-hIgG1, COM701-hIgG4 and SRF813-hIgG1 are the positive controls of this experiment; anti-HA HcAb-hIgG1, anti-CD38 HcAb-hIgG1 and anti-Fluorescein-hIgG1 are the negative controls of this experiment.
FIG. 27A shows the binding activity of tested PVRIG antibodies to human PVRIG on the surface of FlpinCHO-PVRIG cells. The figure shows the binding ability of the tested antibodies PVRIG-A11, A15, A30, A35, A43, A50, A60, A75, A104, A105, A113, A117 and A118 to human PVRIG on the surface of FlpinCHO-PVRIG cells, wherein COM701-hIgG1 and SRF813-hIgG1 are the positive controls; anti-CD38 HcAb-hIgG1 are the negative isotype controls.
FIG. 27B shows the binding activity of tested PVRIG antibodies to cynomolgus monkey PVRIG on the surface of FlpinCHO-PVRIG cells. The figure shows the binding ability of the tested antibodies PVRIG-A11, A15, A30, A35, A43, A50, A60, A75, A104, A105, A113, A117 and A118 to cynomolgus monkey PVRIG on the surface of FlpinCHO-PVRIG cells, wherein COM701-hIgG1 and SRF813-hIgG1 are the positive controls; anti-CD38 HcAb-hIgG1 are the negative isotype control.
FIG. 28 shows the blocking of tested PVRIG antibodies on the interaction between human PVRIG and human PVRL2 recombinant protein. The figure shows the blocking effect of the tested antibodies PVRIG-A11, A15, A30, A35, A43, A50, A60, A75, A104, A105, A113, A117 and A118 on the binding of PVRIG and PVRL2, wherein COM701-hIgG4 and SRF813-hIgG1 are the positive controls of this experiment; anti-HA HcAb-hIgG1 and anti-CD38 HcAb-hIgG1 are the negative controls of this experiment.
FIG. 29 shows the blocking of tested PVRIG antibodies on the binding of CHO-K1-CD112 cells and human PVRIG-mFc proteins. The figure shows the ability of the tested antibodies PVRIG-A11, A15, A30, A35, A43, A50, A60, A75, A104, A105, A113, A117 and A118 to compete with CHO-K1-CD112 cells to bind to human PVRIG-mFc protein, wherein COM701-hIgG4 and SRF813-hIgG1 are the positive controls; anti-CD38 HcAb-hIgG1 is the negative control.
FIG. 30 shows the expression levels of PVR and PVRL2 on the surface of tumor cell line Reh cells, in the graph, the black hollow peak refer to the expression of PVR/PVRL2 on the surface of Reh cells, the gray solid peak refers to the isotype controls corresponding to two detected antibodies.

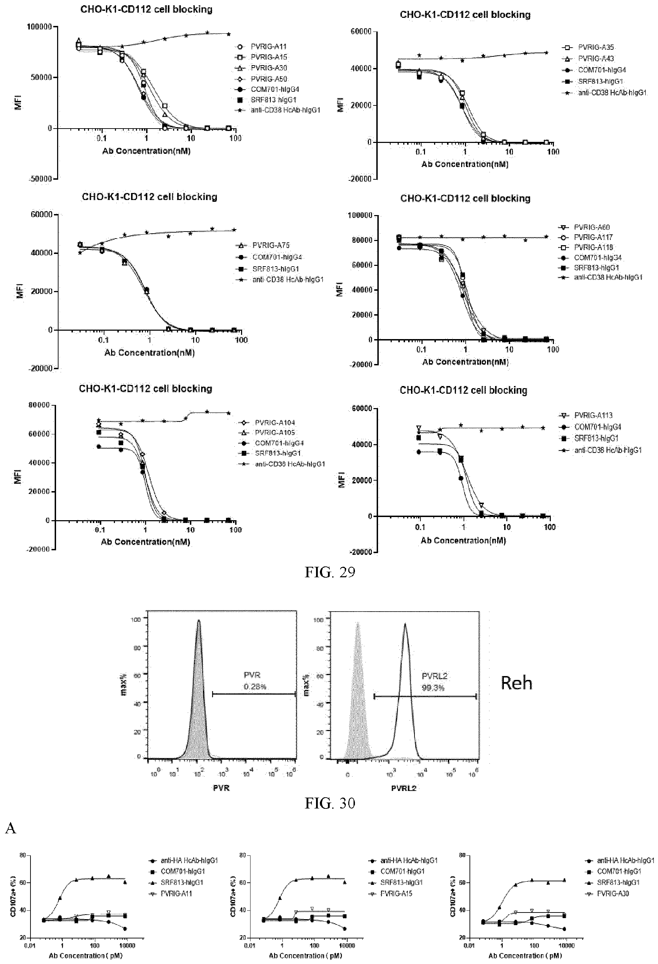

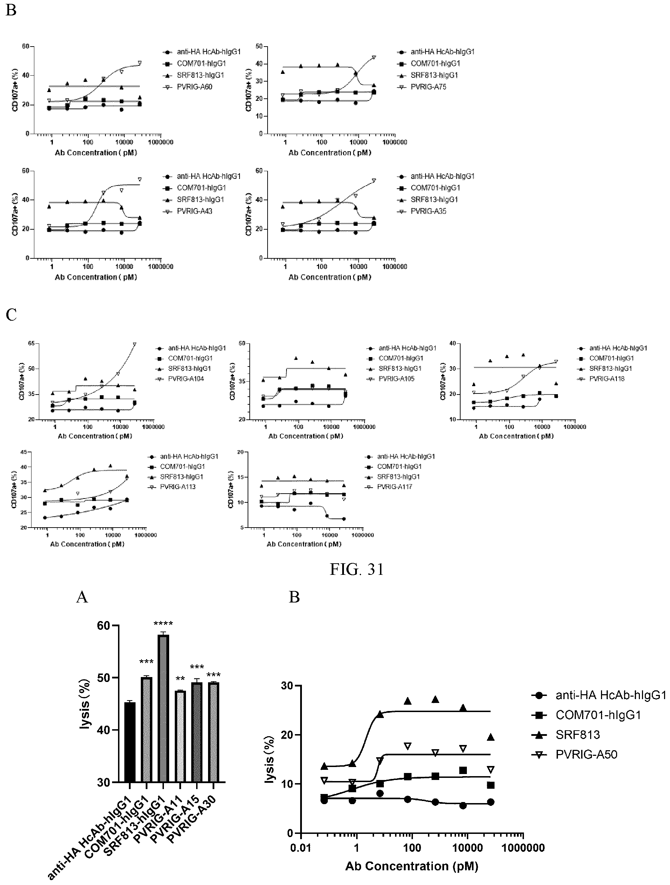

FIG. 31 shows the effect of tested PVRIG antibodies on NK cell degranulation. Fig. A shows the effect of tested antibodies PVRIG-A11, A15, A30 on the expression of CD107a on NK cells when NK cells (donor-010) are incubated with the target cell Reh. Fig. B shows the effect of the tested antibodies PVRIG-A60, A75, A43, A35 on the expression of CD107a on NK cells when NK (donor-010) is incubated with the target cell WIDR. Fig. C shows the effect of the tested antibodies PVRIG-A104, A105, A118, A113, and A117 on the expression of CD107a on NK cells when NK cells (donor-050) are incubated with target cells WIDR. The abscissa is the concentration of tested antibodies, and the ordinate is the percentage of CD107a positive cells, wherein COM701-hIgG1 and SRF813-hIgG1 is positive control antibodies, and anti-HA HcAb-hIgG1 is a negative isotype control antibody.

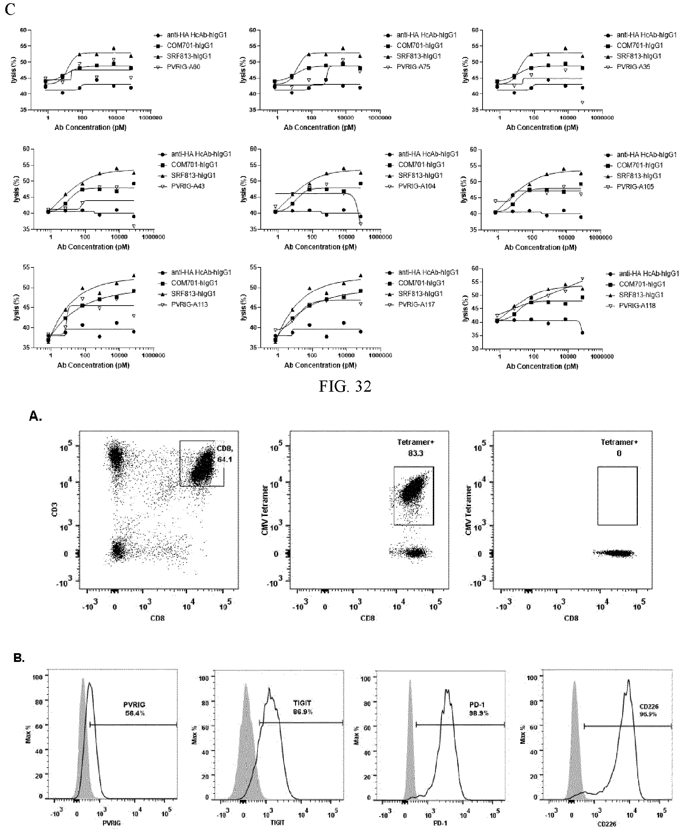

FIG. 32 shows the effect of tested PVRIG antibodies on cytotoxicity for target cells by NK cells. Fig. A shows the promoting effect of tested antibodies PVRIG-A11, A15 and A30 on cytotoxicity for target cells WIDR by NK cells (donor-010) at the concentration of 6.87 nM. There were significant differences between the tested antibodies and the negative isotype control anti-HA HcAb-hIgG1 (**p<0.01, **p<0.001, ***p<0.0001, One-Way ANOVA Analysis). Fig. B shows the promoting effect of tested antibodies PVRIG-A50 on cytotoxicity for target cells WIDR by NK cells (donor-010) at different concentrations. Fig. C shows the promoting effect of tested antibodies PVRIG-A60, A75, A35, A43, A104, A105, A113, A117 and A118 on cytotoxicity for target cells WIDR by NK cells (donor-050) at different concentrations. The abscissa of FIG. B and C is the concentration of tested antibodies, and the ordinate is the death rate of target cells, wherein COM701-hIgG1 and SRF813-hIgG1 are positive control antibodies, and anti-HA HcAb-hIgG1 is a negative isotype control antibody.

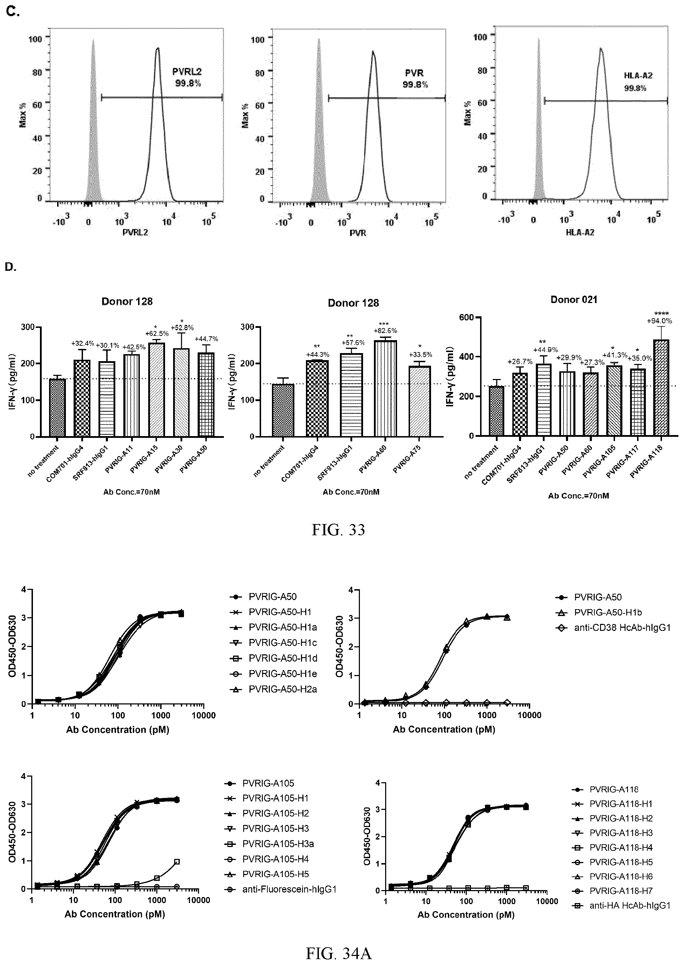

FIG. 33 shows the functional improvement effect of PVRIG antibody on antigen-specific CD8 T cells detected by CMV antigen-recall assay. Fig. A shows the percentage of CD8 T cells and CMV pp65-specific CD8 T cells in CMV IgG positive donor 021 PBMCs after being induced by CMV pp65 (495-503) for 11 days. Fig. B shows the expression of PVRIG, TIGIT, PD-1 and CD226 on CMV pp65-specific CD8 T cells (donor 021). Fig. C shows the expression of PVRL2, PVR and HLA-A2 on colo205. Fig. D shows the secretion level of IFN-γ in cell supernatant after 18 hours co-incubation. The positive control is COM701-hIgG4 and SRF813-hIgG1, and the negative control is no treatment (without any drug treatment) in this experimental system. The final concentration of antibody was 70 nM. Compared with no treatment group, the secretion of IFN-γ in cell supernatant increased significantly after treating with PVRIG-A15, A30, A60, A75, A105, A117 and A118 (*p<0.05, **p<0.01, ***p<0.001, ***p<0.0001, one-way ANOVA analysis).
FIG. 34A shows the binding ability of humanized PVRIG antibodies to human PVRIG recombinant proteins. The figure shows the binding activity of the humanized molecules and the corresponding parental antibodies PVRIG-A50, A105 and A118 to human PVRIG protein, wherein anti-HA HcAb-hIgG1, anti-CD38 HcAb-hIgG1 and anti-Fluorescein-hIgG1 are negative controls.
FIG. 34B shows the binding ability of humanized PVRIG antibodies to cynomolgus monkey PVRIG recombinant proteins. The figure shows the binding activity of the humanized molecules and the corresponding parental antibodies PVRIG-A50, A105 and A118 to cynomolgus monkey PVRIG protein, wherein anti-HA HcAb-hIgG1, anti-CD38 HcAb-hIgG1 and anti-Fluorescein-hIgG 1 are negative controls.
FIG. 35A shows the binding ability of humanized PVRIG antibodies to human PVRIG on the surface of FlpinCHO-PVRIG cells. The figure shows the binding activity of the humanized molecules and the corresponding parental antibodies PVRIG-A50, A105 and A118 to human PVRIG on the surface of FlpinCHO-PVRIG cells, wherein COM701-hIgG1 and SRF813-hIgG1 are positive control antibodies, and anti-CD38 HcAb-hIgG1 and anti-Fluorescein-hIgG 1 are negative isotype control antibody.
FIG. 35B shows the binding ability of humanized PVRIG antibodies to cynomolgus monkey PVRIG on the surface of FlpinCHO-PVRIG cells. The figure shows the binding activity of the humanized molecules and the corresponding parental antibodies PVRIG-A50, A105 and A118 to cynomolgus monkey PVRIG on the surface of FlpinCHO-PVRIG cells, wherein COM701-hIgG1 and SRF813-hIgG1 are positive control antibodies, and anti-CD38 HcAb-hIgG1 and anti-Fluorescein-hIgG 1 are negative isotype control antibody.
FIG. 36 shows the blocking of humanized PVRIG antibodies on the binding of human PVRIG and human PVRL2. The figure shows the blocking effect of the humanized molecules and the corresponding parental antibodies PVRIG-A50, A105 and A118 on the binding of human PVRIG and human PVRL2, wherein anti-HA HcAb-hIgG1 and anti-Fluorescein-hIgG1 are negative controls.
FIG. 37 shows the blocking of humanized PVRIG antibodies on the binding of CHO-K1-CD112 cells and human PVRIG-mFc proteins. The figure shows the ability of the humanized molecules and the corresponding parental antibodies PVRIG-A50, A105 and A118 to compete with CHO-K1-CD112 cells to bind to human PVRIG-mFc protein, wherein COM701-hIgG4 and SRF813-hIgG1 are the positive control antibodies; anti-CD38 HcAb-hIgG1 and anti-Fluorescein-hIgG 1 are the negative isotype control antibodies.
FIG. 38 shows the effect of humanized PVRIG antibodies on cytotoxicity by NK cells. The figures shows the promoting effect of the humanized molecules and the corresponding parental antibodies PVRIG-A50 (A), A105 (B) and A118 (C) on cytotoxicity for target cells WIDR by NK cells (donor-050) at different concentrations. The abscissa is the concentration of tested antibodies, and the ordinate is the death rate of target cells, wherein COM701-hIgG1 and SRF813-hIgG1 are positive control antibodies, and anti-HA HcAb-hIgG1 and anti-Fluorescein-hIgG 1 are negative isotype control antibodies.

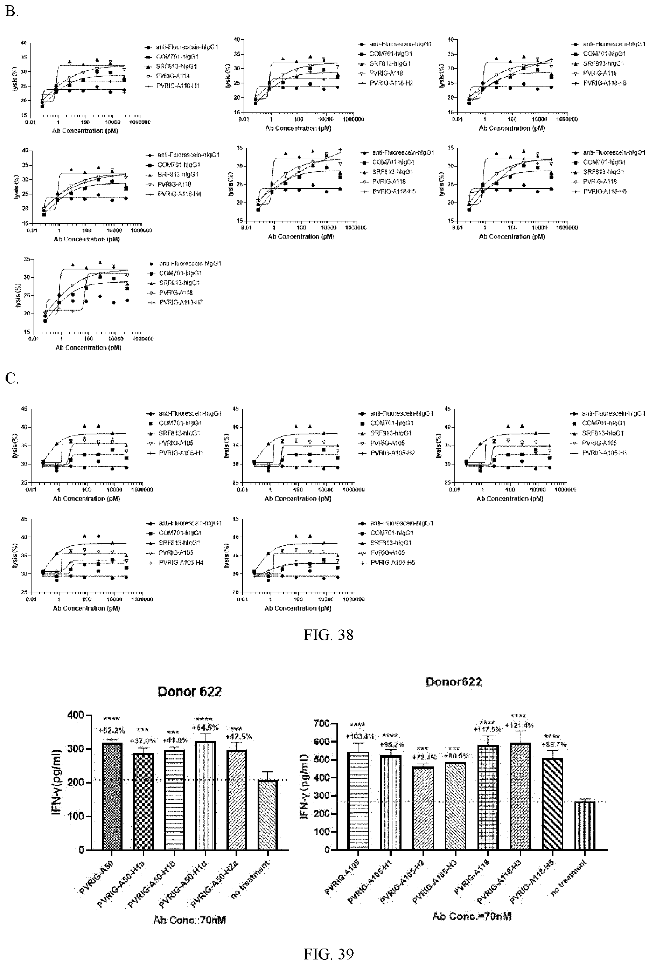

FIG. 39 shows the functional improvement effect of humanized PVRIG antibody on antigen-specific CD8 T cells detected by CMV antigen-recall assay. shows the secretion level of IFN-γ in cell supernatant after 18 hours co-incubation. The positive control is the PVRIG parental antibodies before humanization, and the negative control is no treatment (without any drug treatment) in this experimental system. The final concentration of antibody was 70 nM. *p<0.05, **p<0.01, ***p<0.001, ****p<0.0001 vs. no treatment, one-way ANOVA Analysis. The effect of the 4 humanized antibodies of PVRIG-A50 (PVRIG-A50-H1a, H1b, H1d, H2a) is not significantly different from that of PVRIG-A50 (One-way ANOVA Analysis); the effect of the two humanized antibodies (PVRIG-A118-H3, H5) of PVRIG-A118 is not significantly different from that of PVRIG-A118; the effect of PVRIG-A105-H2 is significantly weaker than that of PVRIG-A105 (*p<0.05, one-way ANOVA analysis), and the other two humanized antibodies (PVRIG-A105-H1, H3) have no significant difference with PVRIG-A105 (one-way ANOVA analysis).
FIG. 40 shows the composition and structure of four humanized bispecific antibodies
FIG. 41 shows the binding of four humanized bispecific antibodies to human PVRIG-ECD-mFc protein detected by ELISA.
FIG. 42 shows the binding of four humanized bispecific antibodies to cynomolgus monkey PVRIG-ECD-mFc protein detected by ELISA.
FIG. 43 shows the binding of four humanized bispecific antibodies to human TIGIT-ECD-mFc protein detected by ELISA.
FIG. 44 shows the binding of four humanized bispecific antibodies to cynomolgus monkey TIGIT-ECD-mFc protein detected by ELISA.
FIG. 45 shows the binding activity of four humanized bispecific antibodies to FlpinCHO-human PVRIG cells detected by FACS.
FIG. 46 shows the binding activity of four humanized bispecific antibodies to FlpinCHO-cynomolgus monkey PVRIG cells detected by FACS.
FIG. 47 shows the binding activity of four humanized bispecific antibodies to CHO-K1-human TIGIT high expression cell strain detected by FACS.
FIG. 48 shows the binding activity of four humanized bispecific antibodies to CHO-K1-human TIGIT medium expression cell strain detected by FACS.
FIG. 49 shows the binding activity of four humanized bispecific antibodies to CHO-K1-human TIGIT low expression cell strain detected by FACS.
FIG. 50 shows the binding activity of four humanized bispecific antibodies to CHO-K1-cynomolgus monkey TIGIT cell strain detected by FACS.
FIG. 51 shows blocking of the binding of PVRIG protein to PVRL2 by four humanized bispecific antibodies detected by HTRF.
FIG. 52 shows blocking of the binding activity of PVRIG-ECD-mFc to CHO-K1-CD112 by four humanized bispecific antibodies detected by FACS.
FIG. 53 shows blocking of the binding activity of TIGIT-ECD-mFc to CHO-K1-CD155 by four humanized bispecific antibodies detected by ELISA.
FIG. 54 shows blocking of the binding activity of Bio-CD155-His protein to CHO-K1 human TIGIT by four humanized bispecific antibodies detected by FACS.
FIG. 55 shows the binding activity of four humanized bispecific antibodies to human PBMC detected by FACS.
FIG.56 shows the co-binding of humanized bispecific antibodies to PVRIG and TIGIT detected by BIAcore. The curves represent the antigen-antibody binding of LC-BsAb-002 humanized bispecific antibody (A) and LC-BsAb-006 humanized bispecific antibody (B) to human TIGIT protein, human PVRIG protein, as well as human TIGIT and human PVRIG protein injected separately and continuously. The black triangle represents the time point of the corresponding protein injection.
FIG. 57 shows the promoting effect of anti-PVRIGxTIGIT humanized bispecific antibodies on NK cell function detected by NK cell degranulation assay. The flow chart of NK cell degranulation assay (A); the expression level of PVRIG and TIGIT in NK cells, and the PVR and PVRL2 in WIDR cells detected by FACS (B); the effect of humanized bispecific antibodies LC-BsAb-002 and LC-BsAb-006 on the expression levels of CD107a on NK cells (EC50, AUC, C); the effect of humanized bispecific antibodies LC-BsAb-002 on the expression levels of CD107a on NK cells (target cells TF-1, D).
FIG. 58 shows the cytotoxicity of NK cells on WIDR cells mediated by the anti-PVRIGxTIGIT humanized bispecific antibodies detected by NK cell cytotoxicity assay. The expression levels of PVRIG and TIGIT on NK cells from different donors (Donor-050, 831, 715) (A); the expression levels of PVR and PVRL2 on target cells WIDR (B); the flow chart of NK cytotoxicity assay (C); the cytotoxicity of NK cells from three different donors on WIDR cells mediated by LC-BsAb -002 and LC-BsAb-006 (EC50, AUC, D), the cytotoxicity of NK cells on TF-1 cells mediated by LC-BsAb -002 (E).
FIG. 59 shows the direct cytotoxic effect of NK cells on human Treg cells mediated by the anti-PVRIGxTIGIT humanized bispecific antibodies detected by NK cell ADCC assay. The flow chart of ADCC cytotoxicity assay mediated by NK cells (A), the expression levels of PVRIG and TIGIT on human Treg cells (B), comparation of ADCC cytotoxicity of LC-BsAb-002 and LC-BsAb-006 with different IgG Fc on human Treg cells (EC50, AUC, C).
FIG. 60 shows the anti-PVRIGxTIGIT humanized bispecific antibodies mediated ADCP activity on human Treg cells

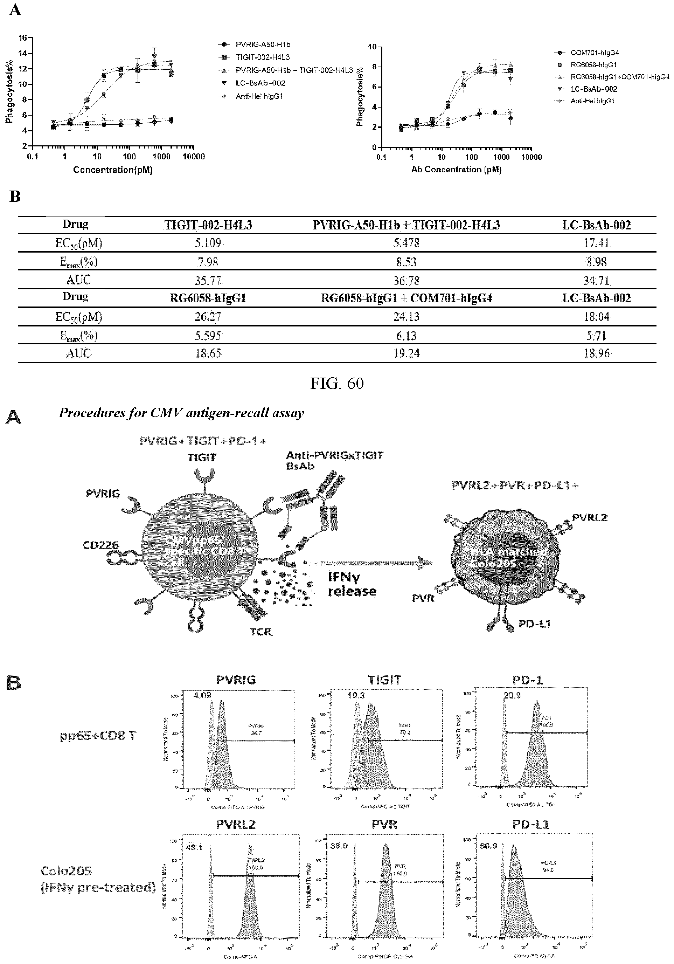

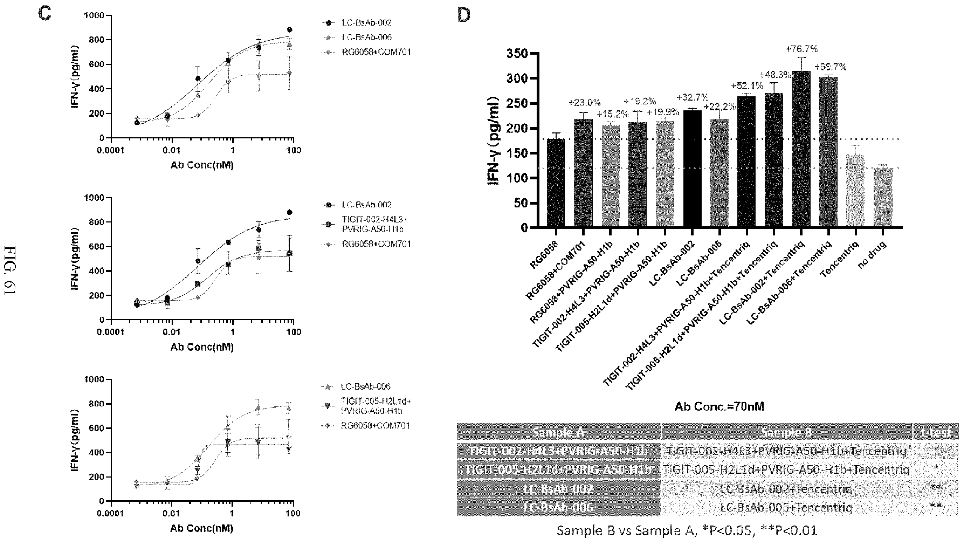

FIG. 61 shows the promoting effect of anti-PVRIGxTIGIT humanized bispecific antibodies on antigen-specific CD8 T cell function detected by CMV antigen-recall assay. The flow chart of CMV antigen-recall assay (A); the expression levels of PVRIG, TIGIT and PD-1 on pp65-specific CD8 T cells, and the expression levels of PVRL2, PVR and PD-L1 on Colo205 cell pretreated with IFN-γ (B); the secretion level of IFN-γ in the cell supernatant after 18 hours of co-incubation. In this experimental system, the controls are anti-TIGIT antibodies (RG6058, TIGIT-002-H4L3 and TIGIT-005-H2L1d) and the anti-PVRIG antibodies (COM701 and PVRIG-A50-H1b), and the negative control is no treatment (without any drug treatment) (C); the secretion level of IFN-γ in the cell supernatant after 18 hours of incubation with humanized bispecific antibodies, combination of corresponding monoclonal antibodies, or their combination with anti-PD-L1 antibody. In this experimental system, the controls are anti-TIGIT antibodies (RG6058, TIGIT-002-H4L3 and TIGIT-005-H2L1d), the anti-PVRIG antibodies (COM701 and PVRIG-A50-H1b), and anti-PD-L1 antibody (Tecentriq), and the negative control is no treatment (without any drug treatment) (D).

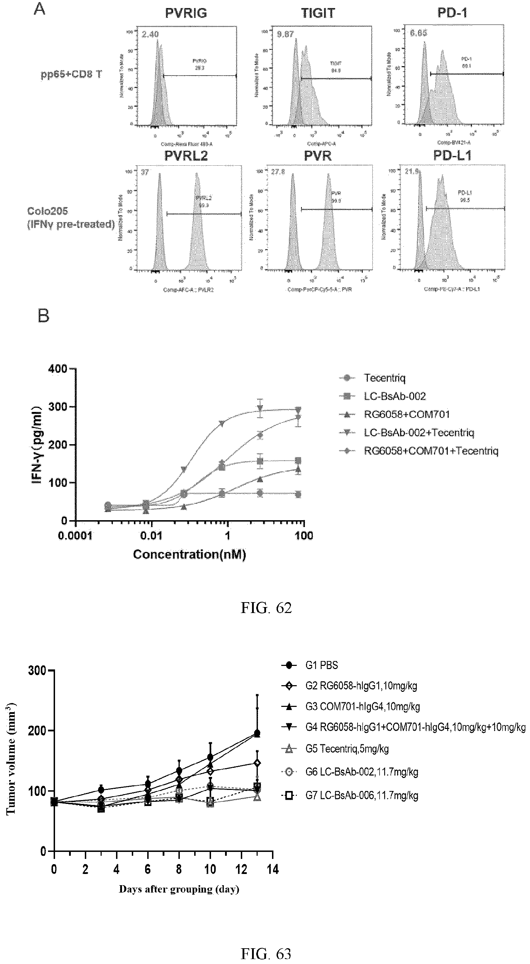

FIG. 62 shows the promoting effect of anti-PVRIGxTIGIT humanized bispecific antibodies combined with Tecentriq on antigen-specific CD8 T cell function detected by CMV antigen-recall assay. The expression levels of PVRIG, TIGIT and PD-1 on pp65-specific CD8 T cells, and the expression levels of PVRL2, PVR and PD-L1 on Colo205 cell pretreated with IFN-γ (A); the secretion level of IFN-γ in the cell supernatant after 18 hours of incubation with humanized bispecific antibodies, combination of corresponding monoclonal antibodies, or their combination with anti-PD-L1 antibody. In this experimental system, the controls are anti-TIGIT antibodies (RG6058), the anti-PVRIG antibodies (COM701), and anti-PD-L1 antibody (Tecentriq) (B)
FIG. 63 shows tumor growth curve of each test group in the A375 hPBMC humanized animal model.
FIG. 64 shows tumor growth curve of a single mouse in each test group in the A375 hPBMC humanized animal model.
FIG. 65 shows the weight changes of mice after administration in each test group in the A375 hPBMC humanized animal model.
FIG. 66 shows tumor growth curve of each test group in different doses of bispecific antibodies alone or combination with Tecentriq in the A375 hPBMC humanized model.
FIG. 67 shows tumor growth curve of single mouse in each test group in different doses of bispecific antibodies alone or combination with Tecentriq in the A375 hPBMC humanized model.
FIG. 68 shows the weight changes of mice in each test group in different doses of bispecific antibodies alone or combination with Tecentriq in the A375 hPBMC humanized model.

### DETAILED DESCRIPTION

The present disclosure will be further illustrated in conjunction with specific embodiments. The advantages and features of the present disclosure will become more apparent with the description. If specific conditions are not indicated in the examples, the conventional conditions or the conditions suggested by the manufacturer shall be followed. If the manufacturer is not indicated, the reagents or instruments used are conventional products that can be purchased commercially.

The following embodiments of this disclosure are merely exemplary and not intended to limit the scope of the present disclosure. It will be understood by those skilled in the art that various modifications or substitutions can be made in details and forms of the technical solution of the present disclosure without departing from or exceeding the spirit or scope of the disclosure, but all such modifications and substitutions are within the scope of the present disclosure.

### EXAMPLE 1 - Preparation of TIGIT antigen

Using human TIGIT protein (NCBI accession number: XP_024309156.1) and cynomolgus monkey TIGIT protein (NCBI: XP _ 015300911.1) as templates of TIGITs in this disclosure, the amino acid sequences of antigens and proteins for detection involved in this disclosure were designed. Optionally, different tags were fused on the basis of the TIGIT proteins. The amino acid sequences were cloned into PTT5 vector (Invitrogen) and then transiently expressed in 293 cells or stably expressed and purified in CHO cells to obtain antigens and proteins for detection involved in present disclosure.

Fusion protein of extracellular domain of human TIGIT and mouse IgG2a Fc fragment (human TIGIT ECD-mFc, for detection): 
SEQ ID NO: 1

MEFGLSWLFLVAILKGVQCMMTGTIETTGNISAEKGGSIILQCHLSSTTAQVTQVNW

EQQDQLLAICNADLGWHISPSFKDRVAPGPGLGLTLQSLTVNDTGEYFCIYHTYPDGTYT

GRIFLEVLESSVAEHGARFQIPEPRGPTIKPCPPCKCPAPNLLGGPSVFIFPPKIKDVLMISLS

PIVTCVVVDVSEDDPDVQISWFVNNVEVHTAQTQTHREDYNSTLRVVSALPIQHQDWMSGKE

FKCKVNNKDLPAPIERTISKPKGSVRAPQVYVLPPPEEEMTKKQVTLTCMVTDFMPEDIYVEW

TNNGKTELNYKNTEPVLDSDGSYFMYSKLRVEKKNWVERNSYSCSVVHEGLHNHHTTKSFSR

TPGK*
 Note: The underlined part is signaling peptide, and the italicized part is mFc.

Fusion protein of extracellular domain of human TIGIT and human IgG1 Fc fragment (human TIGIT ECD-hFc, for detection): SEQ ID NO: 2 
MEFGLSWLFLVAILKGVQCMMTGTIETTGNISAEKGGSIILQCHLSSTTAQVTQVNW
EQQDQLLAICNADLGWHISPSFKDRVAPGPGLGLTLQSLTVNDTGEYFCIYHTYPDGTYT
GRIFLEVLESSVAEHGARFQIPEPKSSDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGFYPSDIAVEWE
SNGQPENNYKTTPPVLDSDGSFFLYSKLTVDKSRWQQGNVFSCSVMHEALHNHYTQKSLSLS
PGK*
 Note: The underlined part is signaling peptide, and the italicized part is Fc.

Fusion protein of extracellular domain of human TIGIT and His-tag (human TIGIT ECD-his, for detection): 
SEQ ID NO: 3

MEFGLSWLFLVAILKGVQCMMTGTIETTGNISAEKGGSIILQCHLSSTTAQVTQVNW

EQQDQLLAICNADLGWHISPSFKDRVAPGPGLGLTLQSLTVNDTGEYFCIYHTYPDGTYT

GRIFLEVLESSVAEHGARFQIP HHHHHH*
 Note: The underlined part is signaling peptide, and the italicized part is His-tag.

Fusion protein of extracellular domain of cynomolgus monkey TIGIT and mouse IgG2a Fc fragment (cynomolgus monkey TIGIT ECD-mFc, for detection): 
SEQ ID NO: 4

MEFGLSWLFLVAILKGVQCMMTGTIETTGNISAKKGGSVILQCHLSSTMAQVTQVN

WEQHDHSLLAIRNAELGWHIYPAFKDRVAPGPGLGLTLQSLTMNDTGEYFCTYHTYPDG

TYRGRIFLEVLESSVAEHSARFQIPEPRGPTIKPCPPCKCPAPNLLGGPSVFIFPPKIKDVLMI

SLSPIVTCVVVDVSEDDPDVQISWFVNNVEVHTAQTQTHREDYNSTLRVVSALPIQHQDWMSG

KEFKCKVNNKDLPAPIERTISKPKGSVRAPQVYVLPPPEEEMTKKQVTLTCMVTDFMPEDIYV

EWTNNGKTELNYKNTEPVLDSDGSYFMYSKLRVEKKNWVERNSYSCSVVHEGLHNHHTTKSF

SRTPGK*
 Note: The underlined part is signaling peptide, and the italicized part is mFc.

Fusion protein of extracellular domain of cynomolgus monkey TIGIT and human IgG1 Fc fragment (cynomolgus monkey TIGIT ECD-hFc, for detection): 
SEQ ID NO: 5

MEFGLSWLFLVAILKGVQCMMTGTIETTGNISAKKGGSVILQCHLSSTMAQVTQVN

WEQHDHSLLAIRNAELGWHIYPAFKDRVAPGPGLGLTLQSLTMNDTGEYFCTYHTYPDG

TYRGRIFLEVLESSVAEHSARFQIPEPKSSDKTHTCPPCPAPELLGGPSVFLFPPKPKDTLMI

SRTPEVTCVVVDVSHEDPEVKFNWYVDGVEVHNAKTKPREEQYNSTYRVVSVLTVLHQDWLN

GKEYKCKVSNKALPAPIEKTISKAKGQPREPQVYTLPPSREEMTKNQVSLTCLVKGFYPSDIAV

EWESNGQPENNYKTTPPVLDSDGSFFLYSKLTVDKSRWQQGNVFSCSVMHEALHNHYTQKSL

SLSPGK*
 Note: The underlined part is signaling peptide, and the italicized part is hFc.

### EXAMPLE 2 - Construction of CHO-K1 engineered cell strain

The nucleotide sequences encoding the full-length amino acid sequence of human TIGIT (NCBI: XP_024309156.1), cynomolgus monkey TIGIT (NCBI: XP_015300911.1), human CD155 (NCBI: NP_006496.4) and human CD112 (NCBI: NP_001036189.1) were cloned into pcDNA3.1 vector (purchased from Clontech) and then the plasmid was prepared. CHO-K1 cell line (purchased from ATCC) was transfected with plasmid (Lipofectamine^{®} 3000 Transfection Kit, purchased from Invitrogen, Cat NO.L3000-015), selectively cultured in DMEM/F12 medium containing 10% (w/w) fetal bovine serum and 10 µg/ml puromycin for 2 weeks. The monoclonal cells were then plated into a 96-well plate and cultured at 37°C with 5% (v/v) CO₂. After about 2 weeks, some monoclonal cells were selected and expanded. The expanded clones were screened by flow cytometry. The monoclonal cell lines with better growth and higher fluorescence intensity were selected for further expansion and then frozen in liquid nitrogen.

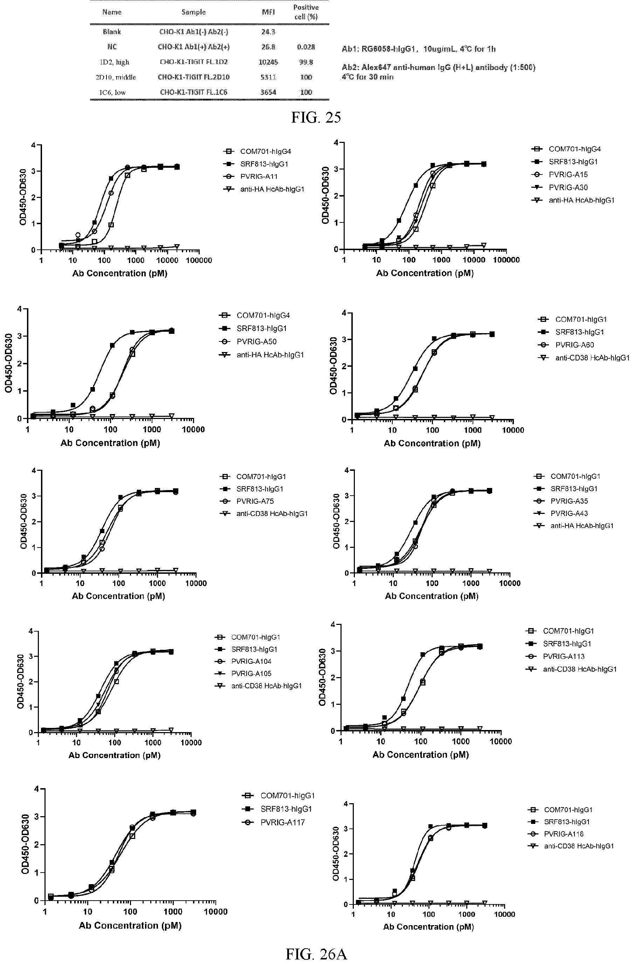

Full-length human TIGIT (NCBI: XP_024309156.1): used to construct the human TIGIT overexpressing cell strain (CHO-K1 human TIGIT). The high expression strain 1D2, medium expression strain 2D10 and low expression strain 1C6 were selected by FACS (Fig. 25). 
SEQ ID NO: 6
MRWCLLLIWAQGLRQAPLASGMMTGTIETTGNISAEKGGSIILQCHLSSTTAQVTQVNWEQQDQLLAI
CNADLGWHISPSFKDRVAPGPGLGLTLQSLTVNDTGEYFCIYHTYPDGTYTGRIFLEVLESSVAEHGAR
FQIPLLGAMAATLVVICTAVIVVVALTRKKKALRIHSVEGDLRRKSAGQEEWSPSAPSPPGSCVQAEAAPAG
LCGEQRGEDCAELHDYFNVLSYRSLGNCSFFTETG*
 Note: Signaling peptide (single underline) + extracellular region + transmembrane region (double underline) + intracellular region (italic part)

Full-length cynomolgus monkey TIGIT (NCBI: XP_015300911.1): used to construct the cynomolgus monkey TIGIT overexpressing cell strain (CHO-K1 cynomolgus monkey TIGIT). 
SEQ ID NO: 7
MRWCLLLIWAQGLRQAPLASGMMTGTIETTGNISAEKGGSIILQCHLSSTTAQVTQVNWEQQDQLLAI
CNADLGWHISPSFKDRVAPGPGLGLTLQSLTVNDTGEYFCIYHTYPDGTYTGRIFLEVLESSVAEHGAR
FQIPLLGAMAATLVVICTAVIVVVALTRKKKALRIHSVEGDLRRKSAGQEEWSPSAPSPPGSCVQAEAAPAG
LCGEQRGEDCAELHDYFNVLSYRSLGNCSFFTETG*
 Note: Signaling peptide (single underline) + extracellular region + transmembrane region (double underline) + intracellular region (italic part)

Full-length human CD155 (NCBI: NP_006496.4): used to construct the human CD155 overexpressing cell strain (CHO-K1 CD155). 
SEQ ID NO: 8
MARAMAAAWPLLLVALLVLSWPPPGTGDVVVQAPTQVPGFLGDSVTLPCYLQVPNMEVTHVSQLTW
ARHGESGSMAVFHQTQGPSYSESKRLEFVAARLGAELRNASLRMFGLRVEDEGNYTCLFVTFPQGSRS
VDIWLRVLAKPQNTAEVQKVQLTGEPVPMARCVSTGGRPPAQITWHSDLGGMPNTSQVPGFLSGTVT
VTSLWILVPSSQVDGKNVTCKVEHESFEKPQLLTVNLTVYYPPEVSISGYDNNWYLGQNEATLTCDAR
SNPEPTGYNWSTTMGPLPPFAVAQGAQLLIRPVDKPINTTLICNVTNALGARQAELTVQVKEGPPSEHS
GISRNAIIFLVLGILVFLILLGIGIYFYWSKCSREVLWHCHLCPSSTEHASASANGHVSYSAVSRENSSSQDPQ
TEGTR*
 Note: Signaling peptide (single underline) + extracellular region + transmembrane region (double underline) + intracellular region (italic part)

Full-length human CD112 (NCBI: NP_001036189.1/NCBI Ref Seq: Q92692): used to construct the human CD112 overexpressing cell strain (CHO-K1 CD112). 
SEQ ID NO: 9
MARAAALLPSRSPPTPLLWPLLLLLLLETGAQDVRVQVLPEVRGQLGGTVELPCHLLPPVPGLYISLVT
WQRPDAPANHQNVAAFHPKMGPSFPSPKPGSERLSFVSAKQSTGQDTEAELQDATLALHGLTVEDEG
NYTCEFATFPKGSVRGMTWLRVIAKPKNQAEAQKVTFSQDPTTVALCISKEGRPPARISWLSSLDWEA
KETQVSGTLAGTVTVTSRFTLVPSGRADGVTVTCKVEHESFEEPALIPVTLSVRYPPEVSISGYDDNWY
LGRTDATLSCDVRSNPEPTGYDWSTTSGTFPTSAVAQGSQLVIHAVDSLFNTTFVCTVTNAVGMGRAE
QVIFVRETPNTAGAGATGGIIGGIIAAIIATAVAATGILICRQQRKEQTLQGAEEDEDLEGPPSYKPPTPKAK
LEAQEMPSQLFTLGASEHSPLKTPYFDAGASCTEQEMPRYHELPTLEERSGPLHPGATSLGSPIPVPPGPPAV
EDVSLDLEDEEGEEEEEYLDKINPIYDALSYSSPSDSYQGKGFVMSRAMYV*
 Note: Signaling peptide (single underline) + extracellular region + transmembrane region (double underline) + intracellular region (italic part)

### EXAMPLE 3 - Production of anti-human TIGIT mouse monoclonal antibodies

The anti-human TIGIT antibody was obtained by hybridoma technology. The mice were immunized with human TIGIT-ECD-mFc fusion protein. The spleen cells of mice were isolated, fused with mouse myeloma cells by electrofusion and cultured in HAT selective culture medium. After identifying the culture supernatant, clones secreting the target antibodies were selected and subcloned. The mouse monoclonal antibodies were eventually obtained by production and purification. The experiment is described in detail as follows:

### A. Animal immunization

SJL white mice, female, 6-8 weeks old, specific-pathogen-free (SPF) grade, (Shanghai SLAC Laboratory Animal Co., Ltd., animal production license number: SCXK (Shanghai) 2017-0005). After purchase, the mice were kept in a laboratory animal room (Obio Technology (Shanghai) Corp., Ltd.) and adapted to the environment for one week, with a 12/12 hours light/dark cycle, an ambient temperature of approximately 20-25°C and humidity of approximately 40-60%. The mice adapted to the environment were immunized according to the following protocol.

Immune antigen: human TIGIT-ECD with mFc tag (Aero Cat No.TIT-H5253). Immunization protocol: cross-immunization with TiterMax^{®} Gold Adjuvant (Sigma Cat No.T2684) and Thermo Imject^{®} Alum (Thermo Cat No.77161) adjuvant. The ratio of antigen to adjuvant (TiterMax^{®} Gold Adjuvant) is 1:1, and the ratio of antigen to adjuvant (Thermo Imject^{®} Alum) is 2:1, 50 µg/mouse/dose (first immunization) and 25 µg/mouse/dose (booster immunization). The antigen was emulsified and inoculated at day 1, 8, 15, 22, 29, 36, 43 and 50. On day 1, 50 µg/mouse emulsified antigen was injected intraperitoneally (I.P.). On day 8, 25 µg/mouse antigen was injected subcutaneously at multiple points (S.C., generally 4 points in the back and groin). Intraperitoneal injection and subcutaneous injection were carried out every other week. Blood samples were collected on days 20, 27, 34 and 48, and the antibody titer in mouse serum was determined by ELISA. After 6-8 times of immunization, the spleen and lymph node cells with a high antibody titer in serum were selected for fusion. Three days before fusion, the mice were injected intraperitoneally with 50 µg/mouse antigen solution prepared with saline for a booster immunization.

### B. B Cell fusion

Spleen and lymph node cells were fused with SP2/0 myeloma cells (ATCC^{®} CRL-1581) by optimized electrofusion (BTX ECM2001+). The fused hybridoma cells were resuspended in the complete culture medium, DMEM (Gibco Cat No.10569044) containing 20% FBS (Excell, Cat No. FND500), 1×HAT (Sigma Cat No.H0262-10VL) and 1×NEAA, at a density of 5×10⁵ /mL. The fused hybridoma cells were inoculated in a 96-well flat bottom plate (100 µL/well) and cultured at 37°C with 5 % CO₂ for 6-7 days. The supernatant was then discarded. Each well was added with 20 µL complete culture medium containing HT supplement (DMEM containing 10% FBS, 1×HT (Sigma Cat No.H0137-10VL) and 1×NEAA), and cultured at 37°C with 5 % CO₂ overnight for ELISA.

### C. Screening of hybridoma cells

After 7-10 days of fusion, the supernatant of hybridoma cells was collected to detect the binding activity to human TIGIT ECD-hFc (In-house production) by ELISA, and the positive clones were selected. On the second day, the binding activity of the positive clone culture supernatant to CHO-K1 human TIGIT (In-house construction) and CHO-K1 cynomolgus monkey TIGIT (In-house construction), and the effect of the positive clone culture supernatant blocking the interaction between human TIGIT ECD-hFc and CHO-K1 CD155 (In-house construction) were detected, and the target clones were selected for subcloning.

The subclones were cultured for 7-10 days and screened by ELISA to obtain the target hybridoma monoclonal cells, which were then expanded to 24 wells. After 2-3 days, the binding activity of culture supernatant to human TIGIT ECD-hFc, CHO-K1 human TIGIT (In-house construction), CHO-K1 cynomolgus monkey TIGIT (In-house construction) and cynomolgus monkey TIGIT ECD-hFc (internal production), and the effect of culture supernatant blocking the interaction between Bio-CD155-His (Sino Biological Inc., 10109-H08H) and CHO-K1 human TIGIT (In-house construction), and blocking the interaction between human TIGIT ECD-hFc and CHO-K1 CD155 (In-house construction) were detected. 116 monoclonal antibodies were produced and purified from the target clones.

### D. Identification of mouse monoclonal antibodies

The 116 monoclonal antibodies obtained above were identified by ELISA, FACS and BIAcore, and 13 antibody candidates were obtained, including: TIGIT-F2-002, TIGIT-F2-005, TIGIT-F2-006, TIGIT-F2-011, TIGIT-F3-034, TIGIT-F4-044, TIGIT-F5-057, TIGIT-F5-067, TIGIT-F5-070, TIGIT-F5-072, TIGIT-F6-084, TIGIT-F6-088 and TIGIT-F6-104.
(a) Detection of the binding activity of anti-TIGIT mouse monoclonal antibodies to human TIGIT ECD-hFc and cynomolgus monkey TIGIT ECD-hFc.

The sheep anti-human IgG antibody (Jackson, CAT: 109-006-098) was diluted to 4 µg/mL with PBS (Hyclone, CAT#SH30256, pH7.4), added (50 µL/well) into an enzyme-labeled plate (corning, CAT#9018) and kept at 4°C overnight. After discarding the coating solution, the plate was added with 5% skim milk powder (Sangon Biotech, CAT#A600669-0250)-PBS, 250 µL/well, and incubated at 37°C for 2-4 hours. After washing three times with 0.05% Tween 20-PBS (Sangon Biotech, CAT#A100777-0500, B548117-0500) on the Microplate Washer (Biotek, CAT#405TUS), the plate was added with human TIGIT ECD-hFc (In-house construction, working concentration: 30ng/mL) and cynomolgus monkey TIGIT-hFc (In-house construction, working concentration: 30ng/mL), 50 µL/well, and incubated at 4°C overnight. After washing three times with 0.05% Tween 20-PBS on the Microplate Washer, the plate was added with purified mouse monoclonal antibodies (diluted to 13 nM with 1% BSA, diluted by 3-fold serial dilution for 12 concentration points), 50 µL/well, incubated at 37°C for 1.5-2 hours. The enzyme-labeled plate was then washed with 0.05% Tween 20-PBS for 3 times on the Microplate Washer, added with 50 µL/well HRP enzyme-labeled antibodies (Jackson, CAT#115-035-003) diluted with 1% BSA (Sangon Biotech, CAT#A500023-0100)-PBS at a dilution ratio of 1:5000 and incubated at 37°C for 1 hour. After washing 3 times with 0.05% Tween 20-PBS on the Microplate Washer, the plate was added with TMB chromogenic solution (KPL, CAT#52-00-03), 50 µL/well, and incubated at room temperature for 7-10 minutes. 1M HCL, 50 µL/well, was then added into the plate to terminate the reaction, and the OD450 nm was read by a microplate reader (Biotek, Powerwave HT). The experimental results show that the anti-TIGIT mouse monoclonal antibodies can effectively bind to human TIGIT ECD-hFc and cynomolgus monkey TIGIT ECD-hFc, compared with the control antibody, anti-TIGIT antibody (Roche RG6058).

The corresponding amino acid sequence of RG6058 is shown below:

### RG6058 VH SEQ ID NO 234:

EVQLQQSGPGLVKPSQTLSLTCAISGDSVSSNSAAWNWIRQSPSRGLEWLGKTYYR
FKWYSDYAVSVKGRITINPDTSKNQFSLQLNSVTPEDTAVFYCTRESTTYDLLAGPFDYW
GQGTLVTVSS

### RG6058 VL SEQ ID NO 235:

DIVMTQSPDSLAVSLGERATINCKSSQTVLYSSNNKKYLAWYQQKPGQPPNLLIYW
ASTRESGVPDRFSGSGSGTDFTLTISSLQAEDVAVYYCQQYYSTPFTFGPGTKVEIK

(b) The binding activity of anti-TIGIT mouse monoclonal antibodies to CHO-K1 human TIGIT and CHO-K1 cynomolgus monkey TIGIT detected by ELISA.

The collected cells were adjusted to a concentration of 5×10⁵/mL with 10% FBS-DMEM/F12 medium (Excell, CAT#FSP500; Gibco, CAT#11330), seeded into a 96-well cell culture plate (corning, CAT#3599), 100 µL/well, and cultured overnight at 37 °C with 5% CO₂. After discarding the culture supernatant, the cells were fixed with a cell fixation solution (Beyotime, CAT#P0098), 50 µL/well, at room temperature for 1 hour. After washing once with 0.05% Tween 20-PBS on the Microplate Washer, the plate was added with 5% skim milk powder-PBS, 250 µL/well, and incubated at 37°C for 2-4 hours. After washing 3 times with 0.05% Tween 20-PBS on the Microplate Washer, the plate was added with purified mouse monoclonal antibodies (diluted to 13 nM with 1% BSA, diluted by 3 fold serial dilution for 12 concentration points), 50 µL/well, incubated at 37°C for 1.5-2 hours. The cell plate was then washed with 0.05% Tween 20-PBS for 3 times on the Microplate Washer, then added with 50 µL/well HRP enzyme-labeled antibodies (Jackson, CAT#115-035-003) diluted with 1% BSA (Sangon Biotech, CAT#A500023-0100)-PBS at a dilution ratio of 1:5000 and incubated at 37°C for 1 hour. After washing 3 times with 0.05% Tween 20-PBS on the Microplate Washer, the plate was added with TMB chromogenic solution (KPL, CAT#52-00-03), 50 µL/well, and incubated at 37°C for 10 minutes. 1M HCL, 50 µL/well, was then added into the plate to terminate the reaction, and the OD450nm was read by a microplate reader (Biotek, Powerwave HT). The experimental results show that the purified anti-TIGIT mouse monoclonal antibodies can effectively bind to CHO-K1 human TIGIT high expression cell strains, CHO-K1 human TIGIT medium expression cell strains, CHO-K1 human TIGIT low expression cell strains and CHO-K1 cynomolgus monkey TIGIT cell strains, compared with the control antibody, anti-TIGIT antibody (Roche RG6058).

(c) The effect of anti-TIGIT mouse monoclonal antibodies blocking the interaction between Bio-CD155-His and CHO-K1 human TIGIT detected by FACS

The collected cells were washed once with PBS (Hyclone, CAT#SH30256), resuspended to 2×10⁵/40 µL with 1% BSA-PBS. The antibodies were diluted to 210 nM with 1% BSA-PBS (diluted by 3-fold serial dilution for 12 concentration points). The Bio-CD155-His (Sino Biological Inc., 10109-H08H) was diluted to 3 µg/mL with 1% BSA-PBS. The 40 µL cells were then mixed with 40 µL diluted antibodies and 40 µL, diluted Bio-CD155-His, and then incubated at 4°C for 60 minutes. The cells were washed twice with PBS, resuspended with APC labeled streptavidin (dilution ratio 1:1700, Biolegend, 405243), 100 µL/well, and incubated at 4°C for 40 minutes. The cell samples were washed twice with PBS, resuspended with 1% BSA-PBS, 100 µL/well and then analyzed by flow cytometry (BD, Canto II). The experimental results show that both the purified mouse monoclonal antibodies and the control antibody (RG6058) can effectively block the binding between the Bio-CD155-His protein and CHO-K1- human TIGIT cells.

(d) The effect of anti-TIGIT mouse monoclonal antibodies blocking the interaction between human TIGIT and CHO-K1 CD155 detected by ELISA

The collected CHO-K1 CD155 cells were adjusted to a concentration of 5×10⁵/mL with 10% FBS-DMEM/F12 medium (Excell, CAT#FSP500; Gibco, CAT#11330), seeded into a 96-well cell culture plate (corning, CAT#3599), 100 µL/well, and cultured overnight at 37 °C with 5% CO₂. After discarding the culture supernatant, the cells were fixed with a cell fixation solution (Beyotime, CAT#P0098), 50 µL/well, at room temperature for 1 hour. After washing once with 0.05% Tween 20-PBS on the Microplate Washer, the plate was added with 5% skim milk powder-PBS, 250 µL/well, and incubated at 37°C for 2-4 hours. The plate was then washed 3 times with 0.05% Tween 20-PBS on the Microplate Washer. The mouse monoclonal antibodies were mixed with human TIGIT ECD-hFc (working concentration: 30 ng/mL) and incubated for 0.5 hour. The mixed solution of antigen and antibody was added to the cell plate (50 µL/well). The cells were incubated at 37°C for 1.5-2 hours and then washed with 0.05% Tween 20-PBS for 3 times on the Microplate Washer. The cell plate was then added with 50 µL/well HRP enzyme-labeled antibodies (Merck, CAT#AP113P) diluted with 1% BSA (Sangon Biotech, CAT#A500023-0100)-PBS at a dilution ratio of 1:5000 and incubated at 37°C for 1 hour. After washing 3 times with 0.05% Tween 20-PBS on the Microplate Washer, the plate was added with TMB chromogenic solution (KPL, CAT#52-00-03), 50 µL/well, and incubated at 37°C for 10 minutes. 1M HCL, 50 µL/well, was then added into the plate to terminate the reaction, and the OD450 nm was read by a microplate reader (Biotek, Powerwave HT). The experimental results show that both purified mouse monoclonal antibodies and control antibody RG6058 can effectively block the binding between the human TIGIT protein and CHO-K1-CD155 cells.

The following table 1 shows that antibodies of all the 13 cell strains have shown a good ability of binding to TIGIT and blocking the interaction between TIGIT and CD155, which reaches or exceeds the control anti-TIGIT antibody, Roche RG6058.

**Table 1. Identification of TIGIT mouse monoclonal antibodies**

| TIGIT monoclonal antibodies | Human TIGIT ECD-His | | | Human TIGIT ECD-hFc /CHO-K 1 CD155 blocking experime nt | Bio-CD1 55-His/C HO-K1 human TIGIT blocking experime nt | CHO-K1 human TI GIT (high expressio n strain) | CHO-K 1 human TIGIT (mediu m expressio n strain) | CHO-K1 human TIGIT (low expressio n strain) | Human TIGIT ECD-hFc binding experime nt | CHO-K1 cynomol gus monkey TIGIT binding experime nt | cynomol gus monkey TIGIT ECD-hF c binding experime nt |
|---|---|---|---|---|---|---|---|---|---|---|---|
| | ka (1/Ms) | kd (1/s) | KD (M) | IC50 (nM) | IC50 (nM) | EC50 (nM) | EC50 (nM) | EC50 (nM) | EC50 (nM) | EC50 (nM) | EC50 (nM) |
| TIGIT-F2-00 2 | 1.70E+0 6 | 8.08E-05 | 4.76E-11 | 0.1411 | 0.2038 | 0.3124 | 0.3162 | 0.2733 | 0.0171 | 0.1095 | 0.0170 |
| TIGIT-F2-00 5 | 1.34E+0 6 | 4.47E-05 | 3.33E-11 | 0.1328 | 0.1738 | 0.3883 | 0.3860 | 0.2310 | 0.0150 | 0.0778 | 0.0147 |
| TIGIT-F2-00 6 | 8.00E+0 5 | 3.32E-05 | 4.14E-11 | 0.1497 | 0.2150 | 0.4796 | 0.5396 | 0.3202 | 0.0208 | 0.0616 | 0.0327 |
| TIGIT-F2-01 1 | 6.29E+0 5 | 2.44E-05 | 3.88E-11 | 0.1685 | 0.3111 | 0.6472 | 0.7398 | 0.4434 | 0.0215 | 0.1252 | 0.0381 |
| TIGIT-F3-03 4 | 1.98E+0 6 | 3.66E-04 | 1.85E-10 | 0.1610 | 0.1790 | 0.4876 | 0.4113 | 0.1788 | 0.0189 | 0.0620 | 0.0252 |
| TIGIT-F4-04 4 | 1.17E+0 6 | 1.17E-03 | 9.97E-10 | 0.1582 | 0.2126 | 0.5412 | 0.4834 | 0.2793 | 0.0180 | 0.0913 | 0.0771 |
| TIGIT-F5-05 7 | 1.13E+0 6 | 1.04E-03 | 9.21E-10 | 0.1281 | 0.2369 | 0.5338 | 0.4975 | 0.2937 | 0.0128 | 0.0698 | 0.0375 |
| TIGIT-F5-06 7 | 1.41E+0 6 | 1.23E-04 | 8.74E-11 | 0.1518 | 0.2252 | 0.4189 | 0.3866 | 0.2318 | 0.0130 | 0.2971 | 0.0836 |
| TIGIT-F5-07 0 | 7.SSE+0 5 | 1.17E-04 | 1.55E-10 | 0.1363 | 0.3395 | 0.6127 | 0.6636 | 0.3750 | 0.0039 | 0.0274 | 0.0043 |
| TIGIT-F5-07 2 | 1.33E+0 6 | 6.54E-04 | 4.92E-10 | 0.1255 | 0.2034 | 0.3662 | 0.3471 | 0.1944 | 0.0050 | 0.0330 | 0.0134 |
| TIGIT-F6-08 4 | 7.37E+0 5 | 9.06E-05 | 1.23E-10 | 0.1343 | 0.3130 | 0.6227 | 0.6107 | 0.4540 | 0.0059 | 0.0328 | 0.0057 |
| TIGIT-F6-08 8 | 5.40E+0 5 | 1.53E-04 | 2.83E-10 | 0.2197 | 0.4289 | 0.9256 | 0.6327 | 0.5585 | 0.0188 | 0.0927 | 0.0183 |
| TIGIT-F6-10 4 | 7.80E+0 5 | 1.01E-04 | 1.29E-10 | 0.1860 | 0.2943 | 0.9268 | 0.7281 | 0.4038 | 0.0204 | 0.0909 | 0.0170 |
| RG6058-mIg G2a | 6.69E+0 5 | 1.41E-04 | 2.11E-10 | 0.2116 | 0.4493 | 1.0500 | 0.9838 | 0.6718 | 0.0108 | 0.0791 | 0.0152 |

### EXAMPLE 4 - Identification of anti-human TIGIT chimeric antibodies

Four chimeric antibodies, TIGIT-CHI-002, TIGIT-CHI-005, TIGIT-CHI-006 and TIGIT-CHI-070, were identified by constructing human-mouse chimeric antibodies with above mouse antibodies. The VH/VL sequences of chimeric antibodies are shown in table 2. The results of KABAT analysis of chimeric antibodies are shown in table 3, and the results of IMGT analysis of chimeric antibodies are shown in table 4.

**Table 2. The VH/VL sequences of chimeric antibodies**

| VH | | |
|---|---|---|
| Antibody No. | Sequence No. | Sequences |
| TIGIT-CHI-002 | SEQ ID NO:10 | EVQLQESGPGLAKPSQTLSLACSVTGYSITSDSWNWIRKFPGNK LEYMGYISYSGNTYYNPSLKSRISITRDTSKNQFYLQLNSVTTED TATYYCARLDFSNYGGAVDYWGQGTSVTVSS |
| TIGIT-CHI-005 | SEQ ID NO:11 | QVQLQQSGAELVRPGTSVKVSCKASGYAFTNYLIEWVKQRPGQ GLEWIGVINPGSGGTNYKEKFKGKATLTADKSSSTAYMQLSSLTS EDSAVYFCARGEYFFFDYWGQGTTLTVSS |
| TIGIT-CHI-006 | SEQ ID NO:12 | EVQLQESGPGLAKPSQTPSLTCSVTGYSMTSDYWNWIRKFPGNK LEYMGYISYSGRTYYNPSLKSRISITRDTSKNQFYLQLNSVTTED TATYYCARGDYSNYGGAMYDWGQGTSVTVSS |
| TIGIT-CHI-070 | SEQ ID NO:13 | QVQLQQPGTELVKPGASVKLSCRASGYTFTNYYMHWVKQRPG RGLEWIGRIDPDSGGSKYNEKFKSKATLTVDKLSSTAYMQLSSLT SEDSAVYYCAREGHYGFYSDYWGQGTTLTVSS |

| VL | | |
|---|---|---|
| TIGIT-CHI-002 | SEQ ID NO:14 | DIVMTQSHTFMSTSVGDRVSITCKASQNVRTAVAWYQQKPGQSP KLMIYSASYRYTGVPDRFTGSGSGTDFTFTISSVQAEDLAVYYCQ QYYTTPWTFGGGAKLEIQ |
| TIGIT-CHI-005 | SEQ ID NO:15 | DIVMTQSHKFMSTSVGDRVSITCKASQHVSNGVAWYQHKPGQS PKLLIYSASYRYTGVPDRFTGTGSGTDFTFTISSVQAEDLAVYYC QQHYNTPHTFGGGTKLEIK |
| TIGIT-CHI-006 | SEQ ID NO:16 | DIVMTQSHKFMSTSVGDRVSITCRASQGVSTTIAWYQQKPGQSP |
| | | KLLIYSASYRYTGVPDRFTGSGSGTDFTFTISGVQAEDLAVYYCQ QYYSSPFTFGGGTKLEIK |
| TIGIT-CHI-070 | SEQ ID NO:17 | DIQMTQSPASLSASVGETVTITCRVSENIYSYLAWYQQKQGKSPQ LLVYNAKTLAEGVPSRFSGSGSGTQFSLKINSLQPEDFGNYYCQ HHYGNPLTFGAGTKLDLK |

**Table 3. The results of KABAT analysis of chimeric antibodies**

| Antibody No. | Light chain CDR | Sequences | Heavy chain CDR | Sequences |
|---|---|---|---|---|
| TIGIT-CHI-002 | LCDR1 | KASQNVRTAVA | HCDR1 | SDSWN |
| | | SEQ ID NO:18 | | SEQ ID NO:21 |
| | LCDR2 | SASYRYT | HCDR2 | YISYSGNTYYNPSLKS |
| | | SEQ ID NO:19 | | SEQ ID NO:22 |
| | LCDR3 | QQYYTTPWT | HCDR3 | LDFSNYGGAVDY |
| | | SEQ ID NO:20 | | SEQ ID NO:23 |
| TIGIT-CHI-006 | LCDR1 | RASQGVSTTIA | HCDR1 | SDYWN |
| | | SEQ ID NO:24 | | SEQ ID NO:27 |
| | LCDR2 | SASYRYT | HCDR2 | YISYSGRTYYNPSLKS |
| | | SEQ ID NO:25 | | SEQ ID NO:28 |
| | LCDR3 | QQYYSSPFT | HCDR3 | GDYSNYGGAMYD |
| | | SEQ ID NO:26 | | SEQ ID NO:29 |
| TIGIT-CHI-005 | LCDR1 | KASQHVSNGVA | HCDR1 | NYLIE |
| | | SEQ ID NO:30 | | SEQ ID NO:33 |
| | LCDR2 | SASYRYT | HCDR2 | VINPGSGGTNYKEKFKG |
| | | SEQ ID NO:31 | | SEQ ID NO:34 |
| | LCDR3 | QQHYNTPHT | HCDR3 | GEYFFFDY |
| | | SEQ ID NO:32 | | SEQ ID NO:35 |
| TIGIT-CHI-070 | LCDR1 | RVSENIYSYLA | HCDR1 | NYYMH |
| | | SEQ ID NO:36 | | SEQ ID NO:39 |
| | LCDR2 | NAKTLAE | HCDR2 | RIDPDSGGSKYNEKFKS |
| | | SEQ ID NO:37 | | SEQ ID NO:40 |
| | LCDR3 | QHHYGNPLT | HCDR3 | EGHYGFYSDY |
| | | SEQ ID NO:38 | | SEQ ID NO:41 |

**Table 4. The results of IMGT analysis of chimeric antibodies**

| Antibody No. | Light chain CDR | Sequences | Heavy chain CDR | Sequences |
|---|---|---|---|---|
| TIGIT-CHI-002 | LCDR1 | QNVRTA | HCDR1 | GYSITSDS |
| | | SEQ ID NO:42 | | SEQ ID NO:45 |
| | LCDR2 | SAS | HCDR2 | ISYSGNT |
| | | SEQ ID NO:43 | | SEQ ID NO:46 |
| | LCDR3 | QQYYTTPWT | HCDR3 | ARLDFSNYGGAVDY |
| | | SEQ ID NO:44 | | SEQ ID NO:47 |
| TIGIT-CHI-006 | LCDR1 | QGVSTT | HCDR1 | GYSMTSDY |
| | | SEQ ID NO:48 | | SEQ ID NO:51 |
| | LCDR2 | SAS | HCDR2 | ISYSGRT |
| | | SEQ ID NO:49 | | SEQ ID NO:52 |
| | LCDR3 | QQYYSSPFT | HCDR3 | ARGDYSNYGGAMYD |
| | | SEQ ID NO:50 | | SEQ ID NO:53 |
| TIGIT-CHI-005 | LCDR1 | QHVSNG | HCDR1 | GYAFTNYL |
| | | SEQ ID NO:54 | | SEQ ID NO:57 |
| | LCDR2 | SAS | HCDR2 | INPGSGGT |
| | | SEQ ID NO:55 | | SEQ ID NO:58 |
| | LCDR3 | QQHYNTPHT | HCDR3 | ARGEYFFFDY |
| | | SEQ ID NO:56 | | SEQ ID NO:59 |
| TIGIT-CHI-070 | LCDR1 | ENIYSY | HCDR1 | GYTFTNYY |
| | | SEQ ID NO:60 | | SEQ ID NO:63 |
| | LCDR2 | NAK | HCDR2 | IDPDSGGS |
| | | SEQ ID NO:61 | | SEQ ID NO:64 |
| | LCDR3 | QHHYGNPLT | HCDR3 | AREGHYGFYSDY |
| | | SEQ ID NO:62 | | SEQ ID NO:65 |

### (a) Detection of binding activity of anti-TIGIT chimeric antibodies to human TIGIT ECD-mFc and cynomolgus monkey TIGIT ECD-mFc

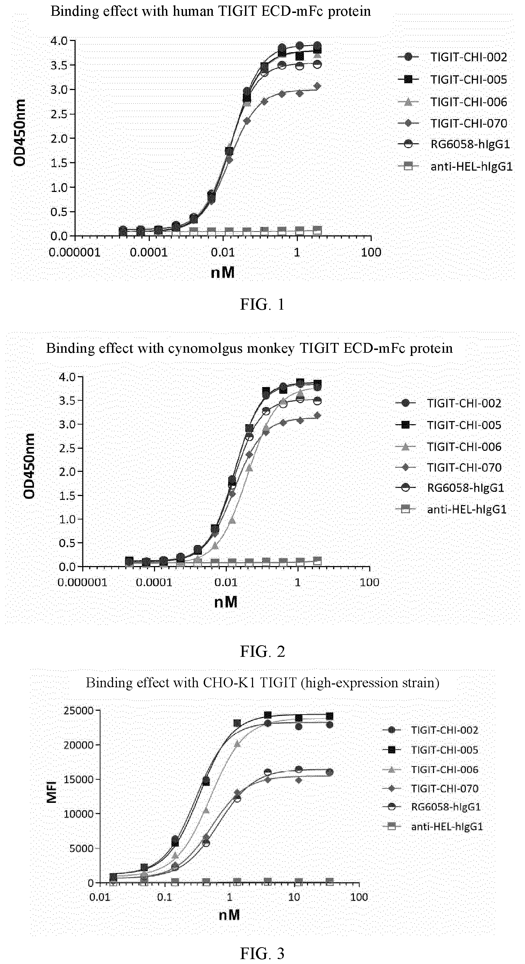

The sheep anti-mouse IgG antibody (Jackson, CAT: 115-006-071) was diluted to 4 µg/mL with PBS (Hyclone, CAT#SH30256, pH7.4), added (50 µL/well) into an enzyme-labeled plate (coming, CAT#9018) and kept at 4°C overnight. After discarding the coating solution, the plate was added with 5% skim milk powder (Sangon Biotech, CAT#A600669-0250)-PBS, 250 µL/well, and incubated at 37°C for 2-4 hours. After washing three times with 0.05% Tween 20-PBS (Sangon Biotech, CAT#A100777-0500, B548117-0500) on the Microplate Washer (Biotek, CAT#405TUS), the plate was added with human TIGIT ECD-mFc (In-house construction, working concentration 30 ng/mL) and cynomolgus monkey TIGIT ECD-mFc (In-house construction, working concentration 30 ng/mL), 50 µL per well, incubated at 4°C overnight. The enzyme-labeled plate was then washed with 0.05% Tween 20-PBS for 3 times on the Microplate Washer, added with tested antibodies diluted to 13nM with 1% BSA (diluted by 3-fold serial dilution for 12 concentration points), 50 µL/well, and incubated at 37°C for 1.5-2 hours. The plate was washed with 0.05% Tween 20-PBS for 3 times on the Microplate Washer, then added with HRP enzyme-labeled antibodies (Merck, CAT#AP113P) diluted with 1% BSA (Sangon Biotech, CAT#A500023-0100)-PBS at a dilution ratio of 1:5000, 50 µL/well, and incubated at 37°C for 1 hour. After washing 3 times with 0.05% Tween 20-PBS on the Microplate Washer, the plate was added with TMB chromogenic solution (KPL, CAT#52-00-03), 50 µL/well, and incubated at room temperature for 7-10 minutes. 1M HCL, 50 µL/well, was then added into the plate to terminate the reaction, and the OD450 nm was read by a microplate reader (Biotek, Powerwave HT). The experimental results show that the anti-TIGIT chimeric antibodies can effectively bind to human TIGIT ECD-mFc (Fig. 1) and cynomolgus monkey TIGIT ECD-mFc (Fig. 2), which is consistent with the control antibody, Roche RG6058.

### (b) The binding activity of anti-TIGIT chimeric antibodies to CHO-K1 human TIGIT (cells with high, medium and low expression levels) and CHO-K1 cynomolgus monkey TIGIT detected by FACS.

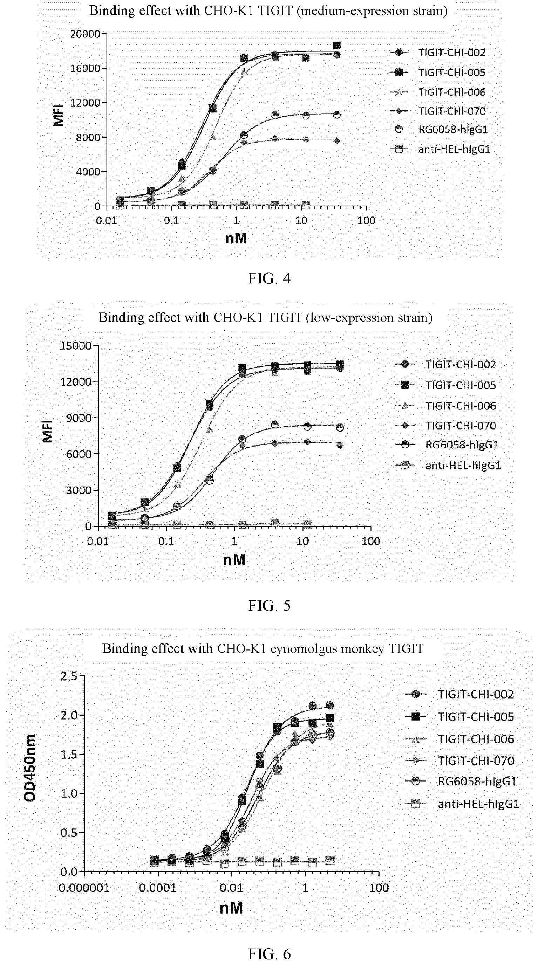

The collected cells were washed once with PBS (Hyclone, CAT#SH30256), resuspended to 2×10⁵/50 µL with 1% BSA-PBS. The antibodies were diluted to 80 nM with 1% BSA-PBS (diluted by 3-fold serial dilution for 12 concentration points). The 50 µL cells were then mixed with 50 µL diluted antibodies, and then incubated at 4°C for 60 minutes. The cells were washed twice with PBS, resuspended with Alexa Fluor^{®} 647 fluorescein labeled secondary antibody (1:800) (Jackson, CAT# 109-605-088), 100 µL/well, and incubated at 4°C for 40 minutes. After washing twice with PBS, the cell samples were resuspended with 1% BSA-PBS, 100 µL/well and then analyzed by flow cytometry (BD, Canto II). The experimental results show that the anti-TIGIT chimeric antibodies can effectively bind to CHO-K1 human TIGIT high expression cell strains (Fig. 3), CHO-K1 human TIGIT medium expression cell strains (Fig. 4), CHO-K1 human TIGIT low expression cell strains (Fig. 5) and CHO-K1 cynomolgus monkey TIGIT cell strains (Fig. 6), which is consistent with the control antibody, Roche RG6058.

### (c) The effect of anti-TIGIT chimeric antibodies blocking the interaction between Bio-CD155-His and CHO-K1 human TIGIT high expression cell strains detected by FACS

The collected cells were washed once with PBS (Hyclone, CAT#SH30256), resuspended to 2×10⁵/40 µL with 1% BSA-PBS. The antibodies were diluted to 210 nM with 1% BSA-PBS (diluted by 3-fold serial dilution for 12 concentration points). The Bio-CD155-His (Sino Biological Inc., 10109-H08H) was diluted to 3 µg/mLwith 1% BSA-PBS. The 40 µL cells were then mixed with 40 µL diluted antibodies and 40 µL diluted Bio-CD155-His, and then incubated at 4°C for 60 minutes. The cells were washed twice with PBS, resuspended with APC labeled streptavidin (dilution ratio 1: 1700, Biolegend, 405243), 100 µL/well, and incubated at 4°C for 40 minutes. The cell samples were washed twice with PBS, resuspended with 1% BSA-PBS, 100 µL/well and then analyzed by flow cytometry (BD, Canto II). As shown in Fig. 7, both the anti-TIGIT chimeric antibodies and the control antibody (RG6058) can effectively block the binding between the Bio-CD155-His protein and CHO-K1- human TIGIT cells.

### (d) The effect of anti-TIGIT chimeric antibodies blocking the interaction between TIGIT ECD-mFc and CHO-K1 CD155 detected by FACS

The collected CHO-K1 CD155 cells were washed once with PBS (Hyclone, CAT#SH30256) and resuspended to 2×10⁵/40 µL with 1% BSA-PBS. The tested antibodies were diluted to 600 nM with 1% BSA-PBS (diluted by 2.5-fold serial dilution for 12 concentration points). The TIGIT ECD-mFc was diluted with 1% BSA-PBS to 6 µg/mL. The 40 µL cells were then mixed with 40 µL diluted antibodies and 40 µL diluted TIGIT ECD-mFc, and then incubated at 4°C for 60 minutes. The cells were washed twice with PBS, resuspended with Alexa Fluor^{®} 647 fluorescein labeled secondary antibody (dilution ratio 1:800, Jackson, CAT# 109-605-003), 100 µL/well, and incubated at 4°C for 40 minutes. The cell samples were washed twice with PBS, resuspended to 100 µL/well with 1% BSA-PBS and then analyzed by flow cytometry (BD, Canto II). As shown in Fig. 8, both the anti-TIGIT chimeric antibodies and the control antibody (RG6058) can effectively block the binding between the human TIGIT protein and CHO-K1-CD155 cells.

**Table 5 shows a summary of the identification of TIGIT chimeric antibodies Table 5. A summary of the identification of TIGIT chimeric antibodies**

| TIGIT chimeric antibody | Human TIGIT ECD-His | | | Human TIGIT ECD-mF c/CHO-K1 CD155 blocking experime nt | Bio-CD1 55-His/C HO-K1 Human TIGIT blocking experime nt | CHO-K1 Human TIGIT (high express ion strain) | CHO-K 1 Human TIGIT (medi um expressi on strain) | CHO-K 1 Human TIGIT (low expressi on strain) | Human TIGIT ECD-m Fc binding experim ent | CHO-K 1 cynomo lgus monkey TIGIT binding experim ent | cynomo lgus monkey TIGIT ECD-m Fc binding experim ent |
|---|---|---|---|---|---|---|---|---|---|---|---|
| | ka (1/Ms) | kd (1/s) | KD (M) | IC50 (nM) | IC50 (nM) | EC50 (nM) | EC50 (nM) | EC50 (nM) | EC50 (nM) | EC50 (nM) | EC50 (nM) |
| TIGIT-CHI-002 | 4.53E+06 | 3.15E-04 | 6.97E-11 | 0.3682 | 0.1519 | 0.2991 | 0.2904 | 0.2183 | 0.0181 | 0.0290 | 0.0165 |
| TIGIT-CHI-005 | 3.59E+06 | 1.92E-04 | 5.36E-11 | 0.3628 | 0.1582 | 0.3421 | 0.3160 | 0.2290 | 0.0173 | 0.0264 | 0.0170 |
| TIGIT-CHI-006 | 1.29E+06 | 1.70E-04 | 1.32E-10 | 0.5723 | 0.2465 | 0.5022 | 0.4810 | 0.3287 | 0.0167 | 0.0731 | 0.0389 |
| TIGIT-CHI-070 | 1.52E+06 | 4.56E-04 | 3.00E-10 | 0.5819 | 0.3837 | 0.5168 | 0.3941 | 0.3524 | 0.0145 | 0.0359 | 0.0163 |
| RG6058-hIgG1 | 9.03E+05 | 1.47E-04 | 1.63E-10 | 0.5658 | 0.3884 | 0.6731 | 0.6164 | 0.4953 | 0.0151 | 0.0509 | 0.0158 |

### (e) The detection of the expression of PVRIG and TIGIT on the surface of NK cells and the expression of PVR and PVRL2 on the surface of WIDR cells

The expression of PVRIG and TIGIT on NK cells (Natural killer cells) was detected by FACS. First, the NK cells were counted by using a cell counter (Beckman Coulter, Vi-CELL). The NK cells were added into three flow cytometry tubes with 1E+5 NK cells in each tube and washed twice with PBS. After discarding the supernatant, one tube was added with 300 µL Staining buffer (PBS+2% FBS) as an unstained tube, and the other two tubes were added with 100 µL staining solution (PBS+1* Zombie Violet (Biolegend, 423114)), mixed well and then incubated at room temperature for 15 minutes. The cells were then washed twice with Staining buffer, and the supernatant was discarded. 50 µL of Fc blocker (Staining buffer + FCX blocker (Biolegend, 422302)) was added to each tube, mixed and then incubated at 4°C for 15 minutes. Each tube was then added with different staining solution, mixed and incubated at 4°C for 30 minutes, wherein, the first tube was added with 50 µL of 2* staining solution (Staining buffer + PE-Cy7 Mouse anti-hCD3 detection antibody + PE Mouse anti-hCD56 detection antibody + APC Mouse anti-hTIGIT detection antibody + AF488 Rabbit anti-hPVRIG detection antibody, CD3 detection antibody: Biolegend 300316, CD56 detection antibody: Biolegend 318306, TIGIT detection antibody: Biolegend 372706, PVRIG detection antibody: RD FAB93651G), and the second tube was added with 50 µL of 2* isotype control staining solution (Staining buffer+PE-Cy7 Mouse anti-hCD3 detection antibody + PE Mouse anti-hCD56 detection antibody + APC Mouse IgG2a κ isotype control antibody + AF488 Rabbit IgG κ isotype control antibody, APC mIgG2a κ isotype control antibody: Biolegend 400222, AF488 Rabbit IgG κ isotype control antibody: RD IC1051G). The samples were then washed twice with Staining buffer, centrifuged and mixed with 300 µL Staining buffer. The samples were detected by a flow cytometer (Thermo Attune NxT) to obtain the percentage of the CD56-positive and CD3-negative cell population in the Zombie Violet negative cell population and read the signals of APC and AF488 of the CD56-positive and CD3-negative cell population in the Zombie Violet negative cell population. Panel A in Figure 9 shows that PVRIG and TIGTI are expressed on the surface of NK cells from different donors (donor-010 and donor-050).

The expression of PVR and PVRL2 on WIDR cells was detected by FACS. First, the WIDR cells were trypsinized to form a cell suspension, and counted by using a cell counter (Beckman Coulter, Vi-CELL). The WIDR cells were added into three flow cytometry tubes with 1E+5 cells in each tube and washed twice with PBS. After centrifugation and discarding the supernatant, one tube was added with 300 µL Staining buffer (PBS+2% FBS) as an unstained tube, and the other two tubes were added with 100 µL staining solution (PBS+1* Zombie Violet (Biolegend, 423114)), mixed well and then incubated at room temperature for 15 minutes. The cells were then washed twice with Staining buffer, and the supernatant was discarded. Each tube was then added with different staining solution, mixed and incubated at 4°C for 30 minutes, wherein, the first tube was added with 100 µL staining solution (Staining buffer + PerCP-Cy5.5 Mouse anti-hPVR detection antibody + APC Mouse anti-hPVRL2 detection antibody, PVR detection antibody: Biolegend 337612, PVRL2 detection antibody: Biolegend 337412), and the second tube was added with 100µL isotype control staining solution (Staining buffer + PerCP-Cy5.5 Mouse IgG1 κ isotype control antibody + APC κ Mouse IgG1 isotype control antibody, PerCP-Cy5.5 mIgG1 κ isotype control antibody: Biolegend 400150 , APC mIgG1 κ isotype control antibody: Biolegend 400122). The samples were then washed twice with Staining buffer, centrifuged and mixed with 300 µL Staining buffer. The samples were detected by a flow cytometer (Thermo Attune NxT) to read the signals of PerCP-Cy5.5 and APC of the Zombie Violet negative cell population. Panel B in Figure 9 shows that PVR and PVRL2 are highly expressed on the surface of WIDR cells.

### (f) The promoting effect of anti-TIGIT chimeric antibodies on NK cell function detected by NK cell degranulation assay

FACS was used to detect the CD107a signal of NK cells (Natural killer cell), which indicated the effect of the tested antibody on the degranulation process of NK cells. The PBMCs were resuscitated one day in advance and NK cells were sorted (Stemcell, 17955). The NK cells were added with 200 IU/ml h-IL2 (RD, 202-IL) and 10 ng/ml h-IL12 (Peprotech, 200-12-50UG) to stimulate overnight and plated for the following experiment on the next day. Firstly, the antibodies were diluted to the highest concentration of 275 nM (four-fold concentration) with assay buffer (RPMI1640-Glutamax + 10% FBS + 1×P/S) and continually diluted with assay buffer by 10-fold gradient dilution. The diluted antibodies were added to an ultra-low attachment, 96-well, U-bottom plate (Costar, 7007), 50 µl per well, for further use. Secondly, the NK cells were counted by using a cell counter (Beckman Coulter, Vi-CELL). A certain number of NK cells were centrifuged at a speed of 350 g for 5 minutes, resuspended to a density of 0.5E+6 cells/ml with assay buffer after discarding the supernatant and added with protein transport inhibitor (Invitrogen, 00498093) and APC mouse anti-human detection antibody (Biolegend, 328620). The antibody coated 96-well, U-bottom plate was added with the treated NK cell suspension (50 µl per well), mixed well and incubated at room temperature for 15 minutes. During the incubation, the target WIDR cells were trypsinized to form a cell suspension and counted by using a cell counter (Beckman Coulter, Vi-CELL). An appropriate number of cells were centrifuged at 200 g for 5 minutes, and resuspended to a density of the 0.25E+6 cells/ml with the assay buffer after discarding the supernatant. After incubation, the target cell suspension was added to the plate, 100 µL per well. At this time, each well contained 25,000 NK cells, 25,000 target cells and different concentrations of tested antibodies. The wells only containing NK cells were served as resting control, while the wells containing NK cells and target cells were served as drug-free control. All the wells were mixed well and incubated in an incubator at 37°C for 16 hours. The change of CD107a was detected by FACS analysis: the cells in the plate were transferred to the same position in a 96-well, V-bottom plate of 96 wells and washed twice with PBS. After discarding the supernatant, each well was added with the staining solution (PBS+2%FBS+ 1 *zombie violet (Biolegend, 423114) + PE mouse anti-CD56 detection antibody (Biolegend, 318306)), mixed well and incubated at 4°C for 30 minutes. Each well was then washed twice with staining buffer and resuspended with 150 µL staining buffer after discarding the supernatant. The samples were detected by a flow cytometer (Thermo Attune NxT) to read the percentage of CD107a strong positive cell population in CD56 positive cells. A higher percentage of CD107a strong positive cells represent a stronger degranulation of NK cells and a higher degree of activation of NK cells. Figure 10 shows that the negative control, anti-HEL-hIgG1, has no effect on CD107a on the surface of NK. The three tested antibodies can improve the expression of CD107a on NK cells to varying degrees, which indicates that the three tested antibodies can effectively promote the activation of NK cells.

### (g) The promoting effect of anti-TIGIT chimeric antibodies on cytotoxicity for target cells by NK cells detected by NK cell cytotoxicity assay

The lysis level of target cells (WIDR) was detected by FACS to infer the effect of the tested antibodies on the cytotoxicity of NK (Natural killer) cells. The PBMCs were resuscitated one day in advance. The NK cells sorted from PBMCs (Stemcell, 17955) were added with 200 IU/mL h-IL2 (RD, 202-IL) and 10 ng/mL h-IL12 (Peprotech, 200-12-50UG) to stimulate overnight and plated for the following experiment on the next day. Firstly, the antibodies were diluted to the highest concentration of 275 nM (four-fold concentration) with assay buffer (RPMI1640-Glutamax + 10% FBS + 1×P/S) and continually diluted with assay buffer by 10-fold gradient dilution. The diluted antibodies were added to an ultra-low attachment, 96-well, U-bottom plate (Costar, 7007), 50 µL per well, for further use. The target cells (WIDR) were trypsinized to form a cell suspension and counted by using a cell counter (Beckman Coulter, Vi-CELL). An appropriate number of WIDR cells were centrifuged at a speed of 200 g for 5 minutes, resuspended with PBS after discarding the supernatant and added with staining solution, CellTrace Violet (Invitrogen, C34557A) with a final concentration of 5 µM. The WIDR suspension with staining solution was mixed evenly, placed in an incubator at 37°C for 10 minutes and shaken during the incubation. At the same time, NK cells were counted with a cell counter. A certain number of NK cells were centrifuged at a speed of 350 g for 5 minutes and resuspended to a density of 0.5E+6 cells/mL with the assay buffer after discarding the supernatant. The antibody coated 96-well, U-bottom plate was added with the treated NK cell suspension (50 µL per well), mixed well and incubated at room temperature for 15 minutes. After the WIDR cell staining, 5 times volume of complete medium (MEM+10%FBS+1*P/S+1*non-essential amino acid+1*sodium glutamate) was added to the cell suspension to stop the reaction. The WIDR cells were centrifuged at a speed of 200 g for 5 minutes and resuspended to a density of 0.25E+6 cells/mL with the assay buffer after discarding the supernatant. After incubating with antibodies and NK cells, the plate was added with WIDR cell suspension, 100 µL per well. At this time, each well contained 25,000 NK cells, 25,000 WIDR cells and various concentrations of tested antibodies. The wells only containing WIDR cells were served as resting control, while the wells containing NK cells and WIDR cells were served as drug-free control. All the wells were mixed well and incubated in an incubator at 37°C for 4 hours. The lysis level of WIDR cells was detected by FACS analysis: each well was added with staining solution (PBS + PI (Propidium Iodide, Invitrogen, P3566)), mixed well and incubated at room temperature for 20 minutes. The samples were then detected by a flow cytometer (Thermo Attune NxT) to read the percentage of PI positive cell population in CTV positive cells. More percentage of PI positive cells represent stronger NK cell cytotoxicity. Figure 11 shows that the negative control, anti-HEL-hIgG1, has no significant effect on the NK cell cytotoxicity. The 4 tested chimeric antibodies can effectively promote the NK cell cytotoxicity for the target cells (WIDR), and the promoting effect of the 4 chimeric antibodies on the NK cell cytotoxicity is not weaker than that of the positive control, RG6058-hIgG1.

### (h) The effect of anti-TIGIT chimeric antibody on improving the functions of antigen-specific CD8 T cells detected by CMV antigen-recall assay

The PBMCs from Anti-CMV IgG positive donor were induced by CMV pp65 (495-503) polypeptide to produce CMV pp65 specific CD8 T cells, which were served as effector cells. The colo205 tumor cell line pulsed with pp65 was used as the target cell. In such experimental system, the effect of TIGIT antibody on improving the functions of the antigen-specific CD8 T cell was detected.

The PBMCs were resuscitated, resuspended to 2×10⁶/mL by using complete medium (RPMI1640-Glutamax + 5% AB serum + 1% P/S+(1×) 2-β mercaptoethanol) containing 1 mg/mL CMV pp65(495-503) peptide (Anaspec, Cat No.AS-28328), 2 ng/mL human IL-2 (R&D, Cat No.IL-202) and 10 ng/mL human IL-7 (Peprotech, Cat No.200-07), inoculated in a 6-well plate (5 mL/well) and incubated at 37°C with 5% CO₂ for 6 days. On day 6, all the PBMCs were collected, and pp65 and IL-7 in the medium were removed. The cells were divided into two portions, resuspended in complete medium containing 100 IU/mL human IL-2, and cultured for another 2 days. On day 8, all the PBMCs were collected and resuspended in complete medium containing 100 IU/mL human IL-2, and the cell density was adjusted to 2×10⁶/mL for continuing cultivation. On day 11, all the PBMCs were collected. The percentage of CD8 T cells and CMV pp65 (495-503) specific CD8 T cells in PBMCs (Figure 12, panel A), and the expression of PVRIG, TIGIT and PD-1 on CMV pp65 (495-503) specific CD8 T cells (Figure 12, panel B) were detected by flow cytometry. The detection antibodies were as follows: Livedead Near IR (Invitrogen, Cat No.L34976), CD8-PerCp Cy5.5 (BD, Cat No.565310), CD3-PE-Cy7 (Biolegend, Cat No.300316), T-select HLA-A*0201 CMV pp65 Tetramer-PE (MBL, Cat No.TS-0010-1C), PVRIG-AF488 (R&D, Cat No.FAB93651G-100UG), TIGIT-APC (Biolegend, Cat No.372706) and PD-1-BV421 (BD, Cat No.562516).

CD8 T cells were isolated from the induced PBMCs as effector cells by CD8 T cell isolation kit (Stemcell, Cat No. 17953) and resuspended with AIM-V to the cell density of 0.4×10⁶/mL. The target cells, Colo205, were digested by TrypLE^{™} Express Enzyme (Gibco, Cat No.12605010), resuspended in AIM-V (Gibco, Cat No.31035-025) containing 50 ng/mL pp65 to the cell density of 1×10⁶/mL and treated at 37°C with 5% CO₂ for 1-2 hours. The target cells were then centrifuged at 250 g for 5 minutes and resuspended in AIM-V to the cell density of 0.2×10⁶/mL after discarding the supernatant. The highly expressed PVRL2 and PVR on Colo205 cells were detected by flow cytometry, as shown in panel C, Fig. 12. Anti-TIGIT antibodies or positive control were diluted with AIM-V to 280 nM. The low-attachment, 96-well, U-bottom plate (Corning, Cat No.7007) was added with 50 µL of antibody, 50 µL of CD8 T cells and 100 µL of pp65-treated colo205 cells in order, mixed well with a multichannel pipette, and incubated at 37°C with 5% CO₂ for 18 hours. In the experimental system, the final concentration of antibodies was 70 nM, CD8 T cells were 20,000/well, and colo205 were 20,000/well. After incubation, the supernatant was collected by centrifugation at 400 g, and the level of human IFN-γ in the supernatant was detected with an ELISA kit (Dakewe, Cat No.1110003). In this system, the positive control was RG6058-hIgG1, and the negative control was no treatment. As shown in Fig. 12 (panel D), the secretion level of IFN-γ in the cell supernatant treated with 3 TIGIT tested antibodies has no statistically significant difference, compared with positive control, RG6058-hIgG1, but is significantly higher than the no treatment group. The percentage on each histogram represents the percentage of IFN-γ secretion increased in the experimental group compared with no treatment group.

The expression of PVRL2 and PVR on colo205 was detected by flow cytometry using following detection antibodies: livedead-BV421 (Invitrogen, Cat No.L34964), PVRL2-APC (Biolegend, Cat No.337412), PVR-PerCp Cy5.5 (Biolegend, Cat No.337612) and PD-L1-PE-Cy7 (BD, Cat No.558017).

### EXAMPLE 5 - Humanization of anti-human TIGIT monoclonal antibodies

By comparing the IMGT (http://imgt.cines.fr) human antibody heavy and light chain variable region germline gene database, the heavy chain and light chain variable region germline genes with high homology with mouse antibody were selected as templates. The CDRs of mouse antibody were grafted into corresponding human templates to form a variable region sequence of FR1-CDR1-FR2-CDR2-FR3-CDR3-FR4. The key amino acids in the skeleton sequence were back mutated to the amino acids corresponding to the murine antibody as needed to ensure the original affinity, and thus to obtain the humanized anti-TIGIT monoclonal antibody. The CDR amino acid residues of the antibody were usually determined and annotated by the Kabat numbering scheme.

### 1. Humanization of TIGIT-002

The humanized light chain templates of the mouse antibody (TIGIT-002) were IGKV2-29*02/IGKV4-1*01 and IGKJ4*01, and the humanized heavy chain templates are IGHV4-38-2*01 and IGHJ6*01. The CDRs of the mouse antibody (TIGIT-002) were grafted into the human template thereof to obtain the corresponding humanized version. The key amino acids in the FR region sequence in the humanized antibody of TIGIT-002 were back mutated to the amino acids corresponding to the mouse antibody as needed to ensure the original affinity. The specific back mutation design is shown in Table 6.

**Table 6. The back mutation design of humanized antibody of TIGIT-002**

| **VL** | | **VH** | |
|---|---|---|---|
| VL1 | Grafted (IGKV2-29*02) + L47M | VH1 | Grafted (IGHV4-38-2*01) + S30T, V71R |
| VL2 | Grafted (IGKV2-29*02) + L37Q, L47M | VH2 | Grafted (IGHV4-38-2*01) + S30T, G44K, V71R |
| VL3 | Grafted (IGKV4-P01) + P43S, L47M | VH3 | Grafted (IGHV4-38-2*01) + S30T, G44K, I48M, V67I, V71R |
| - | - | VH4 | Grafted (IGHV4-38-2*01) + S30T, G44K, W47Y, V71R |

| | | | |
|---|---|---|---|
| Note: Grafted (IGKV2-29*02) represents the insertion of CDRs of the mouse antibody into the FR region sequence in human germline IGKV2-29*02; L47M represents the L at position 47 of Grafted (IGKV2-29*02) is back mutated to M, and so on. The back-mutated amino acids are numbered in natural order. | | | |

The specific variable region sequences in humanized antibody of TIGIT-002 are as follows:
The amino acid sequence of TIGIT-002.VL1 is shown in SEQ ID NO: 66: DIVMTQTPLSLSVTPGQPASISCKASQNVRTAVAWYLQKPGQSPQLMIYSASYRYTGVPD RFSGSGSGTDFTLKISRVEAEDVGVYYCQQYYTTPWTFGGGTKVEIK
The amino acid sequence of TIGIT-002.VL2 is shown in SEQ ID NO: 67: 
DIVMTQTPLSLSVTPGQPASISCKASQNVRTAVAWYQQKPGQSPQLMIYSASYRYTGVPD
RFSGSGSGTDFTLKISRVEAEDVGVYYCQQYYTTPWTFGGGTKVEIK
The amino acid sequence of TIGIT-002.VL3 is shown in SEQ ID NO: 68: 
DIVMTQSPDSLAVSLGERATINCKASQNVRTAVAWYQQKPGQSPKLMIYSASYRYTGVP
DRFSGSGSGTDFTLTISSLQAEDVAVYYCQQYYTTPWTFGGGTKVEIK
The amino acid sequence of TIGIT-002.VH1 is shown in SEQ ID NO: 69: 
EVQLQESGPGLVKPSETLSLTCAVSGYSITSDSWNWIRQPPGKGLEWIGYISYSGNTYYNP
SLKSRVTISRDTSKNQFSLKLSSVTAADTAVYYCARLDFSNYGGAVDYWGQGTTVTVSS
The amino acid sequence of TIGIT-002.VH2 is shown in SEQ ID NO: 70: 
EVQLQESGPGLVKPSETLSLTCAVSGYSITSDSWNWIRQPPGKKLEWIGYISYSGNTYYNP
SLKSRVTISRDTSKNQFSLKLSSVTAADTAVYYCARLDFSNYGGAVDYWGQGTTVTVSS
The amino acid sequence of TIGIT-002.VH3 is shown in SEQ ID NO: 71: 
EVQLQESGPGLVKPSETLSLTCAVSGYSITSDSWNWIRQPPGKKLEWMGYISYSGNTYYN
PSLKSRITISRDTSKNQFSLKLSSVTAADTAVYYCARLDFSNYGGAVDYWGQGTTVTVSS
The amino acid sequence of TIGIT-002.VH4 is shown in SEQ ID NO: 72: 
EVQLQESGPGLVKPSETLSLTCAVSGYSITSDSWNWIRQPPGKKLEYIGYISYSGNTYYNP
SLKSRVTISRDTSKNQFSLKLSSVTAADTAVYYCARLDFSNYGGAVDYWGQGTTVTVSS
The amino acid sequence of the humanized light chain template IGKV2-29*02 is shown in SEQ ID NO: 73: 
DIVMTQTPLSLSVTPGQPASISCKSSQSLLHSDGKTYLYWYLQKPGQSPQLLIYEVSSRFS
GVPDRFSGSGSGTDFTLKISRVEAEDVGVYYCMQGIHLP
The amino acid sequence of the humanized light chain template IGKV4-1*01 is shown in SEQ ID NO: 74: 
DIVMTQSPDSLAVSLGERATINCKSSQSVLYSSNNKNYLAWYQQKPGQPPKLLIYWASTR
ESGVPDRFSGSGSGTDFTLTISSLQAEDVAVYYCQQYYSTP
The amino acid sequence of the humanized light chain template IGKJ4*01 is shown in SEQ ID NO: 75:
   FGGGTKVEIK
The amino acid sequence of the humanized heavy chain template IGHV4-38-2*01 is shown in SEQ ID NO: 76: 
QVQLQESGPGLVKPSETLSLTCAVSGYSISSGYYWGWIRQPPGKGLEWIGSIYHSGSTYY
NPSLKSRVTISVDTSKNQFSLKLSSVTAADTAVYYCAR
The amino acid sequence of the humanized heavy chain template IGHJ6*01 is shown in SEQ ID NO: 77:
   WGQGTTVTVSS

Based on the above-mentioned back mutation design in light chain and heavy chain variable regions in humanized antibody of TIGIT-002, the inventors selected different sequences of light chain and heavy chain for cross combination and finally obtained a variety of humanized antibodies of TIGIT-002. The amino acid sequences of the variable region in various antibodies are as follows:

**Table 7. Amino acid sequences corresponding to variable region in humanized antibodies of TIGIT-002**

| Fv | 002.VH1 | 002.VH2 | 002.VH3 | 002.VH4 |
|---|---|---|---|---|
| 002.VL1 | TIGIT-002-H1L1* | TIGIT-002-H2L1 | TIGIT-002-H3L1 | TIGIT-002-H4L1 |
| 002.VL2 | TIGIT-002-H1L2 | TIGIT-002-H2L2 | TIGIT-002-H3L2 | TIGIT-002-H4L2 |
| 002.VL3 | TIGIT-002-H1L3 | TIGIT-002-H2L3 | TIGIT-002-H3L3 | TIGIT-002-H4L3 |

| | | | | |
|---|---|---|---|---|
| *TIGIT-002-H1L1 indicates that the heavy chain is selected from TIGIT-002.VH1 and the light chain is selected from TIGIT-002.VL1. The same below. | | | | |

According to Kabat numbering scheme, the analysis results of VH and VL sequences in above 12 humanized antibodies are shown in table 8.

**Table 8 Kabat analysis results of VH and VL sequences in humanized antibody of TIGIT-002**

| Light/heavy chain variable regions | CDR1 | CDR2 | CDR3 |
|---|---|---|---|
| VH1/2/3/4 | SDSWN | YISYSGNTYYNPSLKS | LDFSNYGGAVDY |
| | SEQ ID NO:21 | SEQ ID NO:22 | SEQ ID NO:23 |
| VL1/2/3 | KASQNVRTAVA | SASYRYT | QQYYTTPWT |
| | SEQ ID NO:18 | SEQ ID NO:19 | SEQ ID NO:20 |

### 2. Humanization of TIGIT-006

The humanized light chain templates of the mouse antibody (TIGIT-006) were IGKV2-29*02/IGKV4-1*01 and IGKJ4*01, and the humanized heavy chain templates are IGHV4-38-2*01 and IGHJ6*01. The CDRs of the mouse antibody (TIGIT-006) were grafted into the human template thereof to obtain the corresponding humanized version. The key amino acids in the FR region sequence in the humanized antibody of TIGIT-006 were back mutated to the amino acids corresponding to the mouse antibody as needed to ensure the original affinity. The specific back mutation design is shown in Table 9.

**Table 9. The back mutation design of humanized antibody of TIGIT-006**

| **VL** | | **VH** | |
|---|---|---|---|
| VL1 | Grafted(IGKV2-29*02) | VH1 | Grafted(IGHV4-38-2*01) + S30T,V71R |
| VL2 | Grafted(IGKV2-29*02) + L37Q,Q45K | VH2 | Grafted(IGHV4-38-2*01) + I29M,S30T,V71R |
| VL3 | Grafted(IGKV4-1*01) + P43S | VH3 | Grafted(IGHV4-38-2*01) + I29M,S30T,G44K,V71R |
| - | - | VH4 | Grafted(IGHV4-38-2*01) + I29M,S30T,G44K,I48M,V67I,V71R |
| | | VH5 | Grafted(IGHV4-38-2*01) + I29M,S30T,G44K,W47Y,V71R |

| | | | |
|---|---|---|---|
| Note: Grafted (IGKV2-29*02) represents the insertion of CDRs of the mouse antibody into the FR region sequence in human germline IGKV2-29*02; L37Q represents the L at position 37 of Grafted (IGKV2-29*02) is back mutated to Q, and so on. The back-mutated amino acids are numbered in natural order. | | | |

The specific variable region sequences in humanized antibody of TIGIT-006 are as follows:
The amino acid sequence of TIGIT-006.VL1 is shown in SEQ ID NO:78: 
DIVMTQTPLSLSVTPGQPASISCRASQGVSTTIAWYLQKPGQSPQLLIYSASYRYTGVPDR
FSGSGSGTDFTLKISRVEAEDVGVYYCQQYYSSPFTFGGGTKVEIK
The amino acid sequence of TIGIT-006.VL2 is shown in SEQ ID NO:79: 
DIVMTQTPLSLSVTPGQPASISCRASQGVSTTIAWYQQKPGQSPKLLIYSASYRYTGVPDR
FSGSGSGTDFTLKISRVEAEDVGVYYCQQYYSSPFTFGGGTKVEIK
The amino acid sequence of TIGIT-006.VL3 is shown in SEQ ID NO:80: DIVMTQSPDSLAVSLGERATINCRASQGVSTTIAWYQQKPGQSPKLLIYSASYRYTGVPRF SGSGSGTDFTLTISSLQAEDVAVYYCQQYYSSPFTFGGGTKVEIK
The amino acid sequence of TIGIT-006.VH1 is shown in SEQ ID NO:81: 
EVQLQESGPGLVKPSETLSLTCAVSGYSITSDYWNWIRQPPGKGLEWIGYISYSGRTYYN
PSLKSRVTISRDTSKNQFSLKLSSVTAADTAVYYCARGDYSNYGGAMYDWGQGTTVTV
SS
The amino acid sequence of TIGIT-006.VH2 is shown in SEQ ID NO:82: 
EVQLQESGPGLVKPSETLSLTCAVSGYSMTSDYWNWIRQPPGKGLEWIGYISYSGRTYY
NPSLKSRVTISRDTSKNQFSLKLSSVTAADTAVYYCARGDYSNYGGAMYDWGQGTTVT
VSS
The amino acid sequence of TIGIT-006.VH3 is shown in SEQ ID NO:83: 
EVQLQESGPGLVKPSETLSLTCAVSGYSMTSDYWNWIRQPPGKKLEWIGYISYSGRTYY
NPSLKSRVTISRDTSKNQFSLKLSSVTAADTAVYYCARGDYSNYGGAMYDWGQGTTVT
VSS
The amino acid sequence of TIGIT-006.VH4 is shown in SEQ ID NO:84: 
EVQLQESGPGLVKPSETLSLTCAVSGYSMTSDYWNWIRQPPGKKLEWMGYISYSGRTYY
NPSLKSRITISRDTSKNQFSLKLSSVTAADTAVYYCARGDYSNYGGAMYDWGQGTTVTV
SS
The amino acid sequence of TIGIT-006.VH5 is shown in SEQ ID NO:85: 
EVQLQESGPGLVKPSETLSLTCAVSGYSMTSDYWNWIRQPPGKKLEYIGYISYSGRTYYN
PSLKSRVTISRDTSKNQFSLKLSSVTAADTAVYYCARGDYSNYGGAMYDWGQGTTVTV
SS
The amino acid sequence of the humanized light chain template IGKV2-29*02 is shown in SEQ ID NO:73: 
DIVMTQTPLSLSVTPGQPASISCKSSQSLLHSDGKTYLYWYLQKPGQSPQLLIYEVSSRFS
GVPDRFSGSGSGTDFTLKISRVEAEDVGVYYCMQGIHLP
The amino acid sequence of the humanized light chain template IGKV4-1*01 is shown in SEQ ID NO:74: 
DIVMTQSPDSLAVSLGERATINCKSSQSVLYSSNNKNYLAWYQQKPGQPPKLLIYWASTR
ESGVPDRFSGSGSGTDFTLTISSLQAEDVAVYYCQQYYSTP
The amino acid sequence of the humanized light chain template IGKJ4*01 is shown in SEQ ID NO:75:
   FGGGTKVEIK
The amino acid sequence of the humanized heavy chain template IGHV4-38-2*01 is shown in SEQ ID NO:76: 
QVQLQESGPGLVKPSETLSLTCAVSGYSISSGYYWGWIRQPPGKGLEWIGSIYHSGSTYY
NPSLKSRVTISVDTSKNQFSLKLSSVTAADTAVYYCAR
The amino acid sequence of the humanized heavy chain template IGHJ6*01 is shown in SEQ ID NO:77:
   WGQGTTVTVSS

Based on the above-mentioned back mutation design in light chain and heavy chain variable regions in humanized antibody of TIGIT-006, the inventors selected different sequences of light chain and heavy chain for cross combination and finally obtained a variety of humanized antibodies of TIGIT-006. The amino acid sequences of the variable region in various antibodies are as follows:

**Table 10. Amino acid sequences corresponding to variable region in humanized antibodies of TIGIT-006**

| Fv | 006.VH1 | 006.VH2 | 006.VH3 | 006.VH4 | 006.VH5 |
|---|---|---|---|---|---|
| 006.VL1 | TIGIT-006-H1L1* | TIGIT-006-H2L1 | TIGIT-006-H3L1 | TIGIT-006-H4L1 | TIGIT-006-H5L1 |
| 006.VL2 | TIGIT-006-H1L2 | TIGIT-006-H2L2 | TIGIT-006-H3L2 | TIGIT-006-H4L2 | TIGIT-006-H5L2 |
| 006.VL3 | TIGIT-006-H1L3 | TIGIT-006-H2L3 | TIGIT-006-H3L3 | TIGIT-006-H4L3 | TIGIT-006-H5L3 |

| | | | | | |
|---|---|---|---|---|---|
| *TIGIT-006-H1L1 indicates that the heavy chain is selected from TIGIT-006.VH1, and the light chain is selected from TIGIT-006.VL1. The same below. | | | | | |

According to Kabat numbering scheme, the analysis results of VH and VL sequences in above 15 humanized antibodies are shown in Table 11.

**Table 11. Kabat analysis results of VH and VL sequences in humanized antibody of TIGIT-006**

| Light/heavy chain variable regions | CDR1 | CDR2 | CDR3 |
|---|---|---|---|
| VH1/2/3/4/5 | SDYWN | YISYSGRTYYNPSLKS | GDYSNYGGAMYD |
| | SEQ ID NO:27 | SEQ ID NO:28 | SEQ ID NO:29 |
| VL1/2/3 | RASQGVSTTIA | SASYRYT | QQYYSSPFT |
| | SEQ ID NO:24 | SEQ ID NO:25 | SEQ ID NO:26 |

### 3. Humanization of TIGIT-005

The humanized light chain templates of the mouse antibody TIGIT-005 were IGKV4-1*01 and IGKJ4*01, and the humanized heavy chain templates are IGHV1-3*01 and IGHJ6*01. The CDRs of the mouse antibody TIGIT-005 were grafted into the human template thereof to obtain the corresponding humanized version. The key amino acids in the FR region sequence in the humanized antibody of TIGIT-005 were back mutated to the amino acids corresponding to the mouse antibody as needed to ensure the original affinity. The specific back mutation design is shown in Table 12.

**Table 12. The back mutation design of humanized antibody of TIGIT-005**

| **VL** | | **VH** | |
|---|---|---|---|
| VL1 | Grafted(IGKV4-1*01) + Q38H,P43S | VH2 | Grafted(IGHV1-3*01) + T28A,R72A,T74K,A76S |

| | | | |
|---|---|---|---|
| Note: Grafted (IGKV4-1*01) represents the insertion of CDRs of the mouse antibody into the FR region sequence in human germline IGKV4-1*01; Q38H represents the Q at position 38 of Grafted (IGKV4-1*01) is back mutated to H, and so on. The back-mutated amino acids are numbered in natural order. | | | |

The specific variable region sequences in humanized antibody of TIGIT-005 are as follows:
The amino acid sequence of TIGIT-005.VL1 is shown in SEQ ID NO: 86: 
DIVMTQSPDSLAVSLGERATINCKASQHVSNGVAWYQHKPGQSPKLLIYSASYRYTGVP
DRFSGSGSGTDFTLTISSLQAEDVAVYYCQQHYNTPHTFGGGTKVEIK
The amino acid sequence of TIGIT-005.VH2 is shown in SEQ ID NO: 87: 
EVQLVQSGAEVKKPGASVKVSCKASGYAFTNYLIEWVRQAPGQRLEWMGVINPGSGGT
NYKEKFKGRVTITADKSSSTAYMELSSLRSEDTAVYYCARGEYFFFDYWGQGTTVTVSS
The amino acid sequence of the humanized light chain template IGKV4-1*01 is shown in SEQ ID NO: 74: 
DIVMTQSPDSLAVSLGERATINCKSSQSVLYSSNNKNYLAWYQQKPGQPPKLLIYWASTR
ESGVPDRFSGSGSGTDFTLTISSLQAEDVAVYYCQQYYSTP
The amino acid sequence of the humanized light chain template IGKJ4*01 is shown in SEQ ID NO: 75:
   FGGGTKVEIK
The amino acid sequence of the humanized heavy chain template IGHV1-3*01 is shown in SEQ ID NO: 106: 
QVQLVQSGAEVKKPGASVKVSCKASGYTFTSYAMHWVRQAPGQRLEWMGWINAGNG
NTKYSQKFQGRVTITRDTSASTAYMELSSLRSEDTAVYYCAR
The amino acid sequence of the humanized heavy chain template IGHJ6*01 is shown in SEQ ID NO: 77:
   WGQGTTVTVSS

TIGIT-005 antibody is prone to have chemical modifications on NG site. Inventors performed point mutations on NG to eliminate the risk of modification. In five of the examples, Inventors mutated the NG of 005.VL1. The mutation sites are shown in bold. The sequences after mutation are as follows:
The amino acid sequence of TIGIT-005.VL1a is shown in SEQ ID NO: 88: 
DIVMTQSPDSLAVSLGERATINCKASQHVSQGVAWYQHKPGQSPKLLIYSASYRYTGVP

DRFSGSGSGTDFTLTISSLQAEDVAVYYCQQHYNTPHTFGGGTKVEIK
The amino acid sequence of TIGIT-005.VL1b is shown in SEQ ID NO: 89: 
DIVMTQSPDSLAVSLGERATINCKASQHVSTGVAWYQHKPGQSPKLLIYSASYRYTGVPD
RFSGSGSGTDFTLTISSLQAEDVAVYYCQQHYNTPHTFGGGTKVEIK
The amino acid sequence of TIGIT-005.VL1c is shown in SEQ ID NO: 90: 
DIVMTQSPDSLAVSLGERATINCKASQHVSDGVAWYQHKPGQSPKLLIYSASYRYTGVP
DRFSGSGSGTDFTLTISSLQAEDVAVYYCQQHYNTPHTFGGGTKVEIK
The amino acid sequence of TIGIT-005.VL1d is shown in SEQ ID NO: 91: 
DIVMTQSPDSLAVSLGERATINCKASQHVSNAVAWYQHKPGQSPKLLIYSASYRYTGVP
DRFSGSGSGTDFTLTISSLQAEDVAVYYCQQHYNTPHTFGGGTKVEIK
The amino acid sequence of TIGIT-005.VL1e is shown in SEQ ID NO: 92:

DIVMTQSPDSLAVSLGERATINCKASQHVSNYVAWYQHKPGQSPKLLIYSASYRYTGVP
DRFSGSGSGTDFTLTISSLQAEDVAVYYCQQHYNTPHTFGGGTKVEIK

Based on the above-mentioned back mutation design in light chain and heavy chain variable regions in humanized antibody of TIGIT-005, the inventors selected different sequences of light chain and heavy chain for cross combination and finally obtained 6 humanized antibodies of TIGIT-005. The amino acid sequences of the variable region in various antibodies are as follows:

**Table 13. Amino acid sequences corresponding to variable region in humanized antibodies of TIGIT-005**

| Fv | 005.VL1 | 005.VL1a | 005.VL1b | 005.VL1c | 005.VL1d | 005.VL1e |
|---|---|---|---|---|---|---|
| 005.VH2 | TIGIT-005-H2L1* | TIGIT-005-H2L1a | TIGIT-005-H2L1b | TIGIT-005-H2 L1c | TIGIT-005-H2 L1d | TIGIT-005-H2L1e |

| | | | | | | |
|---|---|---|---|---|---|---|
| *TIGIT-005-H2L1 indicates that the heavy chain is selected from TIGIT-005.VH2, and the light chain is selected from TIGIT-005.VL1. The same below. | | | | | | |

According to Kabat numbering scheme, the analysis results of VH and VL sequences in above 6 humanized antibodies are shown in Table 14.

**Table 14. Kabat analysis results of VH and VL sequences in humanized antibody of TIGIT-005**

| Light/heavy chain variable regions | CDR1 | CDR2 | CDR3 |
|---|---|---|---|
| VH2 | NYLIE | VINPGSGGTNYKEKFKG | GEYFFFDY |
| | SEQ ID NO:33 | SEQ ID NO:34 | SEQ ID NO:35 |
| VL1 | KASQHVSNGVA | SASYRYT | QQHYNTPHT |
| | SEQ ID NO:30 | SEQ ID NO:31 | SEQ ID NO:32 |
| VL1a | KASQHVSQGVA | SASYRYT | QQHYNTPHT |
| | SEQ ID NO:93 | SEQ ID NO:31 | SEQ ID NO:32 |
| VL1b | KASQHVSTGVA | SASYRYT | QQHYNTPHT |
| | SEQ ID NO:94 | SEQ ID NO:31 | SEQ ID NO:32 |
| VL1c | KASQHVSDGVA | SASYRYT | QQHYNTPHT |
| | SEQ ID NO:95 | SEQ ID NO:31 | SEQ ID NO:32 |
| VL1d | KASQHVSNAVA | SASYRYT | QQHYNTPHT |
| | SEQ ID NO:96 | SEQ ID NO:31 | SEQ ID NO:32 |
| VL1e | KASQHVSNYVA | SASYRYT | QQHYNTPHT |
| | SEQ ID NO:97 | SEQ ID NO:31 | SEQ ID NO:32 |

### 4. Humanization of TIGIT-070

The humanized light chain templates of the mouse antibody TIGIT-070 were IGKV1-39*01/IGKV4-1*01 and IGKJ3*01, and the humanized heavy chain templates are IGHV1-3*01 and IGHJ6*01. The CDRs of the mouse antibody TIGIT-070 were grafted into the human template thereof to obtain the corresponding humanized version. The key amino acids in the FR region sequence in the humanized antibody of TIGIT-070 were back mutated to the amino acids corresponding to the mouse antibody as needed to ensure the original affinity. The specific back mutation design is shown in Table 15.

**Table 15. The back mutation design of humanized antibody of TIGIT-070**

| **VL** | | **VH** | |
|---|---|---|---|
| VL1 | Grafted(IGKV1-39*01) + A43S | VH1 | Grafted(IGHV1-3*01) + R72V,T74K |
| VL2 | Grafted(IGKV1-39*01) + A43S,I48V | VH2 | Grafted(IGHV1-3*01) + R72V,T74K,S75L,A76S |
| VL3 | Grafted(IGKV4-1*01) + P43S,148V | VH3 | Grafted(IGHV1-3*01) + R44G,R72V,T74K,S75L,A76S |

| | | | |
|---|---|---|---|
| Note: Grafted (IGKV1-39*01) represents the insertion of CDRs of the mouse antibody into the FR region sequence in human germline IGKV1-39*01; A43S represents the A at position 43 of Grafted (IGKV1-39*01) is back mutated to S, and so on. The back-mutated amino acids are numbered in natural order. | | | |

The specific variable region sequences in humanized antibody of TIGIT-070 are as follows:
The amino acid sequence of TIGIT-070.VL1 is shown in SEQ ID NO: 98: 
DIQMTQSPSSLSASVGDRVTITCRVSENIYSYLAWYQQKPGKSPKLLIYNAKTLAEGVPS
RFSGSGSGTDFTLTISSLQPEDFATYYCQHHYGNPLTFGPGTKVDIK
The amino acid sequence of TIGIT-070.VL2 is shown in SEQ ID NO: 99: 
DIQMTQSPSSLSASVGDRVTITCRVSENIYSYLAWYQQKPGKSPKLLVYNAKTLAEGVPS
RFSGSGSGTDFTLTISSLQPEDFATYYCQHHYGNPLTFGPGTKVDIK
The amino acid sequence of TIGIT-070.VL3 is shown in SEQ ID NO: 100: 
DIVMTQSPDSLAVSLGERATINCRVSENIYSYLAWYQQKPGQSPKLLVYNAKTLAEGVPD
RFSGSGSGTDFTLTISSLQAEDVAVYYCQHHYGNPLTFGPGTKVDIK
The amino acid sequence of TIGIT-070.VH1 is shown in SEQ ID NO: 101: 
EVQLVQSGAEVKKPGASVKVSCKASGYTFTNYYMHWVRQAPGQRLEWMGRIDPDSGG
SKYNEKFKSRVTITVDKSASTAYMELSSLRSEDTAVYYCAREGHYGFYSDYWGQGTTVT
VSS
The amino acid sequence of TIGIT-070.VH2 is shown in SEQ ID NO: 102: 
EVQLVQSGAEVKKPGASVKVSCKASGYTFTNYYMHWVRQAPGQRLEWMGRIDPDSGG
SKYNEKFKSRVTITVDKLSSTAYMELSSLRSEDTAVYYCAREGHYGFYSDYWGQGTTVT
VSS
The amino acid sequence of TIGIT-070.VH3 is shown in SEQ ID NO: 103: 
EVQLVQSGAEVKKPGASVKVSCKASGYTFTNYYMHWVRQAPGQGLEWMGRIDPDSG
GSKYNEKFKSRVTITVDKLSSTAYMELSSLRSEDTAVYYCAREGHYGFYSDYWGQGTTV
 TVSS
The amino acid sequence of the humanized light chain template IGKV1-39*01 is shown in SEQ ID NO: 104: 
DIQMTQSPSSLSASVGDRVTITCRASQSISSYLNWYQQKPGKAPKLLIYAASSLQSGVPSR
FSGSGSGTDFTLTISSLQPEDFATYYCQQSYST
The amino acid sequence of the humanized light chain template IGKV4-1*01 is shown in SEQ ID NO: 74: 
DIVMTQSPDSLAVSLGERATINCKSSQSVLYSSNNKNYLAWYQQKPGQPPKLLIYWASTR
ESGVPDRFSGSGSGTDFTLTISSLQAEDVAVYYCQQYYSTP
The amino acid sequence of the humanized light chain template IGKJ3*01 is shown in SEQ ID NO: 105:
   FGPGTKVDIK
The amino acid sequence of the humanized heavy chain template IGHV1-3*01 is shown in SEQ ID NO: 106: 
QVQLVQSGAEVKKPGASVKVSCKASGYTFTSYAMHWVRQAPGQRLEWMGWINAGNG
NTKYSQKFQGRVTITRDTSASTAYMELSSLRSEDTAVYYCAR
The amino acid sequence of the humanized heavy chain template IGHJ6*01 is shown in SEQ ID NO: 77:
   WGQGTTVTVSS

Based on the above-mentioned back mutation design in light chain and heavy chain variable regions in humanized antibody of TIGIT-070, the inventors selected different sequences of light chain and heavy chain for cross combination and finally obtained various humanized antibodies of TIGIT-070. The amino acid sequences of the variable region in various antibodies are as follows:

**Table 16. Amino acid sequences corresponding to variable region in humanized antibodies of TIGIT-070**

| Fv | 070.VH1 | 070.VH2 | 070.VH3 |
|---|---|---|---|
| 070.VL1 | TIGIT-070-H1L1* | TIGIT-070-H2L1 | TIGIT-070-H3L1 |
| 070.VL2 | TIGIT-070-H1L2 | TIGIT-070-H2L2 | TIGIT-070-H3L2 |
| 070.VL3 | TIGIT-070-H1L3 | TIGIT-070-H2L3 | TIGIT-070-H3L3 |

| | | | |
|---|---|---|---|
| *TIGIT-070-H1L1 indicates that the heavy chain is selected from TIGIT-070.VH1, and the light chain is selected from TIGIT-070.VL1. The same below. | | | |

According to Kabat numbering scheme, the analysis results of VH and VL sequences in above 9 humanized antibodies are shown in Table 17.

**Table 17. Kabat analysis results of VH and VL sequences in humanized antibody of TIGIT-070**

| Light/heavy chain variable regions | CDR1 | CDR2 | CDR3 |
|---|---|---|---|
| VH1/2/3 | NYYMH | RIDPDSGGSKYNEKFKS | EGHYGFYSDY |
| | SEQ ID NO:39 | SEQ ID NO:40 | SEQ ID NO:41 |
| VL1/2/3 | RVSENIYSYLA | NAKTLAE | QHHYGNPLT |
| | SEQ ID NO:36 | SEQ ID NO:37 | SEQ ID NO:38 |

### 5. Identification of humanized variants of anti-human TIGIT monoclonal antibodies

The affinity of the above humanized antibodies with human TIGIT ECD-His is detected by BIAcore, and with reference to the methods of Example 4(a), (b), and (d), the binding activity of the above humanized antibodies to human TIGIT ECD-mFc, CHO-K1 human TIGIT, CHO-K1 cynomolgus monkey TIGIT and cynomolgus monkey TIGIT ECD-mFc, and the effect of the humanized antibodies blocking the binding between human TIGIT ECD-mFc and CHO-K1 CD155 are detected, the results are shown in Table 18.

It can be seen that the 4 humanized antibodies (TIGIT-002-H4L3, TIGIT-005-H2L1d, TIGIT-006-H5L3, and TIGIT-070-H1L1) maintain the same affinity, binding activity and blocking effect as the chimeric antibodies, which are not significantly affected by humanization, and their overall performance is better than RG6058.

**Table 18. Identification of humanized variants of anti-human TIGIT monoclonal antibodies derived from SJL mice**

| TIGIT humanized antibodies | Human TIGIT ECD-His | | | | Human TIGIT ECD-hFc/ CHO-K1 CD155 blocking experiment | CHO-K1 human TIGIT(hig h expression strain) | Human TIGIT ECD-mFc binding experiment | CHO-K1 cynomolgu s monkey TIGIT binding experiment | Cynomolg us monkey TIGIT ECD-mFc binding experiment |
|---|---|---|---|---|---|---|---|---|---|
| | ka (1/Ms) | kd (1/s) | KD (M) | Fold change vs chimeric antibodies (KD) | IC50(nM) | EC50(nM) | EC50(nM) | EC50(nM) | EC50(nM) |
| TIGIT-002-H1L1 | no data | no data | no data | no data | 0.5258 | 0.0201 | 0.0155 | 0.1866 | 0.2087 |
| TIGIT-002-H1L2 | no data | no data | no data | no data | 0.4212 | 0.0167 | 0.0158 | 0.2206 | 0.0811 |
| TIGIT-002-H1L3 | 3.59E+06 | 9.73E-04 | 2.71E-10 | 6.44 | 0.3953 | 0.0253 | 0.0157 | 0.1429 | 0.0511 |
| TIGIT-002-H2L1 | no data | no data | no data | no data | 0.6459 | 0.0468 | 0.0188 | 0.1809 | 0.0877 |
| TIGIT-002-H2L2 | no data | no data | no data | no data | 0.5600 | 0.0215 | 0.0222 | 0.1198 | 0.0554 |
| TIGIT-002-H2L3 | 4.03E+06 | 1.07E-03 | 2.66E-10 | 6.30 | 0.5401 | 0.0278 | 0.0159 | 0.1498 | 0.0337 |
| TIGIT-002-H3L1 | no data | no data | no data | no data | 0.4593 | 0.0204 | 0.0120 | 0.2320 | 0.0253 |
| TIGIT-002-H3L2 | no data | no data | no data | no data | 0.3938 | 0.0329 | 0.0170 | 0.1134 | 0.0218 |
| TIGIT-002-H3L3 | 4.52E+06 | 8.52E-04 | 1.88E-10 | 4.47 | 0.3385 | 0.0361 | 0.0173 | 0.0919 | 0.0220 |
| TIGIT-002-H4L1 | no data | no data | no data | no data | 0.4449 | 0.0253 | 0.0164 | 0.0543 | 0.0206 |
| TIGIT-002-H4L2 | no data | no data | no data | no data | 0.4397 | 0.0294 | 0.0179 | 0.0474 | 0.0254 |
| TIGIT-002-H4L3 | 5.65E+06 | 3.00E-04 | 5.31E-11 | 1.26 | 0.3540 | 0.0249 | 0.0138 | 0.0473 | 0.0189 |
| TIGIT-CHI-002 | 7.13E+06 | 3.00E-04 | 4.21E-11 | 1.00 | 0.5635 | 0.0218 | 0.0161 | 0.0372 | 0.0200 |
| TIGIT-005-H1L1 | no data | no data | no data | no data | not binding | 0.0021 | 45632 | 0.0011 | 18459 |
| TIGIT-005-H2L1 | 4.26E+06 | 1.61E-04 | 3.78E-11 | 1.37 | 0.3211 | 0.0281 | 0.0141 | 0.0199 | 0.0145 |
| TIGIT-005-H2L2 | 4.33E+06 | 1.74E-03 | 4.02E-10 | 14.57 | 0.3530 | 0.0232 | 0.0110 | 0.0218 | 0.0107 |
| TIGIT-005-H3L1 | 4.62E+06 | 1.55E-04 | 3.35E-11 | 1.21 | 0.3931 | 0.0223 | 0.0144 | 0.0261 | 0.0151 |
| TIGIT-005-H4L1 | 2.07E+06 | 1.52E-04 | 7.33E-11 | 2.66 | 0.3845 | 0.0257 | 0.0124 | 0.0251 | 0.0136 |
| TIGIT-CHI-005 | 4.76E+06 | 1.13E-04 | 2.76E-11 | 1.00 | 0.3596 | 0.0260 | 0.0190 | 0.0265 | 0.0165 |
| TIGIT-005-H2L1 | 3.54E+06 | 2.63E-04 | 7.42E-11 | 1.00 | 0.3890 | 0.0272 | 0.0247 | 0.0470 | 0.0393 |
| TIGIT-005-H2L1a | 2.83E+06 | 1.05E-02 | 3.71E-09 | 50.01 | 0.7165 | 0.0129 | 0.0289 | 0.0561 | 0.0357 |
| TIGIT-005-H2L1b | 3.38E+06 | 2.48E-03 | 7.34E-10 | 9.89 | 0.3581 | 0.0279 | 0.0238 | 0.0368 | 0.0320 |
| TIGIT-005-H2L1c | 2.49E+06 | 4.59E-04 | 1.84E-10 | 2.48 | 0.3468 | 0.0246 | 0.0268 | 0.0673 | 0.0296 |
| TIGIT-005-H2L1d | 4.03E+06 | 3.61E-04 | 8.96E-11 | 1.21 | 0.3758 | 0.0439 | 0.0291 | 0.0566 | 0.0403 |
| TIGIT-006-H1L1 | 1.22E+05 | 7.57E-04 | 6.21E-09 | 80.27 | 1.5340 | 0.0702 | 0.0186 | not binding | not binding |
| TIGIT-006-H1L2 | 1.17E+05 | 6.66E-04 | 5.71E-09 | 73.78 | 1.5820 | 0.1049 | 0.0183 | not binding | not binding |
| TIGIT-006-H1L3 | 1.44E+05 | 3.84E-04 | 2.66E-09 | 34.43 | 1.2320 | 0.0596 | 0.0185 | not binding | not binding |
| TIGIT-006-H2L1 | 4.03E+05 | 3.82E-04 | 9.50E-10 | 12.28 | 0.5284 | 0.0383 | 0.0106 | 0.3421 | 2.8790 |
| TIGIT-006-H2L2 | 3.63E+05 | 3.24E-04 | 8.93E-10 | 11.54 | 0.9045 | 0.0444 | 0.0117 | 9.02E+14 | 3.3840 |
| TIGIT-006-H2L3 | 4.20E+05 | 2.16E-04 | 5.15E-10 | 6.65 | 0.6332 | 0.0353 | 0.0103 | 0.2553 | 24.4200 |
| TIGIT-006-H3L1 | 3.42E+05 | 2.41E-04 | 7.05E-10 | 9.11 | 0.6367 | 0.0468 | 0.0096 | weak binding | 2.8070 |
| TIGIT-006-H3L2 | 2.79E+05 | 2.08E-04 | 7.48E-10 | 9.66 | 0.7088 | 0.0494 | 0.0099 | 1.3810 | 7.4440 |
| TIGIT-006-H3L3 | 3.65E+05 | 1.76E-04 | 4.82E-10 | 6.23 | 0.6815 | 0.0317 | 0.0095 | 6.2950 | 0.7790 |
| TIGIT-006-H4L1 | 3.93E+05 | 2.58E-04 | 6.57E-10 | 8.49 | 0.7039 | 0.0349 | 0.0086 | 7.63E+09 | 1.5260 |
| TIGIT-006-H4L2 | 3.37E+05 | 2.21E-04 | 6.58E-10 | 8.50 | 0.6708 | 0.0251 | 0.0113 | weak binding | 0.9967 |
| TIGIT-006-H4L3 | 4.41E+05 | 1.88E-04 | 4.28E-10 | 5.52 | 0.6142 | 0.0316 | 0.0101 | 2.1050 | 0.7326 |
| TIGIT-006-H5L1 | 2.29E+06 | 1.63E-04 | 7.13E-11 | 0.92 | 0.5370 | 0.0399 | 0.0108 | 0.1615 | 0.0182 |
| TIGIT-006-H5L2 | 2.30E+06 | 1.84E-04 | 8.00E-11 | 1.03 | 0.5756 | 0.0239 | 0.0111 | 0.0849 | 0.0172 |
| TIGIT-006-H5L3 | 2.51E+06 | 1.60E-04 | 6.39E-11 | 0.83 | 0.5866 | 0.0220 | 0.0092 | 0.0578 | 0.0132 |
| TIGIT-CHI-006 | 2.09E+06 | 1.62E-04 | 7.74E-11 | 1.00 | 0.5789 | 0.0299 | 0.0152 | 0.1498 | 0.0258 |
| TIGIT-070-H1L1 | 1.98E+06 | 1.16E-04 | 5.83E-11 | 0.59 | 0.5025 | 0.0260 | 0.0149 | 0.0596 | 0.0123 |
| TIGIT-070-H1L2 | 1.67E+06 | 1.48E-04 | 8.83E-11 | 0.90 | 0.4206 | 0.0272 | 0.0118 | 0.0879 | 0.0116 |
| TIGIT-070-H1L3 | 1.78E+06 | 1.29E-04 | 7.24E-11 | 0.74 | 0.4451 | 0.0240 | 0.0148 | 0.1121 | 0.0137 |
| TIGIT-070-H2L1 | 1.66E+06 | 1.34E-04 | 8.08E-11 | 0.82 | 0.3927 | 0.0236 | 0.0129 | 0.0799 | 0.0124 |
| TIGIT-070-H2L2 | 1.67E+06 | 1.42E-04 | 8.51E-11 | 0.87 | 0.4478 | 0.0312 | 0.0125 | 0.0662 | 0.0112 |
| TIGIT-070-H2L3 | 1.77E+06 | 1.34E-04 | 7.56E-11 | 0.77 | 0.3504 | 0.0209 | 0.0119 | 0.0774 | 0.0119 |
| TIGIT-070-H3L1 | 1.26E+06 | 1.52E-04 | 1.21E-10 | 1.23 | 0.4630 | 0.0132 | 0.0131 | 0.0741 | 0.0139 |
| TIGIT-070-H3L2 | 1.30E+06 | 1.68E-04 | 1.29E-10 | 1.32 | 0.4233 | 0.0276 | 0.0143 | 0.0665 | 0.0157 |
| TIGIT-070-H3L3 | 1.31E+06 | 1.51E-04 | 1.15E-10 | 1.17 | 0.4166 | 0.0300 | 0.0151 | 0.0556 | 0.0142 |
| TIGIT-CHI-070 | 1.48E+06 | 1.45E-04 | 9.80E-11 | 1.00 | 0.6374 | 0.0330 | 0.0159 | 0.0741 | 0.0184 |

| | | | | | | | | | |
|---|---|---|---|---|---|---|---|---|---|
| No data: not tested | | | | | | | | | |

### EXAMPLE 6 - Identification of anti-TIGIT humanized antibodies

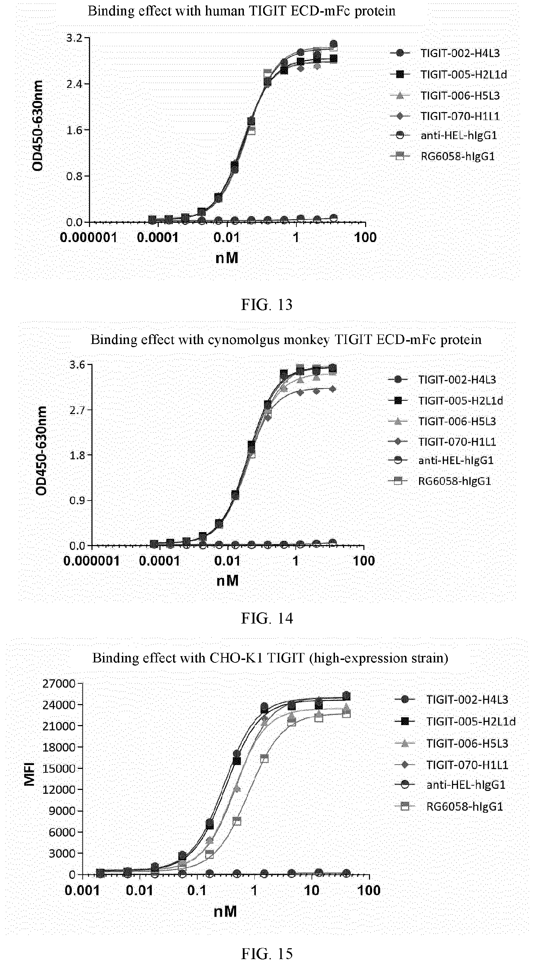

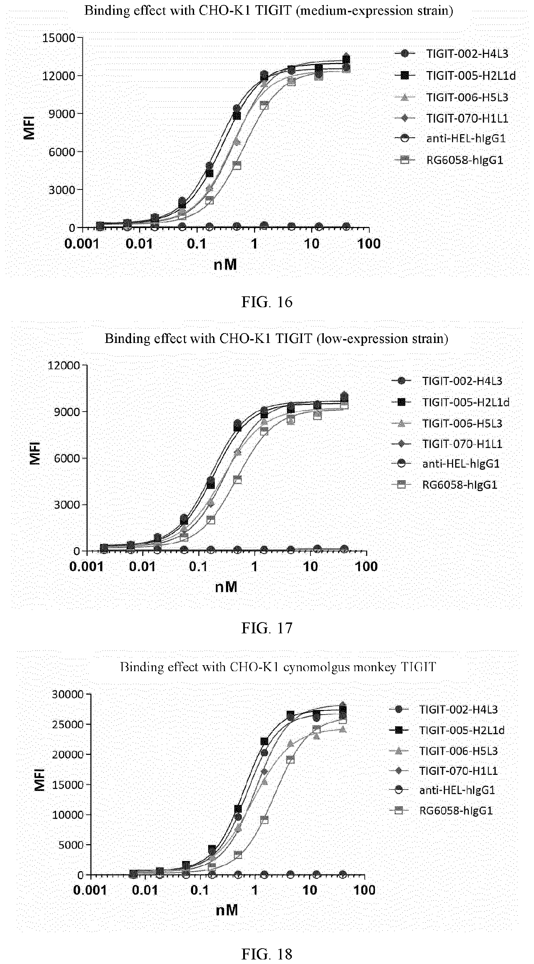

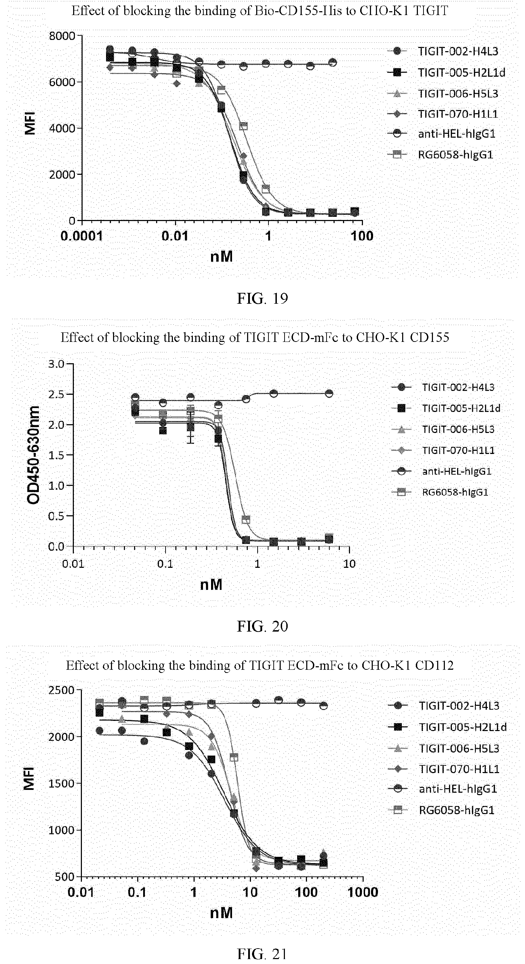

(a) Detection of the binding activity of anti-TIGIT humanized antibodies to human TIGIT ECD-mFc and cynomolgus monkey TIGIT ECD-mFc.
   The methods refer to Example 4 (a). The experimental results show that the anti-TIGIT humanized antibodies can effectively bind to human TIGIT ECD-mFc (Fig. 13) and cynomolgus monkey TIGIT ECD-mFc (Fig. 14), which is consistent with the control antibody, Roche RG6058.
(b) The binding activity of anti-TIGIT humanized antibodies to CHO-K1 human TIGIT (cells with high, medium and low expression levels) and CHO-K1 cynomolgus monkey TIGIT detected by FACS.
   The methods refer to Example 4 (b). The experimental results show that the anti-TIGIT humanized antibodies can effectively bind to CHO-K1 human TIGIT high expression cell strains (Fig. 15), CHO-K1 human TIGIT medium expression cell strains (Fig. 16), CHO-K1 human TIGIT low expression cell strains (Fig. 17) and CHO-K1 cynomolgus monkey TIGIT cell strains (Fig. 18), which is consistent with the control antibody, Roche RG6058.
(c) The effect of anti-TIGIT humanized antibodies blocking the interaction between Bio-CD155-His and CHO-K1 human TIGIT detected by FACS.
   The methods refer to Example 4 (c). As shown in Fig. 19, both the anti-TIGIT humanized antibodies and the control antibody (RG6058) can effectively block the binding between the Bio-CD155-His protein and CHO-K1- human TIGIT cells.
(d) The effect of anti-TIGIT humanized antibodies blocking the interaction between TIGIT ECD-mFc and CHO-K1 CD155 detected by FACS.
   The methods refer to Example 4 (d). As shown in Fig. 20, both the anti-TIGIT humanized antibodies and the control antibody (RG6058) can effectively block the binding between the human TIGIT protein and CHO-K1-CD155 cells.
(e) The effect of anti-TIGIT humanized antibodies blocking the interaction between TIGIT ECD-mFc and CHO-K1 CD112 detected by FACS.
   The collected CHO-K1 CD112 cells were washed once with PBS (Hyclone, CAT#SH30256) and resuspended to 2× 10⁵/40 µL with 1% BSA-PBS. The anti-TIGIT humanized antibodies were diluted to 600 nM with 1% BSA-PBS (diluted by 2.5-fold serial dilution for 12 concentration points). The TIGIT ECD-mFc was diluted with 1% BSA-PBS to 6 µg/mL. The 40 µL cells were then mixed with 40 µL diluted antibodies and 40 µL diluted TIGIT ECD-mFc, and then incubated at 4°C for 60 minutes. The cells were washed twice with PBS, resuspended with Alexa Fluor^{®} 647 fluorescein labeled secondary antibody (dilution ratio 1:800, Jackson, CAT# 109-605-003), 100 µL/well, and incubated at 4°C for 40 minutes. The cell samples were washed twice with PBS, resuspended to 100 µL/well with 1% BSA-PBS and then analyzed by flow cytometry (BD, Canto II). As shown in Fig. 21, both the anti-TIGIT humanized antibodies and the control antibody (RG6058) can effectively block the binding between the human TIGIT protein and CHO-K1-CD112 cells.
(f) The binding activity of anti-TIGIT humanized antibodies to human PBMCs detected by FACS.
   The fresh human PBMCs (AllCells, PB004-C) were adjusted to a density of 5×10⁵/mL, added with SEA (Toxin Technology, Inc., CAT: AT101) to 100 ng/mL, and cultured at 37°C with 5% CO₂ for 3 days. The cells were collected 3 days later, washed once with PBS (Hyclone, CAT#SH30256), added with Fc Block (BD, 564220) and then incubated at 4°C for 10 minutes. After washing twice with PBS, the PBMCs were resuspended to 2×10⁵ /50 µL with 1%BSA-PBS. The humanized antibodies were diluted to 80 nM with 1% BSA-PBS (diluted by 3-fold serial dilution for 12 concentration points). The 50 µL cells were then mixed with 50 µL diluted antibodies, and then incubated at 4°C for 60 minutes. The cells were washed twice with PBS, resuspended with Alexa Fluor^{®} 647 fluorescein labeled secondary antibody (dilution ratio 1:800, Jackson, CAT# 109-605-088), 100 µL/well, and incubated at 4°C for 60 minutes. After washing twice with PBS, the cell samples were resuspended with 1% BSA-PBS, 100 µL/welland then analyzed by flow cytometry (BD, Canto II). As shown in Fig. 22, the anti-TIGIT humanized antibodies can effectively bind to human PBMCs stimulated by SEA, which is consistent with the control antibody, Roche RG6058.

Table 19 summarizes the characteristics of the 4 humanized antibodies. The 4 humanized antibodies (TIGIT-002-H4L3, TIGIT-005-H2L1d, TIGIT-006-H5L3, and TIGIT-070-H1L1) have the same tendency to bind to PBMC and stable transgenic cells with high, medium and low expression levels of human TIGIT, wherein, TIGIT-002-H4L3 and TIGIT-005-H2L1d have a stronger binding activity than TIGIT-006-H5L3 and TIGIT-070-H1L1, but all humanized antibodies have a stronger binding activity than RG6058. Compared with RG6058 (0.1560 nM), the monovalent affinities of 4 humanized antibodies to the human TIGIT ECD-His are 0.0994 nM, 0.0852 nM, 0.1145 nM, 0.2505 nM, respectively, and they all have strong cross-reactivity with cynomolgus monkey TIGIT. Regarding the characterization of blocking effect in vitro, the 4 humanized antibodies all show significant ability to block the interaction between TIGIT-CD155 and TIGIT-CD112.

**Table 19. Biological activities of the four humanized antibodies, TIGIT-002-H4L3, TIGIT-005-H2L1d, TIGIT-006-H5L3 and TIGIT-070-H1L1**

| TIGIT humanized antibodies | Human TIGIT ECD-His | | | Human TIGIT ECD-mF c/CHO-K1 CD155 blocking experime nt | Bio-C D155-His /CHO-K1 human TIGIT blockin g experi ment | Human TIGIT ECD-m Fc/ CHO-K 1 CD 112 blockin g experim ent | Huma n PBM C bindi ng exper iment | CHO-K1 human TIGIT (high expres sion strain) | CHO-K1 human TIGIT (mediu m expres sion strain) | CHO-K1 huma n TIGI T(low expre ssion strain ) | Huma n TIGI T ECD-mFc bindi ng exper iment | CHO-K1 cyno molg us monk ey TIGI T bindi ng exper iment | Cyno molg us monk ey TIGI T ECD-mFc bindi ng exper iment |
|---|---|---|---|---|---|---|---|---|---|---|---|---|---|
| | ka (1/Ms) | kd (1/s) | KD (M) | IC50 (nM) | IC50 (nM) | IC50 (nM) | EC50 (nM) | EC50 (nM) | EC50 (nM) | EC50 (nM) | EC50 (nM) | EC50 (nM) | EC50 (nM) |
| TIGIT-002-H4L3 | 2.95E+06 | 2.93E-04 | 9.94E-11 | 0.4919 | 0.1373 | 3.4930 | 0.0436 | 0.2970 | 0.2282 | 0.1696 | 0.0370 | 0.7234 | 0.0427 |
| TIGIT-005-H2L1d | 3.02E+06 | 2.57E-04 | 8.52E-11 | 0.4666 | 0.1501 | 3.4880 | 0.0602 | 0.3210 | 0.2848 | 0.1856 | 0.0311 | 0.6315 | 0.0395 |
| TIGIT-006-HSL3 | 1.60E+06 | 1.83E-04 | 1.15E-10 | 0.4583 | 0.1714 | 4.6480 | 0.0916 | 0.4430 | 0.3963 | 0.2677 | 0.0319 | 0.9222 | 0.0406 |
| TIGIT-070-HIL1 | 8.75E+05 | 2.19E-04 | 2.51E-10 | 0.4558 | 0.2123 | 4.3030 | 0.1211 | 0.4945 | 0.4500 | 0.3048 | 0.0284 | 1.0490 | 0.0347 |
| RG6058-hIgG1 | 8.66E+05 | 1.35E-04 | 1.56E-10 | 0.5840 | 0.3340 | 60000 | 0.2849 | 0.7945 | 0.6523 | 0.4807 | 0.0393 | 2.2850 | 0.0486 |

(g) The promoting effect of anti-TIGIT humanized antibodies on cytotoxicity for target cells by NK cells detected by NK cell cytotoxicity assay.

The methods refer to Example 4 (d). Figure 23 shows that the negative control, anti-HA HcAb-hIgG1, has no significant effect on the NK cell cytotoxicity. The 4 tested humanized antibodies (TIGIT-002-H4L3, TIGIT-005-H2L1d, TIGIT-006-H5L3 and TIGIT-070-H1L1) can promote the NK cell cytotoxicity for the target cells (WIDR) to varying degrees, wherein, TIGIT-002-H4L3 and TIGIT-005-H2L1d show the same effect on NK cell cytotoxicity as pre-humanized chimeric antibody TIGIT-CHI-002 and TIGIT-CHI-005.

(h) The effect of anti-TIGIT humanized antibody on improving the functions of antigen-specific CD8 T cells detected by CMV antigen-recall assay

The PBMCs were resuscitated, resuspended to 2×10⁶/mL by using complete medium (RPMI1640-Glutamax + 5% AB serum + 1% P/S+(1×) 2-β mercaptoethanol) containing 1 mg/mL CMV pp65(495-503) peptide (Anaspec, Cat No. AS-28328), 2 ng/mL human IL-2 (R&D, Cat No. IL-202) and 10 ng/mL human IL-7 (Peprotech, Cat No. 200-07), inoculated in a 6-well plate (5 mL/well) and incubated at 37°C with 5% CO₂ for 6 days. On day 6, all the PBMCs were collected, and pp65 and IL-7 in the medium were removed. The cells were divided into two portions, resuspended in complete medium containing 100 IU/mL human IL-2, and cultured for another 2 days. On day 8, all the PBMCs were collected and resuspended in complete medium containing 100 IU/mL human IL-2, and the cell density was adjusted to 2×10⁶/mL for continuing cultivation. On day 11, all the PBMCs were collected. The percentage of CD8 T cells and CMV pp65 (495-503) specific CD8 T cells in PBMCs (Figure 24, panel A), and the expression of PVRIG, TIGIT, PD-1 and CD226 on CMV pp65 (495-503) specific CD8 T cells (Figure 24, panel B) were detected by flow cytometry. The detection antibodies were as follows: Livedead Near IR (Invitrogen, Cat No. L34976), CD8-PerCp Cy5.5 (BD, Cat No. 565310), CD3-PE-Cy7 (Biolegend, Cat No. 300316), T-select HLA-A*0201 CMV pp65 Tetramer-PE (MBL, Cat No. TS-0010-1C), PVRIG-AF488 (R&D, Cat No. FAB93651G-100UG), TIGIT-APC (Biolegend, Cat No. 372706) and PD-1-BV421 (BD, Cat No. 562516).

CD8 T cells were isolated from the induced PBMCs as effector cells by CD8 T cell isolation kit (Stemcell, Cat No. 17953) and resuspended with AIM-V to the cell density of 0.4×10⁶/mL. The purity of isolated CD8 T cells and the expression of CD226 on them were detected. The target cells, Colo205, were digested by TrypLE^{™} Express Enzyme (Gibco, Cat No. 12605010), resuspended in AIM-V (Gibco, Cat No. 31035-025) containing 20 ng/mL pp65 to the cell density of 1×10⁶/mL and treated at 37°C with 5% CO₂ for 3 hours. The target cells were then centrifuged at 250 g for 5 minutes and resuspended in AIM-V to the cell density of 0.5×10⁶/mL after discarding the supernatant. Anti-TIGIT antibodies or positive control were diluted with AIM-V to 280 nM. The low-attachment, 96-well, U-bottom plate (Corning, Cat No.7007) was added with 50 µL of antibody, 50 µL of CD8 T and 100 µL of pp65-treated colo205 in order, mixed well with a multichannel pipette, and incubated at 37°C with 5% CO₂ for 18 hours. In the experimental system, the final concentration of antibodies was 70 nM, CD8 T cells were 20,000/well, and colo205 were 50,000/well. After incubation, the supernatant was collected by centrifugation at 400 g, and the level of human IFN-γ in the supernatant was detected with an ELISA kit (Dakewe, Cat No.1110003). In this system, the positive controls were RG6058-hIgG1 and TIGIT pre-humanized antibody (TIGIT-CHI-002), and the negative control was no treatment. As shown in Fig. 24 (panel C), the secretion level of IFN-γ in the cell supernatant treated with 4 TIGIT humanized antibodies (TIGIT-002-H4L3, TIGIT-005-H2L1d, TIGIT-006-H5L3 and TIGIT-070-H1L1) has no statistically significant difference (One-way ANOV Analysis) compared with RG6058-hIgG1; the secretion level of IFN-γ in the cell supernatant has no statistically significant difference (One-way ANOV Analysis) compared with TIGIT pre-humanized antibody (TIGIT-CHI-002), but was significantly higher than the no treatment group. The percentage on each histogram represents the percentage of IFN-γ secretion increased in the experimental group compared with no treatment group.

The purity of isolated CD8 T cells and the expression of CD226 on them were detected by flow cytometry using following detection antibodies: livedead-BV421 (Invitrogen, Cat No. L34964), CD8-FITC (BD, Cat No. 555366) and CD226-PE-Cy7 (Biolegend, Cat No. 338316). The expression of PVRL2 and PVR on colo205 was detected by flow cytometry using following detection antibodies: livedead-BV421 (Invitrogen, Cat No. L34964), PVRL2-APC (Biolegend, Cat No. 337412), PVR-PerCp Cy5.5 (Biolegend, Cat No. 337612), PD-L1- PE-Cy7 (BD, Cat No. 558017) and HLA-A2-PE (Biolegend, Cat No. 343306).

### EXAMPLE 7 - Preparation of PVRIG Alpaca VHH Antibody

Two healthy adult alpacas (Alpaca) (purchased by Chengdu Apack Company) were injected with complete freund's adjuvant (CFA, from SIGMA, Cat No. F5881) mixed with human PVRIG recombinant protein (Acro Biosystems, Cat No. PVG H5257) for the first immunization, and with incomplete freund's adjuvant (IFA, from SIGMA, Cat No. F5506) mixed with the same human PVRIG recombinant protein for the last three immunizations, all of which were injected subcutaneously for four times. Before immunization, 10 mL of blood was taken as negative serum control, after the second immunization, 10 mL of blood was taken to detect serum antibody titer, and after the third and fourth immunization, 50 mL of peripheral blood was taken to separate lymphocytes. According to the amount of lymphocytes, 5 mL RNA iso Plus (Takara, Cat No. 9109) was added and stored in 1.5 mL EP tube at -80 °C. Total RNA was extracted from cryopreserved lymphocytes and reverse transcribed into cDNA, and then two rounds of VHH PCR amplification were carried out using cDNA as template. The VHH products amplified by the second round of PCR were digested to construct a phage library. The established bacterial bank was collected, and the insertion rate and diversity of the bank were sequenced and analyzed.

Two rounds of affinity panning were performed on the phage, and the phage clones that specifically bound to the target protein human PVRIG-his (AcroBiosystems, Cat No. PVG-H52H4) were identified. The optimized clones with better binding to human PVRIG-His protein were sequenced and cloned into the expression vector by homologous recombination method. The CH2 and CH3 constant regions were both derived from human IgG1, and the complete expression sequence was signal peptide-VHH-hinge-CH2-CH3. After a series of physicochemical and functional screening, a total of 13 positive candidate antibody molecules were obtained, and the CDRs of their sequences were analyzed by IMGT and KABAT respectively. The corresponding sequence information is shown in Tables 20 to 22 below. Table 20 shows the VHH sequence of candidate antibody molecules, Table 21 shows the IMGT analysis results of candidate antibody molecules, and Table 22 shows the KABAT analysis results of candidate antibody molecules.

**Table 20 VHH Sequence of Candidate Antibody Molecules**

| Name | SEQ ID | sequence |
|---|---|---|
| PVRIG-A11 | SEQ ID NO. 107 | QLQLVESGGGLVQPGGSLRLSCAASGFTDDYYAIGWFRQAPGKE REGVSCISGSGGSTNYEDSVKGRFAISRDNAKNMVYLQMNDLKP EDTAVYYCAADPFWSAPCTGHNDRYFDVWGQGTLVTVSS |
| PVRIG-A15 | SEQ ID NO. 108 | QVQLVESGGGLVQAGGSLRLSCVASGRTFSANTMAWFRQAPGKE REFVAYTRWTDDNTDYASYADFVKGRFTAFRDNTRNTMYLQMN RLRPEDTAVYYCAAATTRGTYYSAGDYNSWGQGTQVTVSS |
| PVRIG-A30 | SEQ ID NO. 109 | QVQLVESGGGLVQPGGSLRLSCAASGRTDSMNVMGWFRRAPGK EREFVARIKWNGDTTYTAYADFLKGRFTLYGAVARNRVYLQMNS LQPEDTAVYYCAAGEVSGSSYSPDYGMHYWGKGTLVTVSS |
| PVRIG-A50 | SEQ ID NO. 110 | EVQLVESGGGLVQPGGSLRLSCAASGFTFSYYDMSWVRQAPGKG LEWVSTINSNGGRTSYVDSVKGRFTISRDNTKNTLYLQMNSLKPE DTAVYYCVEGDPHNFGLENLSLRDFGSWGQGTQVTVSS |
| PVRIG-A60 | SEQ ID NO. 111 | QVQLVESGGGLVQSGGSLRLSCAASGRTRSMYVMGWFRQAPGK EREFVGRIKWSGDTTYTSYADFVKGRFTLYGNQARNTVYLQMNS LKPEDAAVYYCNLRRLDGVNYWGKGTLVTVSS |
| PVRIG-A75 | SEQ ID NO. 112 | QLQLVESGGGLVQSGGSLRLSCAASGRTRSMYVMGWFRQAPGK EREFVGRIKWSGDTTYTSYADFVKGRFTLYGNQARNTVYLQMNS LKPEDTAVYSCAASKFPVSGVPEHYDYWGQGTQVTVSS |
| PVRIG-A35 | SEQ ID NO. 113 | EVQVVESGGGLVQSGGSLRLSCAASGRTRSMYVMGWFRQAPGK EREFVGRIKWSGDTTYTSYADFVKGRFTLYGNQARNTVYLQMNS |
| | | LKPEDTAVYYCNGRSRFNVINAWGTGTLVTVAS |
| PVRIG-A43 | SEQ ID NO. 114 | QVQLVESGGGLVQSGGSLRLSCAASGRTRSMYVMGWFRQAPGK EREFVGRIKWSGDTTYTSYADFVKGRFTLYGNQARNTVYLQMNS LKVEDTAVYYCAGRVVPLPSRQRDRYDYWGQGTQVTVSS |
| PVRIG-A104 | SEQ ID NO. 115 | EVQVVESGGGLVQAGGSLRLSCAASGRTRSMYVMGWFRQAPGK EREFVGRIKWSGDTTYTSYADFVKGRFTLYGNQARNTVYLQMNS LKPEDTAVYYCYRRSWTNTKLYWGKGIQVTVSS |
| PVRIG-A105 | SEQ ID NO. 116 | EVQLVESGGGLVQAGGSLRLSCAASGRTFDRHTMTWFRQAPGKE REFVATASRIPGDTYYSHSVKGRFTISRDNAKNTVYLQLNNLKPED TAVYYCAATSAYCSEVDCYEKGSWYDNWGQGIQVTVSS |
| PVRIG-A113 | SEQ ID NO. 117 | QLQLVESGGGLVQSGGSLRLSCAASGRTRSMYVMGWFRQAPGK EREFVGRIKWSGDTTYTSYADFVKGRFTLYGNQARNTVYLQMNS LKPEDTAVYYCAALPSDYDYRAASYGVDYWGKGTLVTVSS |
| PVRIG-A117 | SEQ ID NO. 118 | EVQVVESGGGLVQAGGSLRLSCTASEHIYDLYIMGWYRQAPGKD RELVATITYTGSIYIADSVKDRFTISRGNAKNTVSLQMSGLKPEDTA VYYCNADPSGLGRKLYWGQGTQVTVSS |
| PVRIG-A118 | SEQ ID NO. 119 | QVQLVESGGGLVQAGGSLRLSCAASETYFDLYVMGWYRQAPGK DRELVATITYTGSIKIVDSVKDRFTISRGDAKNTVSLQMSSLKPEDT AVYFCNADPSGLGRK VYWGQGTQVTVSS |

**Table 21 IMGT Analysis Results of Candidate Antibody Molecules**

| Name | CDR1-IMGT | CDR2-IMGT | CDR3-IMGT |
|---|---|---|---|
| PVRIG-A11 | GFTDDYYA SEQ ID NO. 120 | ISGSGGST SEQ ID NO. 121 | AADPFWSAPCTGHNDRYFDV SEQ ID NO. 122 |
| PVRIG-A15 | GRTFSANT SEQ ID NO. 123 | TRWTDDNTDYA SEQ ID NO. 124 | AAATTRGTYYSAGDYNS SEQ ID NO. 125 |
| PVRIG-A30 | GRTDSMNV SEQ ID NO. 126 | IKWNGDTTYT SEQ ID NO. 127 | AAGEVSGSSYSPDYGMHY SEQ ID NO. 128 |
| PVRIG-A50 | GFTFSYYD SEQ ID NO. 129 | INSNGGRT SEQ ID NO. 130 | VEGDPHNFGLENLSLRDFGS SEQ ID NO. 131 |
| PVRIG-A60 | GRTRSMYV SEQ ID NO. 132 | IKWSGDTTYT SEQ ID NO. 133 | NLRRLDGVNY SEQ ID NO. 134 |
| PVRIG-A75 | GRTRSMYV SEQ ID NO. 135 | IKWSGDTTYT SEQ ID NO. 136 | AASKFPVSGVPEHYDY SEQ ID NO. 137 |
| PVRIG-A35 | GRTRSMYV SEQ ID NO. 138 | IKWSGDTTYT SEQ ID NO. 139 | NGRSRFNVINA SEQ ID NO. 140 |
| PVRIG-A43 | GRTRSMYV SEQ ID NO. 141 | IKWSGDTTYT SEQ ID NO. 142 | AGRVVPLPSRQRDRYDY SEQ ID NO. 143 |
| PVRIG-A104 | GRTRSMYV SEQ ID NO. 144 | IKWSGDTTYT SEQ ID NO. 145 | YRRSWTNTKLY SEQ ID NO. 146 |
| PVRIG-A105 | GRTFDRHT SEQ ID NO. 147 | ASRIPGDT SEQ ID NO. 148 | AATSAYCSEVDCYEKGSWYDN SEQ ID NO. 149 |
| PVRIG-A113 | GRTRSMYV SEQ ID NO. 150 | IKWSGDTTYT SEQ ID NO. 151 | AALPSDYDYRAASYGVDY SEQ ID NO. 152 |
| PVRIG-A117 | EHIYDLYI SEQ ID NO. 153 | ITYTGSI SEQ ID NO. 154 | NADPSGLGRKLY SEQ ID NO. 155 |
| PVRIG-A118 | ETYFDLYV SEQ ID NO. 156 | ITYTGSI SEQ ID NO. 157 | NADPSGLGRKVY SEQ ID NO. 158 |

**Table 22 KABAT Analysis Results of Candidate Antibody Molecules**

| Name | CDR1-KABAT | CDR2-KABAT | CDR3-KABAT |
|---|---|---|---|
| PVRIG-A11 | YYAIG SEQ ID NO. 159 | CISGSGGSTNYEDSVKG SEQ ID NO. 160 | DPFWSAPCTGHNDRYFDV SEQ ID NO. 161 |
| PVRIG-A15 | ANTMA SEQ ID NO. 162 | YTRWTDDNTDYASYADFVKG SEQ ID NO. 163 | ATTRGTYYSAGDYNS SEQ ID NO. 164 |
| PVRIG-A30 | MNVMG SEQ ID NO. 165 | RIKWNGDTTYTAYADFLKG SEQ ID NO. 166 | GEVSGSSYSPDYGMHY SEQ ID NO. 167 |
| PVRIG-A50 | YYDMS SEQ ID NO. 168 | TINSNGGRTSYVDSVKG SEQ ID NO. 169 | GDPHNFGLENLSLRDFGS SEQ ID NO. 170 |
| PVRIG-A60 | MYVMG SEQ ID NO. 171 | RIKWSGDTTYTSYADFVKG SEQ ID NO. 172 | RRLDGVNY SEQ ID NO. 173 |
| PVRIG-A75 | MYVMG SEQ ID NO. 174 | RIKWSGDTTYTSYADFVKG SEQ ID NO. 175 | SKFPVSGVPEHYDY SEQ ID NO. 176 |
| PVRIG-A35 | MYVMG SEQ ID NO. 177 | RIKWSGDTTYTSYADFVKG SEQ ID NO. 178 | RSRFNVINA SEQ ID NO. 179 |
| PVRIG-A43 | MYVMG SEQ ID NO. 180 | RIKWSGDTTYTSYADFVKG SEQ ID NO. 181 | RVVPLPSRQRDRYDY SEQ ID NO. 182 |
| PVRIG-A104 | MYVMG SEQ ID NO. 183 | RIKWSGDTTYTSYADFVKG SEQ ID NO. 184 | RSWTNTKLY SEQ ID NO. 185 |
| PVRIG-A105 | RHTMT SEQ ID NO. 186 | TASRIPGDTYYSHSVKG SEQ ID NO. 187 | TSAYCSEVDCYEKGSWYDN SEQ ID NO. 188 |
| PVRIG-A113 | MYVMG SEQ ID NO. 189 | RIKWSGDTTYTSYADFVKG SEQ ID NO. 190 | LPSDYDYRAASYGVDY SEQ ID NO. 191 |
| PVRIG-A117 | LYIMG SEQ ID NO. 192 | TITYTGSIYIADSVKD SEQ ID NO. 193 | DPSGLGRKLY SEQ ID NO. 194 |
| PVRIG-A118 | LYVMG SEQ ID NO. 195 | TITYTGSIKIVDSVKD SEQ ID NO. 196 | DPSGLGRKVY SEQ ID NO. 197 |

### EXAMPLE 8 - The specific binding of PVRIG antibodies to human and cynomolgus monkey PVRIG protein detected by ELISA

The ELISA plate was pre-coated with 100 µL/well of 0.5 µg/mL human PVRIG-his (AcroBiosystems, Cat NO.PVG-H52H4) or cynomolgus monkey PVRIG (Novoprotein, Cat NO.C09B). The tested PVRIG antibodies (constructed by connecting human IgG1-Fc with VHH described in Example 7) were serially diluted (initial concentration 20 nM, 3.33-fold serial dilution, or 3nM, 3-fold serial dilution), added into the plate (100 µL/well) and incubated with shaking at room temperature for 1.5 hours. After washing the plate, the mouse anti-human IgG Fc-HRP (Jackson ImmunoResearch, Cat NO.209-035-098)) working solution (dilution ratio 1:10000, 100 µL/well) were added into wells and incubated with shaking at room temperature for 1.0 hour. A HRP substrate, TMB (Thermo, Cat NO.34029) was added into wells after washing the plate again for color development. After adding a termination solution to terminate the reaction, the absorbance value was read by a microplate reader (MD i3x). An antibody binding curve was drawn with concentration of antibodies as the abscissa and the corresponding OD value as the ordinate. The EC50 value was calculated by using a Four Parameter Logistic Fit (GraphPadPrism9). The smaller the EC50 value, the stronger the ability of the antibodies binds to human or cynomolgus monkey PVRIG. The positive control antibodies are COM701-hIgG1 (Patent No.:US20180244774A1), COM701-hIgG4 (patent No.: US20180244774A1) and SRF813-hIgG1 (Patent No.: US20200040081A1); and the negative control antibodies are anti-HA HcAb-hIgG1 (Chengdu NB Biolab, Cat NO. NBR022), anti-CD38 HcAb-hIgG1 (in-house) and anti-Fluorescein-hIgG1 (in house).

The corresponding amino acid sequences of COM701 are as follows:
COM701 VH SEQ ID NO 236: 
QVQLVQSGAEVKKPGASVKVSCKASGYTFTDYNINWVRQAPGQGLEWMGYIYPYIGGS
GYAQKFQGRVTMTRDTSTSTVYMELSSLRSEDTAVYYCAREDKTARNAMDYWGQGTL
VTVSS
COM701 VL SEQ ID NO 237: 
DIQMTQSPSSLSASVGDRVTITCRVSENIYSNLAWYQQKPGKAPKLLIYEATNLAEGVPS
RFSGSGSGTDFTLTISSLQPEDFATYYCQHFWGTPYTFGQGTKLEIK
SRF813 VH SEQ ID NO 238: 
QVQLVQSGAEVKKPGSSVKVSCKASGGTFSSAAISWVRQAPGQGLEWMGNIIPIVGIAN
YAQKFQGRVTITADESTSTAYMELSSLRSEDTAVYYCARDTGRGYTRHFWFDPWGQGTL
VTVSS
SRF813 VL SEQ ID NO 239:

DIQMTQSPSSLSASVGDRVTITCRASQSISSYLNWYQQKPGKAPKLLIYAASSLQSGVPSR
FSGSGSGTDFTLTISSLQPEDFATYYCQQSDILYTFGGGTKVEIK

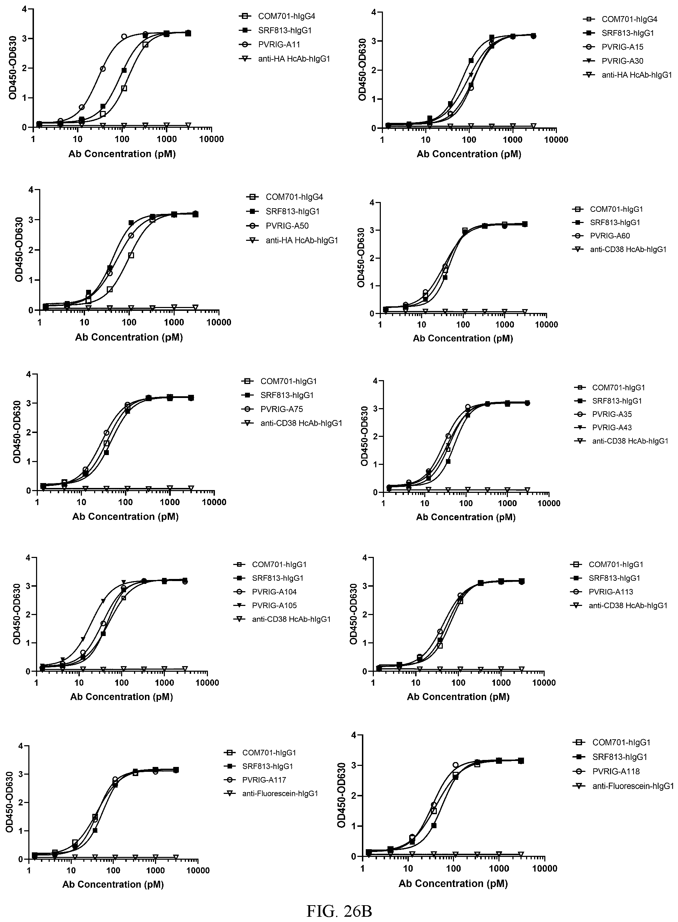

The binding results of the 13 anti-PVRIG antibodies to human PVRIG protein are shown in Fig. 26A and Table 23, and the binding results to cynomolgus monkey PVRIG are shown in Fig. 26B and Table 23. The data shows that all test antibodies can specifically bind to human or cynomolgus monkey PVRIG protein.

**Table 23. The specific binding of anti-PVRIG antibodies to human or cynomolgus monkey PVRIG protein detected by ELISA**

| Name | EC50 (pM) | |
|---|---|---|
| | Bind to human PVRIG protein | Bind to cynomolgus monkey PVRIG protein |
| PVRIG-A11 | 121.40 | 28.91 |
| PVRIG-A15 | 207.70 | 128.60 |
| PVRIG-A30 | 254.70 | 93.00 |
| PVRIG-A50 | 195.70 | 53.35 |
| PVRIG-A60 | 55.66 | 34.29 |
| PVRIG-A75 | 62.11 | 28.83 |
| PVRIG-A35 | 54.31 | 28.83 |
| PVRIG-A43 | 53.65 | 35.99 |
| PVRIG-A104 | 65.93 | 35.33 |
| PVRIG-A105 | 57.07 | 19.24 |
| PVRIG-A113 | 92.00 | 44.31 |
| PVRIG-A117 | 52.84 | 41.81 |
| PVRIG-A118 | 51.35 | 34.10 |

### EXAMPLE 9 - The binding of anti-PVRIG antibodies to FlpinCHO-PVRIG and FlpinCHO-cyno PVRIG detected by FACS.

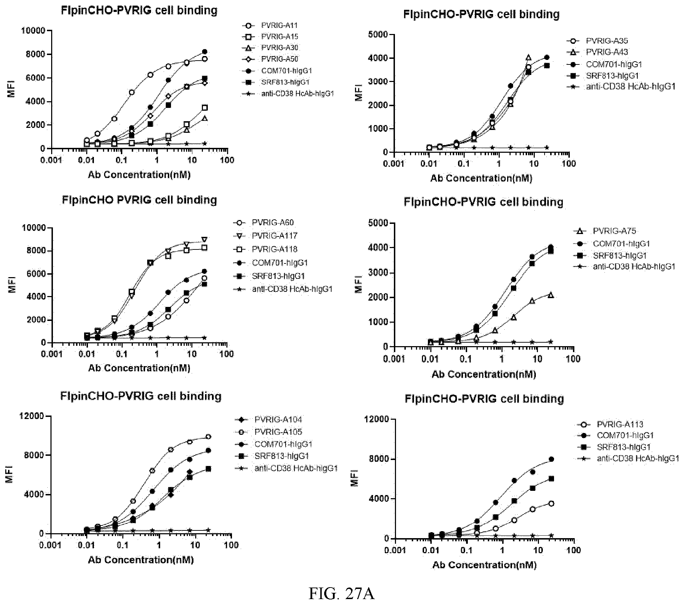

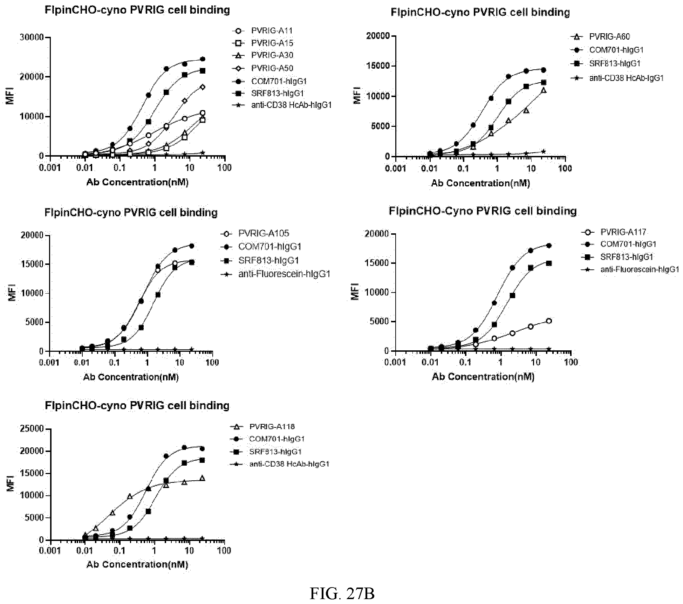

CHO-K1 stable cells transfected with human or cynomolgus monkey PVRIG high expression plasmids were named FlpinCHO-PVRIG and FlpinCHO-cyno PVRIG. Human PVRIG full-length plasmids (NCBI Ref Seq: NP_076975) and cynomolgus monkey PVRIG full-length plasmid (NCBI Ref Seq: XP_014989941) were synthesized by General Biol. The experiment was performed when the cell density did not exceed 80%. After discarding the cell culture medium, the cells were rinsed with PBS and digested for 8-10 minutes by adding 1mL Versene (Gibico, 15040-066). The Ham's F12 (Gibico, 21127-022) complete medium containing 10% FBS was then added to terminate the digestion and acquire cell suspension. After counting with a cell counter (Beckman Coulter, Vi-CELL), an appropriate amount of cell suspension was centrifuged at 350×g to remove the supernatant, washed twice with PBS, stained with Zombie violet (Biolegend, 423114) and incubated at room temperature for 20 minutes. After incubation, the cells were added with staining buffer (2% FBS+PBS) to stop staining, centrifuged at 350×g to remove the supernatant, washed twice and resuspended with staining buffer to a density of 2×10⁶ cells/mL. The cells were plated into a 96-well plate (50µL of cell suspension per well) for further use. The antibodies were diluted from the highest concentration of 46 nM (two-fold concentration) with staining buffer by 3.3-fold serial dilution. The diluted antibodies were added to the well containing 50 µL of cell suspension. The plate was placed on a microplate shaker at 400 rpm for 1 minute to fully mix the antibodies and cells, and then incubated at 4°C for 30 minutes. After the incubation, the cells were washed twice with staining buffer (200 µL per well) and centrifuged at 350×g for 5 minutes to discard the supernatant. The PE goat anti-human IgG Fc antibody (ebioscience, 12-4998-82) was diluted 250 times with staining buffer, added into the washed wells (100 µL per well), mixed well, and stained at 4°C for 30 minutes. After staining, the cells were washed twice with staining buffer, finally resuspended with 200 µL staining buffer and analyzed by flow cytometry (BD, Canto II). The stronger the signal, the stronger the ability of the antibodies binds to PVRIG. An antibody binding curve was drawn with concentration of antibodies as the abscissa and the corresponding Mean Fluorescence Intensity (MFI) as the ordinate. The AUC value of the curve was calculated by using a Four Parameter Logistic Fit (GraphPadPrism9). The stronger the AUC value, the stronger the ability of the antibodies binds to FlpinCHO-PVRIG and FlpinCHO-cyno PVRIG. Fig. 27A and 27B show that all the test antibodies bind to human/cynomolgus monkey PVRIG on the surface of overexpressing cells. The binding activity of the antibodies was normalized to the positive control COM701-hIgG1 and SRF813-hIgG1. The higher the percentage value, the stronger the binding ability of the antibodies. Table 24 shows that the binding activity of the tested antibodies PVRIG-A11, A35, A43, A105, A117 and A118 to human PVRIG is stronger than that of COM701-hIgG1 and SRF813-hIgG1, and the binding activity of PVRIG-A105 and A117 to cynomolgus monkey PVRIG is stronger than COM701-hIgG1 and SRF813-hIgG1.

**Table 24. Binding of anti-PVRIG antibodies to human/cynomolgus monkey PVRIG on the surface of overexpressing cells**

| Name | human PVRIG binding | | cyno PVRIG binding | |
|---|---|---|---|---|
| | %AUC | | %AUC | |
| | to COM701-hIgG1 | to SRF813-hIgG1 | to COM701-hIgG1 | to SRF813-hIgG1 |
| PVRIG-A11 | 116.77 | 142.90 | 41.30 | 48.56 |
| PVRIG-A15 | 36.32 | 42.69 | 24.80 | 29.15 |
| PVRIG-A30 | 29.30 | 34.44 | 28.73 | 33.78 |
| PVRIG-A50 | 89.54 | 114.99 | 59.31 | 69.73 |
| PVRIG-A60 | 63.30 | 68.58 | 63.40 | 82.88 |
| PVRIG-A75 | 50.64 | 54.46 | 47.58 | 62.40 |
| PVRIG-A35 | 121.76 | 133.55 | 19.27 | 25.28 |
| PVRIG-A43 | 165.18 | 181.18 | 19.62 | 25.73 |
| PVRIG-A104 | 18.07 | 23.94 | 18.45 | 23.87 |
| PVRIG-A105 | 122.51 | 162.31 | 121.63 | 157.39 |
| PVRIG-A113 | 42.38 | 57.40 | 38.63 | 52.69 |
| PVRIG-A117 | 133.29 | 180.80 | 127.75 | 174.25 |
| PVRIG-A118 | 152.73 | 232.45 | 66.41 | 80.43 |

### EXAMPLE 10 - The affinity of anti-PVRIG antibodies for human PVRIG proteins detected by BIAcore

Biacore was used to detect the specific binding between anti-PVRIG antibody and human PVRIG protein. Protein A chip was used in the BIAcore assay. The time required for the chip to capture the diluted antibody and saturate the binding antigen to reach Rₘₐₓ (Maximum binding capacity, 50 RU) was measured by manual run. The human PVRIG proteins (Human PVRIG-His, Aero C227P1-9ARF1-T4) were serially diluted to 20, 10, 5, 2.5, 1.25 nM. The affinity of antibody for antigen was measured by multi-cycle kinetics. In each cycle, the antibodies were injected prior to the injection of gradient concentraions of PVRIG proteins allowing the occurrence of antibody-antigen association and dissociation After each cycle, the Protein A chip was regenerated by Glycine, pH 1.5 (to remove the proteins on the chip). The affinity of antibody for antigen was calculated by BIAcore T200 analysis software. As shown in Table 25, all the anti-PVRIG antibodies specifically bind to human PVRIG proteins with high affinity.

**Table 25. BIACore results of specific binding of anti-PVRIG antibodies for human PVRIG proteins**

| **Name** | **Antigen** | **KD (M)** | **ka (1/Ms)** | **kd (1/s)** |
|---|---|---|---|---|
| PVRIG-A11 | Human PVRIG | 1.06E-10 | 3.18E+07 | 3.38E-03 |
| PVRIG-A15 | | 3.77E-10 | 6.12E+04 | 2.31E-05 |
| PVRIG-A30 | | 4.04E-10 | 1.57E+05 | 6.36E-05 |
| PVRIG-A50 | | 2.54E-11 | 5.07E+05 | 1.29E-05 |
| PVRIG-A60 | | 2.83E-10 | 1.41E+05 | 3.97E-05 |
| PVRIG-A75 | | 5.31E-10 | 1.07E+05 | 5.67E-05 |
| PVRIG-A35 | | 8.46E-10 | 8.90E+03 | 7.53E-06 |
| PVRIG-A43 | | 3.66E-10 | 2.34E+04 | 8.58E-06 |
| PVRIG-A104 | | 2.76E-11 | 1.54E+05 | 4.24E-06 |
| PVRIG-A105 | | 8.41E-10 | 5.27E+05 | 4.43E-04 |
| PVRIG-A113 | | 2.03E-10 | 4.42E+05 | 8.96E-05 |
| PVRIG-A117 | | 3.67E-10 | 3.74E+06 | 1.37E-03 |
| PVRIG-A118 | | 4.80E-10 | 2.38E+06 | 1.14E-03 |
| COM701-hIgG1 | | 3.19E-10 | 2.04E+06 | 6.51E-04 |
| SRF813-hIgG1 | | 2.78E-10 | 1.14E+06 | 3.17E-04 |

### EXAMPLE 11 - The effect of anti-PVRIG antibodies blocking the binding between PVRIG and PVRL2 detected by ELISA

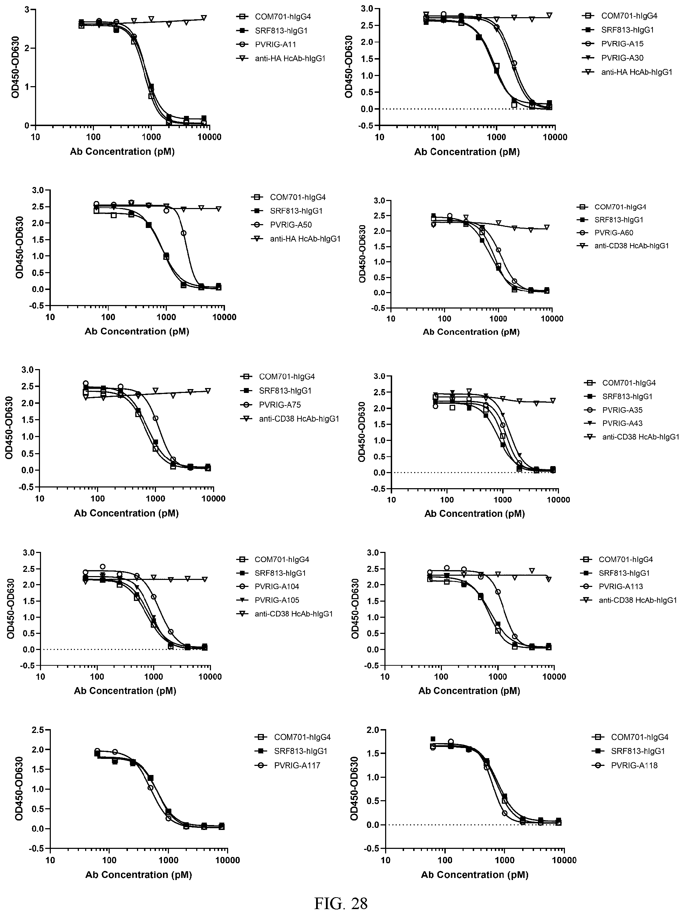

The ELISA plate was pre-coated with 100 µL/well of 0.5 µg/mL human PVRIG-his protein (AcroBiosystems, Cat NO.PVG-H52H4). The tested anti-PVRIG antibodies were diluted from the highest concentration of 16 nM (two-fold concentration). The diluted antibodies were mixed with 18 ng/mL human PVRL2-mFc (AcroBiosystems, Cat NO. CD2-H5257) in equal volume, added into the plate (100 µL/well), and incubated with shaking at room temperature for 2.0 hours. After washing the plate, the Goat anti-mouse IgG Fc-HRP (Jackson ImmunoResearch, Cat NO.115-035-071)) working solution (dilution ratio 1:10000, 50 µL/well) were added into wells and incubated with shaking at room temperature for 1.0 hour. A HRP substrate, TMB (Thermo, Cat NO.34029) was added into wells after washing the plate again for color development. After adding a termination solution to terminate the reaction, the absorbance value was read by a microplate reader (MD i3x). An inhibiting curve was drawn with concentration of antibodies as the abscissa and the corresponding OD value as the ordinate. The IC50 value was calculated by using a Four Parameter Logistic Fit (GraphPadPrism9). The smaller the IC50 value, the stronger the ability of the antibodies inhibits the binding between PVRIG and PVRL2. The positive and negative controls are the same as in Example 8. Fig. 28 shows the blocking curves of the 13 test antibodies, and Table 26 shows the inhibiting activity. As shown in Fig. 28 and Table 26, all the antibodies can significantly inhibit the binding of human PVRIG to human PVRL2 protein.

**Table 26. The effect of test antibodies blocking the binding of PVRIG to PVRL2**

| Name | IC50 (pM) |
|---|---|
| PVRIG-A11 | 121.40 |
| PVRIG-A15 | 207.70 |
| PVRIG-A30 | 254.70 |
| PVRIG-A50 | 195.70 |
| PVRIG-A60 | 55.66 |
| PVRIG-A75 | 62.11 |
| PVRIG-A35 | 54.31 |
| PVRIG-A43 | 53.65 |
| PVRIG-A104 | 65.93 |
| PVRIG-A105 | 57.07 |
| PVRIG-A113 | 92.00 |
| PVRIG-A117 | 52.84 |
| PVRIG-A118 | 51.35 |

### EXAMPLE 12 - The effect of anti-PVRIG antibodies blocking CHO-K1-CD112 cells binding to human PVRIG-mFc proteins detected by FACS

CHO-K1 stable cells transfected with human CD112 high expression plasmid was named CHO-K1-CD112. Human CD112 full-length plasmid (NP_001036189.1/NCBI Ref Seq: Q92692) was synthesized by General Biol. The experiment was performed when the cell density did not exceed 80%. After discarding the cell culture medium, the cells were rinsed with PBS and digested for 2 minutes by adding 1mL trypsin (Gibico, 25200-72). The Ham's F12 (Gibico, 21127-022) complete medium containing 10% FBS was then added to terminate the digestion and acquire cell suspension. After counting with a cell counter (Beckman Coulter, Vi-CELL), an appropriate amount of cell suspension was centrifuged at 350×g to remove the supernatant, washed twice with PBS, stained with Zombie violet (Biolegend, 423114) and incubated at room temperature for 20 minutes. After incubation, the cells were added with staining buffer (2% FBS+PBS) to stop staining, centrifuged at 350×g to remove the supernatant, washed twice and resuspended with staining buffer to a density of 1×10⁶ cells/mL. The cells were plated into a 96-well plate (50µL of cell suspension per well) for further use. A working solution of human PVRIG-mFc protein (Aero, PVG-H5253) prepared with staining buffer, 1µg/mL (four-fold concentration), was added into a 96-well plate (50µL PVRIG-mFc working solution per well). The antibodies were diluted from the highest concentration of 275 nM (four-fold concentration) with staining buffer by 3-fold serial dilution. The diluted antibodies were added to the well containing 50 µL of PVRIG-mFc. The plate was placed on a microplate shaker at 400 rpm for 1 minute to fully mix the antibodies and PVRIG-mFc proteins, and then incubated at 4°C for 30 minutes. After the incubation, the cell suspension prepared above (100µL) was added into the wells, gently mixed with the pipette tip and incubated at 4°C for 30 minutes. After the incubation, the cells were washed twice with staining buffer (200 µL per well) and centrifuged at 350×g for 5 minutes to discard the supernatant. The PE goat anti-mouse IgG Fc antibody (Biolegend, 405337) was diluted 250 times with staining buffer, added into the washed wells (100 µL per well), mixed well, and stained at 4°C for 30 minutes. After staining, the cells were washed twice with staining buffer, finally resuspended with 200 µL staining buffer and analyzed by flow cytometry (BD, Canto II). The weaker the fluorescence signal, the stronger the ability of the antibodies blocks the binding of CHO-K1 human CD112 cells to the PVRIG-mFc protein. An antibody binding curve was drawn with concentration of antibodies as the abscissa and the corresponding Mean Fluorescence Intensity (MFI) as the ordinate. The IC50 value of the antibodies and the AUC value of the binding curve were calculated by using a Four Parameter Logistic Fit (GraphPadPrism9). The smaller the IC50 value and AUC value, the stronger the ability of the antibodies competes with CHO-K1-CD112 cells to bind to human PVRIG-mFc protein. As shown in Fig. 29, all the test antibodies can block the binding of CHO-K1 human CD112 cells to human PVRIG-mFc protein. The competitive blocking activity of the antibodies was normalized to the positive control COM701-hIgG1 and SRF813-hIgG1. The smaller the percentage value, the stronger the blocking ability of the antibodies. Table 27 shows that the blocking activity of the tested antibodies PVRIG-A11, A15, A30 and A50 is stronger than that of COM701-hIgG1 and SRF813-hIgG1.

**Table 27 Anti-PVRIG antibodies compete with CHO-K1-CD112 cells to bind to human PVRIG-mFc protein**

| Name | CHO-K1-CD112 cell blocking | | | |
|---|---|---|---|---|
| | %IC50 | | %AUC | |
| | to COM701-hIgG4 | to SRF813-hIgG1 | to COM701-hIgG4 | to SRF813-hIgG1 |
| PVRIG-A11 | 62.81 | 64.08 | 77.99 | 58.87 |
| PVRIG-A15 | 54.07 | 72.38 | 77.22 | 77.27 |
| PVRIG-A30 | 48.12 | 64.41 | 58.86 | 58.90 |
| PVRIG-A50 | 99.07 | 91.85 | 103.64 | 81.01 |
| PVRIG-A60 | 98.87 | 96.37 | 117.47 | 102.84 |
| PVRIG-A75 | 89.61 | 93.74 | 100.89 | 86.58 |
| PVRIG-A35 | 138.27 | 136.23 | 145.00 | 125.72 |
| PVRIG-A43 | 121.18 | 119.40 | 127.93 | 110.92 |
| PVRIG-A104 | 117.82 | 115.52 | 151.79 | 110.90 |
| PVRIG-A105 | 99.67 | 97.72 | 115.09 | 84.08 |
| PVRIG-A113 | 124.29 | 97.94 | 172.27 | 105.69 |
| PVRIG-A117 | 133.89 | 117.75 | 129.93 | 72.66 |
| PVRIG-A118 | 110.95 | 97.42 | 109.54 | 75.58 |

### EXAMPLE 13 - Detection of The expression of PVRIG and TIGIT on NK cells, and PVR and PVRL2 on Tumor cell lines Reh and WIDR cells

The expression level of PVRIG and TIGIT on NK cells was detected by FACS. The method refer to Example 4 (e). As shown in Fig. 9A, PVRIG and TIGIT are expressed on the surface of NK cells of donor-010 and donor-050.

The expression level of PVR and PVRL2 on Reh/WIDR cells was detected by FACS. The methods refer to Example 4 (e), wherein Reh were directly mixed to prepare the cell suspensions. The expression of PVR and PVRL2 on the surface of WIDR cells was shown in Figure 9B. As shown in Fig. 30, the expression of PVR on the surface of Reh cells is negative and the expression of PVRL2 is positive.

### EXAMPLE 14 - The promoting effect of anti-PVRIG antibodies on NK cell function detected by NK cell degranulation assay

The effect of the tested antibodies on NK cell degranulation was indicated by the signal of CD107a in NK cells (Natural killer cells) detected by FACS. The methods refer to Example 4 (f), wherein Reh were directly mixed to prepare cell suspensions. Fig. 31 shows that the negative control, anti-HEL-hIgG1, has no effect on CD107a on NK cells. 12 PVRIG antibodies and the control antibodies COM701-hIgG1 and SRF813-hIgG1 can improve the expression of CD107a on NK cells to varying degrees, which indicates that the tested antibodies and control antibodies can effectively promote the activation of NK cells.

### EXAMPLE 15 - The cytotoxicity of NK cells for tumor cell line mediated by anti-PVRIG antibodies detected by NK cell cytotoxicity assay

The lysis level of target cells (WIDR) was detected by FACS to infer the effect of the tested antibodies on the cytotoxicity of NK cells. The methods refer to Example 4 (g). Figure 32 shows that the negative control, anti-HA HcAb-hIgG1, has no significant effect on the NK cell cytotoxicity, while 13 tested antibodies can effectively promote the NK cell cytotoxicity for the target cells (WIDR), and except for PVRIG-A35 and A43, the cytotoxicity promoting effect of the remaining 11 antibodies to WIDR cells was stronger than or equal to that of the control antibody COM701-hIgG1.

### EXAMPLE 16 - The effect of anti-PVRIG antibodies on improving the functions of antigen-specific CD8 T cells detected by CMV antigen-recall assay

The PBMCs from anti-CMV IgG positive donor were induced by CMV pp65 (495-503) polypeptide to produce CMV pp65 specific CD8 T cells, which were served as effector cells. The colo205 tumor cell line pulsed with pp65 was used as the target cell. In such experimental system, the effect of anti-PVRIG antibodies on improving the functions of the antigen-specific CD8 T cell was detected.

The CMV IgG+ PBMCs were resuscitated, resuspended to 2×10⁶/mL by using complete medium (RPMI1640-Glutamax + 5% AB serum + 1% P/S+(1 ×) 2-β mercaptoethanol) containing 1 mg/mL CMV pp65(495-503) peptide (Anaspec, Cat No.AS-28328), 2 ng/mL human IL-2 (R&D, Cat No.IL-202) and 10 ng/mL human IL-7 (Peprotech, Cat No.200-07), inoculated in a 6-well plate (5 mL/well) and incubated at 37°C with 5% CO₂ for 6 days. On day 6, all the PBMCs were collected, and pp65 and IL-7 in the medium were removed. The cells were divided into two portions, resuspended in complete medium containing 100 IU/mL human IL-2, and cultured for another 2 days. On day 8, all the PBMCs were collected and resuspended in complete medium containing 100 IU/mL human IL-2, and the cell density was adjusted to 2×10⁶/mL for continuing cultivation. On day 11, all the PBMCs were collected. The percentage of CD8 T cells and CMV pp65 (495-503) specific CD8 T in PBMC and the expression of PVRIG, TIGIT and PD-1 on the cells were detected by flow cytometry, as shown in Fig. 33A, B, after inducing by CMV pp65 (495-503), the percentage of CMV pp65 (495-503) specific CD8 T exceeds 80%. Fig. 33B shows that pp65+CD8+ T (donor021) expresses different levels of PVRIG, TIGIT, PD-1 and CD226. The detection antibodies in the flow cytometry were as follows: Livedead Near IR (Invitrogen, Cat No.L34976), CD8-PerCp Cy5.5 (BD, Cat No.565310), CD3-PE-Cy7 (Biolegend, Cat No.300316), T-select HLA-A*0201 CMV pp65 Tetramer-PE (MBL, Cat No.TS-0010-1C), PVRIG-AF488 (R&D, Cat No.FAB93651G-100UG), TIGIT-APC (Biolegend, Cat No.372706) and PD-1-BV421 (BD, Cat No.562516).

CD8 T cells were isolated from the induced PBMCs as effector cells by CD8 T cell isolation kit (Stemcell, Cat No. 17953) and resuspended with AIM-V to the cell density of 0.4×10⁶/mL. The expression of CD226 and purity of isolated CD8 T were detected. The target cells, Colo205, were digested by TrypLE^{™} Express Enzyme (Gibco, Cat No.12605010), resuspended in AIM-V (Gibco, Cat No.31035-025) containing 20 ng/mL pp65 to the cell density of 1×10⁶/mL and treated at 37°C with 5% CO₂ for 3 hours. The target cells were then centrifuged at 250g for 5 minutes and resuspended in AIM-V to the cell density of 0.5×10⁶/mL after discarding the supernatant. PVRL2, PVR and PD-L1 were highly expressed on Colo205 detected by flow cytometry (Fig. 33C). Anti-PVRIG antibodies and negative controls were diluted with AIM-V to 280 nM. The low-attachment, 96-well, U-bottom plate (Corning, Cat No.7007) was added with 50 µL of antibody, 50 µL of CD8 T and 100 µL of pp65-treated colo205 in order, mixed well with a multichannel pipette, and incubated at 37°C with 5% CO₂ for 18 hours. In the experimental system, the final concentration of antibodies was 70 nM, CD8 T cells were 20,000/well, and colo205 were 20,000/well. After incubation, the supernatant was collected by centrifugation at 400 g, and the level of human IFN-γ in the supernatant was detected with an ELISA kit (Dakewe, Cat No.1110003). In this system, the positive controls were COM701-hIgG4 and SRF813-hIgG1, and the negative control was no treatment. As shown in Figure 33D, compared with the no treatment group, most of the tested PVRIG antibodies significantly increase the secretion of IFN-γ in the cell supernatant.

The detection antibodies used in flow cytometry to detect the purity of isolated CD8 T and the expression of CD226 were as follows: Livedead-BV421 (Invitrogen, Cat No.L34964), CD8-FITC (BD, Cat No.555366) and CD226-PE-Cy7 (Biolegend , Cat No.338316). The detection antibodies used in flow cytometry to detect the expression of PVRL2, PVR, PD-L1 and HLA-A2 on Colo205 cells were as follows: livedead-BV421 (Invitrogen, Cat No.L34964), PVRL2-APC (Biolegend, Cat No.337412), PVR-PerCp Cy5.5 (Biolegend, Cat No.337612), PD-L1-PE-Cy7 (BD, Cat No.558017) and HLA-A2-PE (Biolegend, Cat No.343306).

### EXAMPLE 17 - Humanization of alpaca anti-human PVRIG antibody

By comparing the IMGT (http://imgt.cines.fr) human antibody heavy and light chain variable region germline gene database, the heavy chain and light chain variable region germline genes with high homology with alpaca antibody were selected as templates. The CDRs of alpaca antibody were grafted into corresponding human templates to form a variable region sequence of FR1-CDR1-FR2-CDR2-FR3-CDR3-FR4. The key amino acids in the skeleton sequence were back mutated to the amino acids corresponding to the alpaca antibody as needed to ensure the original affinity, and thus to obtain the humanized anti-PVRIG monoclonal antibody.

### 1. Humanization of PVRIG-A50

The humanized heavy chain templates of the alpaca antibody PVRIG-A50 are IGHV3-23*04 and IGHJ3*01. The CDRs of the alpaca antibody (PVRIG-A50) were grafted into the human template thereof to obtain the corresponding humanized version. The CDRs amino acid residues of the antibody were determined and annotated by the Kabat numbering scheme. The key amino acids in the FR region sequence in the humanized antibody of PVRIG-A50 were back mutated to the amino acids corresponding to the alpaca antibody as needed to ensure the original affinity. PVRIG-A50 antibody is prone to have chemical modifications on NG site and Glycosylation on NLS. Inventors performed point mutations on NG/NLS to eliminate the risk of modification. The specific design is shown in Table 28.

**Table 28. The design of humanized antibody of PVRIG-A50**

| **VHH** | |
|---|---|
| H1 | Grafted(IGHV3-23*04) + A97V,K98E |
| H1a | Grafted(IGHV3-23*04) + A97V,K98E + N54D |
| H1b | Grafted(IGHV3-23*04) + A97V,K98E + N54D,N108S |
| H1c | Grafted(IGHV3-23*04) + A97V,K98E + N54D,S110A |
| H1d | Grafted(IGHV3-23*04) + A97V,K98E + N108S |
| H1e | Grafted(IGHV3-23*04) + A97V,K98E + G55A, N108S |
| H2a | Grafted(IGHV3-23*04) + S75T,A97V,K98E + G55A, N108S |

| | |
|---|---|
| Note: Grafted (IGHV3-23*04) represents the insertion of CDRs of the target antibody into the FR region sequence in human germline IGHV3-23*04; the first + A97V represents the A at position 97 of Grafted is back mutated to V, the second + N54D represents the point mutation on NG site, and so on. The back-mutated amino acids are numbered in natural order, the same below. | |

The specific variable region sequences in humanized antibody of PVRIG-A50 are as follows:
The amino acid sequence of A50.VH1(PVRIG-A50-H1) is shown in SEQ ID NO: 198 
EVQLVESGGGLVQPGGSLRLSCAASGFTFSYYDMSWVRQAPGKGLEWVSTINSNGGRT
SYVDSVKGRFTISRDNSKNTLYLQMNSLRAEDTAVYYCVEGDPHNFGLENLSLRDFGSW
GQGTMVTVSS
The amino acid sequence of A50.VH1a(PVRIG-A50-H1a) is shown in SEQ ID NO: 199 
EVQLVESGGGLVQPGGSLRLSCAASGFTFSYYDMSWVRQAPGKGLEWVSTINSDGGRT
SYVDSVKGRFTISRDNSKNTLYLQMNSLRAEDTAVYYCVEGDPHNFGLENLSLRDFGSW
GQGTMVTVSS
The amino acid sequence of A50.VH1b(PVRIG-A50-H1b) is shown in SEQ ID NO: 200 
EVQLVESGGGLVQPGGSLRLSCAASGFTFSYYDMSWVRQAPGKGLEWVSTINSDGGRT
SYVDSVKGRFTISRDNSKNTLYLQMNSLRAEDTAVYYCVEGDPHNFGLESLSLRDFGSW
GQGTMVTVSS
The amino acid sequence of A50.VH1c(PVRIG-A50-H1c) is shown in SEQ ID NO: 201 
EVQLVESGGGLVQPGGSLRLSCAASGFTFSYYDMSWVRQAPGKGLEWVSTINSDGGRT
SYVDSVKGRFTISRDNSKNTLYLQMNSLRAEDTAVYYCVEGDPHNFGLENLALRDFGS
WGQGTMVTVSS
The amino acid sequence of A50.VH1d(PVRIG-A50-H1d) is shown in SEQ ID NO: 202 
EVQLVESGGGLVQPGGSLRLSCAASGFTFSYYDMSWVRQAPGKGLEWVSTINSNGGRT
SYVDSVKGRFTISRDNSKNTLYLQMNSLRAEDTAVYYCVEGDPHNFGLESLSLRDFGSW
GQGTMVTVSS
The amino acid sequence of VH1e(PVRIG-A50-H1e) is shown in SEQ ID NO: 203 EVQLVESGGGLVQPGGSLRLSCAASGFTFSYYDMSWVRQAPGKGLEWVSTINSNAGRT 
SYVDSVKGRFTISRDNSKNTLYLQMNSLRAEDTAVYYCVEGDPHNFGLESLSLRDFGSW
GQGTMVTVSS
The amino acid sequence of A50.VH2a(PVRIG-A50-H2a) is shown in SEQ ID NO: 204 
EVQLVESGGGLVQPGGSLRLSCAASGFTFSYYDMSWVRQAPGKGLEWVSTINSNAGRT
SYVDSVKGRFTISRDNTKNTLYLQMNSLRAEDTAVYYCVEGDPHNFGLESLSLRDFGSW
GQGTMVTVSS
The amino acid sequence of the humanized heavy chain template IGHV3-23*04 is shown in SEQ ID NO: 205 
EVQLVESGGGLVQPGGSLRLSCAASGFTFSSYAMSWVRQAPGKGLEWVSAISGSGGSTY
YADSVKGRFTISRDNSKNTLYLQMNSLRAEDTAVYYCAK
The amino acid sequence of the humanized heavy chain template IGHJ3*01 is shown in SEQ ID NO: 206
   WGQGTMVTVSS

According to Kabat numbering scheme, the analysis results of VH sequences in above 7 humanized antibodies are shown in Table 29.

**Table 29 Kabat analysis results of VH sequences in humanized antibody of PVRIG-A50**

| Heavy chain | CDR1 | CDR2 | CDR3 |
|---|---|---|---|
| VH1 | YYDMS | TINSNGGRTSYVDSVKG | GDPHNFGLENLSLRDFGS |
| | SEQ ID NO: 168 | SEQ ID NO: 169 | SEQ ID NO: 170 |
| VH1a | YYDMS | TINSDGGRTSYVDSVKG | GDPHNFGLENLSLRDFGS |
| | SEQ ID NO: 168 | SEQ ID NO: 207 | SEQ ID NO: 170 |
| VH1b | YYDMS | TINSDGGRTSYVDSVKG | GDPHNFGLESLSLRDFGS |
| | SEQ ID NO: 168 | SEQ ID NO: 207 | SEQ ID NO: 208 |
| VH1c | YYDMS | TINSDGGRTSYVDSVKG | GDPHNFGLENLALRDFGS |
| | SEQ ID NO: 168 | SEQ ID NO: 207 | SEQ ID NO: 209 |
| VH1d | YYDMS | TINSNGGRTSYVDSVKG | GDPHNFGLESLSLRDFGS |
| | SEQ ID NO: 168 | SEQ ID NO: 169 | SEQ ID NO: 208 |
| VH1e/H2a | YYDMS | TINSNAGRTSYVDSVKG | GDPHNFGLESLSLRDFGS |
| | SEQ ID NO: 168 | SEQ ID NO: 210 | SEQ ID NO: 208 |

### 2. Humanization of PVRIG-A105

The humanized heavy chain templates of the alpaca antibody PVRIG-A105 are IGHV3-7*01 and IGHJ3*01. The CDRs of the alpaca antibody (PVRIG-A105) were grafted into the human template thereof to obtain the corresponding humanized version. The CDRs amino acid residues of the antibody were determined and annotated by the IMGT numbering scheme. The key amino acids in the FR region sequence in the humanized antibody of PVRIG-A105 were back mutated to the amino acids corresponding to the alpaca antibody as needed to ensure the original affinity. PVRIG-A105 antibody has two free cysteines. In order to improve the stability of the antibody, Inventors performed point mutations on Cys. The specific design is shown in Table 30.

**Table 30. The design of humanized antibody of PVRIG-A105**

| **VHH** | |
|---|---|
| H1 | Grafted(IGHV3-7*01) + V37F,G44E,L45R,W47F,N50T |
| H2 | Grafted(IGHV3-7*01) + S35T,V37F,G44E,L45R,W47F,N50T |
| H3 | Grafted(IGHV3-7*01) + S35T,V37F,G44E,L45R,W47F,N50T,L79V |
| H3a | Grafted(IGHV3-7*01) + S35T,V37F,G44E,L45R,W47F,N50T,L79V + C103S,C108S |
| H4 | Grafted(IGHV3-7*01) + S35T,V37F,G44E,L45R,W47F,N50T,V61S,D62H |
| H5 | Grafted(IGHV3-7*01) + S35T,V37F,G44E,L45R,W47F,N50T,T122I,M123Q |

| | |
|---|---|
| Note: Grafted (IGHV3-7*01) represents the insertion of CDRs of the target antibody into the FR region sequence in human germline IGHV3-7*01; the first + V37F represents the V at position 37 of Grafted is back mutated to F, the second + C103S represents the point mutation on Cys site, and so on. The back-mutated amino acids are numbered in natural order, the same below. | |

The specific variable region sequences in humanized antibody of PVRIG-A105 are as follows:
The amino acid sequence of A105.VH1(PVRIG-A105-H1) is shown in SEQ ID NO: 211 
EVQLVESGGGLVQPGGSLRLSCAASGRTFDRHTMSWFRQAPGKEREFVATASRIPGDTY
YVDSVKGRFTISRDNAKNSLYLQMNSLRAEDTAVYYCAATSAYCSEVDCYEKGSWYDN
WGQGTMVTVSS
The amino acid sequence of A105.VH2(PVRIG-A105-H2) is shown in SEQ ID NO: 212 
EVQLVESGGGLVQPGGSLRLSCAASGRTFDRHTMTWFRQAPGKEREFVATASRIPGDTY
YVDSVKGRFTISRDNAKNSLYLQMNSLRAEDTAVYYCAATSAYCSEVDCYEKGSWYDN
WGQGTMVTVSS
The amino acid sequence of A105.VH3(PVRIG-A105-H3) is shown in SEQ ID NO: 213 
EVQLVESGGGLVQPGGSLRLSCAASGRTFDRHTMTWFRQAPGKEREFVATASRIPGDTY
YVDSVKGRFTISRDNAKNSVYLQMNSLRAEDTAVYYCAATSAYCSEVDCYEKGSWYDN
WGQGTMVTVSS
The amino acid sequence of A105.VH4(PVRIG-A105-H4) is shown in SEQ ID NO: 214 
EVQLVESGGGLVQPGGSLRLSCAASGRTFDRHTMTWFRQAPGKEREFVATASRIPGDTY
YSHSVKGRFTISRDNAKNSLYLQMNSLRAEDTAVYYCAATSAYCSEVDCYEKGSWYDN
WGQGTMVTVSS
The amino acid sequence of A105.VH5(PVRIG-A105-H5) is shown in SEQ ID NO: 215 
EVQLVESGGGLVQPGGSLRLSCAASGRTFDRHTMTWFRQAPGKEREFVATASRIPGDTY
YVDSVKGRFTISRDNAKNSLYLQMNSLRAEDTAVYYCAATSAYCSEVDCYEKGSWYDN
WGQGIQVTVSS
The amino acid sequence of A105.VH3a(PVRIG-A105-H3a) is shown in SEQ ID NO: 216 
EVQLVESGGGLVQPGGSLRLSCAASGRTFDRHTMTWFRQAPGKEREFVATASRIPGDTY
YVDSVKGRFTISRDNAKNSVYLQMNSLRAEDTAVYYCAATSAYSSEVDSYEKGSWYDN
WGQGTMVTVSS
The amino acid sequence of the humanized heavy chain template IGHV3-7*01 is shown in SEQ ID NO: 217 
EVQLVESGGGLVQPGGSLRLSCAASGFTFSSYWMSWVRQAPGKGLEWVANIKQDGSEK
YYVDSVKGRFTISRDNAKNSLYLQMNSLRAEDTAVYYCAR
The amino acid sequence of the humanized heavy chain template IGHJ3*01 is shown in SEQ ID NO: 206
   WGQGTMVTVSS

According to IMGT numbering scheme, the analysis results of VH sequences in above 6 humanized antibodies are shown in Table 31.

**Table 31 IMGT analysis results of VH sequences in humanized antibody of PVRIG-A105**

| Heavy chain | CDR1 | CDR2 | CDR3 |
|---|---|---|---|
| VH1/2/3/4/5 | GRTFDRHT | ASRIPGDT | AATSAYCSEVDCYEKGSWYDN |
| | SEQ ID NO: 147 | SEQ ID NO: 148 | SEQ ID NO: 149 |
| VH3a | GRTFDRHT | ASRIPGDT | AATSAYSSEVDSYEKGSWYDN |
| | SEQ ID NO: 147 | SEQ ID NO: 148 | SEQ ID NO: 218 |

### 2. Humanization of PVRIG-A118

The humanized heavy chain templates of the alpaca antibody PVRIG-A118 are IGHV3-7*01 and IGHJ3*01. The CDRs of the alpaca antibody (PVRIG-A118) were grafted into the human template thereof to obtain the corresponding humanized version. The CDRs amino acid residues of the antibody were determined and annotated by the IMGT numbering scheme. The key amino acids in the FR region sequence in the humanized antibody of PVRIG-A118 were back mutated to the amino acids corresponding to the alpaca antibody as needed to ensure the original affinity. The specific design is shown in Table 32.

**Table 32. The design of humanized antibody of PVRIG-A118**

| **VHH** | |
|---|---|
| H1 | Grafted(IGHV3-7*01) + S35G,V37Y,G44D,L45R,W47L,N50T |
| H2 | Grafted(IGHV3-7*01) + S35G,V37Y,G44D,L45R,W47L,N50T,Y58K |
| H3 | Grafted(IGHV3-7*01) + S35G,V37Y,G44D,L45R,W47L,N50T,Y58K,D72G,N73D |
| H4 | Grafted(IGHV3-7*01) + S35G,V37Y,G44D,L45R,W47L,N50T,Y58K,D72G,N73D,Y79S |
| H5 | Grafted(IGHV3-7*01) + S35G,V37Y,G44D,L45R,W47L,N50T,Y58K,D72G,N73D,L78V |
| H6 | Grafted(IGHV3-7*01) + S35G,V37Y,G44D,L45R,W47L,N50T,Y58K,Y59I,D72G,N73D |
| H7 | Grafted(IGHV3-7*01) + S35G,V37Y,G44D,L45R,W47L,N50T,Y58K,D72G,N73D,Y94F |

| | |
|---|---|
| Note: Grafted (IGHV3-7*01) represents the insertion of CDRs of the target antibody into the FR region sequence in human germline IGHV3-7*01; the first + S35G represents the S at position 35 of Grafted is back mutated to G, and so on. The back-mutated amino acids are numbered in natural order, the same below. | |

The specific variable region sequences in humanized antibody of PVRIG-A118 are as follows:
The amino acid sequence of A118.VH1 (PVRIG-A118-H1) is shown in SEQ ID NO: 219 
EVQLVESGGGLVQPGGSLRLSCAASETYFDLYVMGWYRQAPGKDRELVATITYTGSIYY
VDSVKGRFTISRDNAKNSLYLQMNSLRAEDTAVYYCNADPSGLGRKVYWGQGTMVTV
SS
The amino acid sequence of A118.VH2 (PVRIG-A118-H2) is shown in SEQ ID NO: 220 
EVQLVESGGGLVQPGGSLRLSCAASETYFDLYVMGWYRQAPGKDRELVATITYTGSIKY
VDSVKGRFTISRDNAKNSLYLQMNSLRAEDTAVYYCNADPSGLGRKVYWGQGTMVTV
SS
The amino acid sequence of A118.VH3 (PVRIG-A118-H3) is shown in SEQ ID NO: 221 
EVQLVESGGGLVQPGGSLRLSCAASETYFDLYVMGWYRQAPGKDRELVATITYTGSIKY
VDSVKGRFTISRGDAKNSLYLQMNSLRAEDTAVYYCNADPSGLGRKVYWGQGTMVTV
SS
The amino acid sequence of A118.VH4 (PVRIG-A118-H4) is shown in SEQ ID NO: 222 
EVQLVESGGGLVQPGGSLRLSCAASETYFDLYVMGWYRQAPGKDRELVATITYTGSIKY
VDSVKGRFTISRGDAKNSLSLQMNSLRAEDTAVYYCNADPSGLGRKVYWGQGTMVTV
SS
The amino acid sequence of A118.VH5 (PVRIG-A118-H5) is shown in SEQ ID NO: 223 
EVQLVESGGGLVQPGGSLRLSCAASETYFDLYVMGWYRQAPGKDRELVATITYTGSIKY
VDSVKGRFTISRGDAKNSVYLQMNSLRAEDTAVYYCNADPSGLGRKVYWGQGTMVTV
SS
The amino acid sequence of A118.VH6 (PVRIG-A118-H6) is shown in SEQ ID NO: 224 
EVQLVESGGGLVQPGGSLRLSCAASETYFDLYVMGWYRQAPGKDRELVATITYTGSIKIV
DSVKGRFTISRGDAKNSLYLQMNSLRAEDTAVYYCNADPSGLGRKVYWGQGTMVTVSS
The amino acid sequence of A118.VH7 (PVRIG-A118-H7) is shown in SEQ ID NO: 225 
EVQLVESGGGLVQPGGSLRLSCAASETYFDLYVMGWYRQAPGKDRELVATITYTGSIKY
VDSVKGRFTISRGDAKNSLYLQMNSLRAEDTAVYFCNADPSGLGRKVYWGQGTMVTVS
S
The amino acid sequence of the humanized heavy chain template IGHV3-7*01 is shown in SEQ ID NO: 217 
EVQLVESGGGLVQPGGSLRLSCAASGFTFSSYWMSWVRQAPGKGLEWVANIKQDGSEK
YYVDSVKGRFTISRDNAKNSLYLQMNSLRAEDTAVYYCAR
The amino acid sequence of the humanized heavy chain template IGHJ3*01 is shown in SEQ ID NO: 206
   WGQGTMVTVSS

According to IMGT numbering scheme, the analysis results of VH sequences in above 7 humanized antibodies are shown in Table 33.

**Table 33 IMGT analysis results of VH sequences in humanized antibody of PVRIG-A118**

| Heavy chain | CDR1 | CDR2 | CDR3 |
|---|---|---|---|
| VH1/2/3/4/5/6/7 | ETYFDLYV | ITYTGSI | NADPSGLGRKVY |
| | SEQ ID NO: 156 | SEQ ID NO: 157 | SEQ ID NO: 158 |

### EXAMPLE 18 - The specific binding of PVRIG humanized antibodies to human and cynomolgus monkey PVRIG protein detected by ELISA

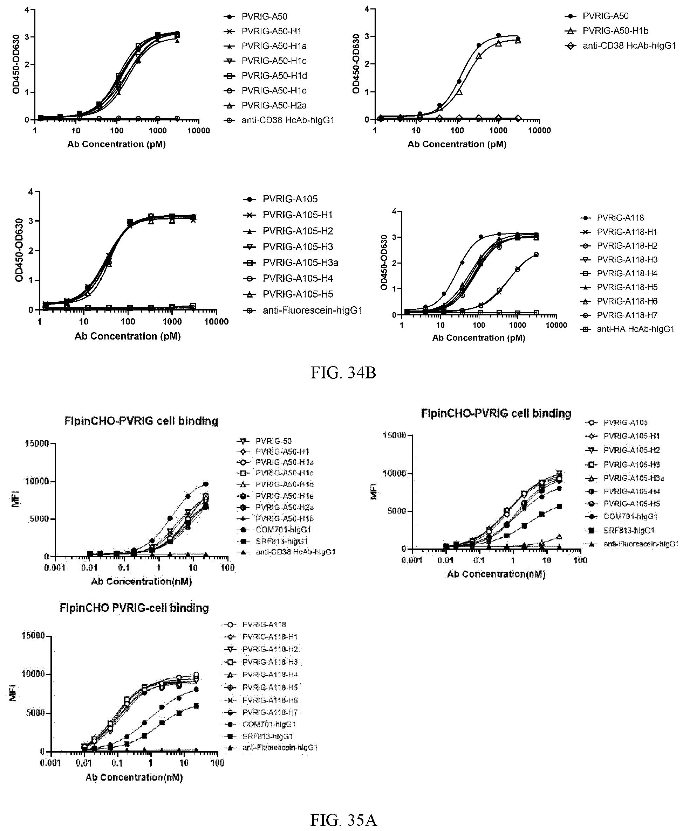

The ELISA plate was pre-coated with 100 µL/well of 0.5 µg/mL human PVRIG-his (AcroBiosystems, Cat NO.PVG-H52H4) or cynomolgus monkey PVRIG (Novoprotein, Cat NO.C09B). The tested anti-PVRIG humanized antibodies were serially diluted (initial concentration 3nM, 3-fold serial dilution), added into the plate (100 µL/well) and incubated with shaking at room temperature for 1.5 hours. After washing the plate, the mouse anti-human IgG Fc-HRP (Jackson ImmunoResearch, Cat NO.209-035-098)) working solution (dilution ratio 1:10000, 100 µL/well) were added into wells and incubated with shaking at room temperature for 1.0 hour. A HRP substrate, TMB (Thermo, Cat NO.34029) was added into wells after washing the plate again for color development. After adding a termination solution to terminate the reaction, the absorbance value was read by a microplate reader (MD i3x). An antibody binding curve was drawn with concentration of antibodies as the abscissa and the corresponding OD value as the ordinate. The EC50 value was calculated by using a Four Parameter Logistic Fit (GraphPadPrism9). The smaller the EC50 value, the stronger the ability of the antibodies binds to human or cynomolgus monkey PVRIG. The binding effect of the humanized antibodies was normalized to the corresponding parental antibodies. If the percentage value is higher than 100%, the binding effect of humanized antibodies is better than that of parental antibody. As shown in Fig. 34A, Fig. 34B and Table 34, most of the humanized antibodies of PVRIG-A50, PVRIG-A105 and PVRIG-A118 specifically bind to the human/cynomolgus monkey PVRIG proteins.

**Table 34. The specific binding of anti-PVRIG humanized antibodies to human or cynomolgus monkey PVRIG protein detected by ELISA**

| No. | Name | Bind to human PVRIG protein | | 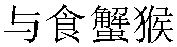 PVRIG 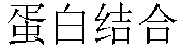 | |
|---|---|---|---|---|---|
| | | EC50 | % relative activity | EC50 | % relative activity |
| | | (pM) | parental antibody* /tested antibody | (pM) | parental antibody /tested antibody |
| 1 | PVRIG-A50-H1 | 87.12 | 105.02% | 129.6 | 95.06% |
| 2 | PVRIG-A50-H1a | 99.04 | 92.38% | 185.5 | 66.42% |
| 3 | PVRIG-A50-H1c | 104.8 | 87.30% | 177.4 | 69.45% |
| 4 | PVRIG-A50-H1d | 77.4 | 118.20% | 110.6 | 111.39% |
| 5 | PVRIG-A50-H1e | 79.06 | 115.72% | 137.6 | 89.53% |
| 6 | PVRIG-A50-H2a | 65.51 | 139.66% | 180.4 | 68.29% |
| 7 | PVRIG-A50-H1b | 81.94 | 111.29% | 161.3 | 75.76% |
| 8 | PVRIG-A105-H1 | 48.71 | 149.85% | 30.28 | 106.51% |
| 9 | PVRIG-A105-H2 | 51.51 | 141.70% | 34.33 | 93.94% |
| 10 | PVRIG-A105-H3 | 58.53 | 124.71% | 35.16 | 91.72% |
| 11 | PVRIG-A105-H3a | 7408 | 0.99% | 1273 | 2.53% |
| 12 | PVRIG-A105-H4 | 52.07 | 140.18% | 36.07 | 89.41% |
| 13 | PVRIG-A105-H5 | 67.76 | 107.72% | 37.65 | 85.66% |
| 14 | PVRIG-A118-H1 | 45.63 | 109.69% | 584.9 | 4.62% |
| 15 | PVRIG-A118-H2 | 49.08 | 101.98% | 593.7 | 4.55% |
| 16 | PVRIG-A118-H3 | 49.97 | 100.16% | 79.41 | 34.05% |
| 17 | PVRIG-A118-H4 | 52.22 | 95.84% | 72.9 | 37.09% |
| 18 | PVRIG-A118-H5 | 48.05 | 104.16% | 67.31 | 40.17% |
| 19 | PVRIG-A118-H6 | 55.14 | 90.77% | 55.9 | 48.37% |
| 20 | PVRIG-A118-H7 | 50.11 | 99.88% | 81.67 | 33.11% |

| | | | | | |
|---|---|---|---|---|---|
| * parental antibody: the corresponding parental antibody before humanization | | | | | |

### EXAMPLE 19 - The binding of anti-PVRIG humanized antibodies to human PVRIG on FlpinCHO-PVRIG and cynomolgus monkey PVRIG on FlpinCHO-cyno PVRIG detected by FACS

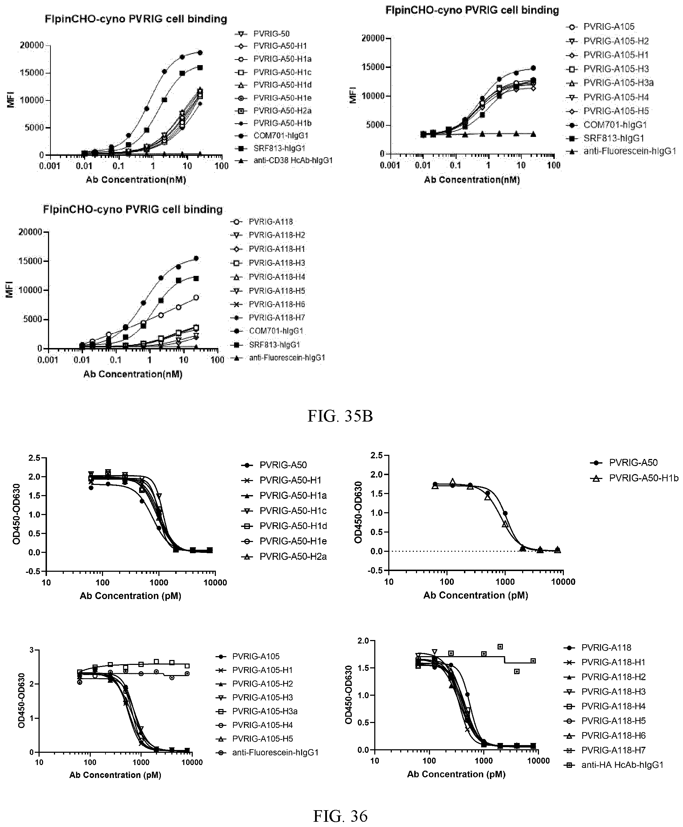

The methods refer to Example 19. The stronger the AUC value, the stronger the ability of the humanized antibodies binds to FlpinCHO-human/cyno PVRIG. Fig. 35A shows that the binding ability of most of the humanized antibodies of PVRIG-A50, PVRIG-A105 and PVRIG-A118 to human PVRIG on the surface of FlpinCHO-PVRIG cells is equivalent to that of their parental antibody. Fig. 35B shows that except for the humanized molecules of PVRIG-A118, the binding of which to cynomolgus monkey PVRIG is significantly weaker than the corresponding parental antibody, most of the humanized molecules of PVRIG-A50 and A105 bind well to cynomolgus monkey PVRIG, and their binding ability is equivalent to that of the corresponding parental antibody. The binding activity of the humanized antibodies was normalized to the positive control COM701-hIgG1 and SRF813-hIgG1. As shown in Table 35, the higher the percentage value, the stronger the binding ability of the antibodies.

**Table 35. Binding of anti-PVRIG humanized antibodies to human/cynomolgus monkey PVRIG on the surface of overexpressing cells**

| PVRIG humanized antibody | human PVRIG binding | | | cyno PVRIG binding | | |
|---|---|---|---|---|---|---|
| | AUC% | | | AUC% | | |
| | to COM701-h IgG1 | to SRF813-hIg G1 | to parental Ab* | to COM701-hI gG1 | to SRF813-hI gG1 | to parental Ab |
| PVRIG-50 | 73.39 | 129.95 | 100.00 | 47.06 | 57.77 | 100.00 |
| PVRIG-A50-H1 | 74.39 | 131.71 | 101.35 | 45.16 | 55.45 | 95.98 |
| PVRIG-A50-H1a | 70.42 | 124.68 | 95.95 | 40.69 | 49.96 | 86.48 |
| PVRIG-A50-H1c | 69.16 | 122.46 | 94.24 | 38.81 | 47.64 | 82.47 |
| PVRIG-A50-H1d | 63.06 | 111.65 | 85.92 | 48.59 | 59.66 | 103.27 |
| PVRIG-A50-H1e | 60.31 | 106.79 | 82.18 | 43.31 | 53.17 | 92.03 |
| PVRIG-A50-H2a | 61.92 | 109.64 | 84.37 | 42.42 | 52.08 | 90.16 |
| PVRIG-A50-H1b | 60.53 | 107.17 | 82.47 | 34.38 | 42.21 | 73.06 |
| PVRIG-A118 | 136.50 | 190.93 | 100.00 | 62.41 | 76.11 | 100.00 |
| PVRIG-A118-H1 | 127.17 | 177.87 | 93.16 | 11.52 | 14.05 | 18.46 |
| PVRIG-A118-H2 | 124.64 | 174.34 | 91.31 | 14.61 | 17.82 | 23.41 |
| PVRIG-A118-H3 | 128.93 | 180.34 | 94.45 | 20.97 | 25.58 | 33.60 |
| PVRIG-A118-H4 | 132.42 | 185.22 | 97.01 | 21.32 | 26.00 | 34.17 |
| PVRIG-A118-H5 | 128.19 | 179.30 | 93.91 | 21.84 | 26.63 | 34.99 |
| PVRIG-A118-H6 | 128.62 | 179.90 | 94.22 | 19.86 | 24.22 | 31.82 |
| PVRIG-A118-H7 | 126.47 | 176.90 | 92.65 | 19.71 | 24.03 | 31.57 |
| PVRIG-A105 | 123.99 | 186.43 | 100.00 | 87.47 | 108.46 | 100.00 |
| PVRIG-A105-H1 | 122.83 | 184.68 | 99.07 | 81.31 | 100.83 | 92.96 |
| PVRIG-A105-H2 | 127.59 | 191.84 | 102.91 | 84.20 | 104.41 | 96.26 |
| PVRIG-A105-H3 | 125.38 | 188.52 | 101.12 | 86.38 | 107.11 | 98.75 |
| PVRIG-A105-H3a | 15.92 | 23.94 | 12.84 | 3.27 | 4.05 | 3.74 |
| PVRIG-A105-H4 | 115.40 | 173.51 | 93.07 | 80.31 | 99.59 | 91.82 |
| PVRIG-A105-H5 | 111.54 | 167.70 | 89.96 | 78.82 | 97.74 | 90.11 |

| | | | | | | |
|---|---|---|---|---|---|---|
| * parental antibody: the corresponding parental antibody before humanization | | | | | | |

### EXAMPLE 20 - The affinity of anti-PVRIG humanized antibodies for human PVRIG proteins detected by BIAcore

The methods refer to Example 10. As shown in Table 36, all the anti-PVRIG humanized antibodies specifically bind to human PVRIG proteins with high affinity.

**Table 36. BIAcore results of specific binding of anti-PVRIG antibodies for human PVRIG proteins**

| **Name** | **Antigen** | **KD (M)** | **ka (1/Ms)** | **kd (1/s)** |
|---|---|---|---|---|
| PVRIG-A50 | Human PVRIG | 4.30E-11 | 4.43E+05 | 1.90E-05 |
| PVRIG-A50-H1 | | 3.62E-11 | 4.10E+05 | 1.48E-05 |
| PVRIG-A50-H1a | | 4.51E-11 | 4.25E+05 | 1.92E-05 |
| PVRIG-A50-H1c | | 4.62E-11 | 4.38E+05 | 2.03E-05 |
| PVRIG-A50-H1d | | 5.45E-11 | 2.62E+05 | 1.43E-05 |
| PVRIG-A50-H1e | | 5.51E-11 | 2.65E+05 | 1.46E-05 |
| PVRIG-A50-H2a | | 5.85E-11 | 2.76E+05 | 1.61E-05 |
| PVRIG-A50-H1b | | 8.04E-11 | 3.50E+05 | 2.82E-05 |
| PVRIG-A118 | | 4.83E-10 | 2.12E+06 | 1.02E-03 |
| PVRIG-A118-H1 | | 4.78E-10 | 1.38E+06 | 6.58E-04 |
| PVRIG-A118-H2 | | 5.17E-10 | 1.59E+06 | 8.20E-04 |
| PVRIG-A118-H3 | | 1.35E-09 | 2.59E+06 | 3.49E-03 |
| PVRIG-A118-H4 | | 1.21E-09 | 2.69E+06 | 3.27E-03 |
| PVRIG-A118-H5 | | 1.37E-09 | 2.79E+06 | 3.83E-03 |
| PVRIG-A118-H6 | | 1.10E-09 | 2.71E+06 | 2.99E-03 |
| PVRIG-A118-H7 | | 1.08E-09 | 2.41E+06 | 2.60E-03 |
| PVRIG-A105 | | 6.75E-10 | 7.21E+05 | 4.86E-04 |
| PVRIG-A105-H1 | | 9.32E-10 | 1.39E+06 | 1.30E-03 |
| PVRIG-A105-H2 | | 6.93E-10 | 1.33E+06 | 9.20E-04 |
| PVRIG-A105-H3 | | 6.22E-10 | 9.25E+05 | 5.76E-04 |
| PVRIG-A105-H4 | | 7.96E-10 | 9.72E+05 | 7.73E-04 |
| PVRIG-A105-HS | | 8.18E-10 | 1.11E+06 | 9.10E-04 |

### EXAMPLE 21 - The effect of anti-PVRIG humanized antibodies blocking the binding between PVRIG and PVRL2 detected by ELISA

The methods refer to Example 11. The inhibiting activity of the humanized antibodies was normalized to the corresponding parental antibodies. If the percentage value is higher than 100%, the inhibiting effect of humanized antibodies is better than that of parental antibodies. Fig. 36 shows the blocking curves of the humanized antibodies, and Table 37 shows the inhibiting activity. As shown in Fig. 36 and Table 37, most humanized antibodies of PVRIG-A50, PVRIG-A105 and PVRIG-A118 can significantly inhibit the binding of human PVRIG to human PVRL2 protein.

**Table 37. The effect of anti-PVRIG humanized antibodies blocking the binding of PVRIG to PVRL2**

| No. | Name | Blocking the binding of PVRIG to PVRL2 | | | |
|---|---|---|---|---|---|
| | | IC50 (pM) | % relative activity | AUC | % relative activity |
| | | | Parental antibody*/test antibody | | Parental antibody/test antibody |
| 1 | PVRIG-A50-H1 | 1003 | 77.45% | 2.423 | 82.62% |
| 2 | PVRIG-A50-H1a | 1064 | 73.01% | 2.426 | 82.52% |
| 3 | PVRIG-A50-H1c | 1178 | 65.94% | 2.625 | 76.27% |
| 4 | PVRIG-A50-H1d | 1035 | 75.05% | 2.405 | 83.24% |
| 5 | PVRIG-A50-H1e | 977.2 | 79.49% | 2.323 | 86.18% |
| 6 | PVRIG-A50-H2a | 910.8 | 85.29% | 2.317 | 86.40% |
| 7 | PVRIG-A50-H1b | 847.3 | 124.63% | 1.999 | 104.60% |
| 8 | PVRIG-A105-H1 | 555.10 | 132.57% | 2.271 | 111.76% |
| 9 | PVRIG-A105-H2 | 577.00 | 127.54% | 2.270 | 111.81% |
| 10 | PVRIG-A105-H3 | 751.70 | 97.90% | 2.523 | 100.59% |
| 11 | PVRIG-A105-H3a | no blocking | | 5.374 | 47.23% |
| 12 | PVRIG-A105-H4 | 686.50 | 107.20% | 2.320 | 109.40% |
| 13 | PVRIG-A105-H5 | 646.90 | 113.76% | 2.397 | 105.88% |
| 14 | PVRIG-A118-H1 | 355.4 | 156.67% | 1.308 | 120.87% |
| 15 | PVRIG-A118-H2 | 357.0 | 155.97% | 1.351 | 117.02% |
| 16 | PVRIG-A118-H3 | 385.8 | 144.32% | 1.494 | 105.82% |
| 17 | PVRIG-A118-H4 | 462.1 | 120.49% | 1.418 | 111.50% |
| 18 | PVRIG-A118-H5 | 380.6 | 146.30% | 1.389 | 113.82% |
| 19 | PVRIG-A118-H6 | 422.4 | 131.82% | 1.467 | 107.77% |
| 20 | PVRIG-A118-H7 | 430.1 | 129.46% | 1.381 | 114.48% |

| | | | | | |
|---|---|---|---|---|---|
| * parental antibody: the corresponding parental antibody before humanization | | | | | |

### EXAMPLE 22 - The effect of anti-PVRIG humanized antibodies blocking CHO-K1-CD112 cells binding to human PVRIG-mFc proteins detected by FACS

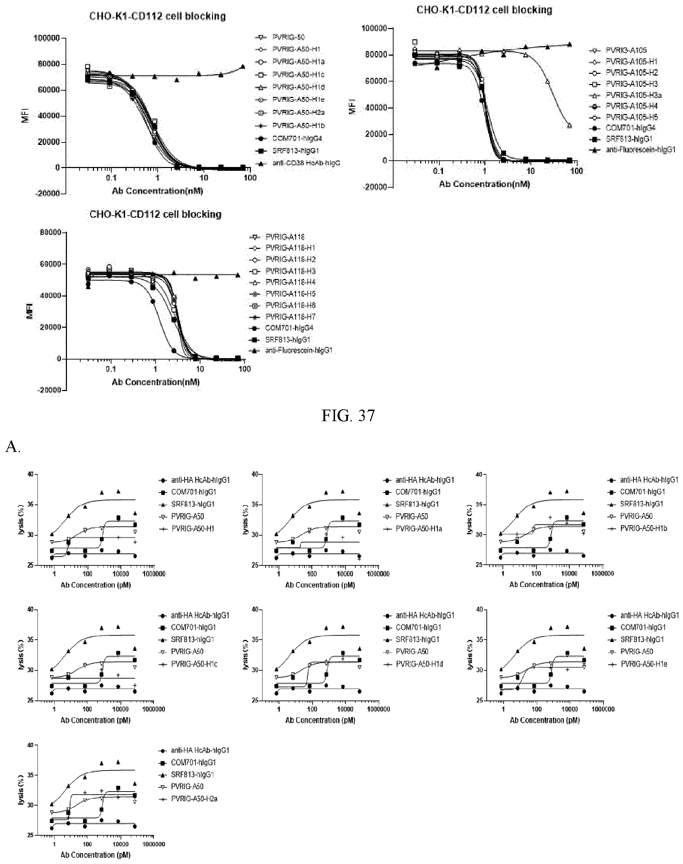

The methods refer to Example 12. As shown in Fig. 37, most humanized antibodies can block the binding of CHO-K1-CD112 cells to human PVRIG-mFc protein. The blocking activity of the humanized antibodies was normalized to the positive control COM701-hIgG1 and SRF813-hIgG1. The smaller the percentage value, the stronger the blocking ability of the antibodies.

**Table 38 Anti-PVRIG humanized antibodies block CHO-K1-CD112 cells from binding to human PVRIG-mFc protein**

| PVRIG humanized antibodies | CHO-K1-CD112 FACS blocking | | | | | |
|---|---|---|---|---|---|---|
| | %IC50 | | | %AUC | | |
| | to COM701-hIgG4 | to S035-hIgG1 | to parental Ab | to COM701-hIgG4 | to SO35-hIgG1 | to parental Ab |
| PVRIG-50 | 117.03 | 90.60 | 100.00 | 122.20 | 72.06 | 100.00 |
| PVRIG-A50-H1 | 100.74 | 77.99 | 86.08 | 114.89 | 67.75 | 94.02 |
| PVRIG-A50-H1a | 120.08 | 92.96 | 102.60 | 124.64 | 73.49 | 101.99 |
| PVRIG-A50-H1c | 122.31 | 94.69 | 104.52 | 131.78 | 77.71 | 107.84 |
| PVRIG-A50-H1d | 115.12 | 89.12 | 98.37 | 120.57 | 71.09 | 98.66 |
| PVRIG-A50-H1e | 130.27 | 100.85 | 111.31 | 125.71 | 74.13 | 102.87 |
| PVRIG-A50-H2a | 118.46 | 91.71 | 101.22 | 111.98 | 66.03 | 91.64 |
| PVRIG-A50-H1b | 95.25 | 73.74 | 81.39 | 104.45 | 61.59 | 85.47 |
| PVRIG-A118 | 140.78 | 95.33 | 100.00 | 118.66 | 61.71 | 100.00 |
| PVRIG-A118-H1 | 140.86 | 95.38 | 100.05 | 119.93 | 62.37 | 101.07 |
| PVRIG-A118-H2 | 161.48 | 109.34 | 114.70 | 141.80 | 73.74 | 119.50 |
| PVRIG-A118-H3 | 145.90 | 98.79 | 103.64 | 126.51 | 65.79 | 106.61 |
| PVRIG-A118-H4 | 140.95 | 95.44 | 100.12 | 116.15 | 60.40 | 97.88 |
| PVRIG-A118-H5 | 143.88 | 97.43 | 102.20 | 127.17 | 66.13 | 107.17 |
| PVRIG-A118-H6 | 144.51 | 97.85 | 102.65 | 118.71 | 61.73 | 100.04 |
| PVRIG-A118-H7 | 143.39 | 97.09 | 101.85 | 123.72 | 64.34 | 104.26 |
| PVRIG-A105 | 104.42 | 88.32 | 100.00 | 113.72 | 65.59 | 100.00 |
| PVRIG-A105-H1 | 104.11 | 88.06 | 99.70 | 111.36 | 64.22 | 97.92 |
| PVRIG-A105-H2 | 109.09 | 92.27 | 104.47 | 115.03 | 66.34 | 101.15 |
| PVRIG-A105-H3 | 108.68 | 91.92 | 104.08 | 109.00 | 62.87 | 95.85 |
| PVRIG-A105-H3a | 3060.00 | 2588.24 | 2930.42 | 3530.47 | 2036.21 | 3104.48 |
| PVRIG-A105-H4 | 110.13 | 93.15 | 105.47 | 111.95 | 64.57 | 98.44 |
| PVRIG-A105-H5 | 110.44 | 93.42 | 105.77 | 118.62 | 68.42 | 104.31 |

### EXAMPLE 23 - The cytotoxicity of NK cells for tumor cell line mediated by anti-PVRIG humanized antibodies detected by NK cell cytotoxicity assay

The methods refer to Example 4 (g). As shown in Fig. 38, all the humanized antibodies can effectively promote the NK cell cytotoxicity for the target cells to varying degrees, wherein Panel A shows that the promoting effect of PVRIG-A50-H1b and PVRIG-A50-H2a on NK cell cytotoxicity for target cells was equal to that of the parental antibody PVRIG-A50, Panel B shows that the promoting effect of PVRIG-A118-H3, H4, H5 and H6 on NK cell cytotoxicity for target cells was equal to that of the parental antibody PVRIG-A118, Panel C shows that the promoting effect of PVRIG-A105-H1, H2 and H3 on NK cell cytotoxicity for target cells was equal to that of the parental antibody PVRIG-A105.

### EXAMPLE 24 - The effect of anti-PVRIG humanized antibodies on improving the functions of antigen-specific CD8 T cells detected by CMV antigen-recall assay

The PBMCs were resuscitated, resuspended to 2×10⁶/mL by using complete medium (RPMI1640-Glutamax + 5% AB serum + 1% P/S+(1×) 2-β mercaptoethanol) containing 1 mg/mL CMV pp65(495-503) peptide (Anaspec, Cat No.AS-28328), 2 ng/mL human IL-2 (R&D, Cat No.IL-202) and 10 ng/mL human IL-7 (Peprotech, Cat No.200-07), inoculated in a 6-well plate (5 mL/well) and incubated at 37°C with 5% CO₂ for 6 days. On day 6, all the PBMCs were collected, and pp65 and IL-7 in the medium were removed. The cells were divided into two portions, resuspended in complete medium containing 100 IU/mL human IL-2, and cultured for another 2 days. On day 8, all the PBMCs were collected and resuspended in complete medium containing 100 IU/mL human IL-2, and the cell density was adjusted to 2×10⁶/mL. On day 11, all the PBMCs were collected. The proportion of CD8 T cells and CMV pp65 (495-503) specific CD8 T in PBMC (Fig. 24A) and the expression of PVRIG, TIGIT and PD-1 on the cells (Fig. 14B) were detected by flow cytometry. The detection antibodies in the flow cytometry were as follows: Livedead Near IR (Invitrogen, Cat No.L34976), CD8-PerCp Cy5.5 (BD, Cat No.565310), CD3-PE-Cy7 (Biolegend, Cat No.300316), T-select HLA-A*0201 CMV pp65 Tetramer-PE (MBL, Cat No.TS-0010-1C), PVRIG-AF488 (R&D, Cat No.FAB93651G-100UG), TIGIT-APC (Biolegend, Cat No.372706) and PD-1-BV421 (BD, Cat No.562516).

CD8 T cells were isolated from the induced PBMCs as effector cells by CD8 T cell isolation kit (Stemcell, Cat No. 17953) and resuspended with AIM-V to the cell density of 0.4×10⁶/mL. The expression of CD226 and purity of isolated CD8 T were detected. The target cells, Colo205, were digested by TrypLE^{™} Express Enzyme (Gibco, Cat No.12605010), resuspended in AIM-V (Gibco, Cat No.31035-025) containing 20 ng/mL pp65 to the cell density of 1×10⁶/mL and treated at 37°C with 5% CO₂ for 3 hours. The target cells were then centrifuged at 250 g for 5 minutes and resuspended in AIM-V to the cell density of 0.5×10⁶/mL after discarding the supernatant. Anti-PVRIG humanized antibodies and negative controls were diluted with AIM-V to 280 nM. The low-attachment, 96-well, U-bottom plate (Corning, Cat No.7007) was added with 50 µL of antibody, 50 µL of CD8 T and 100 µL of pp65-treated Colo205 in order, mixed well and incubated at 37°C with 5% CO₂ for 18 hours. In the experimental system, the final concentration of antibodies was 70 nM, CD8 T cells were 20,000/well, and Colo205 were 50,000/well. After incubation, the supernatant was collected by centrifugation at 400 g, and the level of human IFN-γ in the supernatant was detected with an ELISA kit (Dakewe, Cat No.1110003). In this system, the positive controls were parental antibodies before humanization, and the negative control was no treatment. As shown in Fig. 39, compared with the no treatment group, humanized PVRIG antibodies can significantly increase the level of IFN-γ in the cell supernatant. There was no significant difference between the humanized antibodies and their parental antibodies (one-way ANOVA analysis) in statistics, except that the effect of PVRIG-A105-H2 was significantly weaker than that of PVRIG-A105 (* p < 0.05, one-way ANOVA analysis). The detection antibodies used in flow cytometry to detect the purity of isolated CD8 T and the expression of CD226 were as follows: Live/dead-BV421 (Invitrogen, Cat No.L34964), CD8-FITC (BD, Cat No.555366) and CD226-PE-Cy7 (Biolegend, CatNo.338316). The detection antibodies used in flow cytometry to detect the expression of PVRL2, PVR, PD-L1 and HLA-A2 on Colo205 cells were as follows: live/dead-BV421 (Invitrogen, Cat No.L34964), PVRL2-APC (Biolegend, Cat No.337412), PVR-PerCp Cy5.5 (Biolegend, Cat No.337612), PD-L1-PE-Cy7 (BD, Cat No.558017) and HLA-A2-PE (Biolegend, Cat No.343306).

### EXAMPLE 25 - Construct design of anti-PVRIGxTIGIT humanized bispecific antibodies

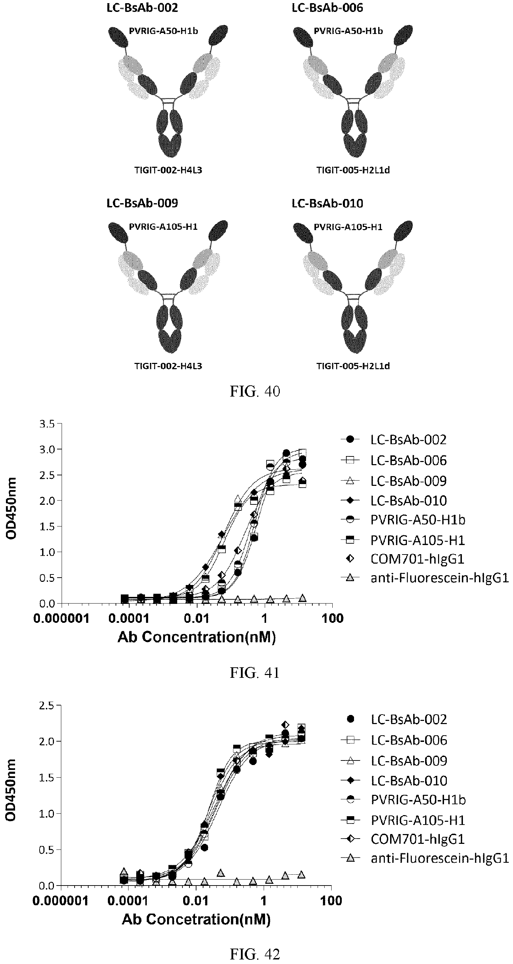

The anti-PVRIG humanized VHH antibodies (PVRIG-A50-H1b, PVRIG-A105-H1) were linked to the N-terminus of the anti-TIGIT humanized monoclonal antibodies (TIGIT-002-H4L3, TIGIT-005-H2L1d) heavy chain by G4S linker peptide to produce anti-PVRIGxTIGIT humanized bispecific antibodies (Fig. 40), which were named LC-BsAb-002, LC-BsAb-006, LC-BsAb-009 and LC-BsAb-010. Table 39 shows the sequences of heavy chain fusion polypeptide (HC) and light chain polypeptide (LC) of four bispecific antibodies.

**Table 39. The fusion polypeptide sequences of bispecific antibodies**

| HC/LC | Sequence No. | Sequences |
|---|---|---|
| LC-BsAb-002 LC | SEQ ID NO: 226 | DIVMTQSPDSLAVSLGERATINCKASQNVRTAVAWYQQKPGQSPKL MIYSASYRYTGVPDRFSGSGSGTDFTLTISSLQAEDVAVYYCQQYY TTPWTFGGGTKVEIKRTVAAPSVFIFPPSDEQLKSGTASVVCLLNNF YPREAKVQWKVDNALQSGNSQESVTEQDSKDSTYSLSSTLTLSKA |
| | | DYEKHKVYACEVTHQGLSSPVTKSFNRGEC Note: TIGIT-002-H4L3 VL (single underline) |
| LC-BsAb-002 HC | SEQ ID NO: 227 | EVQLVESGGGLVQPGGSLRLSCAASGFTFSYYDMSWVRQAPGKGL EWVSTINSDGGRTSYVDSVKGRFTISRDNSKNTLYLQMNSLRAEDT AVYYCVEGDPHNFGLESLSLRDFGSWGQGTMVTVSSGGGGSGGG GSGGGGSGGGGSEVQLQESGPGLVKPSETLSLTCAVSGYSITSDSW NWIRQPPGKKLEYIGYISYSGNTYYNPSLKSRVTISRDTSKNQFSLK LSSVTAADTAVYYCARLDFSNYGGAVDYWGQGTTVTVSSASTKGP SVFPLAPSSKSTSGGTAALGCLVKDYFPEPVTVSWNSGALTSGVHT FPAVLQSSGLYSLSSVVTVPSSSLGTQTYICNVNHKPSNTKVDKKV EPKSCDKTHTCPPCPAPELLGGPSVFLFPPKPKDTLMISRTPEVTCV VVDVSHEDPEVKFNWYVDGVEVHNAKTKPREEQYNSTYRVVSVL TVLHQDWLNGKEYKCKVSNKALPAPIEKTISKAKGQPREPQVYTL PPSRDELTKNQVSLTCLVKGFYPSDIAVEWESNGQPENNYKTTPPV LDSDGSFFLYSKLTVDKSRWQQGNVFSCSVMHEALHNHYTQKSLS LSPGK Note: PVRIG-A50-H1b (double underline) + (G4S)4 Linker (italic) + TIGIT-002-H4L3 VH (single underline) |
| LC-BsAb-006 LC | SEQ ID NO: 228 | DIVMTQSPDSLAVSLGERATINCKASQHVSNAVAWYQHKPGQSPKL LIYSASYRYTGVPDRFSGSGSGTDFTLTISSLQAEDVAVYYCQQHY NTPHTFGGGTKVEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC Note: TIGIT-005-H2L1d VL(single underline) |
| LC-BsAb-006 HC | SEQ ID NO: 229 | EVQLVESGGGLVQPGGSLRLSCAASGFTFSYYDMSWVRQAPGKGL EWVSTINSDGGRTSYVDSVKGRFTISRDNSKNTLYLQMNSLRAEDT AVYYCVEGDPHNFGLESLSLRDFGSWGQGTMVTVSSGGGGSGGG GSGGGGSGGGGSEVQLVQSGAEVKKPGASVKVSCKASGYAFTNY LIEWVRQAPGQRLEWMGVINPGSGGTNYKEKFKGRVTITADKSSS TAYMELSSLRSEDTAVYYCARGEYFFFDYWGQGTTVTVSSASTKG PSVFPLAPSSKSTSGGTAALGCLVKDYFPEPVTVSWNSGALTSGVH TFPAVLQSSGLYSLSSVVTVPSSSLGTQTYICNVNHKPSNTKVDKK VEPKSCDKTHTCPPCPAPELLGGPSVFLFPPKPKDTLMISRTPEVTC VVVDVSHEDPEVKFNWYVDGVEVHNAKTKPREEQYNSTYRVVS VLTVLHQDWLNGKEYKCKVSNKALPAPIEKTISKAKGQPREPQVY TLPPSRDELTKNQVSLTCLVKGFYPSDIAVEWESNGQPENNYKTTPP VLDSDGSFFLYSKLTVDKSRWQQGNVFSCSVMHEALHNHYTQKSL SLSPGK |
| | | Note: PVRIG-A50-H1b (double underline) + (G4S)4 Linker (italic) + TIGIT-005-H2L1d VH (single underline) |
| LC-BsAb-009 LC | SEQ ID NO: 230 | DIVMTQSPDSLAVSLGERATINCKASQNVRTAVAWYQQKPGQSPKL MIYSASYRYTGVPDRFSGSGSGTDFTLTISSLQAEDVAVYYCQQYY TTPWTFGGGTKVEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC Note: TIGIT-002-H4L3 VL (single underline) |
| LC-BsAb-009 HC | SEQ ID NO: 231 | EVQLVESGGGLVQPGGSLRLSCAASGRTFDRHTMSWFRQAPGKER EFVATASRIPGDTYYVDSVKGRFTISRDNAKNSLYLQMNSLRAEDT AVYYCAATSAYCSEVDCYEKGSWYDNWGQGTMVTVSSGGGGSG GGGSGGGGSGGGGSEVQLQESGPGLVKPSETLSLTCAVSGYSITSDS WNWIRQPPGKKLEYIGYISYSGNTYYNPSLKSRVTISRDTSKNQFSL KLSSVTAADTAVYYCARLDFSNYGGAVDYWGQGTTVTVSSASTK GPSVFPLAPSSKSTSGGTAALGCLVKDYFPEPVTVSWNSGALTSGV HTFPAVLQSSGLYSLSSVVTVPSSSLGTQTYICNVNHKPSNTKVDK KVEPKSCDKTHTCPPCPAPELLGGPSVFLFPPKPKDTLMISRTPEVT CVVVDVSHEDPEVKFNWYVDGVEVHNAKTKPREEQYNSTYRVVS VLTVLHQDWLNGKEYKCKVSNKALPAPIEKTISKAKGQPREPQVY TLPPSRDELTKNQVSLTCLVKGFYPSDIAVEWESNGQPENNYKTTPP VLDSDGSFFLYSKLTVDKSRWQQGNVFSCSVMHEALHNHYTQKSL SLSPGK Note: PVRIG-A105-H1 (double underline) + (G4S)4 Linker (italic) + TIGIT-002-H4L3 VH (single underline) |
| LC-BsAb-010 LC | SEQ ID NO: 232 | DIVMTQSPDSLAVSLGERATINCKASQHVSNAVAWYQHKPGQSPKL LIYSASYRYTGVPDRFSGSGSGTDFTLTISSLQAEDVAVYYCQQHY NTPHTFGGGTKVEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC Note: TIGIT-005-H2L1d VL (single underline) |
| LC-BsAb-010 HC | SEQ ID NO: 233 | EVQLVESGGGLVQPGGSLRLSCAASGRTFDRHTMSWFRQAPGKER EFVATASRIPGDTYYVDSVKGRFTISRDNAKNSLYLQMNSLRAEDT AVYYCAATSAYCSEVDCYEKGSWYDNWGQGTMVTVSSGGGGSG GGGSGGGGSGGGGSEVQLVQSGAEVKKPGASVKVSCKASGYAFT NYLIEWVRQAPGQRLEWMGVINPGSGGTNYKEKFKGRVTITADKS SSTAYMELSSLRSEDTAVYYCARGEYFFFDYWGQGTTVTVSSAST KGPSVFPLAPSSKSTSGGTAALGCLVKDYFPEPVTVSWNSGALTSG |
| | | VHTFPAVLQSSGLYSLSSVVTVPSSSLGTQTYICNVNHKPSNTKVD KKVEPKSCDKTHTCPPCPAPELLGGPSVFLFPPKPKDTLMISRTPEV TCVVVDVSHEDPEVKFNWYVDGVEVHNAKTKPREEQYNSTYRV VSVLTVLHQDWLNGKEYKCKVSNKALPAPIEKTISKAKGQPREPQ VYTLPPSRDELTKNQVSLTCLVKGFYPSDIAVEWESNGQPENNYKT TPPVLDSDGSFFLYSKLTVDKSRWQQGNVFSCSVMHEALHNHYTQ KSLSLSPGK Note: PVRIG-A105-H1 (double underline) + (G4S)4 Linker (italic) + TIGIT-005-H2L1d VH (single underline) |

While constructing the bispecific antibody, the positive control antibody was also constructed. The anti-TIGIT positive control antibody is RG6058-hIgG1 (Roche), and the anti-PVRIG positive control antibody is COM701-hIgG4 (Compugen). See above for the corresponding amino acid sequences

### EXAMPLE 26 - The specific binding of anti-PVRIGxTIGIT humanized bispecific antibodies to human and cynomolgus monkey PVRIG protein detected by ELISA

The ELISA plate was pre-coated with 50 µL/well of 1.0 µg/mL human PVRIG (AcroBiosystems, Cat NO.PVG-H52H4) or 50 µL/well of 0.5 µg/mL cynomolgus monkey PVRIG (Novoprotein, Cat NO.C09B). The tested antibodies were serially diluted (initial concentration 13 nM, 3-fold serial dilution, 12 concentration points), added into the plate (50 µL/well) and incubated at 37°C for 2.0 hours. After washing the plate, the Goat anti-human IgG Fc-HRP (Merck, Cat NO.AP113P) working solution (dilution ratio 1:5000, 50 µL/well) were added into wells and incubated at 37°C for 1.0 hour. A HRP substrate, TMB (KPL, Cat NO.5120-0077) was added into wells after washing the plate again and incubated at 37°C for 10 minutes for color development. After adding a termination solution to terminate the reaction, the absorbance value was read by a microplate reader (PE, Ensight-HH3400). An antibody binding curve was drawn with molar concentration of antibodies as the abscissa and the corresponding OD value as the ordinate. The EC50 value was calculated by using a Four Parameter Logistic Fit (GraphPadPrism9). The smaller the EC50 value, the stronger the ability of the antibodies binds to human or cynomolgus monkey PVRIG. The positive control antibodies are COM701-hIgG1, PVRIG-A50-H1b and PVRIG-A105-H1; and the negative control antibodies are anti-Fluorescein-hIgG1 (in house). The binding results of the 4 humanized bispecific antibodies to human PVRIG protein are shown in Fig. 41 and Tables 40 and 41, and the binding results to cynomolgus monkey PVRIG are shown in Fig. 42 and Tables 40 and 41. The data shows that all 4 humanized bispecific antibodies can specifically bind to human or cynomolgus monkey PVRIG protein. The binding of LC-BsAb-002 and LC-BsAb-006 to human PVRIG protein is slightly weaker than that of the corresponding monoclonal antibody (PVRIG-A50-H1b) and the positive control (COM701-hIgG1). The binding of LC-BsAb-009 and LC-BsAb-010 to human or cynomolgus monkey PVRIG protein is comparable to that of the corresponding monoclonal antibody (PVRIG-A105-H1) and superior to the positive control (COM701-hIgG1).

**Table 40. The specific binding of humanized bispecific antibodies to human or cynomolgus monkey PVRIG protein detected by ELISA**

| Name | Bind to human PVRIG protein | | | Bind to cynomolgus monkey PVRIG protein | | |
|---|---|---|---|---|---|---|
| | EC50 | % relative activity | | EC50 | % relative activity | |
| | (nM) | PVRIG-A50-H1b /tested antibody | COM701-hIgG1 / tested antibody | (nM) | PVRIG-A50-H1b / tested antibody | COM701-hIgG1 / tested antibody |
| LC-BsAb-002 | 0.5738 | 66.1% | 43.2% | 0.04402 | 86.4% | 82.5% |
| LC-BsAb-006 | 0.5072 | 74.8% | 48.9% | 0.03851 | 98.7% | 94.3% |
| PVRIG-A50-H1b | 0.3793 | 100.0% | 65.4% | 0.03802 | 100.0% | 95.5% |
| COM701-hIgG1 | 0.2481 | 152.9% | 100.0% | 0.0363 | 104.7% | 100.0% |
| anti-Fluorescein-hI gG1 | ∼40328 | / | / | ∼3.999 | / | / |

**Table 41. The specific binding of humanized bispecific antibodies to human or cynomolgus monkey PVRIG protein detected by ELISA**

| Name | Bind to human PVRIG protein | | | Bind to cynomolgus monkey PVRIG protein | | |
|---|---|---|---|---|---|---|
| | EC50 | % relative activity | | EC50 | % relative activity | |
| | (nM) | PVRIG-A105-H1 /tested antibody | COM701-hIgG1 / tested antibody | (nM) | PVRIG-A105-H1 /tested antibody | COM701-hIgG1 / tested antibody |
| LC-BsAb-009 | 0.06073 | 104.8% | 408.5% | 0.02563 | 93.4% | 141.6% |
| LC-BsAb-010 | 0.06166 | 103.2% | 402.4% | 0.02322 | 103.1% | 156.3% |
| PVRIG-A105-H1 | 0.06362 | 100.0% | 390.0% | 0.02395 | 100.0% | 151.6% |
| COM701-hIgG1 | 0.2481 | 25.6% | 100.0% | 0.0363 | 66.0% | 100.0% |
| anti-Fluorescein-hIgG1 | ∼ 40328 | / | / | ∼ 3.999 | / | / |

### EXAMPLE 27 - The specific binding of anti-PVRIGxTIGIT humanized bispecific antibodies to human and cynomolgus monkey TIGIT protein detected by ELISA

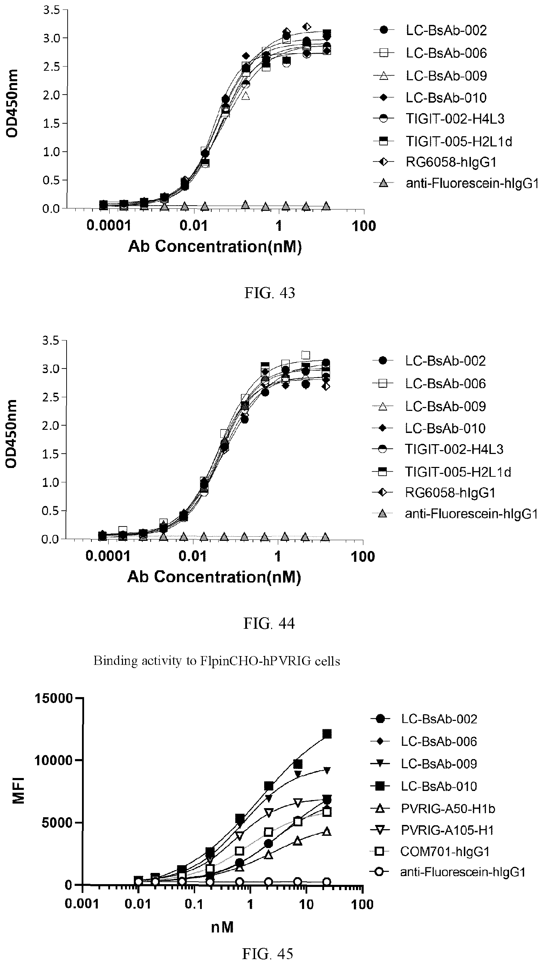

The ELISA plate was pre-coated with 50 µL/well of 4.0 µg/mL goat anti-mouse IgG antibody (Jackson, Cat NO. 115-006-071). After washing with blocking buffer, the plate was added with 50 µL/well of 30 ng/mL human TIGIT ECD-mFc (in house) or cynomolgus monkey TIGIT ECD-mFc (in house) and incubated at 37°C for 2.0 hours. After washing the plate, the serial diluted antibodies (initial concentration 13 nM, 3-fold serial dilution, 12 concentration points) were added into the plate (50 µL/well) and incubated at 37°C for 2.0 hours. After washing the plate, the goat anti-human IgG Fc-HRP (Merck, Cat NO.AP113P)) working solution (dilution ratio 1:5000, 50 µL/well) were added into wells and incubated at 37°C for 1.0 hour. A HRP substrate, TMB (KPL, Cat NO.5120-0077) was added into wells after washing the plate again for color development. After adding a termination solution to terminate the reaction, the absorbance value was read by a microplate reader (PE, Ensight-HH3400). An antibody binding curve was drawn with molar concentration of antibodies as the abscissa and the corresponding OD value as the ordinate. The EC50 value was calculated by using a Four Parameter Logistic Fit (GraphPad Prism9). The smaller the EC50 value, the stronger the ability of the antibodies binds to human or cynomolgus PVRIG. The positive control antibodies are RG6058-hIgG1, TIGIT-002-H4L3 and TIGIT-005-H2L1d; and the negative control antibodies are anti-Fluorescein-hIgG1 (in house). The binding results of the 4 humanized bispecific antibodies to human TIGIT protein are shown in Fig. 43 and Tables 42 and 43, and the binding results to cynomolgus monkey TIGIT are shown in Fig. 44 and Tables 42 and 43. The data shows that all 4 humanized bispecific antibodies can specifically bind to human or cynomolgus monkey TIGIT protein. The binding of LC-BsAb-002 and LC-BsAb-009 to human TIGIT protein is superior to that of the positive control (RG6058-hIgG1), and comparable to that of the corresponding monoclonal antibody (TIGIT-002-H4L3). In terms of binding to cynomolgus monkey TIGIT protein, LC-BsAb-009 is comparable to the corresponding monoclonal antibody (TIGIT-002-H4L3) and the positive control (RG6058-hIgG1); LC-BsAb-002 is slightly weaker than that of both the corresponding monoclonal antibody and the positive control. The binding of LC-BsAb-006 and LC-BsAb-010 to human or cynomolgus monkey TIGIT protein is superior to the corresponding monoclonal antibody (TIGIT-005-H2L1d) and the positive control (RG6058-hIgG1).

**Table 42. The specific binding of humanized bispecific antibodies to human or cynomolgus monkey TIGIT protein detected by ELISA**

| Name | Bind to human TIGIT protein | | | Bind to cynomolgus monkey TIGIT protein | | |
|---|---|---|---|---|---|---|
| | EC50 | %relative activity | | EC50 | %relative activity | |
| | (nM) | TIGIT-002-H4L3 /tested antibody | RG6058-hIgG1 /tested antibody | (nM) | TIGIT-002-H4L3 /tested antibody | RG6058-hIgG1 /tested antibody |
| LC-BsAb-002 | 0.03565 | 105.0% | 141.3% | 0.05535 | 74.9% | 83.2% |
| LC-BsAb-009 | 0.04549 | 82.3% | 110.7% | 0.04185 | 99.1% | 110.0% |
| TIGIT-002-H4L3 | 0.03742 | 100.0% | 134.6% | 0.04146 | 100.0% | 111.1% |
| RG6058-hIgG1 | 0.05038 | 74.3% | 100.0% | 0.04605 | 90.0% | 100.0% |
| anti-Fluorescein-hIgG1 | ∼ 0.000 | / | / | ∼ 0.6872 | / | / |

**Table 43. The specific binding of humanized bispecific antibodies to human or cynomolgus monkey TIGIT protein detected by ELISA**

| Name | Bind to human TIGIT protein | | | Bind to cynomolgus monkey TIGIT protein | | |
|---|---|---|---|---|---|---|
| | EC50 | %relative activity | | EC50 | %relative activity | |
| | (nM) | TIGIT-005-H2L1 d /tested antibody | RG6058-hIg G1 /tested antibody | (nM) | TIGIT-005-H2L1 d /tested antibody | RG6058-hIgG1 /tested antibody |
| LC-BsAb-006 | 0.03563 | 120.5% | 141.4% | 0.04126 | 108.0% | 111.6% |
| LC-BsAb-010 | 0.02761 | 155.5% | 182.5% | 0.03359 | 132.7% | 137.1% |
| TIGIT-005-H2L1d | 0.04292 | 100.0% | 117.4% | 0.04456 | 100.0% | 103.3% |
| RG6058-hIgG1 | 0.05038 | 85.2% | 100.0% | 0.04605 | 96.8% | 100.0% |
| anti-Fluorescein-hIgG1 | ∼ 0.000 | / | / | ∼ 0.6872 | / | / |

### EXAMPLE 28 - The binding activity of anti-PVRIGxTIGIT humanized bispecific antibodies to FlpinCHO human PVRIG and FlpinCHO cynomolgus monkey PVRIG detected by FACS.

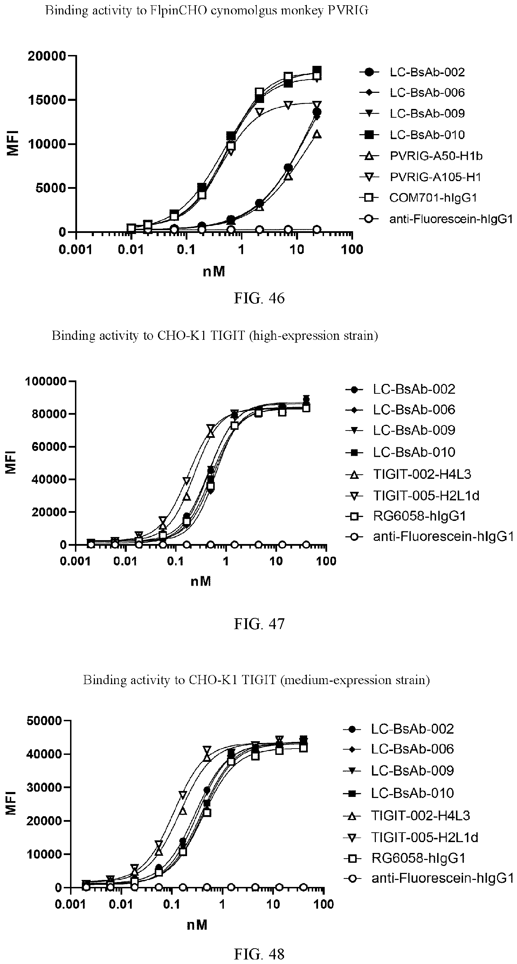

CHO-K1 stable cells (ATCC^{®}CCL-61^{™}) transfected with human or cynomolgus monkey PVRIG high expression plasmids were named FlpinCHO-hPVRIG and FlpinCHO-cynoPVRIG. Human PVRIG full-length plasmids (NCBI Ref Seq: NP_076975) and cynomolgus monkey PVRIG full-length plasmid (NCBI Ref Seq: XP_014989941) were synthesized by General Biol. The experiment was performed when the cell density did not exceed 80%. After discarding the cell culture medium, the cells were rinsed with PBS and digested for 8-10 minutes by adding 1mL Versene (Gibico, 15040-066). The Ham's F12 (Gibico, 21127-022) complete medium containing 10% FBS was then added to terminate the digestion and acquire cell suspension. After cell counting, an appropriate amount of cell suspension was centrifuged at 350×g to remove the supernatant, washed twice with PBS, stained with Zombie violet (Biolegend, 423114) and incubated at room temperature for 20 minutes. After incubation, the cells were added with staining buffer (2% FBS+PBS) to stop staining, centrifuged at 350×g to remove the supernatant, washed twice and resuspended with staining buffer to a density of 2×10⁶ cells/mL. The cells were plated into a 96-well plate (50µL of cell suspension per well) for further use. The antibodies were diluted from the highest concentration of 46 nM (two-fold concentration) with staining buffer by 3.3-fold serial dilution. The diluted antibodies were added to the well containing 50 µL of cell suspension. The plate was placed on a microplate shaker at 400 rpm for 1 minute to fully mix the antibodies and cells, and then incubated at 4°C for 30 minutes. After the incubation, the cells were washed twice with staining buffer (200 µL per well) and centrifuged at 350×g for 5 minutes to discard the supernatant. The PE goat anti-human IgG Fc antibody (ebioscience, 12-4998-82) was diluted 250 times with staining buffer, added into the washed wells (100 µL per well), mixed well, and stained at 4°C for 30 minutes. After staining, the cells were washed twice with staining buffer, finally resuspended with 200 µL staining buffer and analyzed by flow cytometry (BD, Canto II). The stronger the signal, the stronger the ability of the antibodies binds to PVRIG. Fig. 45 shows that the four humanized bispecific antibodies have good binding activity to human PVRIG, as well as cynomolgus monkey PVRIG (Fig. 46), and all of them are superior to their corresponding anti-PVRIG humanized monoclonal antibodies.

### EXAMPLE 29 - The binding activity of anti-PVRIGxTIGIT humanized bispecific antibodies to CHO-K1 human TIGIT (high/medium/low expression cell strain) and CHO-K1 cynomolgus monkey TIGIT cells detected by FACS.

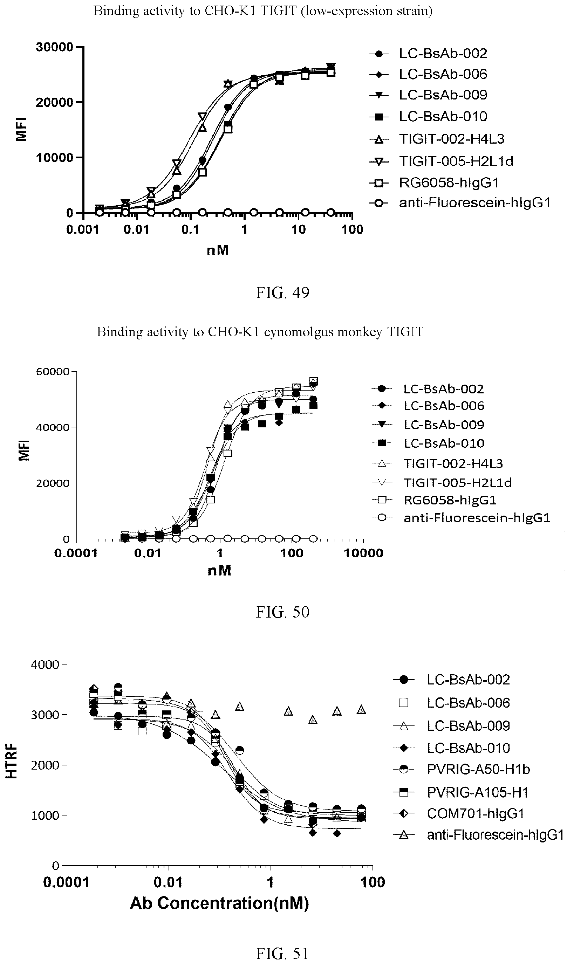

The collected cells were washed once with PBS (Hyclone, Cat NO.SH30256), resuspended to 2×10⁵/50 µL with 1% BSA-PBS. The antibodies were diluted to 80 nM with 1% BSA-PBS (diluted by 3-fold serial dilution for 12 concentration points). The 50 µL cells were then mixed with 50 µL diluted antibodies, and then incubated at 4°C for 60 minutes. The cells were washed twice with PBS, resuspended with Alexa Fluor^{®} 647 fluorescein labeled secondary antibody (1:800) (Jackson, Cat NO.109-605-088), 100 µL/well, and incubated at 4°C for 40 minutes. After washing twice with PBS, the cell samples were resuspended with 1% BSA-PBS, 100 µL/well and then analyzed by flow cytometry (BD, Canto II). The experimental results show that the four humanized bispecific antibodies have good binding activity to CHO-K1 human TIGIT (high/medium/low expression cell strain, Fig. 47, 48 and 49) and CHO-K1 cynomolgus monkey TIGIT cells (Fig. 50).

### EXAMPLE 30 - The effect of anti-PVRIGxTIGIT humanized bispecific antibodies blocking the interaction between PVRIG protein and PVRL2 protein detected by HTRF

PVRIG-mFc (ACRO Biosystems, Cat NO.PVG-H5253) and Bio-CD112-His (Sino Biological, Cat NO.10005-H08H) were diluted to 0.5 µg/mL, respectively. Streptavidin-Tb cryptate (Cisbio) and PAb anti mouse IgG-XL665 (Cisbio) were diluted to 20 µg/mL and 0.8 µg/mL, respectively. The tested antibodies were diluted from the initial concentration of 120 nM, by 3-fold serial dilution for 12 concentration points. The diluted PVRIG-mFc, Bio-CD112-His, Streptavidin-Tb Crytate and PAb anti mouse IgG-XL665 were mixed at 1:1:1:1, and added into a 384-well plate (PE, Cat NO.6007299), 10 µL/well. 10µL/well of serially diluted antibodies was added into wells, centrifuged at 1500 rpm for 30s, and incubated at 37°C for 1 hour. The plate was read on the microplate reader (PE, Envision2105) with wavelengths of 665 nm and 620 nm. Data conversion is performed according to the formula: Ratio=Signal 665 nm/Signal 620 nm × 10⁴. A Four Parameter Logistic Fit was performed with the molar concentration of the antibodies as the abscissa and Ratio as the ordinate to calculate IC50. The smaller the IC50, the better the effect of the antibody block the binding of PVRIG to PVRL2. The positive controls in this experiment are COM701-hIgG1, PVRIG-A50-H1b and PVRIG-A105-H1; and the negative control antibody is anti-Fluorescein-hIgG1 (in house). The effect of 4 humanized bispecific antibodies blocking the binding of PVRIG to PVRL2 is shown in Fig. 51 and Tables 44 and 45. The data shows that all four humanized bispecific antibodies can block the binding of PVRIG to PVRL2 protein. The blocking effect of LC-BsAb-002 is better than its corresponding monoclonal antibody (PVRIG-A50-H1b) and the positive control (COM701-hIgG1). The blocking effect of LC-BsAb-006 is better than its corresponding monoclonal antibody (PVRIG-A50-H1b), but slightly weaker than the positive control (COM701-hIgG1). The blocking effects of LC-BsAb-009 and LC-BsAb-010 are not as good as their corresponding monoclonal antibodies (PVRIG-A105-H1) and the positive control (COM701-hIgG1).

**Table 44. The effect of humanized bispecific antibodies blocking the binding of PVRIG to PVRL2**

| Name | Block the binding of human PVRIG protein to human PVRL2 protein | | |
|---|---|---|---|
| | IC50 | %relative activity | |
| | (nM) | PVRIG-A50-H1b/tested antibody | COM701-hIgG1/tested antibody |
| LC-BsAb-002 | 0.1104 | 186.5% | 124.4% |
| LC-BsAb-006 | 0.1805 | 114.1% | 76.1% |
| PVRIG-A50-H1b | 0.2059 | 100.0% | 66.7% |
| COM701-hIgG1 | 0.1373 | 150.0% | 100.0% |
| anti-Fluorescein-hIgG1 | ∼ 0.03023 | / | / |

**Table 45. The effect of humanized bispecific antibodies blocking the binding of PVRIG to PVRL2**

| Name | Block the binding of human PVRIG protein to human PVRL2 protein | | |
|---|---|---|---|
| | IC50 | %relative activity | |
| | (nM) | PVRIG-A105-H1/ tested antibody | COM701-hIgG1/ tested antibody |
| LC-BsAb-009 | 0.1956 | 65.6% | 70.2% |
| LC-BsAb-010 | 0.1452 | 88.4% | 94.6% |
| PVRIG-A105-H1 | 0.1283 | 100.0% | 107.0% |
| COM701-hIgG1 | 0.1373 | 93.4% | 100.0% |
| anti-Fluorescein-hIgG1 | ∼ 0.03023 | / | / |

### EXAMPLE 31 - The effect of anti-PVRIGxTIGIT humanized bispecific antibodies blocking CHO-K1 human CD112 cells binding to human PVRIG-mFc proteins detected by FACS

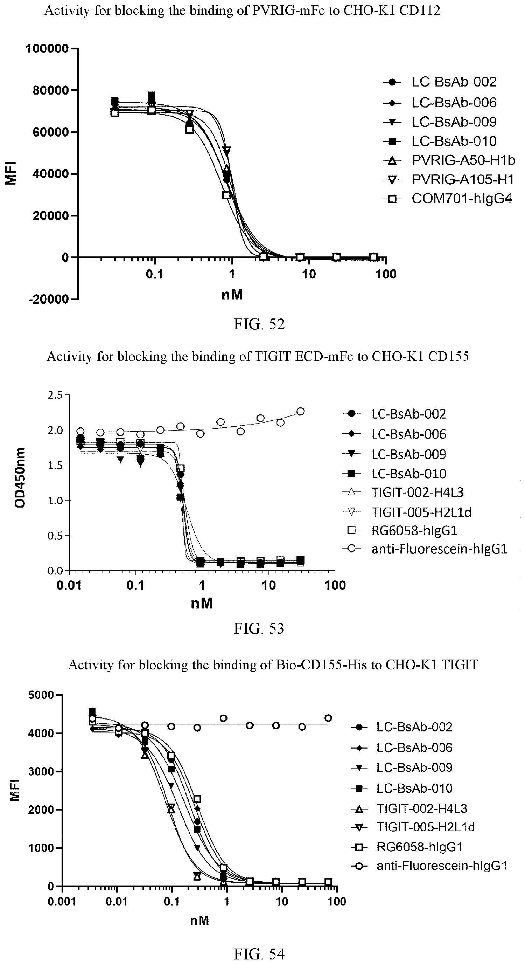

CHO-K1 stable cells transfected with human CD112 high expression plasmid was named CHO-K1-CD112. Human CD112 full-length plasmid (NP_001036189.1/NCBI Ref Seq: Q92692) was synthesized by General Biol. The experiment was performed when the cell density did not exceed 80%. After discarding the cell culture medium, the cells were rinsed with PBS and digested for 2 minutes by adding 1mL trypsin (Gibico, 25200-72). The Ham's F12 (Gibico, 21127-022) complete medium containing 10% FBS was then added to stop the digestion and acquire cell suspension. After counting with a cell counter (Beckman Coulter, Vi-CELL), an appropriate amount of cell suspension was centrifuged at 350×g to remove the supernatant, washed twice with PBS, stained with Zombie violet (Biolegend, 423114) and incubated at room temperature for 20 minutes. After incubation, the cells were added with staining buffer (2% FBS+PBS) to stop staining, centrifuged at 350×g to remove the supernatant, washed twice and resuspended with staining buffer to a density of 2×10⁶ cells/mL. The cells were plated into a 96-well plate (50µL of cell suspension per well) for further use. A working solution of human PVRIG-mFc protein (Aero, PVG-H5253) prepared with staining buffer, 1µg/mL (four-fold concentration), was added into a 96-well plate (50µL PVRIG-mFc working solution per well). The antibodies were diluted from the highest concentration of 275 nM (four-fold concentration) with staining buffer by 3-fold serial dilution. The diluted antibodies were added to the well containing 50 µL of PVRIG-mFc. The plate was placed on a microplate shaker at 400 rpm for 1 minute to fully mix the antibodies and PVRIG-mFc proteins, and then incubated at 4°C for 30 minutes. After the incubation, the cell suspension prepared above (100µL) was added into the wells, gently mixed with the pipette tip and incubated at 4°C for 30 minutes. After the incubation, the cells were washed twice with staining buffer (200 µL per well) and centrifuged at 350×g for 5 minutes to discard the supernatant. The PE goat anti-mouse IgG Fc antibody (Biolegend, 405337) was diluted 250 times with staining buffer, added into the washed wells (100 µL per well), mixed well, and stained at 4°C for 30 minutes. After staining, the cells were washed twice with staining buffer, finally resuspended with 200 µL staining buffer and analyzed by flow cytometry (BD, Canto II). The weaker the fluorescence signal, the stronger the ability of the antibodies blocks the binding of CHO-K1 human CD112 cells to the PVRIG-mFc protein. As shown in Fig.52, all 4 humanized bispecific antibodies can block the binding of CHO-K1 human CD112 cells to human PVRIG-mFc protein.

### EXAMPLE 32 - The effect of anti-PVRIGxTIGIT humanized bispecific antibodies blocking the binding activity of human TIGIT to CHO-K1 CD155 detected by ELISA

The collected CHO-K1 CD155 cells constructed in Example 2 were adjusted to a concentration of 5×10⁵/mL with 10% FBS-DMEM/F12 medium (Excell, FSP500; Gibco, 11330), added into a 96-well cell culture plate (corning, 3599), 100 µL/well, and cultured overnight at 37 °C with 5% CO₂. After discarding the culture supernatant, the cells were fixed with a cell fixation solution (Beyotime, P0098), 50 µL/well, at room temperature for 1 hour. After washing once with 0.05% Tween 20-PBS on the Microplate Washer, the plate was added with 5% skim milk powder-PBS, 250 µL/well, and incubated at 37°C for 2-4 hours. The plate was then washed 3 times with 0.05% Tween 20-PBS on the Microplate Washer. The sample were mixed with human TIGIT ECD-mFc (working concentration: 100 ng/mL) and incubated for 0.5 hour. The mixed solution of antigen and antibody was added to the cell plate (50 µL/well). The cells were incubated with the mixture at 37°C for 1.5-2 hours and then washed with 0.05% Tween 20-PBS for 3 times on the Microplate Washer. The cell plate was then added with 50 µL/well HRP enzyme-labeled antibodies (Jackson, 115-035-003) diluted with 1% BSA (Sangon Biotech, A500023-0100)-PBS at a dilution ratio of 1:5000, and incubated at 37°C for 1 hour. After washing 3 times with 0.05% Tween 20-PBS on the Microplate Washer, the plate was added with TMB chromogenic solution (KPL, 52-00-03), 50 µL/well, and incubated at 37°C for 10 minutes. 1M HCL, 50 µL/well, was then added into the plate to terminate the reaction, and the OD450 nm was read by a microplate reader (Biotek, Powerwave HT). As shown in Fig.53, all 4 humanized bispecific antibodies can block the binding between the human TIGIT and CHO-K1-CD155.

### EXAMPLE 33 - The effect of anti-PVRIGxTIGIT humanized bispecific antibodies blocking the binding activity of Bio-CD155-His to CHO-K1 human TIGIT detected by FACS

The collected cells were washed once with PBS (Hyclone, SH30256), resuspended to 2×10⁵/40 µL with 1% BSA-PBS. The antibodies were diluted to 210 nM with 1% BSA-PBS (diluted by 3-fold serial dilution for 12 concentration points). The Bio-CD155-His (Sino Biological Inc., 10109-H08H) was diluted to 3 µg/mL with 1% BSA-PBS. The 40 µL cells were then mixed with 40 µL diluted antibodies and 40 µL diluted Bio-CD155-His, and then incubated at 4°C for 60 minutes. The cells were washed twice with PBS, resuspended with APC labeled streptavidin (dilution ratio 1:1700, Biolegend, 405243), 100 µL/well, and incubated at 4°C for 40 minutes. The cell samples were washed twice with PBS, resuspended with 1% BSA-PBS (100 µL/well) and then analyzed by flow cytometry (BD, Canto II). As shown in Fig. 54, all 4 humanized bispecific antibodies can block the binding between the Bio-CD155-His and CHO-K1- human TIGIT.

### EXAMPLE 34 - The binding activity of anti-PVRIGxTIGIT humanized bispecific antibodies to human PBMCs detected by FACS

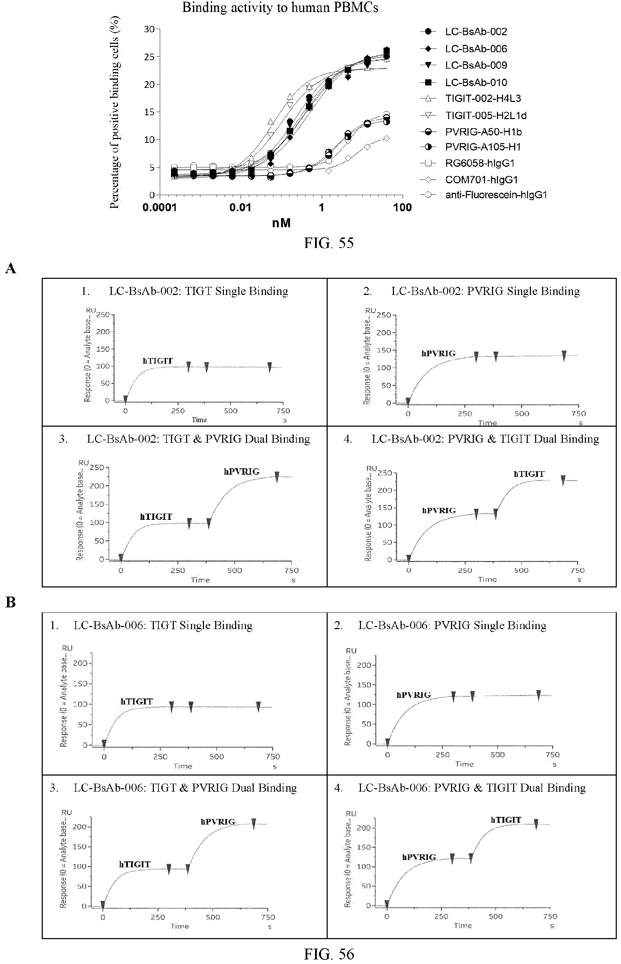

The fresh human PBMCs (AllCells, PB004-C) were adjusted to a density of 5×10⁵/mL, added with SEA (Toxin Technology, Inc., AT101) to 100 ng/mL, and cultured at 37°C with 5% CO₂ for 3 days. The cells were collected 3 days later, washed once with PBS (Hyclone, SH30256), added with Fc Block (BD, 564220) and then incubated at 4°C for 10 minutes. After washing twice with PBS, the PBMCs were resuspended to 2×10⁵ /50 µL with 1% BSA-PBS. The humanized antibodies were diluted to 80 nM with 1% BSA-PBS (diluted by 3-fold serial dilution for 12 concentration points). The 50 µL cells were then mixed with 50 µL diluted antibodies, and then incubated at 4°C for 60 minutes. The cells were washed twice with PBS, resuspended with Alexa Fluor^{®} 647 fluorescein labeled secondary antibody (dilution ratio 1:800, Jackson, 109-605-088), 100 µL/well, and incubated at 4°C for 60 minutes. After washing twice with PBS, the cell samples were resuspended with 1% BSA-PBS, 100 µL/well, and then analyzed by flow cytometry (BD, Canto II). As shown in Fig. 55, all 4 humanized bispecific antibodies have good binding activity to human PBMCs.

### EXAMPLE 35 - The affinity of anti-PVRIGxTIGIT humanized bispecific antibodies for human, cynomolgus monkey and mouse TIGIT and PVRIG proteins detected by BIAcore

Protein A chip was used in the BIAcore assay. The time required for the chip to capture the diluted antibody and saturate the binding antigen to reach Rₘₐₓ (Maximum binding capacity, 50 RU) was measured by manual run. The human, cynomolgus monkey and mouse TIGIT and PVRIG proteins were serially diluted to 20, 10, 5, 2.5, 1.25 nM. The affinity of antibody for antigen was measured by multi-cycle kinetics. In each cycle, the antibodies were injected prior to the injection of gradient concentraions of human, cynomolgus monkey and mouse TIGIT and PVRIG proteins allowing the occurrence of antibody-antigen association and dissociation. After each cycle, the Protein A chip was regenerated by Glycine, pH 1.5 (to remove the proteins on the chip). The affinity KD of antibody for antigen was calculated by BIAcore T200 analysis software. As shown in Table. 46, two humanized bispecific antibodies specifically bind to human and cynomolgus TIGIT and PVRIG proteins with high affinity, but do not bind to mouse TIGIT and PVRIG proteins.

**Table 46. The affinity of humanized bispecific antibodies for TIGIT and PVRIG proteins from various species**

| **Antibody** | **Antigen** | **Binding kinetics** | | | | |
|---|---|---|---|---|---|---|
| | | **ka (1/Ms)** | **kd (1/s)** | **KD (M)** | **Rmax** | **Capture Level** |
| LC-BsAb-002 | hTIGIT | 1.75E+06 | 2.59E-04 | 1.48E-10 | 100.3 | 727 |
| | cynoTIGIT | 4.92E+05 | 6.43E-03 | 1.31E-08 | 112.9 | 726 |
| | mTIGIT | / | / | / | 0.1 | 726 |
| | hPVRIG | 3.09E+05 | 5.87E-05 | 1.90E-10 | 106.6 | 557 |
| | cynoPVRIG | 3.57E+04 | 1.31E-04 | 3.66E-09 | 109.7 | 659 |
| | mPVRIG | / | / | / | 0.1 | 579 |
| LC-BsAb-006 | hTIGIT | 1.65E+06 | 2.14E-04 | 1.30E-10 | 99.4 | 696 |
| | cynoTIGIT | 1.09E+06 | 1.84E-03 | 1.69E-09 | 108.0 | 695 |
| | mTIGIT | / | / | / | 0.9 | 695 |
| | hPVRIG | 3.40E+05 | 5.01E-05 | 1.48E-10 | 103.6 | 538 |
| | cynoPVRIG | 3.60E+04 | 1.42E-04 | 3.95E-09 | 109.8 | 631 |
| | mPVRIG | / | / | / | 0.2 | 1140 |

### EXAMPLE 36 - The co-binding of anti-PVRIGxTIGIT humanized bispecific antibodies to human TIGIT and PVRIG proteins detected by BIAcore

The BIAcore was used to characterize the property of bispecific antibody simultaneously binding to two antigens. Firstly, antibodies, LC-BsAb-002 and LC-BsAb-006, were captured by Protein A chip. TIGIT and PVRIG proteins with his-tag were then injected, respectively, and followed by the continuous injection of TIGIT and PVIRIG, PVRIG and TIGIT. The binding responses of antibodies and antigens were recorded. Finally, the Protein A chip was regenerated by Glycine, pH 1.5. In the assay, the mobile phase was HBS-EP+ (10 mM HEPES, 150 mM NaCl, 3 mM EDTA, 0.05% surfactant P20), the flow rate was 30 µL/min, the binding time to different antigens was 300s, the regeneration time was 30s, the temperature during detection was 25°C, the analytical concentration of hTIGIT was 20 nM, and the analytical concentration of hPVRIG was 50 nM. The data was analyzed by BIAcore 8K analysis software (version 2.0). The capture level of the antibody and the binding responses (RU) of different antigens were recorded. The stoichiometric ratio of antigen and antibody molecules was calculated according to the molecular weight of the antigen and antibody to roughly estimate how many antigens can bind to an antibody molecule. In order to confirm the interaction between the antibody (LC-BsAb-002) and the antigen (TIGIT&PVRIG), the following four-step detection was carried out: only binding to single antigen, hTIGIT; only binding to single antigen, hPVRIG; first binding to hTIGIT and then binding to hPVRIG; first binding to hPVRIG and then binding to hTIGIT, and each antigen reached saturation state. The antibody-antigen binding curves, capture levels of the two bispecific antibodies, and the binding responses of TIGIT and PVRIG in each experiemnt were collected and recorded to calculate the stoichiometric ratio of antigen and antibody molecules. The antibody-antigen binding curves of LC-BsAb-002 and LC-BsAb-006 respectively binding to TIGIT and PVRIG, followed by the continuous injection of TIGIT and PVRIG are shown in Fig.56A and 56B (Table 47). As shown in Table 47, Fig. 56A and 56B, the binding responses generated by the continuous injection of TIGIT and PVRIG were almost same as those generated by injecting TIGIT and PVRIG alone. In addition, the sequence of continuous injection of TIGIT and PVRIG did not affect the binding responses thereof. It suggests that LC-BsAb-002 and LC-BsAb-006 can simultaneously bind to hTIGIT and hPVRIG without mutual influence between the two antigens. Based on the molecular weight of the antibodies and antigens, and the capture level of antibodies and the binding level of antigens, it is roughly estimated that the stoichiometric ratio of TIGIT to LC-BsAb-002 is 1.76; the stoichiometric ratio of TIGIT to LC-BsAb-006 is 1.86; the stoichiometric ratio of PVRIG to LC-BsAb-002 is 2.14, the stoichiometric ratio of PVRIG to LC-BsAb-006 is 2.18, and the stoichiometric ratio of the two antigens and antibodies are both close to 2. Considering the error caused by the detection assay, the inventors expect that an LC-BsAb-002 or an LC-BsAb-006 bispecific antibody molecule can simultaneously bind to two TIGIT molecules and two PVRIG molecules.

**Table 47. The binding of anti-PVRIGxTIGIT humanized bispecific antibodies to human TIGIT and PVRIG proteins detected by BIAcore**

| Antibody | Capture Level (RU) | Antigen 01 | | | Antigen 02 | | |
|---|---|---|---|---|---|---|---|
| | | Name | Binding Responses (RU) | Antibody-Antigen Stoichiometry | Name | Binding Responses (RU) | Antibody-Antigen Stoichiometry |
| LC-BsAb-002 | 673.6 | TIGT | 94.96 | 1.76 | / | / | / |
| | 673.1 | PVRIG | 128.67 | 2.14 | / | / | / |
| | 673.3 | TIGT | 94.87 | 1.76 | PVRIG | 121.11 | 2.02 |
| | 673.7 | PVRIG | 128.79 | 2.15 | TIGT | 91.28 | 1.69 |
| LC-BsAb-006 | 603.7 | TIGT | 89.83 | 1.86 | / | / | / |
| | 602.9 | PVRIG | 117.08 | 2.18 | / | / | / |
| | 603.4 | TIGT | 89.83 | 1.86 | PVRIG | 109.42 | 2.04 |
| | 602.3 | PVRIG | 116.96 | 2.18 | TIGT | 83.88 | 1.74 |

### EXAMPLE 37 - The promoting effect of anti-PVRIGxTIGIT humanized bispecific antibodies on NK cell function detected by NK cell degranulation assay

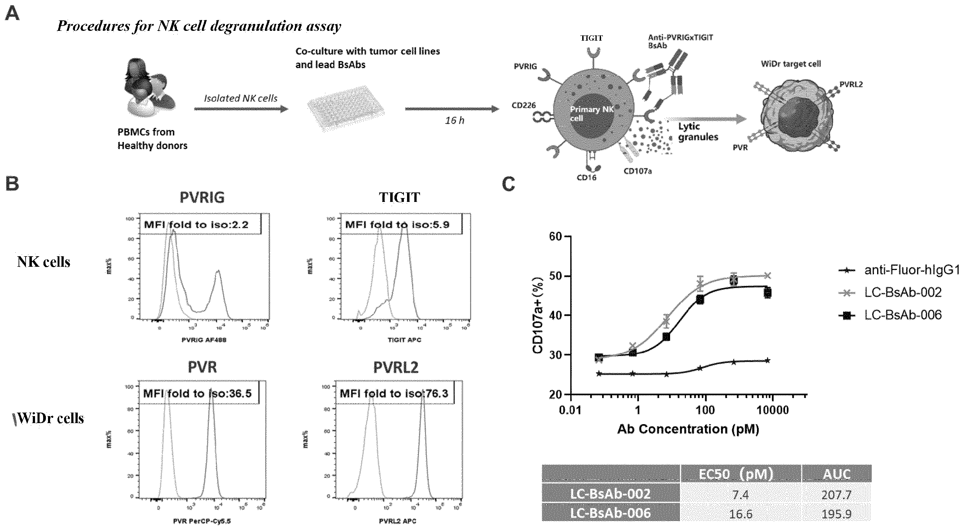

The effect of the tested antibodies on NK cell activation was indicated by the expression level of CD107a in NK cells detected by FACS (The experimental procedures were shown in Fig. 57A)

A. The expression of PVRIG and TIGIT on NK cells (Natural killer cells), and PVR and PVRL2 on WIDR cells was detected by FACS.

Firstly, the NK cells were counted by using a cell counter (Beckman Coulter, Vi-CELL). The NK cells were added into three flow cytometry tubes with 1e+5 NK cells in each tube and washed twice with PBS. After discarding the supernatant, one tube was added with 300 µL Staining buffer (PBS+2% FBS) as an unstained tube, and the other two tubes were added with 100 µL staining solution (PBS+1* Zombie Violet (Biolegend, 423114)), mixed well and then incubated at room temperature for 15 minutes. The cells were then washed twice with staining buffer, and the supernatant was discarded. 50 µL of Fc blocker (Staining buffer + Fcx blocker (Biolegend, 422302)) was added to each tube, mixed and then incubated at 4°C for 15 minutes. Each tube was then added with different staining solution, mixed and incubated at 4°C for 30 minutes, wherein, the first tube was added with 50 µL of 2* staining solution (Staining buffer + PE-Cy7 Mouse anti-hCD3 detection antibody + PE Mouse anti-hCD56 detection antibody + APC Mouse anti-hTIGIT detection antibody + AF488 Rabbit anti-hPVRIG detection antibody, CD3 detection antibody: Biolegend 300316, CD56 detection antibody: Biolegend 318306, TIGIT detection antibody: Biolegend 372706, PVRIG detection antibody: RD FAB93651G), and the second tube was added with 50µL of 2* isotype control staining solution (Staining buffer+PE-Cy7 Mouse anti-hCD3 detection antibody + PE Mouse anti-hCD56 detection antibody + APC Mouse IgG2a κ isotype control antibody + AF488 Rabbit IgG κ isotype control antibody, APC mIgG2a κ isotype control antibody: Biolegend 400222, AF488 Rabbit IgG κ isotype control antibody: RD IC1051G). The samples were then washed twice with Staining buffer, centrifuged and mixed with 300 µL Staining buffer. The samples were detected by a flow cytometer (Thermo Attune NxT) to obtain the percentage of the CD56-positive and CD3-negative cell population in the Zombie Violet negative cell population and read the signals of APC and AF488 of the CD56-positive and CD3-negative cell population in the Zombie Violet negative cell population.

The WIDR cells were trypsinized to form a cell suspension, and counted by using a cell counter (Beckman Coulter, Vi-CELL). The WIDR cells were added into three flow cytometry tubes with 1e+5 cells in each tube and washed twice with PBS. After centrifugation and discarding the supernatant, one tube was added with 300 µL Staining buffer (PBS+2% FBS) as an unstained tube, and the other two tubes were added with 100 µL staining solution (PBS+1* Zombie Violet (Biolegend, 423114)), mixed well and then incubated at room temperature for 15 minutes. The cells were then washed twice with Staining buffer, and the supernatant was discarded. Each tube was then added with different staining solution, mixed and incubated at 4°C for 30 minutes, wherein, the first tube was added with 100 µL staining solution (Staining buffer + PerCP-Cy5.5 Mouse anti-hPVR detection antibody + APC Mouse anti-hPVRL2 detection antibody, PVR detection antibody: Biolegend 337612, PVRL2 detection antibody: Biolegend 337412), and the second tube was added with 100 µL isotype control staining solution (Staining buffer + PerCP-Cy5.5 Mouse IgG1 κ isotype control antibody + APC κ Mouse IgG1 isotype control antibody, PerCP-Cy5.5 mIgG1 κ isotype control antibody: Biolegend 400150 , APC mIgG1 κ isotype control antibody: Biolegend 400122). The samples were then washed twice with Staining buffer, centrifuged and mixed with 300 µL Staining buffer. The samples were detected by a flow cytometer (Thermo Attune NxT) to read the signals of PerCP-Cy5.5 and APC of the Zombie Violet negative cell population. Fig.57B shows that a certain level of PVRIG and TIGIT are expressed on NK cells used in the experiment, while ligands, PVR and PVRL2, are highly expressed on target cells, WIDR.

### B. NK cell degranulation assay (target cells, WIDR)

The PBMCs were resuscitated one day in advance, and human NK cells were sorted by a sorting kit (Stemcell, 17955). The NK cells were added with 200 IU/mL h-IL2 (RD, 202-IL) and 10 ng/mL h-IL12 (Peprotech, 200-12-50UG) to stimulate overnight and plated for the following experiment on the next day. Firstly, the antibodies were diluted to the highest concentration of 275 nM (four-fold concentration) with assay buffer (RPMI1640-Glutamax + 10% FBS + 1×P/S) and continually diluted with assay buffer by 10-fold serial dilution. The diluted antibodies were added to an ultra-low attachment, 96-well, U-bottom plate (Costar, 7007), 50 µL per well, for further use. Secondly, the NK cells were counted by using a cell counter (Beckman Coulter, Vi-CELL). A certain number of NK cells were centrifuged at a speed of 350 g for 5 minutes, resuspended to a density of 0.5E+6 cells/mL with assay buffer after discarding the supernatant and added with protein transport inhibitor (Invitrogen, 00498093) and APC mouse anti-human detection antibody (Biolegend, 328620). The antibody coated 96-well, U-bottom plate was added with the treated NK cell suspension (50µL per well), mixed well and incubated at room temperature for 15 minutes. During the incubation, the target cells (WIDR) were trypsinized to form a cell suspension (Reh cells were directly mixed well) and counted by using a cell counter (Beckman Coulter, Vi-CELL). An appropriate number of cells were centrifuged at 200 g for 5 minutes, and resuspended to a density of the 0.25E+6 cells/mL with the assay buffer after discarding the supernatant. After incubation, the target cell suspension was added to the plate, 100µL per well. At this time, each well contained 25,000 NK cells, 25,000 target cells and different concentrations of tested antibodies. The wells only containing NK cells were served as resting control, while the wells containing NK cells and target cells were served as drug-free control. All the wells were mixed well and incubated in an incubator at 37°C for 16 hours. Finally, FACS staining was performed: the cells in the plate were transferred to the same position in a 96-well, V-bottom plate and washed twice with PBS. After discarding the supernatant, each well was added with the staining solution (PBS+2%FBS+ 1 *zombie violet (Biolegend, 423114) + PE mouse anti-CD56 detection antibody (Biolegend, 318306)), mixed well and incubated at 4°C for 30 minutes. Each well was then washed twice with staining buffer and resuspended with 150 µL staining buffer after discarding the supernatant. The samples were detected by a flow cytometer (Thermo Attune NxT) to read the percentage of CD107a strong positive cell population in CD56 positive cells. A higher percentage of CD107a strong positive cells represent a stronger degranulation of NK cells and a higher degree of activation of NK cells. Fig. 57C shows that the negative control, anti-Fluorescein-hIgG1, has no effect on CD107a on NK cells. The humanized bispecific antibody candidates can improve the expression of CD107a on NK cells to varying degrees, which indicates that the tested antibodies can effectively promote the activation of NK cells.

### C. NK cell degranulation assay (target cells, TF-1)

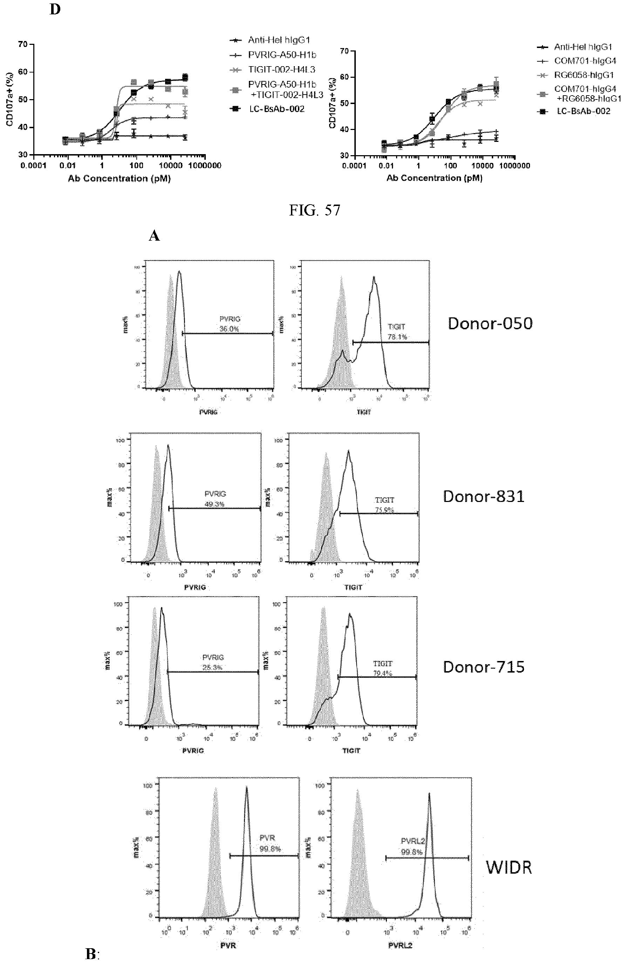

The methods refer to Example 37 B. As shown in Fig. 57D, the effect of the humanized bispecific antibody on degranulation of NK cells was better than that of PVRIG positive control (COM701-hIgG4) and TIGIT positive control (RG6058-hIgG1), and equivalent to that of the combination of COM701-hIgG4 and RG6058-hIgG1. Meanwhile, the effect of the humanized bispecific antibody on degranulation of NK cells was better than that of PVRIG antibody (PVRIG-A50-H1b) and TIGIT antibody (TIGIT-002-H4L3), and comparable to that of the combination of PVRIG-A50-H1b and TIGIT-002-H4L3.

### EXAMPLE 38 - The cytotoxicity of NK cells for tumor cell line mediated by anti-PVRIGxTIGIT humanized bispecific antibodies detected by NK cell cytotoxicity assay

The lysis level of target cells (WIDR) was detected by FACS to infer the effect of the tested antibodies on the cytotoxicity of NK cells for target cells.

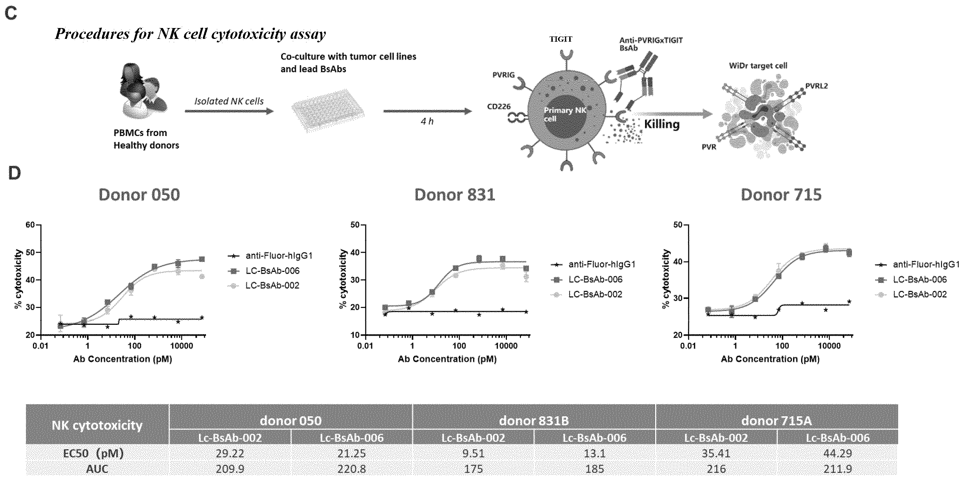

The PBMCs were resuscitated one day in advance. The NK cells were sorted by a sorting kit (Stemcell, 17955), added with 200 IU/mL h-IL2 (RD, 202-IL) and 10 ng/mL h-IL12 (Peprotech, 200-12-50UG) to stimulate overnight and plated for the following experiment on the next day. The expression level of PVRIG and TIGIT on three NK donors (Donor-050, Donor-831, and Donor-715) was detected by the method described in Example 13. Meanwhile, the antibodies were diluted to the highest concentration of 275 nM (four-fold concentration) with assay buffer (RPMI1640-Glutamax + 10% FBS + 1×P/S) and continually diluted with assay buffer by 10-fold serial dilution. The diluted antibodies were added to an ultra-low attachment, 96-well, U-bottom plate (Costar, 7007), 50 µL per well, for further use. Secondly, the target cells (WIDR) were trypsinized to form a cell suspension and counted by using a cell counter (Beckman Coulter, Vi-CELL). An appropriate number of WIDR cells were centrifuged at a speed of 200 g for 5 minutes, resuspended with PBS after discarding the supernatant and added with staining solution, CellTrace Violet (Invitrogen, C34557A) with a final concentration of 5 µM. The WIDR suspension with staining solution was mixed evenly, placed in an incubator at 37°C for 10 minutes and shaken during the incubation. A portion of WIDR cells were used to determine the expression levels of PVR and PVRL2 on WIDR detected by the method described in Example 13. At the same time, NK cells were counted with a cell counter. A certain number of NK cells were centrifuged at a speed of 350 g for 5 minutes and resuspended to a density of 0.5e+6 cells/mL with the assay buffer after discarding the supernatant. The antibody coated 96-well, U-bottom plate was added with the treated NK cell suspension (50 µL per well), mixed well and incubated at room temperature for 15 minutes. After the WIDR cell staining, 5 times volume of complete medium (MEM+10%FBS+1*P/S+1*non-essential amino acid+1*sodium glutamate) was added to the cell suspension to stop the reaction. The WIDR cells were centrifuged at 200 g for 5 minutes and resuspended to a density of 0.25E+6 cells/mL with the assay buffer after discarding the supernatant. After incubating with antibodies and NK cells, the plate was added with WIDR cell suspension, 100 µL per well. At this time, each well contained 25,000 NK cells, 25,000 WIDR cells and various concentrations of tested antibodies. The wells only containing WIDR cells were served as resting control, while the wells containing NK cells and WIDR cells were served as drug-free control. All the wells were mixed well and incubated in an incubator at 37°C for 4 hours. Finally, FACS staining was performed: each well was added with staining solution (PBS + PI (Propidium Iodide, Invitrogen, P3566)), mixed well and incubated at room temperature for 20 minutes. The samples were then detected by a flow cytometer (Thermo Attune NxT) to read the percentage of PI positive cell population in CTV positive cells. More PI positive cells represent stronger NK cell cytotoxicity. Figure 58A shows that a certain level of PVRIG and TIGIT are expressed on all three NK donors (donor-050, donor-831 and donor-715); Figure 58B shows that PVR and PVRL2 are highly expressed on target cells, WIDR; Figure 58C shows brief experimental process of NK cell cytotoxicity assay for WIDR cells; Figure 58D shows the negative control, anti-Fluorescein-hIgG 1, has no significant effect on the NK cell cytotoxicity, while two tested humanized bispecific antibodies can effectively promote the NK cell (from three donors) cytotoxicity for the target cells (WIDR). The EC50 and area under the curve (AUC) of NK cell cytotoxicity (from different NK donors) for WIDR cells mediated by two humanized bispecific antibodies are shown in the Table.

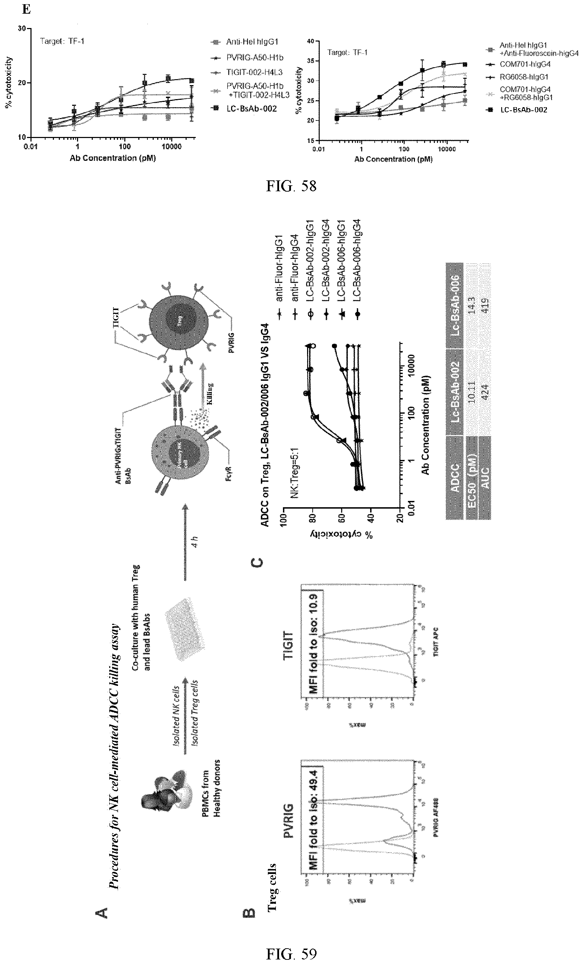

Use TF-1 as a target cell, NK cells cytotoxicity for TF-1 mediated by the anti-PVRIGxTIGIT humanized bispecific antibody was detected. As shown in Figure 58E, the humanized bispecific antibody can promote NK cells to kill tumor cells, with the activity better than that of PVRIG positive control (COM701-hIgG4) and TIGIT positive control (RG6058-hIgG1) and equivalent to the combination of COM701-hIgG4 and RG6058-hIgG1. Meanwhile, the promoting activity of the humanized bispecific antibody is better than that of PVRIG antibody (PVRIG-A50-H1b) and TIGIT antibody (TIGIT-002-H4L3), and comparable to that of the combination of the PVRIG-A50-H1b and TIGIT-002-H4L3.

### EXAMPLE 39 - The cytotoxicity of NK cells for human Treg cells mediated by anti-PVRIGxTIGIT humanized bispecific antibodies detected by ADCC assay

The lysis level of target cells (Treg) was detected by FACS to infer the direct effect of the tested antibodies on antibody-dependent cell-mediated cytotoxicity (ADCC) of NK cells against target cells (Fig.59A).

The PBMCs were resuscitated one day in advance. The NK cells were sorted out as effector cells using a sorting kit (Stemcell, 17955), added with 200 IU/mL h-IL2 (RD, 202-IL) and 10 ng/mL h-IL12 (Peprotech, 200-12-50UG) to stimulate overnight and plated for the following experiment on the next day. Tregs (regulatory T cells) isolated from PBMCs were used as target cells (Stemcell, 18063). Tregs were expanded in vitro 12 days by Dynabeads (Gibco, 11129D) for further use. The expression level of PVRIG and TIGIT on Treg cells was detected by the method and reagents described in example 13. The effector cells and target cells were co-incubated at a ratio of 5:1. The serially diluted humanized bispecific antibodies or isotype control anti-Fluorescein-hIgG 1, anti-Fluorescein-hIgG4 antibodies are added into the cells and incubated in a 37°C incubator for 4 hours before adding PI staining. The percentage of PI-positive Treg cells was read to evaluate the ADCC effect of the bispecific antibodies on Treg cells. The results show that TIGIT and PVRIG are highly expressed on isolated and expanded human Treg cells (Fig.59B). The tested humanized bispecific antibodies only show ADCC activity against Treg cells as anti-human IgG1 Fc antibodies, but the corresponding anti-human IgG4 Fc antibodies does not show obvious ADCC activity against Treg cells, which is equivalent to the two negative control antibodies, anti-Fluorescein-hIgG1 and anti-Fluorescein-hIgG4 (Fig.59C). The EC50 and AUC of the ADCC activity of two tested humanized bispecific antibodies (anti-human IgG1 Fc antibodies) against Treg cells are listed in the table.

### EXAMPLE 40 ADCP activity of anti-PVRIGxTIGIT humanized bispecific antibodies

Monocytes were isolated from donor PBMC and induced with 75 ng/mL GM-CSF for seven days to differentiate to macrophages, which were labeled with CellTrace Violet and used as effector cells. Human Treg cells were sorted by Treg Cells Isolation Kit from human PBMCs. Tregs were expanded and activated in vitro for 13 days by Dynabeads Human Treg Expander and used as target cells. The target cells were stained by CFSE. The effector cells and the target cells were co-incubated at a ratio of 4:1. Tested antibodies with serial dilution, negative control antibodies Hel hIgG1, PVRIG antibody (PVRIG-A50-H1b), TIGIT antibody (TIGIT-002-H4L3) and combination of the two monoantibody were added, incubated at 37°C for 4 hours. After the incubation, cell stain PI was added, and the percentage of CellTrace Violet-positive cells in CFSE-positive Treg cells was detected by flow cytometry to evaluate the ADCP effect of the tested antibodies.

As shown in Fig. 60A, the tested antibodies activate the ADCP action of Treg cells in a dose-dependent manner. PVRIG-A50-H1b or COM701-IgG4 almost has no ADCP activity, TIGIT-002-H4L3 or RG6058-hIgG1 exhibits dose-dependent ADCP activity. Based on the under the curve (AUC) of the ADCP curve, the ADCP activity of tested antibodies was slightly weaker than that of TIGIT-002-H4L3 and the combination of PVRIG-A50-H1b and TIGIT-002-H4L3. The ADCP effect of the tested antibodies are comparable to that of the RG6058-hIgG1 and combination of two positive control antibodies (COM701-hIgG4 + RG6058-hIgG1). According to the Eₘₐₓ of the ADCP curve, the ADCP activity of tested antibodies are comparable to the combination of PVRIG-A50-H1b and TIGIT-002-H4L3, and combination of two positive control antibodies (COM701-hIgG4 + RG6058-hIgG1). (Fig. 60B)

### EXAMPLE 41 The effect of anti-PVRIGxTIGIT humanized bispecific antibodies on cytokine release in human PBMC from healthy donors

The present experiment investigates the effect of the tested antibodies on cytokine secretion of unstimulated PBMCs from healthy individuals. PBMCs from three healthy volunteers were incubated with tested antibodies for 24 hours under liquid or solid phase conditions, and then secretion levels of five cytokines, IFN-γ, IL-2, IL-6, IL-10 and TNF-α in PBMCs supernatants were detected by flow cytometry. Lipopolysaccharide and CD3 monoclonal antibody were used as positive control, Anti-Hel hIgG1 was used as negative control, RG6058-hlgG1 and COM701-hlgG4 were used as monoclonal antibody control of TIGIT and PVRIG respectively.

The results show that the positive control CD3 monoclonal antibodies in liquid or solid phase conditions, and LPS in liquid phase conditions, incubated for 24 hours with unstimulated PBMCs from three healthy volunteers, can increase the secretion levels of 5 cytokines, IFN-γ, IL-2, IL-6, IL-10, and TNF-α to varying degrees. In liquid phase conditions, secretion levels of IFN-γ, IL-2, IL-6, IL-10, and TNF-α in PBMC were comparable to that of the negative control or below the detection limit after incubation of different concentrations of the tested antibodies with unstimulated PBMC for 24 hours. In solid phase conditions, the secretion of IFN-γ, IL-2 and IL-10 in PBMC were comparable to that of the negative control or below the detection limit after incubation of different concentrations of the tested antibodies with unstimulated PBMC for 24 hours. In solid phase conditions, the secretion of TNF-α and IL-6 were significantly higher than that of the negative control or comparable to that of the negative control after the action of tested antibodies at a high concentration point (2850 nM); but the tested antibodies dose not additionally increase the secretion of 5 cytokines, IFN-γ, IL-2, IL-6, IL-10 and TNF-α in unstimulated PBMC from healthy individuals compared with RG6058-hlgG1 and COM701-hlgG4 under the same conditions.

In summary, compared with the TIGIT monoclonal antibody RG6058-hIgG1 and the PVRIG antibody COM701-hIgG4, the tested antibodies, under the same conditions, do not additionally increase the secretion of 5 cytokines, IFN-γ, IL-2, IL-6, IL-10, and TNF-α in unstimulated PBMCs from healthy subjects.

### EXAMPLE 42 - The effect of anti-PVRIGxTIGIT humanized bispecific antibodies on improving the functions of antigen-specific CD8 T cells detected by CMV antigen-recall assay

Principle of this assay: The PBMCs from CMV IgG positive donor were induced by CMV pp65 (495-503) polypeptide to produce CMV pp65 specific CD8 T cells, which were served as effector cells. The colo205 tumor cell line pulsed with pp65 was used as the target cell. In such experimental system, the effect of anti-PVRIG&TIGIT humanized bispecific antibodies on improving the functions of the pp65-specific CD8 T cell was detected (Fig. 61A).

The PBMCs were resuscitated, resuspended to 2×10⁶/mL by using complete medium (RPMI1640-Glutamax + 5% AB serum + 1% P/S+(1×) 2-β mercaptoethanol) containing 1 µg/mL CMV pp65(495-503) peptide (Anaspec, Cat No.AS-28328), 2 ng/mL human IL-2 (R&D, Cat No.IL-202) and 10 ng/mL human IL-7 (Peprotech, Cat No.200-07), inoculated in a 6-well plate (5 mL/well) and cultured at 37°C with 5% CO₂ for 6 days. On day 6, all the PBMCs were collected, and pp65 and IL-7 in the medium were removed. The cells were divided into two portions, resuspended in complete medium containing 100 IU/mL human IL-2, and cultured for another 2 days. On day 8, all the PBMCs were collected and resuspended in complete medium containing 100 IU/mL human IL-2, and the cell density was adjusted to 2×10⁶/mL for continuing cultivation. On day 11, all the PBMCs were collected. The expression of PVRIG, TIGIT and PD-1 on CMV pp65 (495-503) specific CD8 T cells (Fig. 61B) were detected by flow cytometry. The detection antibodies were as follows: Livedead Near IR (Invitrogen, Cat No.L34976), CD8-PerCp Cy5.5 (BD, Cat No.565310), CD3-PE-Cy7 (Biolegend, Cat No.300316), T-select HLA-A*0201 CMV pp65 Tetramer-PE (MBL, Cat No.TS-0010-1C), PVRIG-AF488 (R&D, Cat No.FAB93651G-100UG), TIGIT-APC (Biolegend, Cat No.372706) and PD-1-BV421 (BD, Cat No.562516).

CD8 T cells were isolated from the induced PBMCs as effector cells by CD8 T cell isolation kit (Stemcell, Cat No.17953) and resuspended with AIM-V to the cell density of 0.4×10⁶/mL (If CD8 is cryopreserved after induction, the number of cells needs to be increased to 0.7×10⁶/mL.). The expression of CD226 and purity of isolated CD8 were detected. The target cells, Colo205, were digested by TrypLE^{™} Express Enzyme (Gibco, Cat No.12605010), resuspended in AIM-V (Gibco, Cat No.31035-025) containing 20 ng/mL pp65 to the cell density of 1×10⁶/mL and treated at 37°C with 5% CO₂ for 3 hours. The target cells were then centrifuged at 250 g for 5 minutes and resuspended in AIM-V to the cell density of 0.5×10⁶/mL after discarding the supernatant. The expression of PVRL2, PVR and PD-L1 on Colo205 were detected by flow cytometry (Fig. 61B). Tested antibodies (humanized bispecific antibodies and Tecentriq) were diluted with AIM-V to 280 nM. The low-attachment, 96-well, U-bottom plate (Corning, Cat No.7007) was added with 50 µL of antibody, 50 µL of CD8 T and 100 µL of pp65-treated colo205 in order, mixed well with a multichannel pipette, and incubated at 37°C with 5% CO₂ for 18 hours. In the experimental system, the final concentration of antibodies was 70 nM, CD8 T cells were 20,000/well, and colo205 were 50,000/well. After incubation, the supernatant was collected by centrifugation at 400 g, and the level of human IFN-γ in the supernatant was detected with an ELISA kit (Dakewe, Cat No.1110003). In this system, the positive controls were COM701-hIgG4 and RG6058-hIgG1, and the negative control was no treatment. The detection antibodies used in flow cytometry to detect the purity of isolated CD8 T were as follows: Livedead-BV421 (Invitrogen, Cat No.L34964) and CD8-FITC (BD, Cat No.555366).

As shown in Fig. 61C and Table 48, when adding the bispecific antibodies (LC-BsAb-002 or LC-BsAb-006), there is no statistically significant difference in the secretion level of IFN-γ between the two tested group at each concentration points. The secretion level of IFN-γ is significantly higher with bispecific antibodies at high concentration point(s) (LC-BsAb-002 at 70nM, LC-BsAb-006 at 70 and 7nM) compared with the corresponding monoclonal antibody combination under the same conditions. There is no significant difference between the monoclonal antibody combination corresponding to the two candidate molecules and the antibody combination of positive controls (RG605-hIgG1 and COM701-hIgG4) at each concentration point. Over all, compared with the antibody combination of positive controls (RG605-hIgG1 and COM701-hIgG4), the two bispecific antibodies have a better effect on promoting IFN-γ released from CD8 T cells.

As shown in Fig. 61D, when using the combination of bispecific antibodies (LC-BsAb-002 or LC-BsAb-006) and Tencentriq, the secretion level of human IFN-γ is significantly increased compared with using bispecific antibodies themselves (t-test, **P<0.01). When using the combination of PVRIG monoclonal antibody, TIGIT monoclonal antibody and PD-L1 monoclonal antibody, the secretion level of human IFN-γ is significantly increased compared with using the combination of PVRIG monoclonal antibody and TIGIT monoclonal antibody (t-test, *P<0.05). The percentage of the histogram is the percentage increase in IFN-γ compared with the anti-TIGIT positive control antibody, RG6058-hIgG1.

**Table 48. Statistical data analysis of the detection results of human IFN-γ in the cell supernatant (two-way ANOVA)**

| Two-way ANOVA analysis-01 | | | | | |
|---|---|---|---|---|---|
| Conc.(nM) | LC-BsAb-002 | | LC-BsAb-006 | | two-way ANOVA |
| 0.0007 | 137.4 | 111.7 | 128.3 | 131.5 | ns |
| 0.007 | 197.9 | 165 | 162.5 | 176.2 | ns |
| 0.07 | 554.3 | 411.2 | 371.1 | 338.2 | ns |
| 0.7 | 628.3 | 642.7 | 672 | 539.3 | ns |
| 7 | 784.3 | 691.5 | 820 | 727.9 | ns |
| 70 | 1119.1 | 881.8 | 733.6 | 798.2 | ns |

| Two-way ANOVA analysis-02 | | | | | |
|---|---|---|---|---|---|
| Conc.(nM) | LC-BsAb-002 | | TIGIT-002-H4L3+PVRIG-A50-H1b | | two-way ANOVA |
| 0.0007 | 137.4 | 111.7 | 148.2 | 121 | ns |
| 0.007 | 197.9 | 165 | 132.2 | 146.5 | ns |
| 0.07 | 554.3 | 411.2 | 311 | 280.8 | ns |
| 0.7 | 628.3 | 642.7 | 444.6 | 455.5 | ns |
| 7 | 784.3 | 691.5 | 632.7 | 540.4 | ns |
| 70 | 1119.1 | 881.8 | 648.5 | 440.4 | ** |

| Two-way ANOVA analysis-03 | | | | | |
|---|---|---|---|---|---|
| Conc.(nM) | LC-BsAb-006 | | TIGIT-005-H2L1d+PVRIG-A50-H1b | | two-way ANOVA |
| 0.0007 | 128.3 | 131.5 | 120.3 | 121.6 | ns |
| 0.007 | 162.5 | 176.2 | 151.5 | 146.4 | ns |
| 0.07 | 371.1 | 338.2 | 229.9 | 276.2 | ns |
| 0.7 | 672 | 539.3 | 427.7 | 545.9 | ns |
| 7 | 820 | 727.9 | 571.5 | 388.2 | ** |
| 70 | 733.6 | 798.2 | 441.9 | 415.2 | *** |

| Two-way ANOVA analysis-04 | | | | | |
|---|---|---|---|---|---|
| Conc.(nM) | TIGIT-002-H4L3+PVRIG-A50-H1b | | RG6058+COM701 | | two-way ANOVA |
| 0.0007 | 148.2 | 121 | 173.6 | 148.3 | ns |
| 0.007 | 132.2 | 146.5 | 189.9 | 113.3 | ns |
| 0.07 | 311 | 280.8 | 199.4 | 172.6 | ns |
| 0.7 | 444.6 | 455.5 | 396.7 | 529.4 | ns |
| 7 | 632.7 | 540.4 | 593.6 | 416.1 | ns |
| 70 | 648.5 | 440.4 | 630.3 | 436.7 | ns |

| Two-way ANOVA analysis-05 | | | | | |
|---|---|---|---|---|---|
| Conc.(nM) | TIGIT-005-H2L1d+PVRIG-A50-H1b | | RG6058+COM701 | | two-way ANOVA |
| 0.0007 | 120.3 | 121.6 | 173.6 | 148.3 | ns |
| 0.007 | 151.5 | 146.4 | 189.9 | 113.3 | ns |
| 0.07 | 229.9 | 276.2 | 199.4 | 172.6 | ns |
| 0.7 | 427.7 | 545.9 | 396.7 | 529.4 | ns |
| 7 | 571.5 | 388.2 | 593.6 | 416.1 | ns |
| 70 | 441.9 | 415.2 | 630.3 | 436.7 | ns |

### EXAMPLE 43 - The effect of combination use of anti-PVRIGxTIGIT humanized bispecific antibodies and Tecentriq on improving the functions of antigen-specific CD8 T cells detected by CMV antigen-recall assay

### Principle of this assay: Same as Example 40 (Fig. 61A)

Induction of antigen-specific CD8 T cells: same as Example 40. Expression of PVRIG, TIGIT and PD-1 on inoculated cells was detected by flow cytometry on the day of inoculation (Fig. 62A).

CD8 T cells were isolated from the induced PBMCs as effector cells by CD8 T cell isolation kit (Stemcell, Cat No. 17953) and resuspended with AIM-V to the cell density of 0.8×10⁶/mL (the number of CD8 T cells in microplates was adjused according to the ratio of antigen-specific CD8 T cells). Colo205 cells pretreated overnight with 100ng/mL IFN-γ were added to the complete medium as target cells, and digested by TrypLE^{™} Express Enzyme (Gibco, Cat No.12605010), resuspended in AIM-V (Gibco, Cat No.31035-025) containing 20 ng/mL pp65 to the cell density of 1×10⁶/mL after washed twice and treated at 37°C with 5% CO₂ for 3 hours. The target cells were then centrifuged at 250 g for 5 minutes and resuspended in AIM-V to the cell density of 0.5×10⁶/mL after discarding the supernatant. The expression of PVRL2, PVR and PD-L1 on Colo205 were detected by flow cytometry (Fig. 62A). Tested antibodies (humanized bispecific antibodies, Tecentriq, combination of bispecific antibodies and Tecentriq, combination of two positive control monoclonal antibody (COM701-hIgG4 and RG6058-hIgG1), combination of three monoclonal antibody (COM701-hIgG4, RG6058-hIgG1 and Tecentriq)) were diluted with AIM-V (initial concentration 280 nM(4×), 10-fold serial dilution, 6 concentration points). The low-attachment, 96-well, U-bottom plate (Corning, Cat No.7007) was added with 50 µL of antibody, 50 µL of CD8 T and 100 µL of pp65-treated colo205 in order, mixed well with a multichannel pipette, and incubated at 37°C with 5% CO₂ for 18 hours. In the experimental system, the final concentration of antibodies was 70 nM, 7nM, 0.7nM, 0.07 nM, 0.007 nM and 0.0007 nM, respectively. CD8 T cells were 40,000/well, and colo205 were 50,000/well. After incubation, the supernatant was collected by centrifugation at 400 g, and the level of human IFN-γ in the supernatant was detected with an ELISA kit (Dakewe, Cat No.1110003).

As shown in Fig. 62B and Table 49, sort according to the AUC of the IFN-γ fitting curve: LC-BsAb-002+Tecentriq>RG6058-hIgG1+COM701-hIgG4+Tecentriq>LC-BsAb-002>RG6058 -hIgG1+COM701-hIgG4 > Tecentriq. Higher AUC indicates greater effectiveness, when using the combination of bispecific antibodies (LC-BsAb-002) and Tencentriq, the secretion level of human IFN-γ is significantly increased compared with using LC-BsAb-002 itselves. When using the combination of COM701-hIgG4, RG6058-hIgG1 and Tecentriq, the secretion level of human IFN-γ is significantly increased compared with using the combination of COM701-hIgG4 and RG6058-hIgG1.

**Table 49. Curve fit of human IFN-γ assay results in cell supernatants**

| | Tecentriq | LC-BsAb-002 | RG6058+COM701 | LC-BsAb-002 +Tecentriq | RG6058+COM701 +Tecentriq |
|---|---|---|---|---|---|
| EC50(nM) | -* | 0.1494 | 1.571 | 0.1101 | 0.9041 |
| AUC | 97.17 | 320.4 | 176.4 | 728.4 | 447.9 |

| | | | | | |
|---|---|---|---|---|---|
| *Data curves cannot be fitted | | | | | |

### EXAMPLE 44 - In vivo drug efficacy evaluation of anti-PVRIGxTIGIT humanized bispecific antibody in mice

A375 cells were inoculated subcutaneously on the right side of NPG mice, female, 5-6 weeks old (strain: NPG; Beijing Vitalstar Biotechnology Co., Ltd.) at a concentration of 5×10⁶ cells/0.1mL. One day after the inoculation of A375 cells, Hu PBMC cells were injected into mice at a concentration of 5×10⁶ cells/0.2mL by tail vein. When the tumor grew to about 82 mm³, 56 mice were selected according to the tumor volume and randomly divided into 7 groups with 8 mice in each group: Vehicle (PBS), RG6058-hIgG1 (10 mg/kg), COM701-hIgG4 (10 mg/kg), RG6058-hIgG1+ COM701-hIgG4 (10 mg/kg+10 mg/kg), Tecentriq (5 mg/kg; lot NO. HK65567, Roche), LC-BsAb-002 (11.7mg/kg), LC-BsAb-006 (11.7mg/kg). All groups were administrated by intraperitoneal injection, twice a week for 4 consecutive times. The experiment was ended 3 days after the last administration. The body weight and tumor volume of mice were measured three times a week during administration and observation. The measured values were recorded, and the tumor volume (long diameter × short diameter ²/2) and growth inhibition rate (TGI_{TV} (%) = (1-(Tn) -T0)/(Vn-V0)) × 100% were calculated.

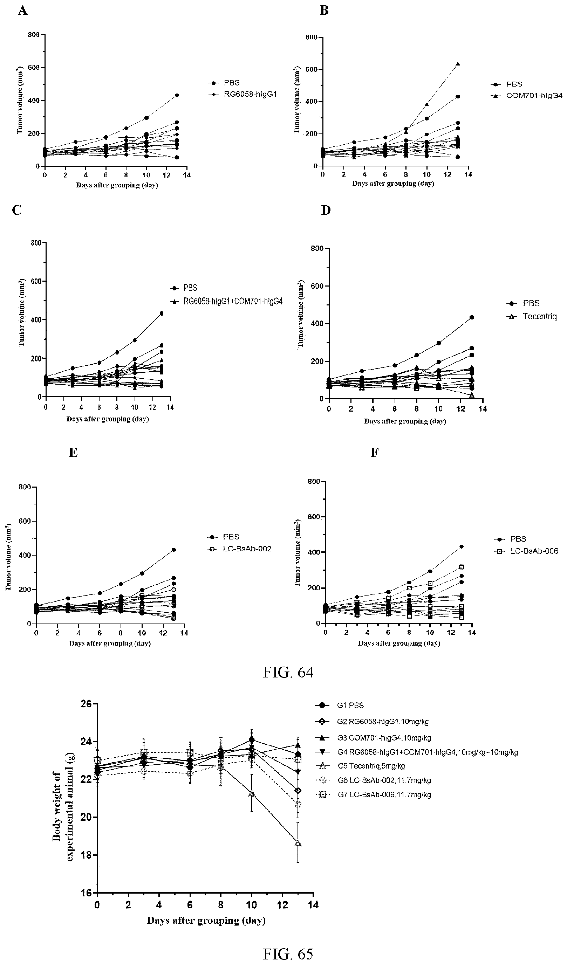

Efficacy results: As shown in Fig. 63, the candidate molecules LC-BsAb-002 and LC-BsAb-006 have a significant inhibitory effect on A375 tumor growth after administration. The inhibitory level of LC-BsAb-002 and LC-BsAb-006 is the same as that of the positive molecule (RG6058-hIgG1+COM701-hIgG4) and Tecentriq. On the day 13 of administration, the tumor inhibition rate (TGI) and difference analysis of each administration group and the negative control group (PBS) were obtained and shown in Table 50. The TGI of LC-BsAb-002 and LC-BsAb-006 were 82.16% and 78.59%, respectively, which are significantly higher than PBS (P<0.005). The level of TGI is better than RG6058-hIgG1 (TGI=42.55), COM701-hIgG4 (TGI=0.23%) alone, and equivalent to the combination of RG6058-hIgG1 and COM701-hIgG4 (TGI=83.32%). The tumor growth curve of single mouse shown in Fig. 64 shows the same trend as Fig. 63.

**Table 50. Effect of the test substances on tumor volume of HuPBMC-NPG mice transplanted with A375 cells.**

| Grou p | Test substance | Tumor volume (mm³) ^{a} | | | P ^{b} |
|---|---|---|---|---|---|
| | | Before administration | Day 13 | TGI (%) | |
| G1 | PBS | 83±4 | 196±41 | - | - |
| G2 | RG6058-hIgG1 | 81±4 | 146±20 | 42.55 | 0.2614 |
| G3 | COM701-hIgG4 | 82±4 | 195±64 | 0.23 | 0.6052 |
| G4 | RG6058-hIgG1+ COM701-hIgG4 | 82±4 | 101±17 | 83.32 | ***0.0004 |
| G5 | Tecentriq | 83±5 | 91±20 | 92.48 | ***0.0002 |
| G6 | LC-BsAb-002 | 82±5 | 103±21 | 82.16 | **0.0035 |
| G7 | LC-BsAb-006 | 82±3 | 106±36 | 78.59 | ***0.0001 |

| | | | | | |
|---|---|---|---|---|---|
| Note: a: mean ± standard error; b: the tumor volume of the administration group and the vehicle control group were statistically compared on the day 13 after administration by two-way ANOVA analysis, *P<0.05, **P<0.01, ***P<0.001, ****P<0.0001. | | | | | |

Results of body weight: as shown in Fig. 65 and table 51, except for the control molecule, Tecentriq, which causes significant weight loss and shows toxic side effects, the body weight change trends of the other control and candidate molecules (LC-BsAb-002 and LC-BsAb-006) are basically the same as that of PBS. The subsequent body weight loss is the GVHD phenomenon caused by PBMC reconstitution.

**Table 51 Effects of test substances on the body weight of HuPBMC-NPG mice transplanted with A375 cells**

| Grou p | Test substance | Body weight (g) ^{a} | | | Body weight change on day 13 (%) |
|---|---|---|---|---|---|
| | | Before administration | Day 13 | P ^{b} | |
| G1 | PBS | 22.5±0.3 | 23.3±0.8 | - | +3.8 |
| G2 | RG6058-hIgG1 | 22.4±0.4 | 21.4±1.1 | 0.8251 | -4.6 |
| G3 | COM701-hIgG4 | 22.7±0.5 | 23.8±0.4 | 0.9999 | +5.4 |
| G4 | RG6058-hIgG1+ COM701-hIgG4 | 22.6±0.6 | 22.4±1.4 | 0.9798 | -1.5 |
| G5 | Tecentriq | 22.7±0.8 | 18.7±1.0 | **0.006 1 | -18.9 |
| G6 | LC-BsAb-002 | 22.2±0.5 | 20.7±0.7 | 0.0744 | -6.4 |
| G7 | LC-BsAb-006 | 23.0±0.6 | 23.1±1.1 | 0.9999 | +1.7 |

| | | | | | |
|---|---|---|---|---|---|
| Note: a: mean ± standard error; b: the body weight of the administration group and the vehicle control group were statistically compared on the day 13 after administration by two-way ANOVA analysis. | | | | | |

The final results shows that the anti-PVRIGxTIGIT humanized bispecific antibody molecules (LC-BsAb-002 and LC-BsAb-006) have a significant inhibitory effect on the growth of subcutaneous xenograft tumors of A375, which is better than the positive drugs RG6058-hIgG1 and COM701-hIgG4 alone, and equivalent to the positive control antibodies (Tecentriq) and the combination of RG6058-hIgG1 and COM701-hIgG4. At the same time, no toxic and side effects of the candidate molecules are observed during the administration, which indicates that the safety and tolerability of candidate molecules under this model.

### EXAMPLE 45 - In vivo drug efficacy evaluation of combination use of anti-PVRIGxTIGIT humanized bispecific antibody and Tecentriq in mice

A375 cells were inoculated subcutaneously on the right side of NPG mice, female, 5-6 weeks old (strain: NPG; Beijing Vitalstar Biotechnology Co., Ltd.) at a concentration of 5×10⁶ cells/0.1mL. One day after the inoculation of A375 cells, Hu PBMC cells were injected into mice at a concentration of 5.5×10⁶ cells/0.2mL by tail vein. When the tumor grew to about 82.78 mm³, 45 mice were selected according to the tumor volume and randomly divided into 5 groups: Vehicle (PBS, 9 mice), LC-BsAb-002 (11.7 mg/kg, 9 mice), LC-BsAb-002 (5.9 mg/kg, 9 mice), Tecentriq (3 mg/kg, 10 mice; lotNO. HK65567, Roche), LC-BsAb-002 + Tecentriq (5.9 mg/kg + 3mg/kg, 8 mice). All groups were administrated by intraperitoneal injection, twice a week for 5 consecutive times. The experiment was ended 3 days after the last administration. The body weight and tumor volume of mice were measured three times a week during administration and observation. The measured values were recorded, and the tumor volume (long diameter × short diameter ²/2) and growth inhibition rate (TGI_{TV} (%) = (1-(Tn) -T0)/(Vn-V0)) × 100% were calculated.

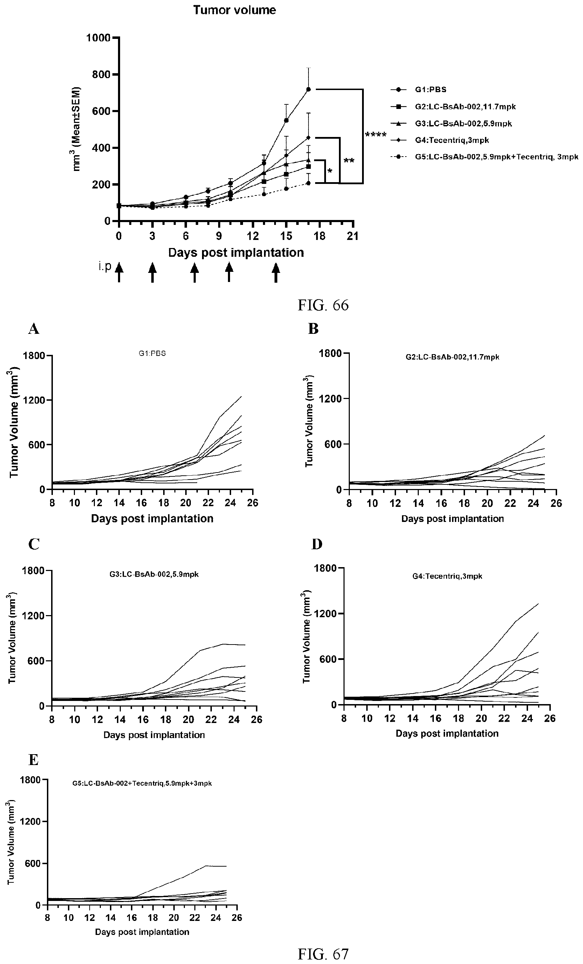

Efficacy results: As shown in Fig. 66, the candidate molecules LC-BsAb-002 have a significant inhibitory effect on A375 tumor growth after administration, and the higher the dose administered, the greater the inhibitory effect on A375 tumor growth. The inhibitory level of combination use of LC-BsAb-002 and Tecentriq is significantly better than LC-BsAb-002 and Tecentriq alone. On the day 17 of administration, the tumor inhibition rate (TGI) and difference analysis of each administration group and the negative control group (PBS) were obtained and shown in Table 52. The TGI of LC-BsAb-002 (11.7 mg/kg), LC-BsAb-002 (5.9 mg/kg) and Tecentriq (3 mpk) were 66.56%, 60.51% and 41.53%, respectively, which are significantly different compared to the PBS (P<0.0001, P=0.0003 and P=0.0015). The TGI of combination use of LC-BsAb-002 and Tecentriq (80.44%) is significantly different compared to the PBS (P<0.0001), and better than that of LC-BsAb-002 (5.9 mg/kg) and Tecentriq(3 mpk) alone. The tumor growth curve of single mouse is shown in Fig. 67, and the tumor growth trends in each group were the same as Fig. 66.

**Table 52. Effect of the test substances on tumor volume of HuPBMC-NPG mice transplanted with A375 cells.**

| Group | Test substance | Tumor volume (mm³) ^{a} | | | P ^{b} |
|---|---|---|---|---|---|
| | | Before administration | Day 17 | TGI (%) | |
| G1 | PBS | 83±3 | 718±116 | - | - |
| G2 | LC-BsAb-002 11.7 mpk | 84±3 | 296±76 | 66.56 | ****<0.0001 |
| G3 | LC-BsAb-002 5.9 mpk | 83±3 | 334±78 | 60.51 | ***0.0003 |
| G4 | Tecentriq 3mpk | 83±3 | 455±134 | 41.53 | **0.0015 |
| G5 | LC-BsAb-002 5.9 mpk +Tecentriq 3 mpk | 81±4 | 206±54 | 80.44 | ****<0.0001 |

| | | | | | |
|---|---|---|---|---|---|
| Note: a: mean ± standard error; b: the tumor volume of the administration group and the vehicle control group were statistically compared on the day 17 after administration by two-way ANOVA analysis, *P<0.05, **P<0.01, ***P<0.001, ****P<0.0001. | | | | | |

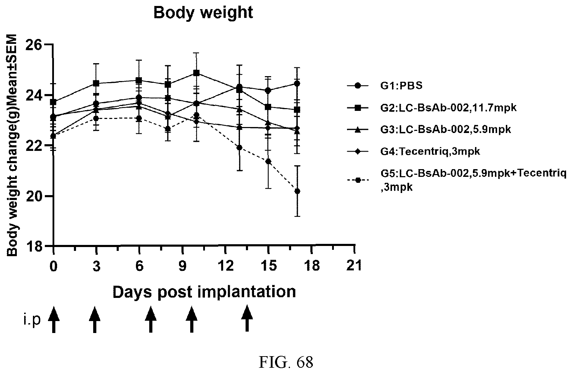

Results of body weight: as shown in Fig. 68 and Table 53, combination use of LC-BsAb-002 and Tecentriq showed a certain decrease in weight, but did not exhibit significant toxic side effects. The body weight change trends of the Tecentrip group, LC-BsAb-002 (11.7 mpk) group and LC-BsAb-002 (5.9 mpk) group are basically the same as that of PBS.

**Table 53 Effects of test substances on the body weight of HuPBMC-NPG mice transplanted with A375 cells**

| Group | Test substance | Body weight (g) ^{a} | | | Body weight change on day 13 (%) |
|---|---|---|---|---|---|
| | | Before administration | Day 17 | P ^{b} | |
| G1 | PBS | 23.1±0.4 | 22.4±0.6 | - | +6.1 |
| G2 | LC-BsAb-002 11.7 mpk | 23.7±0.7 | 23.4±1.2 | 0.9598 | -1.8 |
| G3 | LC-BsAb-002 5.9 mpk | 22.4±0.6 | 22.5±0.6 | 0.2524 | +1.0 |
| G4 | Tecentriq 3mpk | 23.2±0.6 | 22.7±1.0 | 0.1582 | -2.3 |
| G5 | LC-BsAb-002 5.9 mpk +Tecentriq 3 mpk | 22.4±0.5 | 20.2±1.0 | 0.0002 | -10.0 |

| | | | | | |
|---|---|---|---|---|---|
| Note: a: mean ± standard error; b: the body weight of the administration group and the vehicle control group were statistically compared on the day 17 after administration by two-way ANOVA analysis. | | | | | |

The final results shows that the anti-PVRIGxTIGIT humanized bispecific antibody molecules (LC-BsAb-002) have a significant inhibitory effect on the growth of subcutaneous xenograft tumors of A375, and the inhibitory effect showed a significant dose-dependence as the administered dose increased. Combination of LC-BsAb-002 and Tecentriq is better than the respective drugs alone, shows significant combination effect. At the same time, no toxic and side effects of the candidate molecules are observed during the administration, which indicates that the safety and tolerability of candidate molecules under this model.

## Claims

1. An anti-PVRIG/anti-TIGIT bispecific antibody, which comprises:
(a) a first antigen binding fragment, which comprise a heavy chain variable region (VH) and a light chain variable region (VL) that form an anti-TIGIT antigen binding domain; wherein the TIGIT VH comprises HCDR1, HCDR2 and HCDR3 of the VH shown in SEQ ID NO: 72 or 87; the TIGIT VL comprises LCDR1, LCDR2 and LCDR3 of the VL shown in SEQ ID NO: 68 or 91;
(b) a second antigen binding fragment, which comprise VHH that specifically binds to PVRIG, the VHH comprises CDR1, CDR2 and CDR3 of the sequences shown in SEQ ID NO: 200 or 211.

2. The bispecific antibody according to claim 1, wherein,
(a) the HCDR1 of the first antigen binding fragment comprises the sequence shown in any one of SEQ ID NO: 21 or 33; the HCDR2 comprises the sequence shown in any one of SEQ ID NO: 22 or 34; the HCDR3 comprises the sequence shown in any one of SEQ ID NO: 23 or 35;
(b) the LCDR1 of the first antigen binding fragment comprises the sequence shown in any one of SEQ ID NO: 18 or 96; the LCDR2 comprises the sequence shown in any one of SEQ ID NO: 19 or 31; the LCDR3 comprises the sequence shown in any one of SEQ ID NO: 20 or 32;
(c) the CDR1 of the second antigen binding fragment comprises the sequence shown in any one of SEQ ID NO: 168 or 147; the CDR2 comprises the sequence shown in any one of SEQ ID NO: 207 or 148; the CDR3 comprises the sequence shown in any one of SEQ ID NO: 208 or 149.

3. The bispecific antibody according to claim 2, wherein, the first antigen binding fragment comprises HCDR1, HCDR2, HCDR3, LCDR1, LCDR2 and LCDR3 of the following sequences:
(1) SEQ ID NO: 21, 22, 23, 18, 19 and 20, respectively; or
(2) SEQ ID NO: 33, 34, 35, 96, 31 and 32, respectively; or
(3) a sequence having at least 90% identity or having 1, 2, 3 or more amino acid insertions, deletions and/or substitutions compared with the sequences shown in (1) or (2), preferably, the substitutions are conservative amino acid substitutions.

4. The bispecific antibody according to claims 2 to 3, wherein, the second antigen binding fragment comprises CDR1, CDR2 and CDR3 of the following sequences:
(1) SEQ ID NO: 168, 207 and 208 respectively; or
(2) SEQ ID NO: 147, 148 and 149 respectively; or
(3) a sequence having at least 90% identity or having 1, 2, 3 or more amino acid insertions, deletions and/or substitutions compared with the sequences shown in (1) or (2), preferably, the substitutions are conservative amino acid substitutions.

5. The bispecific antibody according to claims 1 to 4, wherein, the VH of the first antigen binding fragment comprises a sequence having at least 90% identity to the amino acid sequence shown in SEQ ID NO: 72 or 87; the VL of the first antigen binding fragment comprises a sequence having at least 90% identity to the amino acid sequence shown in SEQ ID NO: 68 or 91.

6. The bispecific antibody according to claims 1 to 5, wherein, the second antigen binding fragment comprises a sequence having at least 90% identity to the amino acid sequence shown in SEQ ID NO: 200 or 211.

7. The bispecific antibody according to claims 1 to 6, wherein, the first antigen binding fragment is a full-length antibody, comprising two heavy chains and two light chains; the C-terminus of the second antigen binding fragment is fused to the N-terminus of at least one heavy chain of the first antigen binding fragment.

8. The bispecific antibody according to claim 7, wherein, the heavy chain fusion polypeptide comprises PVRIG VHH - (G4S)4 Linker - TIGIT VH - CH1 - hinge - CH2-CH3 from the N-terminus to the C-terminus, and the light chain polypeptide comprises TIGIT VL-CL from the N-terminus to the C-terminus.

9. The bispecific antibody according to claims 7 to 8, wherein, the heavy chain fusion polypeptide comprises a sequence having at least 80% identity to the amino acid sequences shown in SEQ ID NO: 227, 229, 231 or 233, and the light chain polypeptide comprises a sequence having at least 80% identity to the amino acid sequence shown in SEQ ID NO: 226, 228, 230 or 232.

10. The bispecific antibody according to claims 1 to 9, which is a humanized antibody.

11. The bispecific antibody according to claims 1 to 10, which specifically bind to PRVIG or TIGIT protein of human and/or monkey; preferably, the KD value between bispecific antibodies and TIGIT protein of human and/or monkey is better than 1.00E-7M, and the KD value between bispecific antibodies and PRVIG protein of human and/or monkey is better than 1.00E-8M; more preferably, the bispecific antibodies can simultaneously combine with TIGIT and PVRIG.

12. An antibody or antigen-binding fragment specifically binding to TIGIT, which comprises:
(1) a heavy chain variable region (VH), wherein the heavy chain variable region comprises three complementary determining regions (HCDRs): HCDR1, HCDR2 and HCDR3, wherein, according to the Kabat numbering scheme, the HCDR1 comprises the amino acid sequence shown in SEQ ID NO: 21, 27, 33 or 39, the HCDR2 comprises the amino acid sequence shown in SEQ ID NO: 22, 29, 34 or 40, and the HCDR3 comprises the amino acid sequence shown in SEQ ID NO: 23, 29, 35 or 41; according to the IMGT numbering scheme, the HCDR1 comprises the amino acid sequence shown in SEQ ID NO: 45, 51, 57 or 63, the HCDR2 comprises the amino acid sequence shown in SEQ ID NO:46, 52, 58 or 64, and the HCDR3 comprises the amino acid sequence shown in SEQ ID NO:47, 53, 59 or 65; and,
(2) a light chain variable region (VL), wherein the light chain variable region comprises three complementary determining regions (LCDRs): LCDR1, LCDR2 and LCDR3, wherein, according to the Kabat numbering scheme, the LCDR1 comprises the amino acid sequence shown in SEQ ID NO: 18, 24, 30, 36, 93, 94, 95 or 96, the LCDR2 comprises the amino acid sequence shown in SEQ ID NO: 19, 25, 31 or 37, and the LCDR3 comprises the amino acid sequence shown in SEQ ID NO: 20, 26, 32 or 38; according to the IMGT numbering scheme, the LCDR1 comprises the amino acid sequence shown in SEQ ID NO: 42, 48, 54 or 60, the LCDR2 comprises the amino acid sequence shown in SEQ ID NO: 43, 49, 55 or 61, and the LCDR3 comprises the amino acid sequence shown in SEQ ID NO: 44, 50, 56 or 62.

13. The antibody or antigen-binding fragment according to claim 12, which comprises LCDR1, LCDR2, LCDR3, HCDR1, HCDR2 and HCDR3 of the following sequences:
(1) SEQ ID NO: 18, 19, 20, 21, 22 and 23, respectively; or
(2) SEQ ID NO: 24, 25, 26, 27, 28 and 29, respectively; or
(3) SEQ ID NO: 30, 31, 32, 33, 34 and 35, respectively; or
(4) SEQ ID NO: 36, 37, 38, 39, 40 and 41, respectively; or
(5) SEQ ID NO: 42, 43, 44, 45, 46 and 47, respectively; or
(6) SEQ ID NO: 48, 49, 50, 51, 52 and 53, respectively; or
(7) SEQ ID NO: 54, 55, 56, 57, 58 and 59, respectively; or
(8) SEQ ID NO: 60, 61, 62, 63, 64 and 65, respectively; or
(9) SEQ ID NO: 93, 31, 32, 33, 34 and 35, respectively; or
(10) SEQ ID NO: 94, 31, 32, 33, 34 and 35, respectively; or
(11) SEQ ID NO: 95, 31, 32, 33, 34 and 35, respectively; or
(12) SEQ ID NO: 96, 31, 32, 33, 34 and 35, respectively; or
(13) a sequence having at least 80% identity to or having 1, 2, 3 or more amino acid insertions, deletions and/or substitutions with the sequences shown in the above (1) to (12), preferably, the substitutions are conservative amino acid substitutions.

14. The antibody or antigen-binding fragment according to claim 12 or 13, which comprises:
(1) a heavy chain variable region, which comprises amino acid sequences having at least 80% identity to SEQ ID NO: 10, 11, 12, 13, 69, 70, 71, 72, 81, 82, 83, 84, 85, 87, 101,102 or 103; or / and
(2) a light chain variable region, which comprises amino acid sequences having at least 80% identity to SEQ ID NO: 14, 15, 16, 17, 66, 67, 68, 78, 79, 80, 86, 88, 89, 90, 91, 98, 99 or 100.

15. The antibody or antigen-binding fragment according to claims 12 to 14, which comprises:
(1)a heavy chain variable region comprising the amino acid sequence shown in SEQ ID NO:10 and a light chain variable region comprising the amino acid sequence shown in SEQ ID NO:14; or
(2) a heavy chain variable region comprising the amino acid sequence shown in SEQ ID NO:11 and a light chain variable region comprising the amino acid sequence shown in SEQ ID NO:15; or
(3) a heavy chain variable region comprising the amino acid sequence shown in SEQ ID NO:12 and a light chain variable region comprising the amino acid sequence shown in SEQ ID NO:16; or
(4) a heavy chain variable region comprising the amino acid sequence shown in SEQ ID NO:13 and a light chain variable region comprising the amino acid sequence shown in SEQ ID NO:17; or
(5) a heavy chain variable region comprising the amino acid sequence shown in SEQ ID NO:69 and a light chain variable region comprising the amino acid sequence shown in SEQ ID NO:66, 67 or 68; or
(6) a heavy chain variable region comprising the amino acid sequence shown in SEQ ID NO:70 and a light chain variable region comprising the amino acid sequence shown in SEQ ID NO:66, 67 or 68; or
(7) a heavy chain variable region comprising the amino acid sequence shown in SEQ ID NO:71 and a light chain variable region comprising the amino acid sequence comprising SEQ ID NO:66, 67 or 68; or
(8) a heavy chain variable region comprising the amino acid sequence shown in SEQ ID NO:72 and a light chain variable region comprising the amino acid sequence shown in SEQ ID NO:66, 67 or 68; or
(9) a heavy chain variable region comprising the amino acid sequence shown in SEQ ID NO:81, 82, 83, 84 or 85 and a light chain variable region comprising the amino acid sequence shown in SEQ ID NO:78; or
(10) a heavy chain variable region comprising the amino acid sequence shown in SEQ ID NO:81, 82, 83, 84 or 85 and a light chain variable region comprising the amino acid sequence shown in SEQ ID NO:79; or
(11) a heavy chain variable region comprising the amino acid sequence shown in SEQ ID NO:81, 82, 83, 84 or 85 and a light chain variable region comprising the amino acid sequence shown in SEQ ID NO:80; or
(12) a heavy chain variable region comprising the amino acid sequence shown in SEQ ID NO:87 and a light chain variable region comprising the amino acid sequence shown in SEQ ID NO:86, 88, 89, 90 or 91; or
(13) a heavy chain variable region comprising the amino acid sequence shown in SEQ ID NO:101 and a light chain variable region comprising the amino acid sequence shown in SEQ ID NO:98, 99 or 100; or
(14) a heavy chain variable region comprising the amino acid sequence shown in SEQ ID NO:102 and a light chain variable region comprising the amino acid sequence shown in SEQ ID NO:98, 99 or 100
(15) a heavy chain variable region comprising the amino acid sequence shown in SEQ ID NO:103 and a light chain variable region comprising the amino acid sequence shown in SEQ ID NO:98, 99 or 100; or
(16) a sequence having at least 80% identity or at most 20 mutations to the sequences shown in the above (1) to (15); the mutation may be selected from insertion, deletion and/or substitution, and the substitution is preferably a conservative amino acid substitution.

16. The antibody or antigen-binding fragment according to claims 12 to 15, which comprises a heavy chain variable region, wherein the heavy chain variable region has at least a mutation selected from the following group compared with the VH shown in SEQ ID NO.10: S30T, G44K, W47Y, I48M, V67I or V71R; preferably, at least S30T and V71R mutations; more preferably, at least S30T, G44K and V71R mutations; more preferably, at least S30T, G44K, I48M, V67I and V71R mutations; more preferably, at least S30T, G44K, W47Y and V71R mutations, which are numbered in natural order; or
the heavy chain variable region has at least a mutation selected from the following group compared with the VH shown in SEQ ID NO.11: T28A, R72A, T74K or A76S; preferably, at least T28A, R72A, T74K and A76S, which are numbered in natural order; or
the heavy chain variable region has at least a mutation selected from the following group compared with the VH shown in SEQ ID NO.12: I29M, S30T, G44K, W47Y, I48M, V67I or V71R mutations; preferably, at least S30T and V71R mutations; more preferably, at least I29M, S30T and V71R mutations; more preferably, at least I29M, S30T, G44K and V71R mutations; more preferably, at least I29M, S30T, G44K, I48M, V67I and V71R mutations; more preferably, at least I29M, S30T, G44K, W47Y and V71R mutations, which are numbered in natural order; or
the heavy chain variable region has at least a mutation selected from the following group compared with the VH shown in SEQ ID NO.13: R44G, R72V, T74K, S75L or A76S; preferably, at least R72V and T74K mutations; more preferably, at least R72V, T74K, S75L and A76S mutations; more preferably, at least R44G, R72V, T74K, S75L and A76S mutations, which are numbered in natural order.

17. The antibody or antigen-binding fragment according to claims 12 to 16, which comprises a light chain variable region, wherein the light chain variable region has at least a mutation selected from the following group compared with the VL shown in SEQ ID NO.14: L37Q, P43S or L47M; preferably, at least L47M mutation; more preferably, at least L37Q and L47M mutations; more preferably, at least P43S and L47M mutations, which are numbered in natural order; or
the light chain variable region has at least a mutation selected from the following group compared with the VL shown in SEQ ID NO.15: N31Q, N31T, N31D, G32A, Q38H or P43S; preferably, at least Q38H and P43S mutations; more preferably, at least N31Q, Q38H and P43S mutations; more preferably, at least N31T, Q38H and P43S mutations; more preferably, at least N31D, Q38H and P43S mutations; more preferably, at least G32A, Q38H and P43S mutations; more preferably, at least G32A, Q38H and P43S mutations, which are numbered in natural order; or
the light chain variable region has at least a mutation selected from the following group compared with the VL shown in SEQ ID NO.16: L37Q, P43S or Q45K; preferably, at least L37Q and Q45K mutations; more preferably, at least P43S mutation, which are numbered in natural order; or
the light chain variable region has at least a mutation selected from the following group compared with the VL shown in SEQ ID NO. 17: A43S, P43S or I48V; preferably, at least A43S mutation; more preferably, at least A43S and I48V mutations; more preferably, at least P43S and I48V mutations, which are numbered in natural order.

18. The antibody or antigen-binding fragment according to claims 12 to 17, which specifically binds to human and/or monkey TIGIT proteins; preferably, the KD value between the antibody or antigen-binding fragment and human and/or monkey TIGIT proteins is better than 1.00E-8M.

19. The antibody or antigen-binding fragment according to claims 12 to 18, which is mouse antibodies, humanized antibodies, fully human antibodies or chimeric antibodies.

20. The antibody or antigen-binding fragment according to claims 12 to 19, which is selected from monoclonal antibodies, polyclonal antibodies, natural antibodies, engineered antibodies, monospecific antibodies, multispecific antibodies (such as bispecific antibodies), univalent antibodies, multivalent antibodies, intact antibodies, fragments of intact antibodies, naked antibodies, conjugated antibodies, chimeric antibodies, humanized antibodies, fully human antibodies, Fab, Fab', Fab'-SH, F (ab') 2, Fd, Fv, scFv, bispecific antibodies (diabody) or single-domain antibody.

21. A nanobody or antigen-binding fragment specifically binding to PVRIG, which comprises HCDR1, HCDR2 and HCDR3 of the VH sequences shown in SEQ ID NO: 107-119, 198-204, 211-216, 219-225.

22. The nanobody or antigen-binding fragment according to claim 21, wherein, according to the IMGT numbering scheme, LCDR1, LCDR2 and LCDR3 of the nanobody or antigen-binding fragment are selected from, for example, Table 21; according to the Kabat numbering scheme, HCDR1, HCDR2 and HCDR3 are selected from, for example, Table 22 or Table 29.

23. The nanobody or antigen-binding fragment according to any one of claim 21 or 22, wherein, the HCDR1-3 of the VH shown in SEQ ID NO. 107 have sequences as shown in SEQ ID NO: 120-122 or SEQ ID NO: 159-161 according to the IMGT or Kabat numbering system;
the HCDR1-3 of the VH shown in SEQ ID NO. 108 have sequences as shown in SEQ ID NO: 123-125 or SEQ ID NO: 162-164 according to the IMGT or Kabat numbering system;
the HCDR1-3 of the VH shown in SEQ ID NO. 109 have sequences as shown in SEQ ID NO: 126-128 or SEQ ID NO: 165-167 according to the IMGT or Kabat numbering system;
the HCDR1-3 of the VH shown in SEQ ID NO. 110 have sequences as shown in SEQ ID NO: 129-131 or SEQ ID NO: 168-170 according to the IMGT or Kabat numbering system;
the HCDR1-3 of the VH shown in SEQ ID NO. 111 have sequences as shown in SEQ ID NO: 132-134 or SEQ ID NO: 171-173 according to the IMGT or Kabat numbering system;
the HCDR1-3 of the VH shown in SEQ ID NO. 112 have sequences as shown in SEQ ID NO: 135-137 or SEQ ID NO: 174-176 according to the IMGT or Kabat numbering system;
the HCDR1-3 of the VH shown in SEQ ID NO. 113 have sequences as shown in SEQ ID NO: 138-140 or SEQ ID NO: 177-179 according to the IMGT or Kabat numbering system;
the HCDR1-3 of the VH shown in SEQ ID NO. 114 have sequences as shown in SEQ ID NO: 141-143 or SEQ ID NO: 180-182 according to the IMGT or Kabat numbering system;
the HCDR1-3 of the VH shown in SEQ ID NO. 115 have sequences as shown in SEQ ID NO: 144-146 or SEQ ID NO: 183-185 according to the IMGT or Kabat numbering system;
the HCDR1-3 of the VH shown in SEQ ID NO. 116 have sequences as shown in SEQ ID NO: 147-149 or SEQ ID NO: 186-188 according to the IMGT or Kabat numbering system;
the HCDR1-3 of the VH shown in SEQ ID NO. 117 have sequences as shown in SEQ ID NO: 150-152 or SEQ ID NO: 189-191 according to the IMGT or Kabat numbering system;
the HCDR1-3 of the VH shown in SEQ ID NO. 118 have sequences as shown in SEQ ID NO: 153-155 or SEQ ID NO: 192-194 according to the IMGT or Kabat numbering system;
the HCDR1-3 of the VH shown in SEQ ID NO. 119 have sequences as shown in SEQ ID NO: 156-158 or SEQ ID NO: 195-197 according to the IMGT or Kabat numbering system;
the HCDR1-3 of the VH shown in SEQ ID NO. 198 have sequences as shown in SEQ ID NO: 168-170 according to the Kabat numbering system;
the HCDR1-3 of the VH shown in SEQ ID NO. 199 have sequences as shown in SEQ ID NO: 168, 207 and 170 according to the Kabat numbering system;
the HCDR1-3 of the VH shown in SEQ ID NO. 200 have sequences as shown in SEQ ID NO: 168, 207 and 208 according to the Kabat numbering system;
the HCDR1-3 of the VH shown in SEQ ID NO. 201 have sequences as shown in SEQ ID NO: 168, 207 and 209 according to the Kabat numbering system;
the HCDR1-3 of the VH shown in SEQ ID NO. 202 have sequences as shown in SEQ ID NO: 168, 169 and 208 according to the Kabat numbering system;
the HCDR1-3 of the VH shown in SEQ ID NO. 203 or 204 have sequences as shown in SEQ ID NO: 168, 210 and 208 according to the Kabat numbering system;
the HCDR1-3 of the VH shown in SEQ ID NO. 211-215 have sequences as shown in SEQ ID NO: 147-149 according to the IMGT numbering system;
the HCDR1-3 of the VH shown in SEQ ID NO. 216 have sequences as shown in SEQ ID NO: 147, 148 and 218 according to the IMGT numbering system;
the HCDR1-3 of the VH shown in SEQ ID NO. 219-225 have sequences as shown in SEQ ID NO: 156-158 according to the IMGT numbering system.

24. The nanobody or antigen-binding fragment according to any one of claims 21-23, which comprises CDRs sequences having at least 80% identity or having 1, 2, 3 or more amino acid insertions, deletions and/or substitutions compared with the HCDR1, HCDR2 and HCDR3, preferably, the substitutions are conservative amino acid substitutions.

25. The nanobody or antigen-binding fragment according to any one of claims 21-24, which comprises VH as shown in any one of SEQ ID NO. 107-119, 198-204, 211-216, 219-225, or a sequence having at least 80% identity or having at most 20 mutations compared with the VH as shown in any one of SEQ ID NO. 107-119, 198-204, 211-216, 219-225; the mutation may be selected from insertion, deletion and/or substitution, and the substitution is preferably a conservative amino acid substitution.

26. The nanobody or antigen-binding fragment according to claim 25, which comprises at least a mutant sequence selected from the following group compared with the VH shown in SEQ ID NO. 110: A97V, K98E, N54D, N108S, S110A, G55A or S75T; preferably, at least A97V and K98E mutations; more preferably, at least A97V, K98E and N54D mutations; more preferably, at least A97V, K98E, N54D and N108S mutations; more preferably, at least A97V, K98E, N54D and S110A mutations; more preferably, at least A97V, K98E and N108S mutations; more preferably, at least A97V, K98E, G55A and N108S mutations; more preferably, at least S75T, A97V, K98E, G55A and N108S mutations, which are numbered in natural order;
or comprises at least a mutant sequence selected from the following group compared with the VH shown in SEQ ID NO. 116: S35T, V37F, G44E, L45R, W47F, N50T, L79V, V61S, D62H, T122I or M123Q; preferably, at least V37F, G44E, L45R, W47F and N50T mutations; more preferably, at least S35T, V37F, G44E, L45R, W47F and N50T mutations; more preferably, at least S35T, V37F, G44E, L45R, W47F, N50T and L79V mutations; more preferably, at least S35T, V37F, G44E, L45R, W47F, N50T, V61S and D62H mutations; more preferably, at least S35T, V37F, G44E, L45R, W47F, N50T, T122I and M123Q, which are numbered in natural order;
or comprises at least a mutant sequence selected from the following group compared with the VH shown in SEQ ID NO. 119: S35G, V37Y, G44D, L45R, W47L, N50T, Y58K, Y59I, D72G, N73D, Y79S, L78V or Y94F; preferably, at least S35G, V37Y, G44D, L45R, W47L and N50T mutations; more preferably, at least S35G, V37Y, G44D, L45R, W47L, N50T and Y58K mutations; more preferably, at least S35G, V37Y, G44D, L45R, W47L, N50T, Y58K, D72G and N73D mutations; more preferably, at least S35G, V37Y, G44D, L45R, W47L, N50T, Y58K, D72G, N73D and Y79S mutations; more preferably, at least S35G, V37Y, G44D, L45R, W47L, N50T, Y58K, D72G, N73D and L78V mutations; more preferably, at least S35G, V37Y, G44D, L45R, W47L, N50T, Y58K, Y59I, D72G and N73D mutations; more preferably, at least S35G, V37Y, G44D, L45R, W47L, N50T, Y58K, D72G, N73D and Y94F, which are numbered in natural order.

27. The nanobody or antigen-binding fragment according to any one of claims 21-26, which specifically binds to human and/or monkey PVRIG proteins; preferably, the KD value between the nanobody or antigen-binding fragment and human and/or monkey PVRIG proteins is better than 2.00E-9M.

28. The nanobody or antigen-binding fragment according to any one of claims 21-27, which is (1) chimeric nanobody or fragment thereof; (2) humanized nanobody or fragment thereof; or (3) fully human nanobody or fragment thereof.

29. The nanobody or antigen-binding fragment according to any one of claims 21-28, which includes or does not include an antibody heavy chain constant region; optionally, the antibody heavy chain constant region is selected from human, alpaca, mouse, rat, rabbit or sheep; optionally, the antibody heavy chain constant region may be selected from IgG, IgM, IgA, IgE or IgD, and the IgG may be selected from IgG1, IgG2, IgG3 or IgG4; optionally, the heavy chain constant region may be selected from the Fc region, the CH3 region or the intact heavy chain constant region, preferably, the heavy chain constant region is a human Fc region; preferably, the nanobody or antigen binding fragment is a heavy chain antibody.

30. The anti-PVRIG/anti-TIGIT bispecific antibody of any one of claims 1-11, the antibody or antigen-binding fragment specifically binding to TIGIT of any one of claims 12-20, the nanobody or antigen-binding fragment specifically binding to PVRIG of any one of claims 21-29, which are coupled with a therapeutic agent or a tracer; preferably, the therapeutic agent is selected from drugs, toxins, radioisotopes, chemotherapeutic drugs or immunomodulators, and the tracer is selected from radiocontrast agents, paramagnetic ions, metals, fluorescent labels, chemiluminescent labels, ultrasound contrast agents and photosensitizers.

31. A multispecific molecule, which comprises the anti-PVRIG/anti-TIGIT bispecific antibody of any one of claims 1-11, the antibody or antigen-binding fragment specifically binding to TIGIT of any one of claims 12-20, or the nanobody or antigen-binding fragment specifically binding to PVRIG of any one of claims 21-29; preferably, the multispecific molecule may be bispecific, trispecific or tetraspecific, and more preferably, the multispecific molecule may be bivalent, tetravalent or hexavalent.

32. The multispecific molecule of claim 31, which is tandem scFv, bifunctional antibody (Db), single chain bifunctional antibody (scDb), dual-affinity retargeting (DART) antibody, F(ab')2, dual variable domain (DVD) antibody, knob-into-holes (KIH) antibody, dock-and-lock (DNL) antibody, chemically crosslinked antibody, heteropolymeric nanoantibody or heteroconjugate antibody.

33. A chimeric antigen receptor (CAR), which comprises at least an extracellular antigen-binding domain, a transmembrane domain and an intracellular signaling domain, wherein the extracellular antigen-binding domain comprises the antibody or antigen-binding fragment of any one of claims 12-20, or comprises any of the nanobodies or antigen-binding fragments of any one of claims 21-29.

34. An immune effector cell, which expresses the chimeric antigen receptor of claim 33, or comprises a nucleic acid fragment encoding the chimeric antigen receptor of claim 33; preferably, the immune effector cells are selected from T cells, natural killer cells (NK cells), natural killer T cells (NKT cells), double negative T cells (DNT cells), monocytes, macrophages, dendritic cells or mast cells, the T cells are preferably selected from cytotoxic T cells, regulatory T cells or T helper cells; preferably, the immune effector cells are autoimmune effector cells or allogeneic immune effector cells.

35. An isolated nucleic acid fragment, which encodes the bispecific antibody of any one of claims 1-11, the antibody or antigen-binding fragment of any one of claims 12-20, the nanobody or antigen-binding fragment of any one of claims 21-29, the multispecific molecules of any one of claims 31-32, or the chimeric antigen receptor of any one of claim 33.

36. A vector, which comprises the nucleic acid fragment of claim 35.

37. A host cell, which comprises the vector of claim 36; preferably, the cell is a prokaryotic or eukaryotic cell, such as a bacterium (Escherichia coli), fungus (yeast), insect or mammalian cell (CHO or 293T cell line).

38. A method for preparing the bispecific antibody of any one of claims 1-11, the antibody or antigen-binding fragment of any one of claims 12-20, the nanobody or antigen-binding fragment of any one of claims 21-29, or the multispecific molecule of any one of claims 31-32, which comprises culturing the cells of claim 37 and isolating the antibodies or molecules expressed by the cells.

39. A method for preparing the immune effector cell of claim 34, which comprises introducing the nucleic acid fragment encoding the CAR of claim 33 into the immune effector cells, and optionally, further comprises initiating the immune effector cells to express the CAR of claim 33.

40. A pharmaceutical composition, which comprises the bispecific antibody of any one of claims 1-11, the antibody or antigen-binding fragment of any one of claims 12-20, the nanobody or antigen-binding fragment of any one of claims 21-29, the multispecific molecule of any one of claims 31-32, the immune effector cell of claim 34, the nucleic acid fragment of claim 35, the vector of claim 36, the host cell of claim 37, or products prepared by the method of claims 38-39, and pharmaceutically acceptable carriers.

41. The pharmaceutical composition of claim 40, which further comprises an additional therapeutic agent; preferably, the additional therapeutic agent is an antitumor agent; more preferably, the antitumor agent is a PD-1 axis binding antagonist.

42. The use of the bispecific antibody of any one of claims 1-11, the antibody or antigen-binding fragment of any one of claims 12-20, the nanobody or antigen-binding fragment of any one of claims 21-29, the multispecific molecule of any one of claims 31-32, the immune effector cell of claim 34, the nucleic acid fragment of claim 35, the vector of claim 36, the host cell of claim 37, or products prepared by the method of claims 38-39, or the pharmaceutical composition of claims 40-41 in the preparation of drugs for treating cancer or infectious diseases; wherein the cancer is selected from solid tumors and blood tumors, preferably, wherein the tumor is selected from leukemia, multiple myeloma, lymphoma, myelodysplastic syndrome, prostate cancer, liver cancer, colorectal cancer, anal cancer, ovarian cancer, endometrial carcinoma, cervical cancer, abdominal cancer, breast cancer, pancreatic cancer, gastric cancer, head and neck cancer, thyroid cancer, testicular cancer, urinary tract epithelial cancer, lung cancer, melanoma, non-melanoma skin cancer, glioma, kidney cancer, mesothelioma, esophageal cancer, non-small cell lung cancer, small cell lung cancer, bladder cancer, sarcoma, glioblastoma, thymic carcinoma, mycosis fungoides, Merkel cell carcinoma, high MSI cancer and KRAS mutant tumors.

43. The use of claim 42, wherein, the drug is used in combination with an additional therapeutic agent or operation, wherein the additional therapeutic agent or operation is selected from radiotherapy, chemotherapy, oncolytic drugs, cytotoxic agents, cytokines, surgery, immunostimulatory antibodies, immunomodulatory drugs, activators of costimulatory molecules, inhibitors of inhibitory molecules, vaccines or cellular immunotherapy.

44. The use of claim 43, wherein, the additional therapeutic agent is administered before or after the administration of the drug, or simultaneously with the drug.

45. The use of claims 42-44, wherein, the drug is used in combination with a PD-1 axis binding antagonist.

46. The use of claim 45, wherein, the PD-1 axis binding antagonist is selected from the group consisting of a PD-1 binding antagonist, a PD-L1 binding antagonist, and a PD-L2 binding antagonist; preferably, the PD-1 binding antagonist is an anti-PD-1 antibody; more preferably, the PD-1 binding antagonist is selected from the group consisting of MDX 1106 (nivolumab), MK-3475 (pembrolizumab), CT-011 (pidilizumab), MEDI-0680 (AMP-514), PDR001, REGN2810, and BGB-108; preferably, the PD-L1 binding antagonist is an anti-PD-L1 antibody; more preferably, the PD-L1 binding antagonist is selected from the group consisting of MPDL3280A (atezolizumab), YW243.55.S70, MDX-1105, MEDI4736 (durvalumab), Tecentriq and MSB0010718C (avelumab); preferably, the PD-L2 binding antagonist is an anti-PD-L2 antibody; more preferably, the PD-L2 binding antagonist is an immunoadhesin.

47. A method for treating cancer or infectious diseases, which comprises administering an effective amount of the bispecific antibody of any one of claims 1-11, the antibody or antigen-binding fragment of any one of claims 12-20, the nanobody or antigen-binding fragment of any one of claims 21-29, the multispecific molecule of any one of claims 31-32, the immune effector cell of claim 34, the nucleic acid fragment of claim 35, the vector of claim 36, the host cell of claim 37, or products prepared by the method of claims 38-39, or the pharmaceutical composition of claims 40-41 to patients in need; wherein the cancer is selected from solid tumors and blood tumors, preferably, wherein the tumor is selected from leukemia, multiple myeloma, lymphoma, myelodysplastic syndrome, prostate cancer, liver cancer, colorectal cancer, anal cancer, ovarian cancer, endometrial carcinoma, cervical cancer, abdominal cancer, breast cancer, pancreatic cancer, gastric cancer, head and neck cancer, thyroid cancer, testicular cancer, urinary tract epithelial cancer, lung cancer, melanoma, non-melanoma skin cancer, glioma, kidney cancer, mesothelioma, esophageal cancer, non-small cell lung cancer, small cell lung cancer, bladder cancer, sarcoma, glioblastoma, thymic carcinoma, mycosis fungoides, Merkel cell carcinoma, high MSI cancer and KRAS mutant tumors.

48. The method of claim 47, wherein, the method further comprises administering an effective amount of a PD-1 axis binding antagonist to patients in need, wherein the PD-1 axis binding antagonist is selected from the group consisting of a PD-1 binding antagonist, a PD-L1 binding antagonist, and a PD-L2 binding antagonist; preferably, the PD-1 binding antagonist is an anti-PD-1 antibody; more preferably, the PD-1 binding antagonist is selected from the group consisting of MDX 1106 (nivolumab), MK-3475 (pembrolizumab), CT-011 (pidilizumab), MEDI-0680 (AMP-514), PDR001, REGN2810, and BGB-108; preferably, the PD-L1 binding antagonist is an anti-PD-L1 antibody; more preferably, the PD-L1 binding antagonist is selected from the group consisting of MPDL3280A (atezolizumab), YW243.55.S70, MDX-1105, MEDI4736 (durvalumab), Tecentriq and MSB0010718C (avelumab); preferably, the PD-L2 binding antagonist is an anti-PD-L2 antibody; more preferably, the PD-L2 binding antagonist is an immunoadhesin.

49. The bispecific antibody of any one of claims 1-11, the antibody or antigen-binding fragment of any one of claims 12-20, the nanobody or antigen-binding fragment of any one of claims 21-29, the multispecific molecule of any one of claims 31-32, the immune effector cell of claim 34, the nucleic acid fragment of claim 35, the vector of claim 36, the host cell of claim 37, or products prepared by the method of claims 38-39, or the pharmaceutical composition of claims 40-41 for treating cancer or infectious diseases, wherein the cancer is selected from solid tumors and blood tumors, preferably, wherein the tumor is selected from leukemia, multiple myeloma, lymphoma, myelodysplastic syndrome, prostate cancer, liver cancer, colorectal cancer, anal cancer, ovarian cancer, endometrial carcinoma, cervical cancer, abdominal cancer, breast cancer, pancreatic cancer, gastric cancer, head and neck cancer, thyroid cancer, testicular cancer, urinary tract epithelial cancer, lung cancer, melanoma, non-melanoma skin cancer, glioma, kidney cancer, mesothelioma, esophageal cancer, non-small cell lung cancer, small cell lung cancer, bladder cancer, sarcoma, glioblastoma, thymic carcinoma, mycosis fungoides, Merkel cell carcinoma, high MSI cancer and KRAS mutant tumors.
